(19) Europäisches Patentamt / European Patent Office / Office européen des brevets

(11) **EP 3 779 000 A1**

(12) **EUROPEAN PATENT APPLICATION**
published in accordance with Art. 153(4) EPC

(43) Date of publication:
**17.02.2021 Bulletin 2021/07**

(21) Application number: **19782228.1**

(22) Date of filing: **03.04.2019**

(51) Int Cl.:
*D01F 8/02* (2006.01)  *C07K 14/435* (2006.01)
*D01F 4/02* (2006.01)

(86) International application number:
**PCT/JP2019/014867**

(87) International publication number:
**WO 2019/194243 (10.10.2019 Gazette 2019/41)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **03.04.2018 JP 2018071955**

(71) Applicant: **Spiber Inc.
Tsuruoka-shi, Yamagata 997-0052 (JP)**

(72) Inventors:
• **ABE, Yunosuke
Tsuruoka-shi, Yamagata 997-0052 (JP)**
• **TOGASHI, Shota
Tsuruoka-shi, Yamagata 997-0052 (JP)**

(74) Representative: **Handley, Matthew Edward
Venner Shipley LLP
200 Aldersgate
London EC1A 4HD (GB)**

(54) **COMPOSITE FIBERS AND METHOD FOR MANUFACTURING SAME**

(57)    Provided is a side-by-side type composite fiber having a latent crimping ability, including a first component containing a modified fibroin and a second component containing a structural protein, in which the first component and the second component are joined to each other.

*Fig.7*

(a)    (b)

EP 3 779 000 A1

**Description**

**Technical Field**

The present invention relates to a composite fiber and a method for manufacturing the composite fiber.

**Background Art**

Crimping ability is a very important property for performing spinning. In the case of thermoplastic synthetic fibers such as nylon and a polyester fiber, it is possible to manufacture a fiber having permanently crimping ability by utilizing heat. In addition, raw silk obtained from silkworm is covered with sericin and is converted into a silk fiber (fibroin) which is soft and glossy by a scouring process.

On the other hand, a silk fiber from which sericin has been completely removed has no elasticity and is not suitable for a twisted yarn. Therefore, in order to manufacture a twisted yarn from a silk fiber, a method in which scouring is performed, without completely removing sericin, such that 80% to 90% of sericin remains to maintain an appropriate elasticity is used. In addition, various contrivances have been made, such as carrying out the scouring process at the yarn stage, carrying out the scouring process at the textile stage, not carrying out the scouring process, and weaving a sericin-fixed yarn and a sericin-unfixed yarn (For example, Patent Literature 1 and Non-Patent Literature 1). In recent years, a method in which sericin is insolubilized with a modifier without removing sericin and twisting process is performed has been reported (for example, Patent Literature 2).

**Citation List**

**Patent Literature**

[Patent Literature 1] Japanese Unexamined Patent Publication No. H5-93317
[Patent Literature 2] Japanese Unexamined Patent Publication No. 2016-23389

**Non Patent Literature**

[Non-Patent Literature 1] Ling Peng et al. Macromol. Mater. Eng. 2016, 301, 48-55.

**Summary of Invention**

**Technical Problem**

However, it was necessary to adjust the content rate of sericin to the desired range.

In consideration of the above circumstances, an object of the present invention is to provide a new fiber having crimping ability and a method for manufacturing the fiber having crimping ability.

**Solution to Problem**

The present invention relates to, for example, each of the following inventions.

[1] A side-by-side type composite fiber having a latent crimping ability, including a first component containing a modified fibroin and a second component containing a structural protein, in which the first component and the second component are joined to each other.

[2] The composite fiber according to [1], in which a composition ratio of the first component to the second component is 9:1 to 1:9 based on a mass of the composite fiber.

[3] The composite fiber according to [1] or [2], in which the structural protein is at least one selected from the group consisting of a silk fibroin, a spider silk fibroin, collagen, resilin, elastin, and keratin.

[4] The composite fiber according to any of [1] to [3], in which the structural protein is at least one selected from the group consisting of a silk fibroin, a spider silk fibroin, and keratin.

[4-1] The composite fiber according to any of [1] to [4], in which the modified fibroin is a modified fibroin (a third modified fibroin) including a domain sequence represented by Formula 1: $[(A)_n$ motif - $REP]_m$, and

the domain sequence has an amino acid sequence in which a content of the $(A)_n$ motif is reduced, the amino acid sequence being equivalent to an amino acid sequence in which at least one or a plurality of the $(A)_n$ motifs are deleted, as compared with a naturally occurring fibroin.

[In Formula 1, the $(A)_n$ motif represents an amino acid sequence composed of 2 to 27 amino acid residues and the number of alanine residues with respect to a total number of amino acid residues in the $(A)_n$ motif is 83% or more. The REP represents an amino acid sequence composed of 10 to 200 amino acid residues. m represents an integer of 2 to 300. A plurality of the $(A)_n$ motifs may have the same amino acid sequence or amino acid sequences different from each other. A plurality of the REPs may have the same amino acid sequence or amino acid sequences different from each other.]

[4-2] The composite fiber according to any of [1] to [4], in which the modified fibroin is a modified fibroin (a fourth modified fibroin) including a domain sequence represented by Formula 1: $[(A)_n \text{ motif - REP}]_m$, and

the domain sequence has an amino acid sequence in which a content of a glycine residue is reduced, the amino acid sequence being equivalent to an amino acid sequence in which at least one or a plurality of the glycine residues in the REP are substituted with other amino acid residues, as compared with a naturally occurring fibroin.

[In Formula 1, the $(A)_n$ motif represents an amino acid sequence composed of 2 to 27 amino acid residues and the number of alanine residues with respect to a total number of amino acid residues in the $(A)_n$ motif is 83% or more. The REP represents an amino acid sequence composed of 10 to 200 amino acid residues. m represents an integer of 2 to 300. A plurality of the $(A)_n$ motifs may have the same amino acid sequence or amino acid sequences different from each other. A plurality of the REPs may have the same amino acid sequence or amino acid sequences different from each other.]

[4-3] The composite fiber according to any of [1] to [4], in which the modified fibroin is a modified fibroin (a fifth modified fibroin) including a domain sequence represented by Formula 1: $[(A)_n \text{ motif - REP}]_m$, and

the domain sequence has an amino acid sequence locally including a region having a high hydropathy index, the amino acid sequence being equivalent to an amino acid sequence in which one or a plurality of amino acid residues in the REP are substituted with amino acid residues having a high hydropathy index and/or one or a plurality of amino acid residues having a high hydropathy index are inserted into the REP, as compared with a naturally occurring fibroin.

[In Formula 1, the $(A)_n$ motif represents an amino acid sequence composed of 2 to 27 amino acid residues and the number of alanine residues with respect to a total number of amino acid residues in the $(A)_n$ motif is 83% or more. The REP represents an amino acid sequence composed of 10 to 200 amino acid residues. m represents an integer of 2 to 300. A plurality of the $(A)_n$ motifs may have the same amino acid sequence or amino acid sequences different from each other. A plurality of the REPs may have the same amino acid sequence or amino acid sequences different from each other.]

[4-4] The composite fiber according to any of [1] to [4], in which the modified fibroin a modified fibroin (a sixth modified fibroin) including a domain sequence represented by Formula 1: $[(A)_n \text{ motif - REP}]_m$ or Formula 2: $[(A)_n \text{ motif - REP}]_m - (A)_n$ motif, and

the domain sequence has an amino acid sequence in which a content of a glutamine residue is reduced, the amino acid sequence being equivalent to an amino acid sequence in which one or a plurality of the glutamine residues in the REP are deleted or substituted with other amino acid residues, as compared with a naturally occurring fibroin.

[In Formula 1 and Formula 2, the $(A)_n$ motif represents an amino acid sequence composed of 2 to 27 amino acid residues and the number of alanine residues with respect to a total number of amino acid residues in the $(A)_n$ motif is 80% or more. The REP represents an amino acid sequence composed of 10 to 200 amino acid residues. m represents an integer of 2 to 300. A plurality of the $(A)_n$ motifs may have the same amino acid sequence or amino acid sequences different from each other. A plurality of the REPs may have the same amino acid sequence or amino acid sequences different from each other.]

[4-5] The composite fiber according to any one of [1] to [4], in which the modified fibroin has a limiting oxygen index (LOI) value of 26.0 or more.

[4-6] The composite fiber according to any of [1] to [4], in which the modified fibroin has a highest hygroscopic heat generation degree of more than 0.025 °C/g, the highest hygroscopic heat generation degree being determined according to Expression A.

Expression A: highest hygroscopic heat generation degree = {(highest temperature of a sample when the sample has been transferred to a high humidity environment after being placed in a low humidity environment until a temperature of the sample reaches equilibrium) - (temperature of the sample when the sample is being transferred to the high humidity environment after being placed in the low humidity environment until the temperature of the sample reaches equilibrium)} ($°C$)/sample weight (g)

[In Expression A, the low humidity environment means an environment of a temperature of 20°C and a relative humidity of 40%, and the high humidity environment means an environment of a temperature of 20°C and a relative humidity of 90%.]

[5] A method for manufacturing a composite fiber, comprising:

preparing a first doping liquid containing a modified fibroin and a solvent;
preparing a second doping liquid containing a structural protein and a solvent;
discharging the first doping liquid and the second doping liquid from a spinneret and joining the first doping liquid and the second doping liquid to form an undrawn composite fiber in a coagulation liquid; and
bringing the undrawn composite fiber into contact with an aqueous medium.

[6] The method according to [5], further including drawing the undrawn composite fiber.
[7] The method according to [5] or [6], in which a concentration of the modified fibroin in the first doping liquid is 5% to 40% by mass based on a total mass of the first doping liquid, and
a concentration of the structural protein in the second doping liquid is 5% to 40% by mass based on a total mass of the second doping liquid.
[8] The method according to any of [5] to [7], in which the solvent contains at least one selected from the group consisting of hexafluoroisopropanol, hexafluoroacetone, dimethyl sulfoxide, N,N-dimethylformamide, N,N-dimethylacetamide, 1,3-dimethyl-2-imidazolidone, N-methyl-2-pyrrolidone, acetonitrile, N-methylmorpholine-N-oxide, formic acid, and an aqueous solution containing at least one selected from the group consisting of urea, guanidine, sodium dodecyl sulfate, lithium bromide, calcium chloride, and lithium thiocyanate.
[9] The method according to any of [5] to [8], in which the coagulation liquid is at least one selected from the group consisting of a lower alcohol having 1 to 5 carbon atoms and acetone.
[10] The method according to any of [5] to [9], in which the structural protein is at least one selected from the group consisting of a silk fibroin, a spider silk fibroin, collagen, resilin, elastin, and keratin.
[11] The method according to any of [5] to [10], in which the structural protein is at least one selected from the group consisting of a silk fibroin, a spider silk fibroin, and keratin.

[11-1] The method according to any of [5] to [11], in which the modified fibroin is a modified fibroin including a domain sequence represented by Formula 1: $[(A)_n$ motif - $REP]_m$, and
the domain sequence has an amino acid sequence in which a content of the $(A)_n$ motif is reduced, the amino acid sequence being equivalent to an amino acid sequence in which at least one or a plurality of the $(A)_n$ motifs are deleted, as compared with a naturally occurring fibroin.
[In Formula 1, the $(A)_n$ motif represents an amino acid sequence composed of 2 to 27 amino acid residues and the number of alanine residues with respect to a total number of amino acid residues in the $(A)_n$ motif is 83% or more. The REP represents an amino acid sequence composed of 10 to 200 amino acid residues. m represents an integer of 2 to 300. A plurality of the $(A)_n$ motifs may have the same amino acid sequence or amino acid sequences different from each other. A plurality of the REPs may have the same amino acid sequence or amino acid sequences different from each other.]

[11-2] The method according to any of [5] to [11], in which the modified fibroin includes a domain sequence represented by Formula 1: $[(A)_n \text{ motif - REP}]_m$, and

the domain sequence has an amino acid sequence in which a content of a glycine residue is reduced, the amino acid sequence being equivalent to an amino acid sequence in which at least one or a plurality of the glycine residues in the REP are substituted with other amino acid residues, as compared with a naturally occurring fibroin. [In Formula 1, the $(A)_n$ motif represents an amino acid sequence composed of 2 to 27 amino acid residues and the number of alanine residues with respect to a total number of amino acid residues in the $(A)_n$ motif is 83% or more. The REP represents an amino acid sequence composed of 10 to 200 amino acid residues. m represents an integer of 2 to 300. A plurality of the $(A)_n$ motifs may have the same amino acid sequence or amino acid sequences different from each other. A plurality of the REPs may have the same amino acid sequence or amino acid sequences different from each other.]

[11-3] The method according to any of [5] to [11], in which the modified fibroin includes a domain sequence represented by Formula 1: $[(A)_n \text{ motif - REP}]_m$, and

the domain sequence has an amino acid sequence locally including a region having a high hydropathy index, the amino acid sequence being equivalent to an amino acid sequence in which one or a plurality of amino acid residues in the REP are substituted with amino acid residues having a high hydropathy index and/or one or a plurality of amino acid residues having a high hydropathy index are inserted into the REP, as compared with a naturally occurring fibroin.

[In Formula 1, the $(A)_n$ motif represents an amino acid sequence composed of 2 to 27 amino acid residues and the number of alanine residues with respect to a total number of amino acid residues in the $(A)_n$ motif is 83% or more. The REP represents an amino acid sequence composed of 10 to 200 amino acid residues. m represents an integer of 2 to 300. A plurality of the $(A)_n$ motifs may have the same amino acid sequence or amino acid sequences different from each other. A plurality of the REPs may have the same amino acid sequence or amino acid sequences different from each other.]

[11-4] The method according to any of [5] to [11], in which the modified fibroin includes a domain sequence represented by Formula 1: $[(A)_n \text{ motif - REP}]_m$ or Formula 2: $[(A)_n \text{ motif - REP}]_m - (A)_n \text{ motif}$, and

the domain sequence has an amino acid sequence in which a content of a glutamine residue is reduced, the amino acid sequence being equivalent to an amino acid sequence in which one or a plurality of the glutamine residues in the REP are deleted or substituted with other amino acid residues, as compared with a naturally occurring fibroin.

[In Formula 1 and Formula 2, the $(A)_n$ motif represents an amino acid sequence composed of 2 to 27 amino acid residues and the number of alanine residues with respect to a total number of amino acid residues in the $(A)_n$ motif is 80% or more. The REP represents an amino acid sequence composed of 10 to 200 amino acid residues. m represents an integer of 2 to 300. A plurality of the $(A)_n$ motifs may have the same amino acid sequence or amino acid sequences different from each other. A plurality of the REPs may have the same amino acid sequence or amino acid sequences different from each other.]

[11-5] The method according to any of [5] to [11], in which the modified fibroin has a limiting oxygen index (LOI) value of 26.0 or more.

[11-6] The method according to any of [5] to [11], in which the modified fibroin has a highest hygroscopic heat generation degree of more than 0.025 °C/g, the highest hygroscopic heat generation degree being determined according to Expression A.

Expression A: highest hygroscopic heat generation degree = {(highest temperature of a sample when the sample has been transferred to a high humidity environment after being placed in a low humidity environment until a temperature of the sample reaches equilibrium) - (temperature of the sample when the sample is being transferred to the high humidity environment after being placed in the low humidity environment until the temperature of the sample reaches equilibrium)} (°C)/sample weight (g)

[In Expression A, the low humidity environment means an environment of a temperature of 20°C and a relative humidity of 40%, and the high humidity environment means an environment of a temperature of 20°C and a relative humidity of 90%.]

[12] A product including the composite fiber according to any of [1] to [4] and [4-1] to [4-6], in which the product is one selected from the group consisting of fiber, yarn, fabric, knit, braid, non-woven fabric, paper, and cotton.

[13] A side-by-side type composite fiber including a first component and a second component, in which the first component and the second component are joined to each other, and one of the first component and the second component contains a modified fibroin.

[14] A side-by-side type composite fiber including a first component and a second component, in which the first component and the second component are joined to each other, and one of the first component and the second component contains a modified fibroin having a latent crimping ability.

**Advantageous Effects of Invention**

According to the present invention, a new fiber having a crimping ability can be provided. According to the present invention, it is possible to provide a composite fiber which is excellent in the crimping ability and is useful as a crimped yarn or a spun yarn. Further, according to the present invention, the composite fiber exhibits high strength and toughness since the composite fiber contains a modified fibroin.

**Brief Description of Drawings**

Fig. 1 is a schematic diagram showing a domain sequence of a modified fibroin.
Fig. 2 is a graph showing the distribution of z/w (%) values of a naturally occurring fibroin.
Fig. 3 is a graph showing the distribution of x/y (%) values of a naturally occurring fibroin.
Fig. 4 is a schematic diagram showing a domain sequence of a modified fibroin according to one embodiment.
Fig. 5 is a schematic diagram showing a domain sequence of a modified fibroin according to one embodiment.
Fig. 6 is an illustrative view schematically showing an example of a spinning device for manufacture a composite fiber.
Fig. 7 is a schematic diagram of a composite fiber according to one embodiment of the present invention.
Fig. 8 is a graph showing an example of a result of a hygroscopic heat generating property test.

**Description of Embodiments**

Hereinafter, embodiments for carrying out the present invention will be described in detail. However, the present invention is not limited to the following embodiments.

A composite fiber according to one embodiment of the present invention includes a first component containing a modified fibroin and a second component containing a structural protein, which are joined to each other in a side-by-side type manner. That is, the cross-sectional shape of the composite fiber according to the present embodiment is a shape in which the first component and the second component are joined to each other, for example, a circular shape in which a semicircle of the first component and a semicircle of the second component are combined. In the cross-sectional shape of the composite fiber, the ratio of the first component to the second component may not be 50:50 but may be properly changed to 30:70, 20:80, or the like. Further, the first component and the second component may be drawn in parallel along the composite fiber or may be twisted spirally. In this case, for example, in one part of the composite fiber, the first component and the second component are arranged one over the other, whereas in the other part of the composite fiber, the first component and the second component are arranged side by side while the joint surface gradually rotates. The hydrophobicity of the modified fibroin and the hydrophobicity of structural protein are different from each other.

The first component of the composite fiber according to the present embodiment contains a modified fibroin. The modified fibroin contained in the first component has a property of shrinking when brought into contact with an aqueous medium described later (hereinafter, also referred to as "water shrinkability"). Further, for example, when a fiber containing the modified fibroin is brought into contact with an aqueous medium in a state where no tension is applied, the fiber is shrunk and crimped. As a result, the latent crimping ability is exhibited in the fiber containing a modified fibroin.

(Modified fibroin)

The modified fibroin is a protein including a domain sequence represented by Formula 1: $[(A)_n$ motif - REP$]_m$. In the modified fibroin, an amino acid sequence (an N-terminal sequence and a C-terminal sequence) may be further added to one or both of the N-terminal side and the C-terminal side of the domain sequence. The N-terminal sequence and the C-terminal sequence, although not limited thereto, are typically regions that do not have repetitions of amino acid motifs characteristic of fibroin and consist of amino acids of about 100 residues. In the present embodiment, in a case where the modified fibroin is a modified spider silk fibroin, the heat retaining property, the hygroscopic heat generating property and/or the flame retardancy are/is more excellent.

The term "modified fibroin" in the present specification means an artificially produced fibroin (an artificial fibroin). The modified fibroin may be a fibroin in which the domain sequence is different from the amino acid sequence of a naturally occurring fibroin or may be the same as the amino acid sequence of a naturally occurring fibroin. The "naturally occurring fibroin" referred to in the present specification is also a protein including a domain sequence represented by Formula 1: $[(A)_n$ motif - REP$]_m$.

As the "modified fibroin", an amino acid sequence of a naturally occurring fibroin may be directly used, a fibroin whose amino acid sequence has been modified based on an amino acid sequence of a naturally occurring fibroin (for example, a fibroin whose amino acid sequence has been modified by modifying a cloned gene sequence of a naturally occurring fibroin) may be used, or a fibroin artificially designed and synthesized independently of a naturally occurring fibroin (for example, a fibroin having a desired amino acid sequence by chemically synthesizing a nucleic acid encoding the designed amino acid sequence) may be used, as long as it has the amino acid sequence specified in the present invention.

The term "domain sequence" as used herein refers to an amino acid sequence which produces a crystalline region (typically, corresponds to $(A)_n$ motif of an amino acid sequence) and a non-crystalline region (typically, corresponds to REP of an amino acid sequence) peculiar to fibroin and means an amino acid sequence represented by Formula 1: $[(A)_n$ motif - REP$]_m$. Here, the $(A)_n$ motif represents an amino acid sequence mainly composed of alanine residues. n may be an integer of 2 to 20, preferably an integer of 4 to 20, more preferably 8 to 20, still more preferably 10 to 20, even still more 4 to 16, even still further preferably 8 to 16, and particularly preferably 10 to 16. In addition, the proportion of the number of alanine residues may be 40% or more, preferably 60% or more, more preferably 70% or more, still more preferably 80% or more, and even still more preferably 90% or more, and even still further preferably 100% (which means that the $(A)_n$ motif is composed of only alanine residues), with respect to the total number of amino acid residues in the $(A)_n$ motif. The REP indicates an amino acid sequence composed of 2 to 200 amino acid residues and may be an amino acid sequence composed of 10 to 40 amino acid residues, 10 to 60 amino acid residues, 10 to 80 amino acid residues, 10 to 100 amino acid residues, 10 to 120 amino acid residues, 10 to 140 amino acid residues, 10 to 160 amino acid residues, or 10 to 180 amino acids. m represents an integer of 2 to 300 and may be an integer of 8 to 300, 10 to 300, 20 to 300, 40 to 300, 60 to 300, 80 to 300, 10 to 200, 20 to 200, 20 to 180, 20 to 160, 20 to 140, or 20 to 120. A plurality of the $(A)_n$ motifs may have the same amino acid sequence or amino acid sequences different from each other. The plurality of the REPs may have the same amino acid sequence or amino acid sequences different from each other. As a specific example of the protein derived from the large spinal canal bookmark silk, a protein including the amino acid sequence set forth in SEQ ID NO: 13 (PRT410) can be mentioned.

The modified fibroin can be obtained, for example, by carrying out the modification of an amino acid sequence equivalent to the substitution, deletion, insertion and/or addition of one or a plurality of amino acid residues with respect to, for example, a cloned gene sequence of a naturally occurring fibroin. The substitution, deletion, insertion, and/or addition of an amino acid residue may be carried out by methods well known to those skilled in the art, such as site-directed mutagenesis. Specifically, the modifications may be carried out by methods described in literature such as Nucleic Acid Res. 10, 6487 (1982) and Methods in Enzymology, 100, 448 (1983).

The naturally occurring fibroin is a protein including a domain sequence represented by Formula 1: $[(A)_n$ motif - $REP]_m$, and specifically, for example, a fibroin produced by insects or spiders.

Examples of the fibroin produced by insects include silk proteins produced by silkworms such as Bombyx mori, Bombyx mandarina, Antheraea yamamai, Anteraea pernyi, Eriogyna pyretorum, Pilosamia Cynthia ricini, Samia cynthia, Caligura japonica, Antheraea mylitta, and Antheraea assama; and hornet silk proteins discharged by larvae of Vespa simillima xanthoptera.

More specific examples of the fibroin produced by insects include a silkworm fibroin L chain (GenBank Accession No. M76430 (base sequence) and AAA27840.1 (amino acid sequence)).

Examples of the fibroin produced by spiders include spider silk proteins produced by spiders belonging to the genus Araneus such as Araneus ventricosus, Araneus diadematus, Araneus pinguis, Araneus pentagrammicus and Araneus nojimai, spiders belonging to the genus Neoscona such as Neoscona scylla, Neoscona nautica, Neoscona adianta and Neoscona scylloides, spiders belonging to the genus Pronus such as Pronous minutes, spiders belonging to the genus Cyrtarachne such as Cyrtarachne bufo and Cyrtarachne inaequalis, spiders belonging to the genus Gasteracantha such as Gasteracantha kuhli and Gasteracantha mammosa, spiders belonging to the genus Ordgarius such as Ordgarius hobsoni and Ordgarius sexspinosus, spiders belonging to the genus Argiope such as Argiope amoena, Argiope minuta and Argiope bruennich, spiders belonging to the genus Arachnura such as Arachnura logio, spiders belonging to the genus Acusilas such as Acusilas coccineus, spiders belonging to the genus Cytophora such as Cyrtophora moluccensis, Cyrtophora exanthematica and Cyrtophora unicolor, spiders belonging to the genus Poltys such as Poltys illepidus, spiders belonging to the genus Cyclosa such as Cyclosa octotuberculata, Cyclosa sedeculata, Cyclosa vallata and Cyclosa atrata, and spiders belonging to the genus Chorizopes such as Chorizopes nipponicus; and spider silk proteins produced by spiders belonging to the genus Tetragnatha such as Tetragnatha praedonia, Tetragnatha maxillosa, Tetragnatha extensa and Tetragnatha squamata, spiders belonging to the genus Leucauge such as Leucauge magnifica, Leucauge blanda and Leucauge subblanda, spiders belonging to the genus Nephila such as Nephila clavata and Nephila pilipes, spiders belonging to the genus Menosira such as Menosira ornata, spiders belonging to the genus Dyschiriognatha such as Dyschiriognatha tenera, spiders belonging to the genus Latrodectus such as Latrodectus mactans, Latrodectus hasseltii, Latrodectus geometricus and Latrodectus tredecimguttatus, and spiders belonging to the family Tetragnathidae such as spiders belonging to the genus Euprosthenops. Examples of spider silk proteins include traction yarn proteins such as MaSp (MaSp1 and MaSp2) and ADF (ADF3 and ADF4), and MiSp (MiSp1 and MiSp2).

More specific examples of the fibroin produced by spiders include fibroin-3 (adf-3) [derived from Araneus diadematus] (GenBank Accession No. AAC47010 (amino acid sequence), U47855 (base sequence)), fibroin-4 (adf-4) [derived from Araneus diadematus] (GenBank Accession No. AAC47011 (amino acid sequence), U47856 (base sequence)), dragline silk protein spidroin 1 [derived from Nephila clavipes] (GenBank Accession No. AAC04504 (amino acid sequence), U37520 (base sequence)), major ampullate spidroin 1 [derived from Latrodectus hesperus] (GenBank Accession No. ABR68856 (amino acid sequence), EF595246 (base sequence)), dragline silk protein spidroin 2 [derived from Nephila clavata] (GenBank Accession No. AAL32472 (amino acid sequence), AF441245 (base sequence)), major ampullate spidroin 1 [derived from Euprosthenops australis] (GenBank Accession No. CAJ00428 (amino acid sequence), AJ973155 (base sequence)) and major ampullate spidroin 2 [Euprosthenops australis] (GenBank Accession No. CAM32249.1 (amino acid sequence), AM490169 (base sequence)), minor ampullate silk protein 1 [Nephila clavipes] (GenBank Accession No. AAC14589.1 (amino acid sequence), minor ampullate silk protein 2 [Nephila clavipes] (GenBank Accession No. AAC14591.1 (amino acid sequence)), and minor ampullate spidroin-like protein [Nephilengys cruentata] (GenBank Accession No. ABR37278.1 (amino acid sequence)).

As a further specified example of the naturally occurring fibroin, a fibroin whose sequence information is registered in NCBI GenBank may be mentioned. For example, sequences thereof may be confirmed by extracting sequences in which spidroin, ampullate, fibroin, "silk and polypeptide", or "silk and protein" is described as a keyword in DEFINITION among sequences including INV as DIVISION in sequence information registered in NCBI GenBank, sequences in which a specific character string of a product is described from CDS or sequences in which a specific character string is described from SOURCE to TISSUE TYPE.

The modified fibroin may be a modified silk fibroin (a modified silk protein obtained by modifying an amino acid sequence of a silk protein produced by silkworm), and a modified spider silk fibroin (a modified spider silk protein obtained by modifying an amino acid sequence of a spider silk protein produced by spiders). The modified fibroin is preferably a modified spider silk fibroin.

Specific examples of the modified fibroin include: a modified fibroin (first modified fibroin) derived from a large spinal canal bookmark silk protein produced in a major ampullate gland of a spider; a modified fibroin (second modified fibroin) in which the content of the glycine residue is reduced; a modified fibroin (third modified fibroin) in which the content of the $(A)_n$ motif is reduced; and a modified fibroin (fourth modified fibroin) in which a content of glycine residue and the content of the $(A)_n$ motif are reduced. These modified fibroins are excellent in flame retardancy, hygroscopic heat generating property, and heat retaining property and suitable for using for fireproof clothes (for example, for fireman uniforms and for rescue), fireproof gloves (for example, for laboratory, for industries, and for cooking), winter clothes (cold protection clothes) such as gloves, mufflers, sweaters, outerwear and jackets, batting for cold protection clothes, innerwear, sportswear, shirts, bedding, and batting for bedding.

The first modified fibroin include a protein including a domain sequence represented by Formula 1: $[(A)_n$ motif-$REP]_m$. In the first modified fibroin, the number of amino acid residues in the $(A)_n$ motif is preferably an integer of 3 to 20, more preferably an integer of 4 to 20, still more preferably an integer of 8 to 20, even more preferably an integer of 10 to 20, even further more preferably an integer of 4 to 16, particularly preferably an integer of 8 to 16, and most preferably an integer of 10 to 16. In the first modified fibroin, the number of amino acid residues constituting REP in Formula 1 is preferably 10 to 200 residues, more preferably 10 to 150 residues, and still more preferably 20 to 100 residues, and even more preferably 20 to 75 residues. In the first modified fibroin, the total number of glycine residues, serine residues, and alanine residues contained in the amino acid sequence represented by Formula 1: $[(A)_n$ motif - $REP]_m$ is preferably 40% or more, more preferably 60% or more, and still more preferably 70% or more with respect to the total number of amino acid residues.

The first modified fibroin may be a polypeptide including an amino acid sequence unit represented by Formula 1: $[(A)_n$ motif - $REP]_m$, and having a C-terminal sequence which is the amino acid sequence set forth in any of SEQ ID NOs: 1 to 3 or an amino acid sequence having 90% or more homology with the amino acid sequence set forth in any of SEQ ID NOs: 1 to 3.

The amino acid sequence set forth in SEQ ID NO: 1 is identical to the amino acid sequence consisting of 50 amino acid residues at the C-terminal of the amino acid sequence of ADF3 (GI: 1263287, NCBI). The amino acid sequence set forth in SEQ ID NO: 2 is identical to the amino acid sequence obtained by removing 20 residues from the C-terminal of the amino acid sequence set forth in SEQ ID NO: 1. The amino acid sequence set forth in SEQ ID NO: 3 is identical to the amino acid sequence obtained by removing 29 residues from the C-terminal of the amino acid sequence set forth in SEQ ID NO: 1.

More specific examples of the first modified fibroin can include a modified fibroin including (1-i) the amino acid sequence set forth in SEQ ID NO: 4 (recombinant spider silk protein ADF3KaiLargeNRSH1) and (1-ii) an amino acid sequence having 90% or more sequence identity with the amino acid sequence set forth in SEQ ID NO: 4. The sequence identity is preferably 95% or more.

The amino acid sequence set forth in SEQ ID NO: 4 is an amino acid sequence obtained by approximately doubling repeating regions from the first repeating region to the 13th repeating region and performing mutation so that translation is terminated at the 1154th amino acid residue in an amino acid sequence obtained by adding the amino acid sequence (SEQ ID NO: 5) consisting of a start codon, a His10 tag, and a recognition site for HRV3C protease (human rhinovirus 3C protease) to the N-terminal of ADF3. The C-terminal amino acid sequence of the amino acid sequence set forth in SEQ ID NO: 4 is identical to the amino acid sequence set forth in SEQ ID NO: 3.

The modified fibroin of (1-i) may consist of the amino acid sequence set forth in SEQ ID NO: 4.

The domain sequence of the second modified fibroin has an amino acid sequence in which the content of the glycine residue is reduced, as compared with naturally occurring fibroin. It can be said that the second modified fibroin has an amino acid sequence equivalent to an amino acid sequence in which at least one or a plurality of glycine residues in the REP are substituted with other amino acid residues, as compared with naturally occurring fibroin.

The domain sequence of the second modified fibroin may have an amino acid sequence equivalent to an amino acid sequence in which one glycine residue in at least one or the plurality of motif sequences, at least one of which is selected from GGX and GPGXX (where G represents a glycine residue, P represents a proline residue, and X represents any amino acid residue other than glycine) in the REP, is substituted with other amino acid residue, as compared with naturally occurring fibroin.

In the second modified fibroin, the proportion of the motif sequences in which the above-described glycine residue is substituted with other amino acid residue may be 10% or more with respect to the entire motif sequences.

The second modified fibroin may include a domain sequence represented by Formula 1: $[(A)_n$ motif - $REP]_m$ and have an amino acid sequence in which z/w is 30% or more, 40% or more, 50% or more, or 50.9% or more, in a case where the total number of amino acid residues in the amino acid sequence consisting of XGX (where X represents any amino acid residue other than glycine) included in all REPs in a sequence excluding the sequence from the $(A)_n$ motif located at the most C-terminal side to the C-terminal of the domain sequence from the domain sequence is denoted by z, and the total number of amino acid residues in the sequence excluding the sequence from the $(A)_n$ motif located at the most C-terminal side to the C-terminal of the domain sequence from the domain sequence is denoted by w. The number of alanine residues may be 83% or more with respect to the total number of amino acid residues in the $(A)_n$ motif, preferably 86% or more, more preferably 90% or more, still more preferably 95% or more, and even still more preferably 100% (which means that the $(A)_n$ motif is composed of only alanine residues).

In the second modified fibroin, the content proportion of an amino acid sequence consisting of XGX is preferably increased by substituting one glycine residue in GGX motif with other amino acid residue. In the second modified fibroin, the content proportion of an amino acid sequence consisting of GGX in the domain sequence is preferably 30% or less, more preferably 20% or less, still more preferably 10% or less, even still more preferably 6% or less, still further preferably 4% or less, and particularly preferably 2% or less. The content proportion of an amino acid sequence consisting of GGX in a domain sequence can be calculated by the same method as the following method for calculating the content proportion (z/w) of the amino acid sequence consisting of XGX.

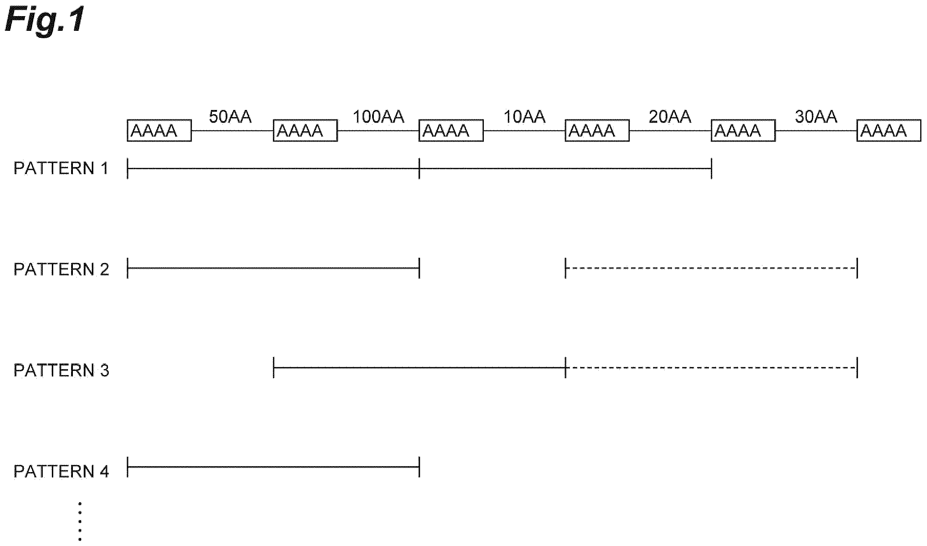

The calculation method for z/w will be described in more detail. First, in a fibroin (a modified fibroin or a naturally occurring fibroin) including a domain sequence represented by Formula 1: $[(A)_n$ motif - $REP]_m$ - $(A)_n$ motif], the amino acid sequence consisting of XGX is extracted from all REPs included in a sequence excluding a sequence from the $(A)_n$ motif located closest to the C-terminal side to the C-terminal of the domain sequence from the domain sequence. The total number of amino acid residues constituting XGX is z. For example, in a case where 50 amino acid sequences consisting of XGX (without overlap) are extracted, z is $50 \times 3 = 150$. Further, for example, in a case where there exists an X (a central X) contained in two XGXs, as in the case of an amino acid sequence consisting of XGXGX, the calculation is performed by subtracting the overlapping portion (in the case of XGXGX, it is counted as 5 amino acid residues). w is the total number of amino acid residues included in the sequence excluding a sequence from the $(A)_n$ motif located closest to the C terminal to the C terminal of the domain sequence from the domain sequence. For example, in the case of the domain sequence illustrated in Fig. 1, w is $4 + 50 + 4 + 100 + 4 + 10 + 4 + 20 + 4 + 30 = 230$ (the $(A)_n$ motif located closest to the C-terminal side is excluded.). Next, z/w (%) can be calculated by dividing z by w.

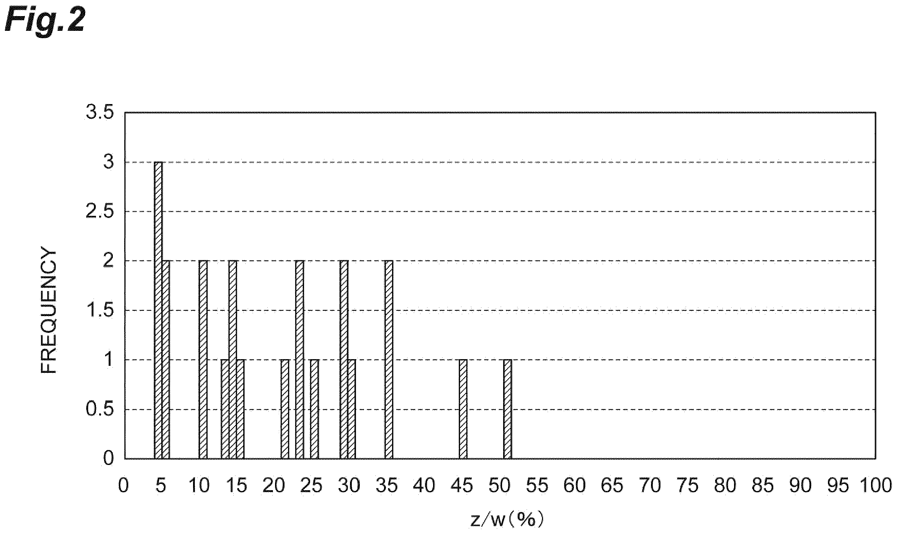

Here, z/w in a naturally occurring fibroin will be described. First, 663 types of fibroins (among them, 415 types of fibroins derived from spiders) were extracted by confirming fibroins with amino acid sequence information registered in NCBI GenBank according to the method exemplified as described above. By the calculation method described above, z/w was calculated from the amino acid sequence of each naturally occurring fibroin, among all of the extracted fibroins, which includes the domain sequence represented by Formula 1: $[(A)_n$ motif - $REP]_m$ and in which a content proportion of the amino acid sequence consisting of GGX in fibroin is 6% or less. The results are shown in Fig. 2. The horizontal axis in Fig. 2 indicates z/w (%), and the vertical axis indicates frequency. As is clear from Fig. 2, z/w in the naturally occurring fibroin is less than 50.9% (the highest z/w is 50.86%).

In the second modified fibroin, z/w is preferably 50.9% or more, more preferably 56.1% or more, still more preferably 58.7% or more, even still more preferably 70% or more, and still further preferably 80% or more. The upper limit of z/w is not particularly limited, but, for example, it may be 95% or less.

The second modified fibroin can be obtained by, for example, modifying a cloned naturally occurring fibroin gene sequence such that at least a part of a base sequence encoding a glycine residue is substituted with other amino acid residue to encode other amino acid residue. In this case, one glycine residue in GGX motif and GPGXX motif may be selected as the glycine residue to be modified or may be substituted so that z/w is 50.9% or more. Alternatively, a modified fibroin may also be obtained, for example, by designing an amino acid sequence satisfying the above-described aspect based on the amino acid sequence of a naturally occurring fibroin and chemically synthesizing a nucleic acid encoding the designed amino acid sequence. In any case, with respect to the amino acid sequence of a naturally occurring fibroin, in addition to the modification corresponding to the substitution of the glycine residue in the REP with other amino acid residue, further modification of amino acid sequence corresponding to substitution, deletion, insertion and/or addition of one or a plurality of amino acid residues may be carried out.

The other amino acid residue described above is not particularly limited as long as it is any amino acid residue other than glycine residue, but it is preferably a hydrophobic amino acid residue such as valine (V) residue, leucine (L) residue, isoleucine (I) residue, methionine (M) residue, proline (P) residue, phenylalanine (F) residue, and tryptophan (W) residue, or a hydrophilic amino acid residues such glutamine (Q) residue, asparagine (N) residue, serine (S) residue, lysine (K) residue, and glutamic acid (E) residue, more preferably valine (V) residue, phenylalanine (F), leucine (L) residue, isoleucine (I) residue, and glutamine (Q) residue, and still more preferably glutamine (Q) residue.

A more specific example of the fibroin can include a modified fibroin including (2-i) the amino acid sequence set forth in SEQ ID NO: 6 (Met-PRT380), SEQ ID NO: 7 (Met-PRT410), SEQ ID NO: 8 (Met-PRT525), or SEQ ID NO: 9 (Met-PRT799), or (2-ii) an amino acid sequence having 90% or more sequence identity with the amino acid sequence set forth in SEQ ID NO: 6, SEQ ID NO: 7, SEQ ID NO: 8, or SEQ ID NO: 9.

The modified fibroin of (2-i) will be described. The amino acid sequence set forth in SEQ ID NO: 6 is obtained by substituting all GGXs in the REP of the amino acid sequence set forth in SEQ ID NO: 10 (Met-PRT313) equivalent to a naturally occurring fibroin with GQX. The amino acid sequence set forth in SEQ ID NO: 7 is obtained by deleting one of every two $(A)_n$ motifs from the N-terminal side to the C-terminal side in the amino acid sequence set forth in SEQ ID NO: 6 and further inserting one $[(A)_n$ motif - REP] just before the C-terminal sequence. The amino acid sequence set forth in SEQ ID NO: 8 is obtained by inserting two alanine residues at the C-terminal side of each $(A)_n$ motif of the amino acid sequence set forth in SEQ ID NO: 7, and further substituting a part of glutamine (Q) residues with serine (S) residues and deleting a part of amino acids on the C-terminal side. The amino acid sequence set forth in SEQ ID NO: 9 is an amino acid sequence obtained by adding a Hinge and a His tag to the C-terminal of a sequence obtained by repeating, four times, a region of 20 domain sequences (where several amino acid residues on the C-terminal side of the region are substituted) present in the amino acid sequence set forth in SEQ ID NO: 7.

The value of z/w in the amino acid sequence set forth SEQ ID NO: 10 (equivalent to a naturally occurring fibroin) is 46.8%. The values of z/w in the amino acid sequences set forth in SEQ ID NO: 6, the amino acid sequence set forth in SEQ ID NO: 7, the amino acid sequence set forth in SEQ ID NO: 8, and the amino acid sequence set forth in SEQ ID NO: 9 are respectively 58.7%, 70.1%, 66.1%, and 70.0%. In addition, the values of x/y with a Giza ratio (described later) of 1:1.8 to 11.3 in the amino acid sequences set forth in SEQ ID NO: 10, SEQ ID NO: 6, SEQ ID NO: 7, SEQ ID NO: 8, and SEQ ID NO: 9 are respectively 15.0%, 15.0%, 93.4%, 92.7%, and 89.8%.

The modified fibroin of (2-i) may consist of the amino acid sequence set forth in SEQ ID NO: 6, SEQ ID NO: 7, SEQ ID NO: 8, or SEQ ID NO: 9.

The modified fibroin of (2-ii) includes an amino acid sequence having 90% or more sequence identity with the amino acid sequence set forth in SEQ ID NO: 6, SEQ ID NO: 7, SEQ ID NO: 8, or SEQ ID NO: 9. The modified fibroin of (2-ii) is also a protein including a domain sequence represented by Formula 1: $[(A)_n$ motif - $REP]_m$. The sequence identity is preferably 95% or more.

The modified fibroin of (2-ii) preferably has 90% or more sequence identity with the amino acid sequence set forth in SEQ ID NO: 6, SEQ ID NO: 7, SEQ ID NO: 8, or SEQ ID NO: 9, and in a case where the total number of amino acid residues in the amino acid sequence consisting of XGX (where X represents any amino acid residue other than glycine) included in the REP is z, and the total number of amino acid residues in the REP in the domain sequence is w, z/w is preferably 50.9% or more.

The second modified fibroin may include a tag sequence at one or both of the N-terminal and C-terminal. This makes it possible to isolate, immobilize, detect, and visualize the modified fibroin.

The tag sequence may be, for example, an affinity tag utilizing specific affinity (binding property, affinity) with another molecule. As a specific example of the affinity tag, a histidine tag (a His tag) can be mentioned. The His tag is a short peptide in which about 4 to 10 histidine residues are arranged and has a property of specifically binding to a metal ion such as nickel, and thus it can be used for isolation of a modified fibroin by a chelating metal chromatography. A specific example of the tag sequence may include the amino acid sequence set forth in SEQ ID NO: 11 (amino acid sequence including a His tag sequence and a hinge sequence).

In addition, a tag sequence such as glutathione-S-transferase (GST) that specifically binds to glutathione or a maltose binding protein (MBP) that specifically binds to maltose can also be used.

Further, an "epitope tag" utilizing an antigen-antibody reaction can also be used. By adding a peptide (an epitope) showing antigenicity as a tag sequence, an antibody against the epitope can be bound. Examples of the epitope tag include an HA (peptide sequence of hemagglutinin of influenza virus) tag, a myc tag, and a FLAG tag. The modified fibroin can be easily purified with high specificity by utilizing an epitope tag.

It is also possible to use a tag sequence which can be cleaved with a specific protease. By treating a protein adsorbed via the tag sequence with a protease, it is also possible to recover a modified fibroin cleaved from the tag sequence.

A more specific example of the modified fibroin having a tag sequence can include a modified fibroin including (2-iii) the amino acid sequence set forth in SEQ ID NO: 12 (Met-PRT380), SEQ ID NO: 13 (Met-PRT410), SEQ ID NO: 14 (Met-PRT525), or SEQ ID NO: 15 (Met-PRT799), or (2-iv) an amino acid sequence having 90% or more sequence identity with the amino acid sequence set forth in SEQ ID NO: 12, SEQ ID NO: 13, SEQ ID NO: 14, or SEQ ID NO: 15.

The amino acid sequences set forth in SEQ ID NO: 16 (PRT313), SEQ ID NO: 12, SEQ ID NO: 13, SEQ ID NO: 14, and SEQ ID NO: 15 are respectively amino acid sequences obtained by adding the amino acid sequence (including a His tag sequence and a hinge sequence) set forth in SEQ ID NO: 11 to the N-terminal of the amino acid sequences set forth in SEQ ID NO: 10, SEQ ID NO: 6, SEQ ID NO: 7, SEQ ID NO: 8, and SEQ ID NO: 9.

The modified fibroin of (2-iii) may consist of the amino acid sequence set forth in SEQ ID NO: 12, SEQ ID NO: 13, SEQ ID NO: 14, or SEQ ID NO: 15.

The modified fibroin of 2-iv) includes an amino acid sequence having 90% or more sequence identity with the amino acid sequence set forth in SEQ ID NO: 12, SEQ ID NO: 13, SEQ ID NO: 14, or SEQ ID NO: 15. The modified fibroin of (2-iv) is also a protein including a domain sequence represented by Formula 1: $[(A)_n$ motif - $REP]_m$. The sequence identity is preferably 95% or more.

The modified fibroin of (2-iv) preferably has 90% or more sequence identity with the amino acid sequence set forth in SEQ ID NO: 12, SEQ ID NO: 13, SEQ ID NO: 14, or SEQ ID NO: 15, and in a case where the total number of amino acid residues in the amino acid sequence consisting of XGX (where X represents any amino acid residue other than glycine) included in the REP is z, and the total number of amino acid residues in the REP in the domain sequence is w, z/w is preferably 50.9% or more.

The second modified fibroin may include a secretory signal for releasing the protein produced in the recombinant protein production system to the outside of a host. The sequence of the secretory signal can be appropriately set depending on the type of the host.

The domain sequence of the third modified fibroin has an amino acid sequence in which the content of the $(A)_n$ motif is reduced, as compared with naturally occurring fibroin. It can be said that the domain sequence of the third modified fibroin has an amino acid sequence equivalent to an amino acid sequence in which at least one or a plurality of the $(A)_n$ motifs are deleted, as compared with naturally occurring fibroin.

The third modified fibroin may have an amino acid sequence equivalent to an amino acid sequence in which 10% to 40% of the $(A)_n$ motifs are deleted from naturally occurring fibroin.

The domain sequence of the third modified fibroin may have an amino acid sequence equivalent to an amino acid sequence obtained by deleting at least one of every one to three $(A)_n$ motifs from the N-terminal side to the C-terminal side, as compared with naturally occurring fibroin.

The domain sequence of the third modified fibroin may have an amino acid sequence equivalent to an amino acid sequence obtained by repeating deletion of at least two consecutive $(A)_n$ motifs and deletion of one $(A)_n$ motif in this order from the N-terminal side to the C-terminal side, as compared with naturally occurring fibroin.

The domain sequence of the third modified fibroin may have an amino acid sequence equivalent to an amino acid sequence obtained by deleting at least one of every two $(A)_n$ motifs from the N-terminal side to the C-terminal side.

The third modified fibroin may include a domain sequence represented by Formula 1: $[(A)_n$ motif - REP$]_m$, and may have an amino acid sequence in which x/y is 20% or more, 30% or more, 40% or more, or 50% or more, in a case where the number of amino acid residues of the REP of two $[(A)_n$ motif - REP] units adjacent to each other is sequentially compared from the N-terminal side to the C-terminal side and then the number of amino acid residues of one REP having a small number of amino acid residues is set to 1, the maximum total value of the added number of amino acid residues of two $[(A)_n$ motif - REP] units adjacent to each other, in which the ratio of the number of amino acid residues of the other REP is 1.8 to 11.3, is denoted by x, and the total number of amino acid residues in the domain sequence is denoted by y. The number of alanine residues may be 83% or more with respect to the total number of amino acid residues in the $(A)_n$ motif, preferably 86% or more, more preferably 90% or more, still more preferably 95% or more, and even still more preferably 100% (which means that the $(A)_n$ motif is composed of only alanine residues).

The method for calculating x/y will be described in more detail with reference to Fig. 1. Fig. 1 illustrates a domain sequence obtained by removing an N-terminal sequence and a C-terminal sequence from a modified fibroin. The domain sequence has a sequence of, from the N-terminal side (left side), $(A)_n$ motif - first REP (50 amino acid residues) - $(A)_n$ motif - second REP (100 amino acid residues) - $(A)_n$ motif - third REP (10 amino acid residues) - $(A)_n$ motif - fourth REP (20 amino acid residues) - $(A)_n$ motif - fifth REP (30 amino acid residues) - $(A)_n$ motif sequence.

Two $[(A)_n$ motif - REP] units adjacent to each other are sequentially selected from the N-terminal side toward the C-terminal side so that the units are not overlapped with each other. In this case, an unselected $[(A)_n$ motif - REP] unit may be present. In Fig. 1, pattern 1 (comparison of first REP and second REP, and comparison of third REP and fourth REP), pattern 2 (comparison of first REP and second REP, and comparison of fourth REP and fifth REP), pattern 3 (comparison of second REP and third REP, and comparison of fourth REP and fifth REP), and pattern 4 (comparison of first REP and second REP). There are other selection methods other than these methods.

Subsequently, for each pattern, the number of amino acid residues of each REP in two selected $[(A)_n$ motif - REP] units adjacent to each other is compared. The comparison is performed by determining the ratio of the number of amino acid residues of one REP to the number of amino acid residues of the other REP in a case where the smaller number of amino acid residues of the REP is set to 1. For example, in a case of comparing the first REP (50 amino acid residues) and the second REP (100 amino acid residues), in a case where the first REP having the smaller number of amino acid residues is set to 1, the ratio of the number of amino acid residues of the second REP is 100/50 = 2. Similarly, in a case of comparing the fourth REP (20 amino acid residues) and the fifth REP (30 amino acid residues), in a case where the fourth REP having the smaller number of amino acid residues is set to 1, the ratio of the number of amino acid residues of the fifth REP is 30/20 = 1.5.

In Fig. 1, in a case where one group of $[(A)_n$ motif - REP] units having the smaller number of amino acid residues is set to 1, the other group in which the ratio of the number of amino acid residues is 1.8 to 11.3 is indicated by a solid line. In the present specification, this ratio is referred to as the Giza ratio. In a case where one group of $[(A)_n$ motif - REP] units having the smaller number of amino acid residues is set to 1, the other group in which the ratio of the number of amino acid residues is less than 1.8 or more than 11.3 is indicated by a broken line.

In each pattern, the total numbers of amino acid residues of two $[(A)_n$ motif - REP] units adjacent to each other indicated by solid lines are added (not only the number of the REPs but also the number of amino acid residues in the $(A)_n$ motif are added.) Then, the added total values are compared, and the total value (maximum value of the total values) of the pattern having the maximum total value is denoted by x. In the example illustrated in Fig. 1, the total value of the pattern 1 is the maximum.

Next, x/y (%) can be calculated by dividing x by y which is the total number of amino acid residues of the domain sequence.

In the third modified fibroin, x/y is preferably 50% or more, more preferably 60% or more, still more preferably 65% or more, even still more preferably 70% or more, still further preferably 75% or more, and particularly preferably 80% or more. The upper limit of x/y is not particularly limited, but for example, it may be 100% or less. In a case where the Giza ratio is 1:1.9 to 11.3, x/y is preferably 89.6% or more. In a case where the Giza ratio is 1:1.8 to 3.4, x/y is more preferably 77.1% or more. In a case where the Giza ratio is 1:1.9 to 8.4, x/y is still more preferably 75.9% or more. In a case where the Giza ratio is 1:1.9 to 4.1, x/y is even still more preferably 64.2% or more.

In a case where the third modified fibroin is a modified fibroin in which at least seven $(A)_n$ motifs present in the domain sequence are composed of only alanine residues, x/y is preferably 46.4% or more, more preferably 50% or more, still more preferably 55% or more, even still more preferably 60% or more, still further preferably 70% or more, and particularly preferably 80% or more. The upper limit of x/y is not particularly limited as long as it is 100% or less.

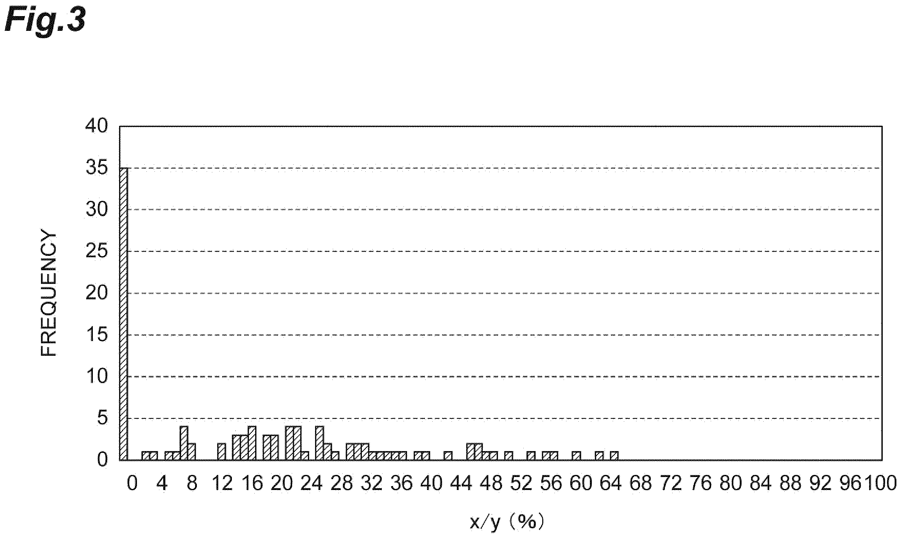

Here, x/y in a naturally occurring fibroin will be described. First, 663 types of fibroins (among them, 415 types of fibroins derived from spiders) were extracted by confirming fibroins with amino acid sequence information registered in NCBI GenBank according to the method exemplified as described above. By the calculation method described above, x/y was calculated from the amino acid sequence of each naturally occurring fibroin, among all of the extracted fibroins, composed of the domain sequence represented by Formula 1: $[(A)_n$ motif - REP$]_m$. The results in the case where the Giza ratio is 1:1.9 to 4.1 are shown in Fig. 3.

The horizontal axis in Fig. 3 indicates x/y (%), and the vertical axis indicates frequency. As is clear from Fig. 3, x/y in the naturally occurring fibroin is less than 64.2% (the highest x/y is 64.14%).

The third modified fibroin, for example, can be obtained by deleting one or a plurality sequences encoding $(A)_n$ motif from a cloned gene sequence of naturally occurring fibroin such that x/y is 64.2% or more. Alternatively, the third modified fibroin may also be obtained, for example, by designing an amino acid sequence equivalent to an amino acid sequence obtained by deleting one or a plurality $(A)_n$ motifs so that x/y is 64.2% or more based on the amino acid sequence of a naturally occurring fibroin and chemically synthesizing a nucleic acid encoding the designed amino acid sequence. In any case, with respect to the amino acid sequence of a naturally occurring fibroin, in addition to the modification corresponding to the deletion of the $(A)_n$ motif, further modification of amino acid sequence equivalent to substitution, deletion, insertion and/or addition of one or a plurality of amino acid residues may be carried out.

A more specific example of the third modified fibroin can include a modified fibroin including (3-i) the amino acid sequence set forth in SEQ ID NO: 17 (Met-PRT399), SEQ ID NO: 7 (Met-PRT410), SEQ ID NO: 8 (Met-PRT525), or SEQ ID NO: 9 (Met-PRT799), or (3-ii) an amino acid sequence having 90% or more sequence identity with the amino acid sequence set forth in SEQ ID NO: 17, SEQ ID NO: 7, SEQ ID NO: 8, or SEQ ID NO: 9.

The modified fibroin of (3-i) will be described. The amino acid sequence set forth in SEQ ID NO: 17 is obtained by deleting one of every two $(A)_n$ motifs from the N-terminal side to the C-terminal side in the amino acid sequence set forth in SEQ ID NO: 10 (Met-PRT313) equivalent to a naturally occurring fibroin and by further inserting one $[(A)_n$ motif - REP] just before the C-terminal sequence. The amino acid sequence set forth in SEQ ID NO: 7, SEQ ID NO: 8, or SEQ ID NO: 9 is as described in the second modified fibroin.

The value of x/y with a Giza ratio of 1:1.8 to 11.3 in the amino acid sequence set forth in SEQ ID NO: 10 (equivalent to a naturally occurring fibroin) is 15.0%. Both the values of x/y in the amino acid sequences set forth in SEQ ID NO: 17 and the value of x/y in the amino acid sequence set forth in SEQ ID NO: 7 are 93.4%. The value of x/y in the amino acid sequence set forth in SEQ ID NO: 8 is 92.7%. The value of x/y in the amino acid sequence set forth in SEQ ID NO: 9 is 89.8%. The values of z/w in the amino acid sequences set forth in SEQ ID NO: 10, SEQ ID NO: 17, SEQ ID NO: 7, SEQ ID NO: 8, and SEQ ID NO: 9 are respectively 46.8%, 56.2%, 70.1%, 66.1%, and 70.0%.

The modified fibroin of (3-i) may consist of the amino acid sequence set forth in SEQ ID NO: 17, SEQ ID NO: 7, SEQ ID NO: 8, or SEQ ID NO: 9.

The modified fibroin of (3-ii) includes an amino acid sequence having 90% or more sequence identity with the amino acid sequence set forth in SEQ ID NO: 17, SEQ ID NO: 7, SEQ ID NO: 8, or SEQ ID NO: 9. The modified fibroin of (3-ii) is also a protein including a domain sequence represented by Formula 1: $[(A)_n$ motif - REP$]_m$. The sequence identity is preferably 95% or more.

The modified fibroin of (3-ii) preferably has 90% or more sequence identity with the amino acid sequence set forth in SEQ ID NO: 17, SEQ ID NO: 7, SEQ ID NO: 8, or SEQ ID NO: 9, and in a case where the number of amino acid residues of the REP of two $[(A)_n$ motif - REP] units adjacent to each other is sequentially compared from the N-terminal side to the C-terminal side, then the number of amino acid residues of one REP having a small number of amino acid residues is set to 1, and the maximum total value of the added numbers of amino acid residues of two $[(A)_n$ motif - REP] units adjacent to each other, in which the ratio (1:1.8 to 11.3 as a Giza ratio) of the number of amino acid residues of the other REP is 1.8 to 11.3, is denoted by x, and the total number of amino acid residues in the domain sequence is denoted by y, x/y is preferably 64.2% or more.

The third modified fibroin may include a tag sequence described above at one or both of the N-terminal and C-terminal.

A more specific example of the modified fibroin having a tag sequence can includes a modified fibroin including (3-iii) the amino acid sequence set forth in SEQ ID NO: 18 (Met-PRT399), SEQ ID NO: 13 (Met-PRT410), SEQ ID NO: 14 (Met-PRT525), or SEQ ID NO: 15 (Met-PRT799), or (3-iv) an amino acid sequence having 90% or more sequence identity with the amino acid sequence set forth in SEQ ID NO: 18, SEQ ID NO: 13, SEQ ID NO: 14, or SEQ ID NO: 15.

The amino acid sequences set forth in SEQ ID NO: 18, SEQ ID NO: 13, SEQ ID NO: 14, and SEQ ID NO: 15 are respectively amino acid sequences obtained by adding the amino acid sequence (including a His tag sequence and a hinge sequence) set forth in SEQ ID NO: 11 to the N-terminal of the amino acid sequences set forth in SEQ ID NO: 17, SEQ ID NO: 7, SEQ ID NO: 8, and SEQ ID NO: 9.

The modified fibroin of (3-iii) may consist of the amino acid sequence set forth in SEQ ID NO: 18, SEQ ID NO: 13, SEQ ID NO: 14, or SEQ ID NO: 15.

The modified fibroin of (3-iv) includes an amino acid sequence having 90% or more sequence identity with the amino acid sequence set forth in SEQ ID NO: 18, SEQ ID NO: 13, SEQ ID NO: 14, or SEQ ID NO: 15. The modified fibroin of (3-iv) is also a protein including a domain sequence represented by Formula 1: $[(A)_n$ motif - $REP]_m$. The sequence identity is preferably 95% or more.

The modified fibroin of (3-iv) preferably has 90% or more sequence identity with the amino acid sequence set forth in SEQ ID NO: 18, SEQ ID NO: 13, SEQ ID NO: 14, or SEQ ID NO: 15, and in a case where the number of amino acid residues of the REP of two $[(A)_n$ motif - REP] units adjacent to each other is sequentially compared from the N-terminal side to the C-terminal side, then the number of amino acid residues of one REP having a small number of amino acid residues is set to 1, the maximum total value of the added numbers of amino acid residues of two $[(A)_n$ motif - REP] units adjacent to each other, in which the ratio of the number of amino acid residues of the other REP is 1.8 to 11.3, is denoted by x, and the total number of amino acid residues in the domain sequence is denoted by y, x/y is preferably 64.2% or more.

The third modified fibroin may include a secretory signal for releasing the protein produced in the recombinant protein production system to the outside of a host. The sequence of the secretory signal can be appropriately set depending on the type of the host.

The domain sequence of the fourth modified fibroin has an amino acid sequence having not only a reduced content of the $(A)_n$ motif but also a reduced content of the glycine residue, as compared with naturally occurring fibroin. It can be said that the domain sequence of the fourth modified fibroin has an amino acid sequence equivalent to an amino acid sequence in which at least one or a plurality of the $(A)_n$ motifs are deleted and at least one or a plurality of glycine residues in the REP are further substituted with other amino acid residues, as compared with naturally occurring fibroin. That is, the fourth modified fibroin is a modified fibroin having the characteristics of the second modified fibroin and the third modified fibroin described above. Specific aspects and the like of the fourth modified fibroin are as described in the second modified fibroin and the third modified fibroin.

A more specific example of the fourth modified fibroin can include a modified fibroin including (4-i) the amino acid sequence set forth in SEQ ID NO: 7 (Met-PRT410), SEQ ID NO: 8 (Met-PRT525), or SEQ ID NO: 9 (Met-PRT799), SEQ ID NO: 13 (PRT410), SEQ ID NO: 14 (PRT525), or SEQ ID NO: 15 (PRT799), or (4-ii) an amino acid sequence having 90% or more sequence identity with the amino acid sequence set forth in SEQ ID NO: 7, SEQ ID NO: 8, SEQ ID NO: 9, SEQ ID NO: 13, SEQ ID NO: 14, or SEQ ID NO: 15. Specific aspects of the modified fibroin including the amino acid sequence set forth SEQ ID NO: 7, SEQ ID NO: 8, SEQ ID NO: 9, SEQ ID NO: 13, SEQ ID NO: 14, or SEQ ID NO: 15 are as described above.

The structural protein contained in the second component may be a synthetic fiber or a hydrophobic modified fibroin.

The synthetic fiber is generally hard to be wetted by water and has poor water absorbency and poor hygroscopicity. Examples of the synthetic fiber include polyesters such as polyethylene terephthalate, polyamides such as a polycaproamide (nylon 6) and nylon 66, polyacryl, and polyvinyl formal (vinylon). The synthetic fiber generally has the property of not easily being shrunk even when brought into contact with an aqueous medium.

The second component may be a chemical fiber. Examples of the chemical fibers include a natural polymer (a natural fiber), a semi-synthetic polymer (a semi-synthetic fiber), and a synthetic polymer (a synthetic fiber). Examples of the natural polymer (the natural fiber) include regenerated cellulose fibers such as rayon, cupra, polynosic, and lyocell.

Examples of the semi-synthetic polymer (semi-synthetic fiber) include acetate fibers such as an acetate (diacetate) fiber and a triacetate fiber, and PROMIX.

Examples of the synthetic polymer (synthetic fiber) include the above-described polyesters such as polyethylene terephthalate, polyamides such as a polycaproamide (nylon 6) and nylon 66, polyacryl, polyvinyl formal (vinylon), and polyurethane (spandex).

In a case where a regenerated cellulose fiber, an acetate fiber, PROMIX, polyacryl, polyvinyl formal, polyurethane, or the like is used as the second component, it is dissolved in a known solvent (solution) to prepare a spinning solution (doping liquid), which is discharged from the spinneret and joined to the first component (modified fibroin). Then, the first component (modified fibroin) and the second component (chemical fiber) are solidified in a coagulation liquid, whereby a side-by-side type composite fiber is obtained.

In a case where polyester or polyamide is used as the second component, a raw material thereof is melted to form a liquid, which is discharged from the spinneret and joined to the first component (modified fibroin). Then, the first component (modified fiboroin) and the second component (chemical fiber) are solidified in a coagulation liquid, whereby a side-by-side type composite fiber is obtained.

In the present specification, the "hydrophobic modified fibroin" means a modified fibroin fiber having poor water absorbency or hygroscopicity, and the hydrophobicity may be determined, for example, by using a hydropathy index of each amino acid (hydropathy index, hereinafter also referred to as "HI"). The high hydropathy index increases the hydrophobicity of the fiber itself, and thus the shrinkage rate can be reduced even in a case where the fiber is brought into contact with an aqueous medium.

The structural protein is not particularly limited, and it may be one produced by a microorganism or the like by a genetic recombination technique, or one produced synthetically. Alternatively, the structural protein may be a purified naturally occurring structural protein.

The structural protein may be, for example, a structural protein or an artificial structural protein derived from the structural protein. The structural protein means a structural protein that forms or retains a structure and morphology in a living body. That is, the structural protein may be a naturally occurring structural protein and a modified protein in which a part of the amino acid sequence (for example, 10% or less of the amino acid sequence) is modified depending on the amino acid sequence of the naturally occurring structural protein. Examples of the structural protein include fibroin, keratin, collagen, elastin, and, resilin.

The fibroin may be, for example, one or more selected from the group consisting of a silk fibroin, a spider silk fibroin, and a hornet silk fibroin. Particularly, the structural protein may be a silk fibroin or a spider silk fibroin, or a combination thereof. In a case where the silk fibroin and the spider silk fibroin are used in combination, the proportion of the silk fibroin may be, for example, 40 parts by mass or less, 30 parts by mass or less, or 10 parts by mass or less, with respect to 100 parts by mass of the spider silk fibroin.

A silk yarn is a fiber (cocoon yarn) obtained from a cocoon made by a silkworm (Bombyx mori). In general, one thread of the cocoon yarn is composed of two threads of the silk fibroin and a glue substance (sericin) that covers the silk fibroin from the outside. The silk fibroin is composed of a number of fibrils. The silk fibroin is covered by four layers of sericin. Practically, a silk filament obtained by dissolving and removing the outer sericin by scouring is used for clothing. A general silk yarn has a specific gravity of 1.33, an average fineness of 3.3 decitex, and a fiber length of about 1,300 to 1,500 m. The silk fibroin can be obtained by using a natural or indoor silkworm cocoon or a used or discarded silk fabric as the raw material.

The silk fibroin may be a sericin-removed silk fibroin, a sericin-unremoved silk fibroin, or a combination thereof. The sericin-removed silk fibroin is a purified silk fibroin obtained by removing sericin covering the silk fibroin and other fatty materials. The silk fibroin purified in this manner is preferably used in the form of a lyophilized powder. The sericin-unremoved silk fibroin is unpurified silk fibroin in which sericin and the like have not been removed.

The spider silk fibroin may contain a spider silk polypeptide selected from the group consisting of a natural spider silk structural protein and a polypeptide derived from the natural spider silk structural protein (an artificial spider silk structural protein).

Examples of the natural spider silk structural proteins include a large spinal canal bookmark silk structural protein, a weft protein, and a minor ampullate gland structural protein. Since the large spinal canal bookmark silk has a repeating region consisting of a crystalline region and a non-crystalline region (also referred to as an amorphous region), the large spinal canal bookmark silk has high stress and elasticity. The weft of the spider silk is characterized by having no crystalline region but having a repeating region consisting of the non-crystalline region. The stress of the weft is inferior to that of the large spinal canal bookmark silk, but the wet has high elasticity.

The large spinal canal bookmark silk structural protein is produced in the major ampullate gland of the spider and has a characteristic of excellent toughness. Examples of the large spinal canal bookmark silk structural protein include major ampullate spidroins MaSp1 and MaSp2 which are derived from the American golden orb-weaving spider (Nephila clavipes), and ADF3 and ADF4 which are derived from Araneus diadematus. ADF3 is one of the two major bookmark silk proteins of an orb-weaving spider. The polypeptides derived from the natural spider silk structural proteins may be polypeptides derived from these bookmark silk structural proteins. A polypeptide derived from ADF3 is relatively easy to be synthesized and has excellent properties in terms of high elongatability and toughness.

The weft structural protein is produced in the flagelliform gland of the spider. An example of the weft structural protein includes a flagellar form silk protein (flagelliform silk protein) derived from the American golden orb-weaving spider (Nephila clavipes).

The polypeptide derived from the natural spider silk structural protein may be a recombinant spider silk structural protein. Examples of the recombinant spider silk structural protein include a mutant, an analog, or a derivative of the natural spider silk structural protein. A preferred example of such a polypeptide is a recombinant spider silk structural protein (also referred to as "polypeptide derived from the large spinal canal bookmark silk structural protein") of the large spinal canal bookmark silk protein.

An example of the structural protein derived from the large spinal canal bookmark silk and the structural protein derived from silkworm silk, which are fibroin-like structural proteins, includes a protein including a domain sequence represented by Formula 1: $[(A)_n$ motif - $REP1]_m$. Here, Formula 1, A in the $(A)_n$ motif represents an alanine residue, n is preferably an integer of 2 to 27 and may be an integer of 4 to 20, 8 to 20, 10 to 20, 4 to 16, 8 to 16, and 10 to 16, and the number of alanine residues may be 40% or more, 60% or more, 70% or more, 80% or more, 90% or more, and 100% (which means that the $(A)_n$ motif is composed of only alanine residues), with respect to the total number of amino acid residues in the $(A)_n$ motif. The REP1 represents an amino acid sequence composed of 10 to 200 amino acid residues. m represents an integer of 10 to 300. A plurality of the $(A)_n$ motifs may have the same amino acid sequence or amino acid sequences different from each other. The plurality of the REP1 may have the same amino acid sequence or amino acid sequences different from each other.

The structural protein described above may be a structural protein obtained by deleting the $(A)_n$ motif in Formula 1 to improve industrial productivity while maintaining strength and elongatability. Regarding the frequency of deletion, in a case where the number of amino acid residues of the REP of two $[(A)_n$ motif - REP1] units adjacent to each other is sequentially compared from the N-terminal side to the C-terminal side, then the number of amino acid residues of one REP having a small number of amino acid residues is set to 1, the maximum total value of the added numbers of amino acid residues of the described-above two $[(A)_n$ motif - REP1] units adjacent to each other, in which the ratio of the number of amino acid residues of the other REP is 1.8 to 11.3, is denoted by x, and the total number of amino acid residues in the domain sequence described above is denoted by y, a structural protein, for example, having 50% or more x/y can be mentioned.

Further, the structural protein may be a structural protein having an amino acid sequence in which the content of the glycine residue is reduced, the amino acid sequence being equivalent to an amino acid sequence in which at least one or a plurality of glycine residues in the REP are substituted with other amino acid residues in the REP in Formula 1. As such a structural protein, a structural protein in which the proportion of the motif sequences in which the glycine residue is substituted with other amino acid residue is 10% or more with respect to the entire motif sequences can be mentioned.

As a specific example of the structural protein derived from the large spinal canal bookmark silk, a structural protein including the amino acid sequence set forth in SEQ ID NO: 13 and SEQ ID NO: 15 can be mentioned.

As the structural protein derived from the weft structural protein, for example, a structural protein including a domain sequence represented by Formula 2: $[REP2]_o$ (Here, the REP2 in Formula 2 represents an amino acid sequence composed of Gly-Pro-Gly-Gly-X, where X represents one amino acid selected from the group comoposed of alanine (Ala), serine (Ser), tyrosine (Tyr), and valine (Val), and o represents an integer of 8 to 300) can be mentioned. Specifically, a structural protein including the amino acid sequence set forth in SEQ ID NO: 13 can be mentioned. The amino acid sequence represented by SEQ ID NO: 41 (PRT215) is an amino acid sequence obtained by linking an amino acid sequence (referred to as PR1 sequence), equivalent to the repeat portion and the motif, from 1,220th to 1,659th residues from the N-terminal of the partial sequence (NCBI accession No.: AAF36090, GI: 7106224) of the flagellar form silk protein of the American golden orb-weaving spider, which is obtained from the NCBI database, with a C-terminal amino acid sequence from 816th to 907th residues from the C-terminal of the partial sequence (NCBI accession No.: AAC38847, GI: 2833649) of the flagellar form silk protein of the American golden orb-weaving spider, which is obtained from the NCBI database, and further adding the amino acid sequence set forth in SEQ ID NO: 11 (a tag sequence and a hinge sequence) to the N-terminal of the linked sequence.

As a structural protein derived from collagen, for example, a structural protein including a domain sequence represented by Formula 3: $[REP3]_p$ (Here, p in Formula 3 represents an integer of 5 to 300. REP3 represents an amino acid sequence composed of Gly-X-Y, where X and Y represent any amino acid residues other than Gly. A plurality of the REP3 may have the same amino acid sequence or amino acid sequences different from each other) can be mentioned. Specifically, a structural protein including the amino acid sequence set forth in SEQ ID NO: 42 can be mentioned. The amino acid sequence set forth in SEQ ID NO: 42 is obtained by adding the amino acid sequence set forth in SEQ ID NO: 11 (a tag sequence and a hinge sequence) to the N-terminal of the amino acid sequence from the 301th residue to the 540th residue, which corresponds to the repeat portion and motif of the partial sequence of human collagen type 4 (NCBI GenBank Accession No.: CAA56335.1, GI: 3702452) obtained from the NCBI database.

As the structural protein derived from resilin, for example, a structural protein including the domain sequence represented by Formula 4: $[REP4]_q$ (Here, q in Formula 4 represents an integer of 4 to 300. REP4 represents an amino acid sequence composed of Ser-J-J-J-Tyr-Gly-U-Pro. J represents any amino acid residue and particularly preferably an amino acid residue selected from the group consisting of Asp, Ser, and Thr. U represents any amino acid residue and particularly preferably an amino acid residue selected from the group consisting of Pro, Ala, Thr, and Ser. A plurality of the REP4 may have the same amino acid sequence or amino acid sequences different from each other.) cab be mentioned. Specifically, a structural protein including the amino acid sequence set forth in SEQ ID NO: 43 can be mentioned. The amino acid sequence set forth in SEQ ID NO: 43 is obtained by adding the amino acid sequence set forth in SEQ ID NO: 11 (a tag sequence and a hinge sequence) to the N-terminal of the amino acid sequence from 19th residue to 321th residue of the amino acid sequence of resilin (NCBI GenBank Accession No. NP611157, GI: 24654243), in which Thr at the 87th residue is substituted with Ser, and Asn at the 95th residue is substituted with Asp.

Examples of the structural protein derived from elastin include structural proteins having amino acid sequences such as NCBI GenBank Accession No.s, AAC98395 (human), 147076 (sheep), and NP786966 (bovine). Specifically, a structural protein including the amino acid sequence set forth in SEQ ID NO: 44 can be mentioned. The amino acid sequence set forth in SEQ ID NO: 44 is obtained by adding the amino acid sequence set forth in SEQ ID NO: 11 (a tag sequence and a hinge sequence) to the N-terminal of the amino acid sequence from 121th residue to 390th residue of the amino acid sequence of NCBI GenBank Accession No. AAC98395.

An example of the structural protein derived from keratin include a type I keratin of Capra hircus. Specifically, a structural protein including the amino acid sequence set forth in SEQ ID NO: 45 can be mentioned. The amino acid sequence set forth in SEQ ID NO: 45 (PRT798) is obtained by adding the amino acid sequence set forth in SEQ ID NO: 11 (a tag sequence and a hinge sequence) to the N-terminal of the amino acid sequence of NCBI GenBank Accession No. ACY30466.

The structural protein or a modified structural protein derived from the structural protein can be used alone or in a combination of two or more thereof. The hydrophobicity as a whole may be adjusted to the desired value by combining two or more structural proteins.

The modified fibroin may be a fibroin in which the domain sequence is different from the amino acid sequence of a naturally occurring fibroin or may be the same as the amino acid sequence of a naturally occurring fibroin. The modified fibroin used in the outermost layer may be, for example, a naturally occurring fibroin having a modified domain sequence obtained by artificially modifying a domain sequence to impart hydrophobicity.

As the specific example of the modified fibroin used in the second component, a modified fibroin having a domain sequence locally including a region having a high hydropathy index (fifth modified fibroin) or a modified fibroin having a domain sequence in which the content of the glutamine residue (sixth modified fibroin) is reduced can be mentioned. The hydropathy index of each amino acid will be described later.

The domain sequence of the fifth modified fibroin has an amino acid sequence locally including a region having a high hydropathy index, the amino acid sequence being equivalent to an amino acid sequence in which one or a plurality of amino acid residues in the REP are substituted with amino acid residues having a high hydropathy index and/or one or a plurality of amino acid residues having a high hydropathy index are inserted into the REP, as compared with a naturally occurring fibroin.

It is preferable that the region locally high in the hydropathy index is composed of two to four consecutive amino acid residues. The above-described "region having a high hydropathy index" means a region in which the sum or average of the hydropathy indices of consecutive 2 to 4 amino acid residues is higher than the sum or average of the hydropathy indices of the amino acid residues at the same position in the corresponding naturally occurring fibroin.

The "amino acid residue having a high hydropathy index" is an amino acid residue having a higher hydropathy index than the amino acid residue at the same position in the corresponding naturally occurring fibroin. It is more preferable that the above-described amino acid residues having a high hydropathy index are selected from isoleucine (I), valine (V), leucine (L), phenylalanine (F), cysteine (C), methionine (M), and alanine (A).

The fifth modified fibroin may further include the modification of the amino acid sequence equivalent to an amino acid sequence in which one or a plurality of amino acid residues are substituted, deleted, inserted and/or added, as compared with naturally occurring fibroin, in addition to the modification of the amino acid sequence in which one or a plurality of amino acid residues in the REP are substituted with amino acid residues having a high hydropathy index and/or one or a plurality of amino acid residues having a high hydropathy index are inserted into REP, as compared with naturally occurring fibroin.

The fifth modified fibroin may be obtained by, with respect to a cloned gene sequence of naturally occurring fibroin, substituting one or a plurality of hydrophilic amino acid residues in the REP (for example, amino acid residues having a negative hydropathy index) with a hydrophobic amino acid residue (for example, amino acid residues having a positive hydropathy index), and/or inserting one or a plurality of hydrophobic amino acid residues into REP. Further, for example, the modified fibroin may also be obtained by designing an amino acid sequence equivalent to an amino acid sequence in which with respect to the amino acid sequence of a naturally occurring fibroin, one or a plurality of hydrophilic amino acid residues in the REP are substituted with hydrophobic amino acid residues and/or one or a plurality of hydrophobic amino acid residues are inserted into REP, and chemically synthesizing a nucleic acid encoding the designed amino acid sequence. In any case, with respect to the amino acid sequence a naturally occurring fibroin, in addition to the modification corresponding to the substitution of one or a plurality of hydrophilic amino acid residues in the REP with hydrophobic amino acid residues and/or insertion of one or a plurality of hydrophobic amino acid residues into REP, further modification of amino acid sequence equivalent to substitution, deletion, insertion and/or addition of one or a plurality of amino acid residues may be carried out.

A fifth modified fibroin may include a domain sequence represented by Formula 1: $[(A)_n \text{ motif - REP}]_m$ and have an amino acid sequence in which p/q is 6.2% or more, in a case where in all REPs included in a sequence excluding a sequence from an (A)$_n$ motif located to most C-terminal side to the C-terminal of the domain sequence from the domain sequence, the total number of amino acid residues contained in a region where an average value of hydropathy indices of four consecutive amino acid residues is 2.6 or more is denoted by p, and the total number of amino acid residues contained in the sequence excluding the sequence from the (A)$_n$ motif located the most C-terminal side to the C-terminal of the domain sequence from the domain sequence is denoted by q.

Regarding the hydropathy index of amino acid residues, known indices from (Hydropathy index: Kyte J, & Doolittle R (1982)"A simple method for displaying the hydropathic character of a protein", J. Mol. Biol., 157, pp. 105-132) may be used as a reference. Specifically, the hydropathy index (hereinafter, also referred to as "HI") of each amino acid is as shown in Table 1 below.

[Table 1]

| Amino acid | HI | Amino acid | HI |
|---|---|---|---|
| Isoleucine (Ile) | 4.5 | Tryptophan (Trp) | -0.9 |
| Valine (Val) | 4.2 | Tyrosine (Tyr) | -1.3 |
| Leucine (Leu) | 3.8 | Proline (Pro) | -1.6 |
| Phenylalanine (Phe) | 2.8 | Histidine (His) | -3.2 |
| Cysteine (Cys) | 2.5 | Asparagine (Asn) | -3.5 |
| Methionine (Met) | 1.9 | Aspartic acid (Asp) | -3.5 |
| Alanine (Ala) | 1.8 | Glutamine (Gln) | -3.5 |
| Glycine (Gly) | -0.4 | Glutamic acid (Glu) | -3.5 |
| Threonine (Thr) | -0.7 | Lysine (Lys) | -3.9 |
| Serine (Ser) | -0.8 | Arginine (Arg) | -4.5 |

The calculation method for p/q will be described in more detail. In the calculation, the sequence (hereinafter, also referred to as "sequence A") excluding a sequence from the (A)$_n$ motif located closest to the C-terminal side to the C-terminal of the domain sequence from the domain sequence represented by Formula 1: ([(A)$_n$ motif - REP]$_m$ - (A)$_n$ motif] is used. First, in all REPs included in the sequence A, average values of hydropathy indices of the four consecutive amino acid residues are calculated. The average value of the hydropathy indices is obtained by dividing the total sum of HI of each of the amino acid residues contained in the four consecutive amino acid residues by 4 (the number of amino acid residues). The average value of the hydropathy indices is obtained for all of the four consecutive amino acid residues (each of the amino acid residues is used for calculating the average value 1 to 4 times). Next, a region where the average value of the hydropathy indices of the four consecutive amino acid residues is 2.6 or more is specified. Even in a case where a plurality of certain amino acid residues correspond to the "four consecutive amino acid residues having an average value of the hydropathy indices of 2.6 or more", the amino acid residue is counted as one amino acid residue in the region. The total number of amino acid residues included in the region is denoted by p. The total number of amino acid residues included in the sequence A is denoted by q.

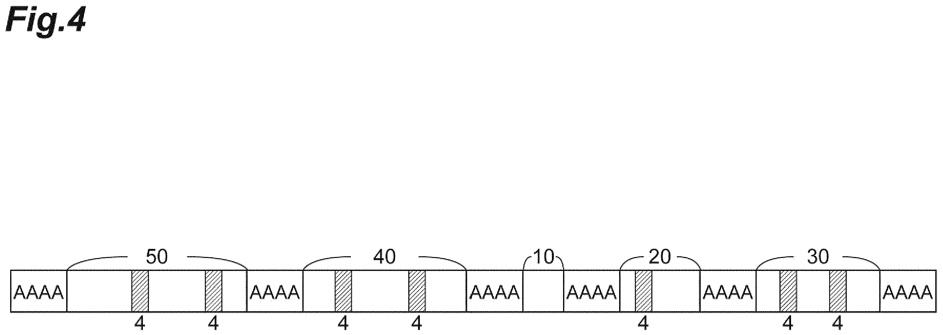

For example, in a case where the "four consecutive amino acid residues whose average value of the hydropathy indices is 2.6 or more" are extracted from 20 places (without overlap), in the region where the average value of the hydropathy indices of the four consecutive amino acid residues is 2.6 or more, the number of the four consecutive amino acid residues (without overlap) is 20, and thus p is 20 × 4 = 80. In addition, for example, in a case where two of the "four consecutive amino acid residues having an average value of the hydropathy indices of 2.6 or more" overlap by only one amino acid residue, in the region where the average value of the hydropathy indices of the four consecutive amino acid residues is 2.6 or more, the number of amino acid residues being included is 7 (p = 2 × 4 - 1 = 7. "-1" corresponds to the subtraction of the overlapping portion). For example, in the case of the domain sequence shown in Fig. 4, since the number of the "four consecutive amino acid residues having an average value of the hydropathy indices of 2.6 or more", which do not overlap, is 7, p is 7 × 4 = 28. Further, for example, in the case of the domain sequence illustrated in Fig. 4, q is 4 + 50 + 4 + 40 + 4 + 10 + 4 + 20 + 4 + 30 = 170 (the (A)$_n$ motif present closest to the C-terminal side can not be included). Next, p/q (%) can be calculated by dividing p by q. In the case of Fig. 4, p/q (%) is 28/170 = 16.47%.

In the fifth modified fibroin, p/q is preferably 6.2% or more, more preferably 7% or more, still more preferably 10% or more, even still more preferably 20% or more, and still further preferably 30% or more. The upper limit of p/q is not particularly limited, but for example, it may be 45% or less.

The fifth modified fibroin may be obtained by, for example, modifying an amino acid sequence of cloned naturally occurring fibroin into an amino acid sequence locally including a region having a high hydropathy index by substituting one or a plurality of hydrophilic amino acid residues in the REP (for example, amino acid residues having a negative hydropathy index) with hydrophobic amino acid residues (for example, amino acid residues having a positive hydropathy index), and/or inserting one or a plurality of hydrophobic amino acid residues into REP, such that the p/q condition is satisfied. Alternatively, the modified fibroin may also be obtained, for example, by designing an amino acid sequence satisfying the p/q condition based on the amino acid sequence of a naturally occurring fibroin and chemically synthesizing a nucleic acid encoding the designed amino acid sequence. In any case, in addition to the modification corresponding to the substitution of one or a plurality of amino acid residues in the REP with amino acid residues having a high hydropathy index and/or insertion of one or a plurality of amino acid residues having a high hydropathy index into REP, as compared with the amino acid sequence of a naturally occurring fibroin, further modification corresponding to substitution, deletion, insertion, and/or addition of one or a plurality of amino acid residues may be carried out.

The amino acid residue having a high hydropathy index is preferably isoleucine (I), valine (V), leucine (L), phenylalanine (F), cysteine (C), methionine (M), and alanine (A), and more preferably valine (V), leucine (L), and isoleucine (I), but is not particularly limited thereto.

A more specific example of the fifth modified fibroin can include a modified fibroin including (5-i) the amino acid sequence set forth in SEQ ID NO: 19 (Met-PRT720), SEQ ID NO: 20 (Met-PRT665), or SEQ ID NO: 21 (Met-PRT666), or (5-ii) an amino acid sequence having 90% or more sequence identity with the amino acid sequence set forth in SEQ ID NO: 19, SEQ ID NO: 20, or SEQ ID NO: 21.

The modified fibroin of (5-i) will be described. The amino acid sequence set forth in SEQ ID NO: 19 is obtained by inserting an amino acid sequence consisting of three amino acid residues (VLI) at two sites for each REP with respect to the amino acid sequence set forth in SEQ ID NO: 7 (Met-PRT410), except for the end of the C-terminal side, and further substituting a part of glutamine (G) residues with serine (S) residues and deleting a part of amino acids on the C-terminal side. The amino acid sequence set forth in SEQ ID NO: 20 is obtained by inserting an amino acid sequence consisting of three amino acid residues (VLI) at one site for every other REP with respect to the amino acid sequence set forth in SEQ ID NO: 8 (Met-PRT525). The amino acid sequence set forth in SEQ ID NO: 21 is obtained by inserting an amino acid sequence consisting of three amino acid residues (VLI) at two sites for every other REP with respect to the amino acid sequence set forth in SEQ ID NO: 8.

The modified fibroin of (5-i) may consist of the amino acid sequence set forth in SEQ ID NO: 19, SEQ ID NO: 20, or SEQ ID NO: 21.

The modified fibroin of (5-ii) includes an amino acid sequence having 90% or more sequence identity with the amino acid sequence set forth in SEQ ID NO: 19, SEQ ID NO: 20, or SEQ ID NO: 21. The modified fibroin of (5-ii) is also a protein including a domain sequence represented by Formula 1: $[(A)_n$ motif - $REP]_m$. The sequence identity is preferably 95% or more.

The modified fibroin of (5-ii) preferably has 90% or more sequence identity with the amino acid sequence set forth in SEQ ID NO: 19, SEQ ID NO: 20, or SEQ ID NO: 21, and preferably has an amino acid sequence in which p/q is 6.2% or more, in a case where in all REPs included in a sequence excluding a sequence from the $(A)_n$ motif located closest to the C-terminal side to the C-terminal of the domain sequence from the domain sequence, the total number of amino acid residues contained in a region where an average value of hydropathy indices of the four consecutive amino acid residues is 2.6 or more is denoted by p, and the total number of amino acid residues contained in the sequence excluding a sequence from the $(A)_n$ motif located closest to the C-terminal side to the C-terminal of the domain sequence from the domain sequence is denoted by q.

The fifth modified fibroin may include a tag sequence at one or both of the N-terminal and C-terminal.

A more specific example of the modified fibroin having a tag sequence can include a modified fibroin including (5-iii) the amino acid sequence set forth in SEQ ID NO: 22 (PRT720), SEQ ID NO: 23 (PRT665), or SEQ ID NO: 24 (PRT666), or (5-iv) an amino acid sequence having 90% or more sequence identity with the amino acid sequence set forth in SEQ ID NO: 22, SEQ ID NO: 23, or SEQ ID NO: 24.

The amino acid sequences set forth in SEQ ID NO: 22, SEQ ID NO: 23, and SEQ ID NO: 24 are respectively amino acid sequences obtained by adding the amino acid sequence (including a His tag sequence and a hinge sequence) set forth in SEQ ID NO: 11 to the N-terminal of the amino acid sequences set forth in SEQ ID NO: 19, SEQ ID NO: 20, and SEQ ID NO: 21.

The modified fibroin of (5-iii) may consist of the amino acid sequence set forth in SEQ ID NO: 22, SEQ ID NO: 23, or SEQ ID NO: 24.

The modified fibroin of (5-iv) includes an amino acid sequence having 90% or more sequence identity with the amino acid sequence set forth in SEQ ID NO: 22, SEQ ID NO: 23, or SEQ ID NO: 24. The modified fibroin of (5-iv) is also a protein including a domain sequence represented by Formula 1: $[(A)_n$ motif - $REP]_m$. The sequence identity is preferably 95% or more.

The modified fibroin of (5-iv) preferably has 90% or more sequence identity with the amino acid sequence set forth in SEQ ID NO: 22, SEQ ID NO: 23, or SEQ ID NO: 24, and preferably has an amino acid sequence in which p/q is 6.2% or more, in a case where in all REPs included in a sequence excluding a sequence from the $(A)_n$ motif located closest to the C-terminal side to the C-terminal of the domain sequence from the domain sequence, the total number of amino acid residues contained in a region where an average value of hydropathy indices of the four consecutive amino acid residues is 2.6 or more is denoted by p, and the total number of amino acid residues contained in the sequence excluding a sequence from the $(A)_n$ motif located closest to the C-terminal side to the C-terminal of the domain sequence from the domain sequence is denoted by q.

The fifth modified fibroin may include a secretory signal for releasing the protein produced in the recombinant protein production system to the outside of a host. The sequence of the secretory signal can be appropriately set depending on the type of the host.

The sixth modified fibroin has an amino acid sequence with a reduced content of glutamine residue, as compared with a naturally occurring fibroin.

The sixth modified fibroin preferably includes at least one motif selected from GGX motif and GPGXX motif in the amino acid sequence of the REP.

In a case where the sixth modified fibroin includes a GPGXX motif in the REP, a GPGXX motif content rate is usually 1% or more, may be 5% or more, and is preferably 10% or more. The upper limit of the GPGXX motif content rate is not particularly limited, may be 50% or less, and may be 30% or less.

In the present specification, the "GPGXX motif content rate" is a value calculated by the following method.

In a fibroin (a modified fibroin or a naturally occurring fibroin) including a domain sequence represented by Formula 1: $[(A)_n$ motif - $REP]_m$ or Formula 2: $[(A)_n$ motif - $REP]_m$ - $(A)_n$ motif, in a case where the number obtained by tripling the total number of the GPGXX motifs included in all REPs included in a sequence excluding the sequence from the $(A)_n$ motif located at the most C-terminal side to the C-terminal of the domain sequence from the domain sequence (that is, equivalent to the total number of G and P in the GPGXX motifs) is denoted by s, and the total number of amino acid residues in all REPs excluding the sequence from the $(A)_n$ motif located at the most the C-terminal side to the C-terminal of the domain sequence from the domain sequence and further excluding $(A)_n$ motifs is denoted by t, the GPGXX motif content rate is calculated as s/t.

For the calculation of the GPGXX motif content rate, the "sequence excluding a sequence from the $(A)_n$ motif located closest to the C-terminal side to the C-terminal of the domain sequence from the domain sequence" is used to exclude the effect occurring due to the fact that the "sequence from the $(A)_n$ motif located closest to the C-terminal side to the C-terminal from the domain sequence" (sequence equivalent to REP) may include a sequence that is weakly correlated with the sequence characteristics of fibroin, which influences the calculation result of the GPGXX motif content rate in a case where m is small (that is, in a case where the domain sequence is short). In a case where a "GPGXX motif" is located at the C-terminal of the REP, it is treated as "GPGXX motif" even in a case where "XX" is, for example, "AA".

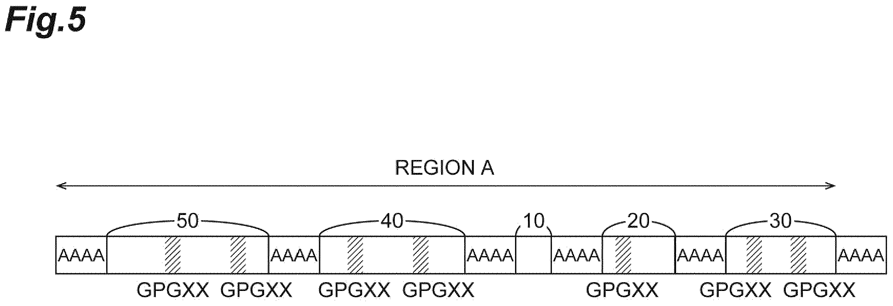

Fig. 5 is a schematic diagram showing a domain sequence of a modified fibroin. The calculation method for the GPGXX motif content rate will be specifically described with reference to Fig. 5. First, in a domain sequence (which is an $[(A)_n$ motif - $REP]_m$ - $(A)_n$ motif] type) of a modified fibroin illustrated in Fig. 5, since all REPs are included in the "sequence excluding a sequence from the $(A)_n$ motif located closest to the C-terminal side to the C-terminal of the domain sequence from the domain sequence" (in Fig. 5, shown as "region A"), the number of GPGXX motifs for calculating s is 7, and s is $7 \times 3 = 21$. Similarly, since all REPs are included in the "sequence excluding a sequence from the $(A)_n$ motif located closest to the C-terminal side to the C-terminal of the domain sequence from the domain sequence" (in Fig. 5, shown as "region A"), t which is the total number of amino acid residues in all REPs excluding a sequence from the $(A)_n$ motif located closest to the C-terminal side to the C-terminal of the domain sequence from the domain sequence and further excluding $(A)_n$ motifs, is $50 + 40 + 10 + 20 + 30 = 150$. Next, s/t (%) can be calculated by dividing s by t and is $21/150 = 14.0\%$ in the case of the modified fibroin of Fig. 5.

In the sixth modified fibroin, a glutamine residue content rate is preferably 9% or less, more preferably 7% or less, still more preferably 4% or less, and particularly preferably 0%.

In the present specification, the "glutamine residue content rate" is a value calculated by the following method.

In a fibroin (a modified fibroin or a naturally occurring fibroin) including a domain sequence represented by Formula 1: $[(A)_n$ motif - $REP]_m$ or Formula 2: $[(A)_n$ motif - $REP]_m$ - $(A)_n$ motif, in a case where the total number of glutamine residues included in all REPs included in a sequence (sequence equivalent to "region A" in Fig. 5) excluding the sequence from the $(A)_n$ motif located at the most C-terminal side to the C-terminal of the domain sequence from the domain sequence is denoted by u, and the total number of amino acid residues in all REPs excluding the sequence from the $(A)_n$ motif located at the most the C-terminal side to the C-terminal of the domain sequence from the domain sequence and further excluding $(A)_n$ motifs is denoted by t, the glutamine residue content rate is calculated as u/t. For the calculation of the glutamine residue content rate, the "sequence excluding a sequence from the $(A)_n$ motif located closest to the C-terminal side to the C-terminal of the domain sequence from the domain sequence" is used for same reason described above.

The domain sequence of the sixth modified fibroin may include an amino acid sequence equivalent to an amino acid sequence in which one or a plurality of glutamine residues in the REP are deleted or substituted with other amino acid residues, as compared with a naturally occurring fibroin.

The "other amino acid residue" may be an amino acid residue other than a glutamine residue but is preferably an amino acid residue having a higher hydropathy index than that of a glutamine residue. The hydropathy indices of amino acid residues are as shown in Table 1.

As shown in Table 1, amino acid residues having a higher hydropathy index than a glutamine residue include an amino acid residue selected from isoleucine (I), valine (V), leucine (L), phenylalanine (F), cysteine (C), methionine (M), alanine (A), glycine (G), threonine (T), serine (S), tryptophan (W), tyrosine (Y), proline (P) and histidine (H). Among these, an amino acid residue selected from isoleucine (I), valine (V), leucine (L), phenylalanine (F), cysteine (C), methionine (M), and alanine (A) is more preferable, and an amino acid residue selected from isoleucine (I), valine (V), leucine (L), and phenylalanine (F) is still more preferable.

In the sixth modified fibroin, the hydrophobicity of REP is preferably -0.8 or more, more preferably -0.7 or more, still more preferably 0 or more, even still more preferably 0.3 or more, and particularly preferably 0.4 or more. The upper limit of the hydrophobicity of the REP is not particularly limited, may be 1.0 or less, and may be 0.7 or less.

In the present specification, the "hydrophobicity of REP" is a value calculated by the following method.

In a fibroin (a modified fibroin or a naturally occurring fibroin) including a domain sequence represented by Formula 1: $[(A)_n \text{ motif} - \text{REP}]_m$ or Formula 2: $[(A)_n \text{ motif} - \text{REP}]_m - (A)_n$ motif, in a case where the sum of the hydropathy indices of each amino acid residue included in all REPs included in a sequence (sequence equivalent to "region A" in Fig. 5) excluding the sequence from the $(A)_n$ motif located at the most C-terminal side to the C-terminal of the domain sequence from the domain sequence is denoted by v, and the total number of amino acid residues in all REPs excluding the sequence from the $(A)_n$ motif located at the most the C-terminal side to the C-terminal of the domain sequence from the domain sequence and further excluding $(A)_n$ motifs is denoted by t, the hydrophobicity of the REP is calculated as v/t. For the calculation of the hydrophobicity of the REP, the "sequence excluding a sequence from the $(A)_n$ motif located closest to the C-terminal side to the C-terminal of the domain sequence from the domain sequence" is used for the same reason described above.

The domain sequence of the sixth modified fibroin may further include an amino acid sequence equivalent to an amino acid sequence in which one or a plurality of amino acid residues are substituted, deleted, inserted and/or added, in addition to the modification of the amino acid sequence in which one or a plurality of glutamine residues in the REP are deleted and/or one or a plurality of glutamine residues in the REP are substituted with other amino acid residues, as compared with a naturally occurring fibroin.

The sixth modified fibroin can be obtained by, for example, with respect to a cloned gene sequence of a naturally occurring fibroin, deleting one or a plurality of glutamine residues in the REP and/or by substituting one or a plurality of glutamine residues in the REP with other amino acid residues. Further, for example, the modified fibroin may also be obtained by designing an amino acid sequence equivalent to an amino acid sequence in which with respect to the amino acid sequence of a naturally occurring fibroin, one or a plurality of glutamine residues in the REP are deleted and/or one or a plurality of glutamine residues in the REP are substituted with other amino acid residues, and chemically synthesizing a nucleic acid encoding the designed amino acid sequence.

A more specific example of the sixth modified fibroin can include a modified fibroin including (6-i) the amino acid sequence set forth in SEQ ID NO: 25 (Met-PRT888), SEQ ID NO: 26 (Met-PRT965), SEQ ID NO: 27 (Met-PRT889), SEQ ID NO: 28 (Met-PRT916), SEQ ID NO: 29 (Met-PRT918), SEQ ID NO: 30 (Met-PRT699), SEQ ID NO: 31 (Met-PRT698), SEQ ID NO: 32 (Met-PRT1009), or SEQ ID NO: 46 (Met-PRT966), or (6-ii) an amino acid sequence having 90% or more sequence identity with the amino acid sequence set forth in SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, or SEQ ID NO: 46.

The modified fibroin of (6-i) will be described. The amino acid sequence set forth in SEQ ID NO: 25 is obtained by substituting all QQs in the amino acid sequence set forth in SEQ ID NO: 7 (Met-PRT410) with VLs. The amino acid sequence set forth in SEQ ID NO: 26 is obtained by substituting all QQs in the amino acid sequence set forth in SEQ ID NO: 7 with TSs and substituting the remaining Qs with As. The amino acid sequence set forth in SEQ ID NO: 27 is obtained by substituting all QQs in the amino acid sequence set forth in SEQ ID NO: 7 with VLs and substituting the remaining Qs with Is. The amino acid sequence set forth in SEQ ID NO: 28 is obtained by substituting all QQs in the amino acid sequence set forth in SEQ ID NO: 7 with VIs and substituting the remaining Qs with Ls. The amino acid sequence set forth in SEQ ID NO: 29 is obtained by substituting all QQs in the amino acid sequence set forth in SEQ ID NO: 7 with VFs and substituting the remaining Qs with Is.

The amino acid sequence set forth in SEQ ID NO: 30 is obtained by substituting all QQs in the amino acid sequence set forth in SEQ ID NO: 8 (Met-PRT525) with VLs. The amino acid sequence set forth in SEQ ID NO: 31 is obtained by substituting all QQs in the amino acid sequence set forth in SEQ ID NO: 8 with VLs and substituting the remaining Qs with Is.

The amino acid sequence set forth in SEQ ID NO: 32 is an amino acid sequence obtained by repeating, four times, a region of 20 domain sequences (where several amino acid residues on the C-terminal side of the region are substituted) present in the amino acid sequence set forth in SEQ ID NO: 7 (Met-PRT410) and substituting all QQs in the repeated sequences with VFs and substituting the remaining Qs with Is.

The glutamine residue content rate of any of the amino acid sequences set forth in SEQ ID NO: 25, SEQ ID NO: 26, SEQ ID NO: 27, SEQ ID NO: 28, SEQ ID NO: 29, SEQ ID NO: 30, SEQ ID NO: 31, and SEQ ID NO: 32 is 9% or less (Table 2).

[Table 2]

| Modified fibroin | Glutamine residue content rate | GPGXX motif content rate | Hydrophobicity of REP |
|---|---|---|---|
| Met-PRT410 (SEQ ID NO: 7) | 17.7% | 27.9% | -1.52 |
| Met-PRT888 (SEQ ID NO: 25) | 6.3% | 27.9% | -0.07 |
| Met-PRT965 (SEQ ID NO: 26) | 0.0% | 27.9% | -0.65 |
| Met-PRT889 (SEQ ID NO: 27) | 0.0% | 27.9% | 0.35 |
| Met-PRT916 (SEQ ID NO: 28) | 0.0% | 27.9% | 0.47 |
| Met-PRT918 (SEQ ID NO: 29) | 0.0% | 27.9% | 0.45 |
| Met-PRT699 (SEQ ID NO: 30) | 3.6% | 26.4% | -0.78 |
| Met-PRT698 (SEQ ID NO: 31) | 0.0% | 26.4% | -0.03 |
| Met-PRT1009 (SEQ ID NO: 32) | 0.0% | 27.9% | 0.35 |

The modified fibroin of (6-i) may consist of the amino acid sequence set forth in SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, or SEQ ID NO: 46.

The modified fibroin of (6-ii) includes an amino acid sequence having 90% or more sequence identity with the amino acid sequence set forth in SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, or SEQ ID NO: 46. The modified fibroin of (6-ii) is also a protein including a domain sequence represented by Formula 1: $[(A)_n \text{ motif - REP}]_m$ or Formula 2: $[(A)_n \text{ motif - REP}]_m - (A)_n$ motif. The sequence identity is preferably 95% or more.

The modified fibroin of (6-ii) preferably has the glutamine residue content rate of 9% or less. In addition, the modified fibroin of (6-ii) preferably has the GPGXX motif content rate of 10% or more.

The sixth modified fibroin may include a tag sequence at one or both of the N-terminal and C-terminal. This makes it possible to isolate, immobilize, detect, and visualize the modified fibroin.

A more specific example of the modified fibroin having a tag sequence can include a modified fibroin including (6-iii) the amino acid sequence set forth in SEQ ID NO: 33 (PRT888), SEQ ID NO: 34 (PRT965), SEQ ID NO: 35 (PRT889), SEQ ID NO: 36 (PRT916), SEQ ID NO: 37 (PRT918), SEQ ID NO: 38 (PRT699), SEQ ID NO: 39 (PRT698), SEQ ID NO: 40 (PRT1009), or SEQ ID NO: 47 (PRT966), or (6-iv) an amino acid sequence having 90% or more sequence identity with the amino acid sequence set forth in SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, or SEQ ID NO: 47.

The amino acid sequences set forth in SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, and SEQ ID NO: 47 are respectively amino acid sequences obtained by adding the amino acid sequence (including a His tag sequence and a hinge sequence) set forth in SEQ ID NO: 11 to the N-terminal of the amino acid sequences set forth in SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, and SEQ ID NO: 46. Since only the tag sequence is added to the N-terminal, the glutamine residue content rate are not changed, and any of the amino acid sequences set forth in SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, and SEQ ID NO: 47 has the glutamine residue content rate of 9% or less (Table 3).

[Table 3]

| Modified fibroin | Glutamine residue content rate | GPGXX motif content rate | Hydrophobicity of REP |
|---|---|---|---|
| PRT888 (SEQ ID NO: 33) | 6.3% | 27.9% | -0.07 |
| PRT965 (SEQ ID NO: 34) | 0.0% | 27.9% | -0.65 |
| PRT889 (SEQ ID NO: 35) | 0.0% | 27.9% | 0.35 |

(continued)

| Modified fibroin | Glutamine residue content rate | GPGXX motif content rate | Hydrophobicity of REP |
|---|---|---|---|
| PRT916 (SEQ ID NO: 36) | 0.0% | 27.9% | 0.47 |
| PRT918 (SEQ ID NO: 37) | 0.0% | 27.9% | 0.45 |
| PRT699 (SEQ ID NO: 38) | 3.6% | 26.4% | -0.78 |
| PRT698 (SEQ ID NO: 39) | 0.0% | 26.4% | -0.03 |
| PRT1009 (SEQ ID NO: 40) | 0.0% | 27.9% | 0.35 |
| PRT966 (SEQ ID NO: 47) | 0.0% | 28.0% | 0.35 |

The modified fibroin of (6-iii) may consist of the amino acid sequence set forth in SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, or SEQ ID NO: 47.

The modified fibroin of (6-iv) includes an amino acid sequence having 90% or more sequence identity with the amino acid sequence set forth in SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, or SEQ ID NO: 47. The modified fibroin of (6-iv) is also a protein including a domain sequence represented by Formula 1: $[(A)_n$ motif - $REP]_m$ or Formula 2: $[(A)_n$ motif - $REP]_m$ - $(A)_n$ motif. The sequence identity is preferably 95% or more.

The modified fibroin of (6-iv) preferably has the glutamine residue content rate of 9% or less. In addition, the modified fibroin of (6-iv) preferably has the GPGXX motif content rate of 10% or more.

The sixth modified fibroin may include a secretory signal for releasing the protein produced in the recombinant protein production system to the outside of a host. The sequence of the secretory signal can be appropriately set depending on the type of the host.

The limiting oxygen index (LOI) value of the modified (artificial) fibroin fiber may be 18 or more, 20 or more, 22 or more, 24 or more, 26 or more, 28 or more, 29 or more, and 30 or more. The LOI value described above is the value measured in accordance with the "testing method for powdery or low melting point synthetic resin" described in "Fire and Disaster Management Agency, Dangerous Materials Regulation Section Manager, Fire and Disaster No. 50 (on May 31th, 1995)".

The highest hygroscopic heat generation degree of the modified (artificial) fibroin fiber, which is determined according to Expression A described later, may be more than 0.025 °C/g, 0.026 °C/g or more, 0.027 °C/g, 0.028 °C/g or more, 0.029 °C/g or more, 0.030 °C/g or more, 0.035 °C/g or more, and 0.040 °C/g more. The upper limit of the highest hygroscopic heat generation degree is not particularly limited, but it is usually 0.060 °C/g or less.

Expression A: highest hygroscopic heat generation degree = {(highest temperature of a sample when the sample has been transferred to a high humidity environment after being placed in a low humidity environment until a temperature of the sample reaches equilibrium) - (temperature of the sample when the sample is being transferred to the high humidity environment after being placed in the low humidity environment until the temperature of the sample reaches equilibrium)} (°C)/sample weight (g)

[In Expression A, the low humidity environment means an environment of a temperature of 20°C and a relative humidity of 40%, and the high humidity environment means an environment of a temperature of 20°C and a relative humidity of 90%.]

The heat retaining property index of the modified fibroin fiber may be 0.22 or more, 0.24 or more, 0.26 or more, 0.28 or more, 0.30 or more, and 0.32 or more. The upper limit of the heat retaining property index is not particularly limited, but, for example, it may be 0.60 or less or 0.40 or less.

The modified fibroin fiber preferably has excellent heat retaining property, and the heat retaining property index determined according to Formula C may be 0.20 or more.

$$\text{Formula C: Heat retaining property index} = \text{heat retention rate}$$

$$(\%)/\text{weight of sample } (g/m^2).$$

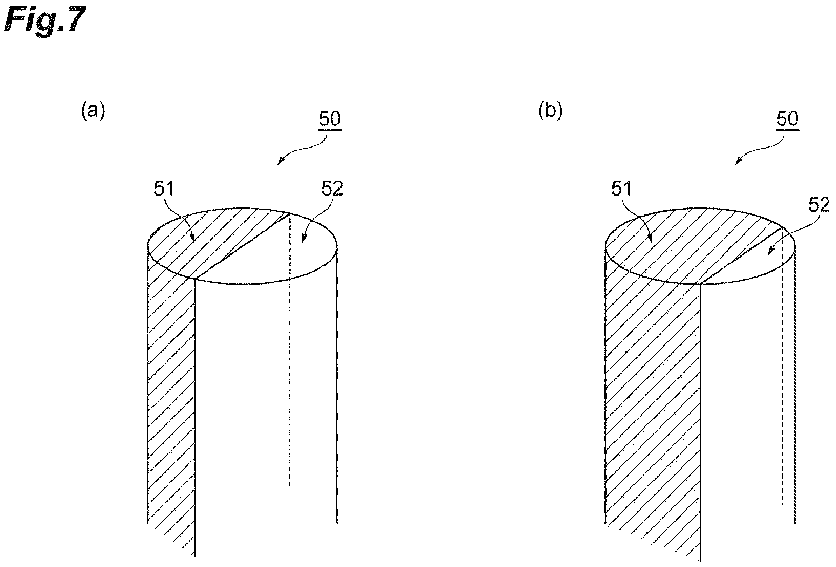

One example of a composite fiber according to one embodiment of the present invention is shown in Fig. 7. Fig. 7(a) is a schematic diagram showing a composite fiber 50 having a first component 51 and a second component 52. The first component 51 contains a modified fibroin having water shrinkability and can be shrunk by being brought into contact with an aqueous medium. On the other hand, the second component 52 has reduced water shrinkability, and the shrinkage rate due to being brought into contact with water is lower than that of the first component. Fig. 7(b) is a schematic diagram showing a composite fiber in which the configuration ratio of the first component to the second component is changed.

By appropriately changing the combination of fibers used for the first component and the second component, or changing the configuration ratio of the first component to the second component, it is possible to prepare a composite fiber having a desired crimping ability.

Due to the difference in water shrinkability between the first component and the second component, in a case where the composite fiber according to the present embodiment is brought into contact with an aqueous medium, the first component shrinks much more than the second component, and thus the crimping processing and spinning can be carried out easily. That is, the composite fiber according to the present embodiment has an excellent crimping ability and is useful as a crimped yarn or a spun yarn. The crimped yarn is excellent in spinnability, bulkiness, elasticity, flexibility, and resiliency, and can impart good feeling, soft texture, and a moisture retaining property.

The hydrophobicity of the modified fibroin contained in the first component and the hydrophobicity of the structural protein contained in the second component is different from each other. The hydrophobicity of the modified fibroin or the structural protein is a value obtained by calculating the sum of the each HI of the amino acid residues (where, the amino acid residues corresponding to the tag sequence and the hinge sequence are excluded) constituting the modified fibroin or the structural protein, and then dividing the sum of the HIs by the number of amino acid residues. The hydrophobicity of the modified fibroin contained in the first component is, for example, preferably -0.8 or less and more preferably -0.55 or less. The hydrophobicity of the structural protein contained in the second component is, for example, preferably more than -0.8 and more preferably more than -0.55.

In a case where the first component contains a plurality of modified fibroins, the hydrophobicity of each component may be obtained as an average value by calculating the hydrophobicity of each modified fibroin contained in the first component and averaging the values based on the ratio of each component. For example, a value obtained by summing the numerical values obtained by multiplying the hydrophobicity of each modified fibroin by the content rate of the modified fibroin in the first component and dividing by the number of modified fibroins may be used.

In a case where the second component contains a plurality of structural proteins, the hydrophobicity of each component may be obtained as an average value by calculating the hydrophobicity of each structural protein contained in the second component and averaging the values based on the ratio of each component. For example, a value obtained by summing the numerical values obtained by multiplying the hydrophobicity of each structural protein by the content rate of the structural protein in the second component and dividing by the number of structural proteins may be used.

Further, as described above, in a case where the first component contains a plurality of modified fibroins or in a case where the second component contains a plurality of structural proteins, the hydrophobicity of each component may be calculated without considering a modified fibroin or a structural protein contained in a low content rate (for example, a content rate of 10% or less) since the modified fibroin or the structural protein contained in a low content rate has a sufficiently small contribution to the entire hydrophobicity of the modified fibroins or the structural proteins. For example, silk yarn is composed of about 75% of silk fibroin and about 25% of sericin (UniProt database, Entry No. P07856). Silk fibroin is composed of a fibroin H chain (UniProt database, Entry No. P05790), a fibroin L chain (UniProt database, Entry No. P21828), and fibrohexamelin (UniProt database, Entry No. P04148), and the fibroin H chain quantitatively occupies most of the silk fibroin. In a case where silk fibroin of an indoor silkworm from which sericin has been removed is used as a natural structural protein, the hydrophobicity of the silk fibroin is obtained by calculating the sum of each HI of each amino acid residue of the fibroin H chain, which is the main component, and dividing the sum by the number of amino acid residues. The hydrophobicity obtained in this manner may be 0.216.

The hydrophobicity of each of the amino acid sequences set forth in SEQ ID NO: 13, SEQ ID NO: 14, SEQ ID NO: 15, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, and SEQ ID NO: 47 is shown in Table 4. In calculating the hydrophobicity of each amino acid sequence, the calculation was performed by excluding the sequence irrelevant to the modified fibroin (that is, the sequence equivalent to the amino acid sequence represented by SEQ ID NO: 11).

[Table 4]

| Amino acid sequence | Hydrophobicity |
|---|---|
| Amino acid sequence set forth in SEQ ID NO:13 | -0.80 |
| Amino acid sequence set forth in SEQ ID NO: 14 | -0.56 |
| Amino acid sequence set forth in SEQ ID NO: 15 | -0.80 |
| Amino acid sequence set forth in SEQ ID NO:33 | 0.07 |
| Amino acid sequence set forth in SEQ ID NO:34 | -0.16 |
| Amino acid sequence set forth in SEQ ID NO:35 | 0.55 |
| Amino acid sequence set forth in SEQ ID NO:36 | 0.54 |
| Amino acid sequence set forth in SEQ ID NO:37 | 0.49 |
| Amino acid sequence set forth in SEQ ID NO:38 | 0.21 |
| Amino acid sequence set forth in SEQ ID NO:39 | 0.48 |
| Amino acid sequence set forth in SEQ ID NO:40 | 0.49 |
| Amino acid sequence set forth in SEQ ID NO:47 | 0.49 |

The hydrophobicity of each of the amino acid sequences represented by SEQ ID NO: 42, SEQ ID NO: 43, SEQ ID NO: 44, and SEQ ID NO: 45 is shown in Table 5. In calculating the hydrophobicity of each amino acid sequence, the calculation was performed by excluding the sequence irrelevant to the structural protein (that is, the sequence equivalent to the amino acid sequence represented by SEQ ID NO: 11).

[Table 5]

| Amino acid sequence | Hydrophobicity |
|---|---|
| Amino acid sequence set forth in SEQ ID NO:42 | -0.74 |
| Amino acid sequence set forth in SEQ ID NO:43 | -1.20 |
| Amino acid sequence set forth in SEQ ID NO:44 | 0.47 |
| Amino acid sequence set forth in SEQ ID NO:45 | -0.53 |

(Method for producing modified fibroin)

A modified fibroin can be produced, for example, by expressing a nucleic acid in a host transformed with an expression vector having a nucleic acid sequence encoding the modified fibroin and one or a plurality of regulatory sequences operably linked to the nucleic acid sequence.

The method for producing a nucleic acid encoding a modified fibroin is not particularly limited. For example, the nucleic acid is produced by cloning a gene encoding the natural fibroin by amplification with polymerase chain reaction (PCR) or the like and modifying the gene by a genetic engineering method, by chemically synthesizing the nucleic acid. The method for chemically synthesizing a nucleic acid is not particularly limited, and for example, the gene can be chemically synthesized by a method in which oligonucleotides are automatically synthesized by AKTA oligopilot plus 10/100 (GE Healthcare Japan Corporation) or the like and are linked by PCR or the like, based on the amino acid sequence information of the fibroin obtained from the NCBI web database or the like. In this case, in order to facilitate purification and/or confirmation of the modified fibroin, a nucleic acid may be synthesized such that a modified fibroin having an amino acid sequence obtained by adding an amino acid sequence consisting of a start codon and a His10 tag to the N-terminal of the above amino acid sequence is encoded.

The regulatory sequence is a sequence (for example, a promoter, an enhancer, a ribosome binding sequence, or a transcription termination sequence) that controls the expression of a modified fibroin in a host, and can be appropriately selected depending on the type of the host. As a promoter, an inducible promoter that functions in a host cell and is capable of inducing the expression of a modified fibroin may be used. An inducible promoter is a promoter that can control transcription by the presence of an inducer (an expression inducer), the absence of a repressor molecule, or physical factors such as an increase or decrease in temperature, osmotic pressure, or pH value.

The type of the expression vector such as a plasmid vector, a viral vector, a cosmid vector, a fosmid vector, or an artificial chromosome vector can be appropriately selected depending on the type of the host. As the expression vector, an expression vector that can autonomously replicate in a host cell or can be incorporated into a chromosome of a host and which contains a promoter at a position capable of transcribing the nucleic acid that encodes a modified fibroin is suitably used.

Both prokaryotes and eukaryotes such as yeast, filamentous fungi, insect cells, animal cells, and plant cells can be suitably used as a host.

Preferred examples of the prokaryotic host cells include bacteria belonging to the genus Escherichia, the genus Brevibacillus, the genus Serratia, the genus Bacillus, the genus Microbacterium, the genus Brevibacterium, the genus Corynebacterium, and the genus Pseudomonas. Examples of microorganisms belonging to the genus Escherichia include Escherichia coli. Examples of the microorganisms belonging to the genus Brevibacillus include Brevibacillus agri. Examples of microorganisms belonging to the genus Serratia include Serratia liquefaciens. Examples of microorganisms belonging to the genus Bacillus include Bacillus subtilis. Examples of microorganisms belonging to the genus Microbacterium include Microbacterium ammoniaphilum. Examples of microorganisms belonging to the genus Brevibacterium include Brevibacterium divaricatum. Examples of microorganisms belonging to the genus Corynebacterium include Corynebacterium ammoniagenes. Examples of microorganisms belonging to the genus Pseudomonas include Pseudomonas putida.

In a case where a prokaryote is used as a host, examples of a vector into which a nucleic acid encoding a modified fibroin is introduced include pBTrp2 (manufactured by Boehringer Mannheim), pGEX (manufactured by Pharmacia), pUC18, pBluescriptII, pSupex, pET22b, pCold, pUB110, and pNCO2. (Japanese Unexamined Patent Publication No. 2002-238569).

Examples of eukaryotic hosts include yeast and filamentous fungi (mold and the like). Examples of yeasts include yeasts belonging to the genus Saccharomyces, the genus Pichia, and the genus Schizosaccharomyces. Examples of filamentous fungi include filamentous fungi belonging to the genus Aspergillus, the genus Penicillium, and the genus Trichoderma.

In a case where a eukaryote is used as a host, examples of the vector into which a nucleic acid encoding a modified fibroin is introduced include YEp13 (ATCC37115) and YEp24 (ATCC37051). As a method for introducing an expression vector into the above host cell, any method can be used as long as the method introduces DNA into the host cell. Examples thereof include a method using calcium ions [Proc. Natl. Acad. Sci. USA, 69, 2110 (1972)], electroporation method, spheroplast method, protoplast method, lithium acetate method, and competent method.

As for the method for expressing a nucleic acid using a host transformed with an expression vector, secretory production, fusion protein expression, or the like, in addition to the direct expression, can be carried out according to the method described in Molecular Cloning, 2nd edition.

The modified fibroin can be produced, for example, by culturing a host transformed with the expression vector in a culture medium, producing and accumulating the modified fibroin in the culture medium, and then collecting the modified fibroin from the culture medium. The method for culturing a host in a culture medium can be carried out according to a method commonly used for culturing a host.

In the case where the host is a prokaryote such as Escherichia coli or a eukaryote such as yeast, any of a natural medium and a synthetic medium may be used as a culture medium of the host as long as the medium contains a carbon source, a nitrogen source, inorganic salts and the like which can be utilized by the host and the medium can be used for efficiently culturing the host.

As the carbon source, any carbon source that can be utilized by the transformed microorganism may be used. Examples of the carbon source that can be utilized include glucose, fructose, sucrose, and molasses containing them, carbohydrates such as starch and a hydrolyzate thereof, organic acids such as acetic acid and propionic acid, and alcohols such as ethanol and propanol. Examples of the nitrogen source that can be utilized include ammonium salts of inorganic or organic acids such as ammonia, ammonium chloride, ammonium sulfate, ammonium acetate, and ammonium phosphate, other nitrogen-containing compounds, peptone, meat extract, yeast extract, corn steep liquor, casein hydrolyzate, soybean cake and soybean cake hydrolyzate, and various fermented microbial cells and digested products thereof. Examples of the inorganic salt that can be utilized include potassium dihydrogen phosphate, dipotassium phosphate, magnesium phosphate, magnesium sulfate, sodium chloride, ferrous sulfate, manganese sulfate, copper sulfate, and calcium carbonate.

Culture of a prokaryote such as Escherichia coli or a eukaryote such as yeast can be carried out under aerobic conditions such as shaking culture or deep aeration stirring culture. The culture temperature is, for example, 15°C to 40°C. The culture time is usually 16 hours to 7 days. It is preferable to maintain the pH of the culture medium during the culture at 3.0 to 9.0. The pH of the culture medium can be adjusted using an inorganic acid, an organic acid, an alkali solution, urea, calcium carbonate, ammonia, or the like.

In addition, antibiotics such as ampicillin and tetracycline may be added to the culture medium as necessary during the culture. In a case of culturing a microorganism transformed with an expression vector using an inducible promoter as a promoter, an inducer may be added to the medium as necessary. For example, in a case of culturing a microorganism transformed with an expression vector using a lac promoter, isopropyl-$\beta$-D-thiogalactopyranoside or the like is used, and in a case of culturing a microorganism transformed with an expression vector using a trp promoter, indole acrylic acid or the like may be added to the medium.

The expressed modified fibroin can be isolated and purified by a commonly used method. For example, in a case where the modified fibroin is expressed in a dissolved state in cells, the host cells are recovered by centrifugation after the completion of the culture, suspended in an aqueous buffer solution, and then disrupted using an ultrasonicator, a French press, a Manton-Gaulin homogenizer, a Dyno-Mill, or the like to obtain a cell-free extract. From the supernatant obtained by centrifuging the cell-free extract, a purified preparation can be obtained by a method commonly used for isolation and purification of the modified fibroin, that is, a solvent extraction method, a salting-out method using ammonium sulfate or the like, a desalting method, a precipitation method using an organic solvent, an anion exchange chromatography method using a resin such as diethylaminoethyl (DEAE)-Sepharose or DIAION HPA-75 (manufactured by Mitsubishi Kasei Kogyo Kabushiki Kaisha), an cation exchange chromatography method using a resin such as S-Sepharose FF (manufacture by Pharmacia Corporation), a hydrophobic chromatography method using a resin such as butyl sepharose or phenyl sepharose, a gel filtration method using a molecular sieve, an affinity chromatography method, a chromatofocusing method, or an electrophoresis method such as isoelectric focusing or the like, using the above methods singly or in combination thereof.

In the case where the modified fibroin is expressed by the formation of an insoluble body in the cell, similarly, the host cells are recovered, disrupted and centrifuged to recover the insoluble body of the modified fibroin as a precipitated fraction. The recovered insoluble body of the modified fibroin can be solubilized with a protein denaturing agent. After this operation, a purified preparation of modified fibroin can be obtained by the same isolation and purification method as described above. In a case where the modified fibroin is secreted extracellularly, the modified fibroin can be recovered from the culture supernatant. That is, a culture supernatant is obtained by treating the culture by a technique such as centrifugation, and a purified preparation can be obtained from the culture supernatant by using the same isolation and purification method as described above.

<Doping liquid>

A first doping liquid contains a modified fibroin and a solvent. The second doping liquid contains a structural protein and a solvent.

The concentration of the modified fibroin in the first doping liquid is not particularly limited and may be appropriately set depending on factors such as the desired crimping ability and fiber diameter of the composite fiber, and the combination with the structural protein contained in the second component. For example, the concentration of the modified fibroin is preferably 5% to 40% by mass, based on the total mass of the first doping liquid (in a case where the total mass of the first doping liquid is set to 100% by mass), more preferably 7% to 40% by mass, more preferably 10% to 40%, more preferably 7% to 35% by mass, more preferably 10% to 35% by mass, more preferably 12% to 35% by mass, more preferably 15% to 35% by mass, still more preferably 15% to 30% by mass, even still more preferably 20% to 35% by mass, and even still further preferably 20% to 30% by mass. In a case where the concentration of the modified fibroin is 5% by mass or more, the productivity of the composite fiber tends to be further improved. In a case where the concentration of the modified fibroin is 40% by mass or less, the doping liquid can be more stably discharged from the spinneret, and thus the productivity tends to be further improved.

The concentration of the structural protein in the second doping liquid is not particularly limited and may be appropriately set depending on factors such as the desired crimping ability and fiber diameter of the composite fiber, and the combination with the structural protein contained in the first component. For example, the concentration of the modified fibroin is preferably 5% to 40% by mass, based on the total mass of the second doping liquid (in a case where the total mass of the second doping liquid is set to 100% by mass), more preferably 7% to 40% by mass, more preferably 10% to 40%, more preferably 7% to 35% by mass, more preferably 10% to 35% by mass, more preferably 12% to 35% by mass, more preferably 15% to 35% by mass, still more preferably 15% to 30% by mass, even still more preferably 20% to 35% by mass, and even still further preferably 20% to 30% by mass. In a case where the concentration of the modified fibroin is 5% by mass or more, the productivity of the composite fiber tends to be further improved. In a case where the concentration of the modified fibroin is 40% by mass or less, the doping liquid can be more stably discharged from the spinneret, and thus the productivity tends to be further improved.

The solvent of the first doping liquid may be any solvent that can dissolve a modified fibroin. In addition, the solvent of the second doping liquid may be any solvent that can dissolve a structural protein. Examples of such a solvent include hexafluoroisopropanol (HFIP), hexafluoroacetone (HFA), dimethyl sulfoxide (DMSO), N,N-dimethylformamide (DMF), N,N-dimethylacetamide (DMA), 1,3-dimethyl-2-imidazolidone (DMI), N-methyl-2-pyrrolidone (NMP), acetonitrile, N-methylmorpholine-N-oxide (NMO), and formic acid. In addition, the solvent may be an aqueous solution, and specifically, an aqueous solution containing at least one selected from the group consisting of urea, guanidine, sodium dodecyl sulfate (SDS), lithium bromide, calcium chloride, and lithium thiocyanate can be mentioned. These solvents may be used alone or in a combination of two or more thereof.

The doping liquid may be prepared by a method known to those skilled in the art, and the solvent may be mixed with a modified fibroin or a structural protein in random order.

As necessary, an inorganic salt may be added to the first doping liquid and the second doping liquid. An inorganic salt may function as a dissolution accelerator of a modified fibroin. Examples of the inorganic salt include an alkali metal halide, an alkaline earth metal halide, and, an alkaline earth metal nitrate. Specific examples of the inorganic salt include lithium carbonate, lithium chloride, calcium chloride, calcium nitrate, lithium bromide, barium bromide, calcium bromide, barium chlorate, sodium perchlorate, lithium perchlorate, barium perchlorate, calcium perchlorate, and magnesium perchlorate.

The viscosity of the doping liquid can be appropriately adjusted depending on the application of the composite fiber and the spinning method. The viscosity of the doping liquid may be at 20°C, for example, 5,000 to 40,000 mPa·sec, 7,000 to 40,000 mPa·sec, 10,000 to 40,000 mPa·sec, 7,000 to 35,000 mPa·sec, 10,000 to 35,000 mPa·sec, 10,000 to 30,000 mPa·sec, or 10,000 to 25,000 mPa·sec. The viscosity of the doping liquid can be measured using, for example, an "EMS viscometer" (trade name) manufactured by Kyoto Electronics Manufacturing Co., Ltd.

The doping liquid may be stirred or shaken for some time in order to promote dissolution. At that time, heating may be performed to a temperature at which a modified fibroin or a structural protein is dissolved in the solvent. The doping liquid may be heated to, for example, 30°C or higher, 40°C or higher, 50°C or higher, 60°C or higher, 70°C or higher, 80°C or higher, or 90°C or higher. The upper limit of the heating temperature is, for example, the boiling point of the solvent or lower.

<Spinning>

The composite fiber can be manufactured by a known spinning method. For example, the composite fiber can be obtained by using the first doping liquid and the second doping liquid by spinning using a known spinning method such as dry type spinning, melt spinning, wet type spinning, and dry-wet type spinning. As a preferred spinning method, wet type spinning and dry-wet type spinning can be mentioned.

In wet type spinning or dry-wet type spinning, the first doping liquid and the second doping liquid are discharged from a spinneret (nozzle) and joined to each other, and the first component (modified fibroin) and the second component (structural protein) are solidified in a coagulation liquid, whereby a composite fiber is obtained in the state of undrawn yarn.

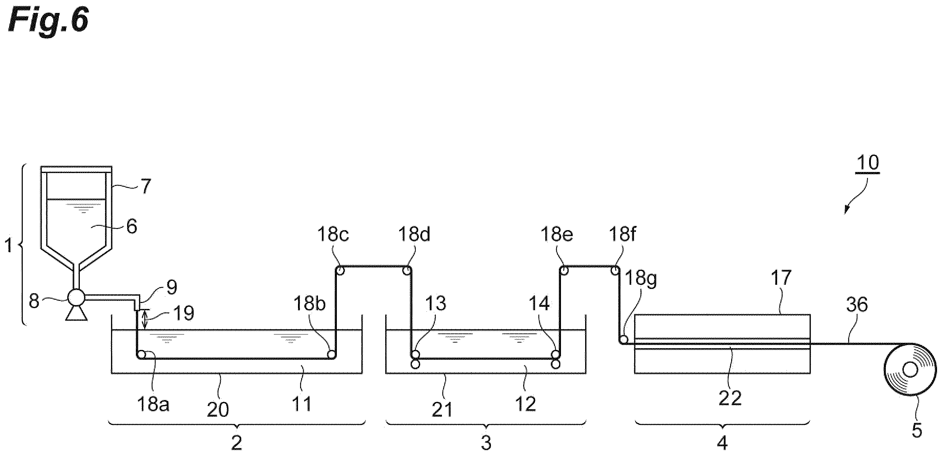

Fig. 6 is an illustrative view schematically showing an example of a spinning device for manufacturing a composite fiber. A spinning device 10 shown in Fig. 6 is an example of a spinning device for dry-wet type spinning and includes an extrusion device 1, an undrawn yarn manufacturing device 2, a wet heat drawing device 3, and a drying device 4.

A spinning method using the spinning device 10 will be described. First, a doping liquid 6 stored in a storage tank 7 is extruded out from a mouthpiece 9 by a gear pump 8. In the laboratory scale, the doping liquid may be filled in a cylinder and extruded from a nozzle using a syringe pump. Next, the extruded doping liquid 6 is supplied into a coagulation liquid 11 in a coagulation liquid bath 20 via an air gap 19, the solvent is removed, a protein is coagulated, and a fibrous coagulate is formed. Then, the fibrous coagulate is supplied into a warm water 12 in a drawing bath 21 and is drawn. A drawing rate is determined according to a speed ratio of a supply nip roller 13 to a withdrawing nip roller 14. Thereafter, the drawn fibrous coagulate is supplied to a drying device 4 and dried in a yarn path 22, and a composite fiber 36 is obtained as a wound yarn body 5. Reference signs 18a to 18g indicate yarn guides.

As the spinneret (nozzle) for manufacturing a composite fiber, for example, a nozzle for manufacturing a composite fiber having a side-by-side structure is known. In the nozzle for manufacturing a composite fiber having a side-by-side structure, a first mouthpiece for extruding the first doping liquid (corresponding to the first component of the composite fiber) is disposed in a central portion of the nozzle, and a second mouthpiece for extruding the second doping liquid (corresponding to the second component of the composite fiber) is disposed in the vicinity of the first mouthpiece. The second mouthpiece may be tilted toward the first mouthpiece such that the second doping liquid is combined with the flux of the first doping liquid extruded from the first mouthpiece. Further, it is preferable that the flux of the first mouthpiece and the flux of the second mouthpiece are parallel. The second mouthpiece may be substantially in contact with the first mouthpiece. Further, the nozzle may be one in which the first mouthpiece and the second mouthpiece are integrated and the discharge port of the first doping liquid and the discharge port of the second doping liquid are separated by a partition wall. It is preferable that each mouthpiece is designed such that the discharge is maintained at a constant amount by controlling the size and the temperature of the mouthpiece. The second doping liquid extruded from the second mouthpiece is combined with the first doping liquid extruded from the first mouthpiece to be integrated with each other, and the integrated liquids come into contact with the coagulation liquid to form an undrawn yarn having a side-by-side structure.

The position of each mouthpiece can be appropriately adjusted depending on the spinning conditions such as the type of the fiber raw material used, the viscosity of each doping liquid, the extrusion rate, the temperature, and the like.

The coagulation liquid 11 may be any liquid that can be desolvated, and examples thereof include lower alcohols having 1 to 5 carbon atoms such as methanol, ethanol, and 2-propanol, and acetone. The coagulation liquid 11 may appropriately contain water. The temperature of the coagulation liquid 11 is preferably 0°C to 30°C. The distance that the coagulated protein passes through the coagulation liquid 11 (substantially, the distance from the yarn guide 18a to the yarn guide 18b) may be a length that allows efficient desolvation, for example, 200 to 500 mm. The retention time in the coagulation liquid 11 may be any time as long as the doping solvent is removed from the undrawn yarn. In addition, drawing (pre-drawing) may be performed in the coagulation liquid 11. The coagulation liquid bath 20 may be provided in multiple stages, and the drawing may be performed in each stage or in a specific stage as necessary. In order to suppress the evaporation of the lower alcohol, the coagulation liquid may be kept at a low temperature, and yarn may be withdrawn in an undrawn state. In addition, the undrawn yarn may be drawn in the coagulation liquid (pre-drawing) .

The undrawn yarn (or pre-drawn yarn) obtained by the above method may be in the state of the drawn yarn (composite fiber) by the drawing process. As the drawing method, wet heat drawing, dry heat drawing, and the like can be mentioned.

The wet heat drawing can be performed in warm water, in a solution obtained by adding an organic solvent or the like to warm water, or in heated steam. The temperature may be, for example, may be 40°C to 200°C, 50°C to 180°C, 50°C to 150°C, or 75°C to 90°C. The drawing rate in wet heat drawing may be, for example, 1 to 30 times, 2 to 25 times, 2 to 20 times, 2 to 15 times, 2 to 10 times, 2 to 8 times, 2 to 6 times, or 2 to 4 times, with respect to the undrawn yarn (or pre-drawn yarn). However, the drawing rate is not limited as long as the desired fiber thickness and the desired mechanical properties and other characteristics can be obtained.

The dry heat drawing can be performed using a device such as a contact type hot plate and a non-contact type furnace but is not particularly limited, and any device capable of heating the fiber to the predetermined temperature and capable of drawing at the predetermined rate may be used. The temperature may be, for example, may be 100°C to 270°C, 140°C to 230°C, 140°C to 200°C, 160°C to 200°C, or 160°C to 180°C.

The drawing rate in the dry heat drawing process may be, for example, 1 to 30 times, 2 to 30 times, 2 to 20 times, and 3 to 15 times, and is preferably 3 to 10 times, more preferably 3 to 8 times, and still more preferably 4 to 8 times, with respect to the undrawn yarn (or pre-drawn yarn). However, the drawing rate is not limited as long as the desired fiber thickness and the desired mechanical properties and other characteristics can be obtained.

In the drawing process, wet heat drawing and dry heat drawing may be carried out individually or may be carried out in multistage or in combination. That is, as the drawing process, wet heat drawing is performed as the first stage drawing and dry heat drawing is performed as the second stage drawing, or wet heat drawing is performed as the first stage drawing and wet heat drawing is performed as the second stage drawing, and then dry heat drawing may be further performed as the third stage drawing. As the drawing process, wet heat drawing and dry heat drawing may be performed appropriately combining them.

The lower limit of the final drawing rate of the composite fiber that has undergone the drawing process may be preferably 1 time, 2 times, 3 times, 4 times, 5 times, 6 times, 7 times, 8 times, and 9 times, with respect to the undrawn yarn (or pre-drawn yarn). The upper limit of the final drawing rate of the composite fiber that has undergone the drawing process may be preferably 40 times, 30 times, 20 times, 15 times, 14 times, 13 times, 12 times, 11 times, and 10 times. In addition, the final drawing rate may be, for example, 3 to 40 times, 3 to 30 times, 5 to 30 times, 5 to 20 times, 5 to 15 times, or 5 to 13 times.

In the spinning process, the mouthpiece shape, the hole shape, the number of holes, and the like of the spinneret are not particularly limited and can be appropriately selected depending on the desired fiber diameter and the number of single yarns.

Before or after the drying, an oil agent may be applied to the undrawn yarn (or pre-drawn yarn) or the drawn yarn for the purpose of imparting an antistatic property, a bundling property, lubricity and the like, as necessary. The type and amount of the oil agent to be applied are not particularly limited and can be appropriately adjusted in consideration of the application of the composite fiber, the handleability of the mating fiber, and the like.

In a case where the hole shape of the spinneret is circular, the hole diameter is, for example, 0.1 mm to 0.6 mm. In a case where the hole diameter is 0.1 mm or more, pressure loss can be reduced and equipment cost can be saved. In a case where the hole diameter is 0.6 mm or less, the drawing operation for reducing the fiber diameter can be omitted, and the possibility of rupture (drawing break) between discharging and withdrawing can be further reduced.

The temperature in a case of passing through the spinneret and the temperature of the spinneret are not particularly limited and may be appropriately adjusted depending on the concentration and viscosity of the doping liquid used, the type of solvent, and the like. The temperature of the spinneret is preferably 30°C to 100°C from the viewpoint of preventing the deterioration of the modified fibroin and structural proteins, and the like. In addition, the upper limit of the temperature is preferably equal to or lower than the boiling point of the solvent used, from the viewpoint of further reducing the pressure increase due to the volatilization of the solvent and the possibility of clogging inside the pipe due to the solidification of the doping liquid. This improves process stability.

The method according to the present embodiment may further include a process (filtering process) of filtering the doping liquid before discharging the doping liquid, and/or a process (defoaming process) of defoaming the doping liquid before discharging.

The crimping process is a process of crimping (hereinafter, may be referred to as "water crimping") by bringing the composite fiber having a latent crimping ability into contact with an aqueous medium.

By bringing into contact with an aqueous medium, the composite modified fibroin fiber can be crimped without depending on the external force. The aqueous medium is a medium of a liquid or gas (steam) containing water (including steam). The aqueous medium may be water or a mixed liquid of water and a hydrophilic solvent. As the hydrophilic solvent, for example, a volatile solvent such as ethanol and methanol, or a vapor thereof can be mentioned. The aqueous medium may be a mixed liquid of water and a volatile solvent such as ethanol or methanol and is preferably water or a mixed liquid of water and ethanol. By using an aqueous medium containing a volatile solvent or a vapor thereof, it is possible to improve the drying speed after the water crimping, and furthermore impart a soft texture to a crimped staple or a crimped fiber finally obtained.

The ratio of water to the volatile solvent or the vapor thereof is not particularly limited, and, for example, water:volatile solvent may be 10:90 to 90:10 by mass ratio. The content of water is preferably 30% by mass or more and may be 40% by mass or 50% by mass or more, based on the total mass of the aqueous medium. In a case where the aqueous medium is a liquid, it is preferable to disperse an oil agent in the aqueous medium. In this case, the water crimping and the oil agent adhering can be performed at the same time. As the oil agent, any oil agent can be used as long it is a known oil agent used for general purposes including process passability and function impartability, such as an antistatic property, a friction reduction property, a flexibility imparting property, and a water repellency imparting property. The amount of the oil agent is not particularly limited and may be, for example, 1% to 10% by mass or 2% to 5% by mass with respect to the total mass of the oil agent and the aqueous medium.

The aqueous medium is preferably a liquid or gas containing water (including steam) and having a temperature of 10°C to 230°C. The temperature of the aqueous medium may be 10°C or higher, 25°C or higher, 40°C or higher, 60°C or higher, or 100°C or higher, and may be 230°C or lower, 120°C or lower, or 100°C or lower. More specifically, in a case where the aqueous medium is a gas (steam), the temperature of the aqueous medium is preferably 100 to 230°C, more preferably 100 to 120°C. In a case where the steam of the aqueous medium is 230°C or less, the heat denaturation of the composite fiber can be prevented. In a case where the aqueous medium is a liquid, the temperature of the aqueous medium is preferably 10°C or higher, 25°C or higher, or 40°C or higher from the viewpoint of efficiently imparting crimpness, and is preferably 60°C or lower from the viewpoint of highly maintaining fiber strength of the composite fiber.

The time of bringing into contact with the aqueous medium is not particularly limited, but may be 30 seconds or longer, 1 minute or longer, or 2 minutes or longer, and preferably 10 minutes or shorter from the viewpoint of productivity. In the case of steam, it is considered that a high shrinkage rate can be obtained in a short time in comparison with a liquid. The contact with the aqueous medium may be carried out under normal pressure or under reduced pressure (for example, vacuum).

As a method for bringing into contact with an aqueous medium, a method for immersing the composite fiber in an aqueous medium, a method for spraying the steam of an aqueous medium to the composite fiber, a method for exposing the composite fiber to an environment filled with the steam of an aqueous medium, and the like can be mentioned. In a case where the aqueous medium is steam, the composite fiber can be brought into contact with the aqueous medium by using a general steam setting device. Specific examples of the steam setting device include a device of product name: FMSA type steam setter (manufactured by Fukushin Kyougyo Co., Ltd.) and a device of a product name: EPS-400 (manufactured by Tsujii Dyeing Machine Manufacturing Co., Ltd.). As the specific example of the method for crimping the composite fiber with the steam of the aqueous medium, a method for accommodating the composite fiber in the predetermined accommodation chamber, introducing the steam of the aqueous medium into the accommodation chamber, and bring the composite fiber into contact with steam while adjusting the temperature in the accommodation chamber at the predetermined temperature (for example, 100°C to 230°C) described above can be mentioned.

The crimping process of the composite fiber by bringing into contact with the aqueous medium is preferably performed in a state in which no tensile force is applied to the composite fiber (no tension is applied in the fiber axis direction) or a predetermined amount of tensile force is applied (a predetermined amount of tension is applied in the fiber axis direction). At that time, it is possible to control the extent of crimping by adjusting the tensile force applied to the composite fiber. As the method for adjusting the tensile force applied to the composite fiber, for example, a method for adjusting the load applied to the fiber by hanging weights of various weights on the composite fiber, a method for identifying both ends of the fiber in the slackened state and variously changing the slackening amount, and a method for winding a fiber around a wound body such as a paper tube or bobbin and appropriately changing the winding force (tightening force on the paper tube or bobbin) at the time of winding can be mentioned.

Further, the composite fiber may be dried after being brought into contact with the aqueous medium. The drying method is not particularly limited, and the drying may be natural drying, hot storm drying, or hot roller drying. The drying temperature is not particularly limited and may be, for example, 20°C to 150°C, preferably 40°C to 120°C, and more preferably 60°C to 100°C.

In the composite fiber according to the present embodiment, the first component containing a modified fibroin and the second component containing a structural protein are different in hydrophobicity with each other.

The difference in the hydrophobicity between the first component and the second component (the difference between the hydrophobicity of the modified fibroin and the hydrophobicity of the structural protein) in the composite fiber can be appropriately selected depending on the desired crimping ability, which is preferably 0.1 or more, more preferably 0.2 or more, more preferably 0.3 or more, more preferably 0.4 or more, more preferably 0.5 or more, more preferably 0.6 or more, more preferably 0. 7 or more, more preferably 0.8 or more, more preferably 0.9 or more, more preferably 1.0 or more, more preferably, more preferably 1.1 or more, still more preferably 1.2, and particularly preferably 1.3 or more. The greater the hydrophobicity difference is, the more stably the latent crimping ability can be imparted.

In the composite fiber, the shrinkage rates due to water shrinkage are different between the first component and the second component. The modified fibroin used for the first component is shrunk by being brought into contact with an aqueous medium. On the other hand, the structural protein used for the second component is not shrunk even when brought into contact with an aqueous medium or has a shrinkage rate lower than that of the first component.

Table 6 shows the shrinkage rate of the modified fibroin fiber obtained by spinning the modified fibroin under the same conditions, with respect to the aqueous medium. The shrinkage rate was calculated by the following method.

<Shrinkage rate>

A plurality of modified fibroin fibers having a length of about 30 cm are bundled to form a fiber bundle having a fineness of 150 denier. This fiber bundle, with a lead weight of 0.8 g being attached thereto, is immersed in water at 40°C for 10 minutes to be shrunk, and the shrinkage due to the residual stress derived from the manufacturing process is removed. The fiber bundle is taken out of the water and dried at room temperature for 2 hours with 0.8 g of lead weight attached. After drying, the length of the fiber bundle is measured. Again, the fiber bundle is immersed in water at 40°C for 10 minutes to be shrunk, and the length of the fiber bundle is measured in water. This wetting and drying are repeated at least three times, and an average length when wetted (Lwet) and an average length when dried (Ldry) are determined. The shrinkage rate is calculated according to the following expression.

$$\text{Expression: Shrinkage rate (\%)} = (1 - (\text{Ldry}/\text{Lwet})) \times 100$$

[Table 6]

| Modified fibroin | Shrinkage rate (%) |
| --- | --- |
| PRT410 (SEQ ID NO: 13) | 12.0% |
| PRT888 (SEQ ID NO: 33) | 8.0% |
| PRT965 (SEQ ID NO: 34) | 8.2% |
| PRT889 (SEQ ID NO: 35) | 5.6% |
| PRT916 (SEQ ID NO: 36) | 4.2% |
| PRT918 (SEQ ID NO: 37) | 4.6% |

The composition ratio of the first component to the second component in the composite fiber is not particularly limited, and it is appropriately set depending on the combination of the first component and the second component, the desired crimping ability, the composite configuration, and the like. The composition ratio of the first component and the second component may be, for example in terms of mass, in the range of 90:10 to 10:90, in the range of 80:20 to 20:80, in the range of 75:25 to 25:75, in the range of 75:25 to 35:65, in the range of 70:30 to 30:70, in the range of 65:35 to 35:65, in the range of 65:35 to 45:55, and in the range of 60:40 to 40:60.

In a case where the composite configuration of the composite fiber is the side-by-side type, a more excellent latent crimping ability can be imparted. The transverse section of the composite fiber (the interface between the first component and the second component) may be straight or curved.

The cross-sectional shape of the composite fiber is not particularly limited, and it may be a round cross section, a triangular cross section, a multi-lobar cross section, a Daruma type cross section, a flat cross section, or any other known cross-sectional shape, but crimp developability. However, in a case where the balance of crimping exhibitability and texture is important, a cross-sectional shape such as a round cross section or a semicircular side-by-side type having a Daruma type cross section is preferred.

The fiber diameter of the composite fiber is not particularly limited, and it can be appropriately set according to the application or the like. The fiber diameter may be, for example, 10 to 125 $\mu$m, 10 to 100 $\mu$m, or 10 to 80 $\mu$m, 10 to 60 $\mu$m, 10 to 40 $\mu$m, 10 to 35 $\mu$m, or 10 to 30 $\mu$m . In a case where the fiber diameter is 125 $\mu$m or less, the desolvation rate in the spinning process is hard to be increased. In a case where the fiber diameter is 10 $\mu$m or more, the composite fiber is easy to obtained stably.

The number of crimpings of the composite fiber can be appropriately set depending on the application and the like. For example, the number of crimpings may be 5 crimpings/25 mm or more, 10 crimpings/25 mm or more, 15 crimpings/25 mm or more, 20 crimpings/25 mm or more, or 25 crimpings/25 mm or more.

Further, the composite fiber may be chemically crosslinked between polypeptide molecules in the composite fiber depending on the application and the like. Examples of functional groups that can be crosslinked include an amino group, a carboxyl group, a thiol group, and a hydroxy group. For example, an amino group of a lysine side chain contained in the polypeptide can be crosslinked through an amide bond by dehydration condensation with a carboxyl group of a glutamic acid or aspartic acid side chain. The crosslinking may be performed by performing a dehydration condensation reaction under vacuum heating, or by a dehydration condensation agent such as carbodiimides.

The crosslinking between polypeptide molecules may be performed using a crosslinking agent such as carbodiimides or glutaraldehyde, or may be performed using an enzyme such as transglutaminase. Carbodiimides are compounds represented by the general formula $R^1N=C=NR^2$ (where $R^1$ and $R^2$ each independently represent an organic group containing an alkyl group having 1 to 6 carbon atoms or a cycloalkyl group). Specific examples of carbodiimides include 1-ethyl-3-(3-dimethylaminopropyl) carbodiimide hydrochloride (EDC), N,N'-dicyclohexylcarbodiimide (DCC), 1-cyclohexyl-3-(2-morpholinoethyl) carbodiimide, and diisopropyl carbodiimide (DIC). Among these, EDC and DIC are preferable because they have a high ability to form an amide bond between polypeptide molecules and easily perform a crosslinking reaction.

The crosslinking treatment is preferably performed by applying a crosslinking agent to the composite fiber and performing crosslinking by vacuum heating and drying. As the crosslinking agent, a pure product may be applied to the composite fiber, or a product diluted with a lower alcohol having 1 to 5 carbon atoms, a buffer solution, or the like to a concentration of 0.005 to 10 mass% may be applied to the composite fiber. The crosslinking treatment is preferably performed at a temperature of 20°C to 45°C for 3 to 42 hours. Higher stress (strength) can be imparted to the composite fiber by the crosslinking treatment.

(Evaluation of crimping ability of composite fiber)

The crimping ability of the composite fiber can be evaluated by, for example, checking the number of crimpings in a certain length of the composite fiber which has undergone the crimping process to exhibit a latent crimping ability.

[Product]

The composite fiber according to the present embodiment can be applied to various products. Examples of such products include fiber, yarn, fabric, knit, braid, non-woven fabric, paper, and batting. Examples of the fiber include a long fiber, a short fiber, a monofilament, and a multifilament, and examples of the yarn include a spun yarn, a twisted yarn, a pre-twisted yarn, a processed yarn, a mixed fiber yarn, and a mixed spinning yarn. Further, from these fibers and yarns, it is possible to manufacture products such as woven fabric, knit, braid, non-woven fabric, paper, batting, and the like. These products can be manufactured by a known method.

Examples

Hereinafter, the present invention will be described more specifically based on Examples and the like. However, the present invention is not limited to the following Examples.

1. Production of modified fibroin and structural protein

(1) Preparation of expression vector

As the modified fibroin, modified structural proteins each having amino acid sequences set forth in SEQ ID NO:

12 (PRT380), SEQ ID NO: 13 (PRT410), SEQ ID NO: 18 (PRT399), SEQ ID NO: 15 (PRT799), SEQ ID NO: 37 (PRT918), SEQ ID NO: 47 (PRT966), and SEQ ID NO: 40 (PRT1009) (hereinafter, also referred to as respectively "PRT799", "PRT918", "PRT966", and "PRT1009") were designed, based on the base sequence and the amino acid sequence of a fibroin (GenBank accession No.s: P46804.1, GI: 1174415) derived from Nephila clavipes. The amino acid sequence set forth in SEQ ID NO: 15 has an amino acid sequence obtained by substituting, inserting, and deleting an amino acid residue for the purpose of improving productivity with respect to the amino acid sequence of the fibroin derived from Nephila clavipes, and furthermore, adding the amino acid sequence set forth in SEQ ID NO: 11 (tag sequence and hinge sequence) to the N-terminal of the sequence. The amino acid sequence set forth in SEQ ID NO: 40 (PRT1009) is an amino acid sequence obtained by repeating, four times, a region of 20 domain sequences present in the amino acid sequence (amino acid sequence before adding the amino acid sequence set forth in SEQ ID NO: 11 to the N-terminus) set forth in SEQ ID NO: 7, substituting all QQs in the repeated sequences with VFs, and substituting the remaining Qs with Is, and furthermore, adding the amino acid sequence set forth in SEQ ID NO: 11 (tag sequence and hinge sequence) to the N-terminal of the sequence, for the purpose of improving hydrophobicity. Further, as the structural protein, a modified structural protein having the amino acid sequence set forth in SEQ ID NO: 45 (hereinafter, also referred to as "PRT798") was designed, based on the base sequence and the amino acid sequence of a structural protein of Caora hircus (GenBank Accession No. NP001272643.1).

Next, nucleic acids encoding PRT380, PRT410, PRT399, PRT799, PRT918, PRT966, and PRT1009 (modified fibroins), and PRT798 (structural protein) were synthesized. In the nucleic acid, an NdeI site was added to the 5' terminal and an EcoRI site was added downstream of the stop codon. The nucleic acid was cloned into a cloning vector (pUC118). Thereafter, each nucleic acid was enzymatically cleaved by treatment with NdeI and EcoRI and then recombinated into a protein expression vector pET-22b(+) to obtain an expression vector.

(2) Expression of modified fibroin and structural protein

Escherichia coli BLR(DE3) was transformed with each of the expression vectors obtained in (1) above. The above transformed Escherichia coli was cultured in 2 mL of an LB medium containing ampicillin for 15 hours. The culture solution was added to 100 mL of a seed culture medium (Table 7) containing ampicillin so that the $OD_{600}$ was 0.005. While maintaining the temperature of the culture solution at 30°C, flask culturing was carried out (for about 15 hours) until the $OD_{600}$ reached 5, thereby obtaining each seed culture solution.

[Table 7]

| Seed culture medium | |
| --- | --- |
| Reagent | Concentration (g/L) |
| Glucose | 5.0 |
| $KH_2PO_4$ | 4.0 |
| $K_2HPO_4$ | 9.3 |
| Yeast Extract | 6.0 |
| Ampicillin | 0.1 |

The seed culture solution was added to a jar fermenter containing 500 mL of a production medium (Table 8) so that the $OD_{600}$ was 0.05. The culture was carried out while maintaining the temperature of culture solution at 37°C and controlling the pH constant at 6.9. Further, the concentration of dissolved oxygen in the culture solution was maintained at 20% of the dissolved oxygen saturation concentration.

[Table 8]

| Production medium | |
| --- | --- |
| Reagent | Concentration (g/L) |
| Glucose | 12.0 |
| $KH_2PO_4$ | 9.0 |
| $MgSO_4 \cdot 7H_2O$ | 2.4 |
| Yeast Extract | 15 |
| $FeSO_4 \cdot 7H_2O$ | 0.04 |
| $MnSO_4 \cdot 5H_2O$ | 0.04 |

(continued)

| Production medium | |
| --- | --- |
| Reagent | Concentration (g/L) |
| CaCl$_2 \cdot$ 2H$_2$O | 0.04 |
| GD-113 (anti-foaming agent) | 0.1 (mL/L) |

Immediately after glucose in the production medium was completely consumed, a feed solution (455 g/1L of glucose and 120 g/1L of Yeast Extract) was added at a rate of 1 mL/min. The culture was carried out while maintaining the temperature of culture solution at 37°C and controlling the pH constant at 6.9. Further, the concentration of dissolved oxygen in the culture solution was maintained at 20% of the dissolved oxygen saturation concentration, and the culture was carried out for 20 hours. Thereafter, 1 M isopropyl-β-thiogalactopyranoside (IPTG) was added to the culture solution to a final concentration of 1 mM to induce the expression of each of the modified fibroin and the structural protein. 20 hours after the addition of IPTG, the culture solution was centrifuged to recover the bacterial cells. SDS-PAGE was carried out using bacterial cells prepared from the culture solution before the addition of IPTG and after the addition of IPTG, and the expression of each of the targeted modified fibroin and the targeted structural protein was checked by the IPTG addition-dependent appearance of a band corresponding to the size of each of the targeted modified fibroin and the targeted structural protein.

(3) Purification of modified fibroin and structural protein

The bacterial cells recovered 2 hours after the addition of IPTG were washed with a 20 mM Tris-HCl buffer solution (pH 7.4). The bacterial cells after washing were suspended in 20 mM Tris-HCl buffer solution (pH 7.4) containing about 1 mM PMSF, and the cell suspension was disrupted with a high-pressure homogenizer (manufactured by GEA Niro Soavi SpA). The disrupted cells were centrifuged to obtain a precipitate. The obtained precipitate was washed with a 20 mM Tris-HCl buffer solution (pH 7.4) until the obtained precipitate was highly pure. The precipitate after washing was suspended in 8 M guanidine buffer solution (8 M guanidine hydrochloride, 10 mM sodium dihydrogen phosphate, 20 mM NaCl, 1 mM Tris-HCl, pH 7.0) so that the concentration of the suspension was 100 mg/mL, and dissolved by stirring with a stirrer at 60°C for 30 minutes. After dissolving, dialysis was carried out in water using a dialysis tube (cellulose tube 36/32 manufactured by Sanko Junyaku Co., Ltd.). The modified fibroins (PRT380, PRT410, PRT399, PRT799, PRT918, PRT966, and PRT1009) and the structural protein (PRT798) were obtained by recovering the white aggregated protein obtained after dialysis by centrifugation, removing the water content with a freeze dryer, and recovering the freeze-dried powder.

2. Production of silk fibroin (structural protein)

(1) Preparation of silk fibroin powder

Silkworm cocoons from a natural silkworm (Bombvx mori), from which the contents therein were removed, were cut into small pieces. The pieces were boiled for about 30 minutes in boiled water containing 0.5% by mass of Marcel soap (Marcel soap finely ground with a grater was used), and then boiled for 30 minutes in boiled water. Further, this procedure was repeated two times (three times in total). Finally the pieces were boiled for 30 minutes in boiled water to completely remove sericin covering the silk fibroin, and the silk fibroin after removing sericin was dried overnight in an environment of 37°C. The dried silk was weighed, and an aqueous solution of lithium bromide (9 mol/L) was added such that the weight of the silk was 10% by mass/volume, and the silk was dissolved at 40°C for 2 hours. This aqueous solution was put into a cellulose dialysis membrane (Seamless Cellulose Tubing, 36/32, manufactured by VISKASESELESCOAP Company), and dialyzed against distilled water for 3 to 4 days. The recovered solution after dialysis was centrifuged at 15,000 rpm and 20°C for 1 hour to remove undissolved residues and impurities. Further, the precipitate was diluted with Milli water such that the concentration was 2% by mass or less. After the dilution, the solution was passed through a 150 μm filter manufactured by Toyo Roshi Kaisha, Ltd. to completely remove impurities. The aqueous solution of silk fibroin was frozen at -80°C and freeze-dried overnight. By confirming that the water content was sufficiently removed, the silk fibroin powder was obtained.

3. Manufacturing and evaluation of composite fiber

<Example 1>

(1) Preparation of doping liquid

24% by mass of the modified fibroin (PRT799, hydrophobicity: -0.80) obtained in the above-described production process of the modified fibroin, as the first component, was mixed with 76% by mass of formic acid (purity 98%) as a solvent, heated with an aluminum block heater at 40°C for 1 hour to be dissolved, filtered with a metal filter having a mesh size of 1 $\mu$m, and defoamed, whereby the first doping liquid was prepared.

The second doping liquid was prepared in the same manner as the first doping liquid, except that 24% by mass of the modified fibroin (PRT1009, hydrophobicity: 0.49) obtained in the above-described production process of the modified fibroin was used as the second component.

(2) Dry-wet type spinning

Dry-wet type spinning was performed using a table-top spinning device. A reserve tank was charged with the first doping liquid and the second doping liquid, prepared as described above. While keeping the temperature at 40°C, the first doping liquid was discharged from a mono-hole nozzle having a diameter of 0.2 mm using a gear pump, then the second doping liquid was discharged from a mono-hole nozzle having the same diameter such that the second doping liquid was joined to the first doping liquid, and the joined liquids were discharged into 100% by mass of methanol in a coagulation liquid bath. After coagulation, drawing was performed in the coagulation liquid bath, further dry heat drawing was performed, followed by washing in a water washing bath to exhibit latent crimping, and a composite fiber of a side-by-side type, having a composite weight ratio of 1:1 (first component: second component), was obtained and wound.

The dry-wet type spinning conditions are as follows.

Extrusion nozzle diameter: 0.2 mm
Dry heat drawing ratio: 6 times
Temperature of coagulation liquid (methanol): 5°C
Drying temperature: 60°C

(3) Evaluation of crimping ability

When the composite fiber obtained in (2) above was again subjected to a repeated process of being brought into contact with water in a water bath and then being dried, it has been confirmed that the crimping confirmed in (2) above was maintained and shown excellent bulkiness and a crimping ability.

<Example 2>

(1) Preparation of spinning stock solution

The first doping liquid was prepared in the same manner as in Example 1.

The second doping liquid was prepared in the same manner as in Example 1, except that 24% by mass of the structural protein (PRT798, hydrophobicity: 0.49) obtained in the above-described production process of the structural protein was used as the second component.

(2) Dry-wet type spinning

A reserve tank was charged with the first doping liquid and the second doping liquid, prepared as described above. Dry-wet type spinning was performed in the same manner as in Example 1 to exhibit latent crimping, and a composite fiber of a side-by-side type, having a composite mass ratio of 1:1 (first component: second component), was obtained and wound.

(3) Evaluation of crimping ability

When the composite fiber obtained above was again subjected to a repeated process of being brought into contact with water in a water bath and then being dried, it has been confirmed that the crimping confirmed in (2) above was maintained and shown excellent bulkiness and a crimping ability.

<Example 3>

(1) Preparation of doping liquid

The first doping liquid was prepared in the same manner as in Example 1.

The second doping liquid was prepared in the same manner as in Example 1, except that 24% by weight of the silk fibroin obtained in the above-described production process of the silk fibroin (structural protein) was used as the second component.

(2) Dry-wet type spinning

A reserve tank was charged with the first doping liquid and the second doping liquid, prepared as described above. Dry-wet type spinning was performed in the same manner as in Example 1 to exhibit latent crimping, and a composite fiber of a side-by-side type, having a composite mass ratio of 1:1 (first component: second component), was obtained and wound.

(3) Evaluation of crimping ability

When the composite fiber obtained above was again subjected to a repeated process of being brought into contact with water in a water bath and then being dried, it has been confirmed that the crimping confirmed in (2) above was maintained and shown excellent bulkiness and a crimping ability.

Reference Example 1: Combustibility test of modified fibroin

A freeze-dried powder of the modified fibroin (PRT799) was added to a dimethyl sulfoxide solution of lithium chloride (concentration: 4.0% by mass) such that the concentration of the modified fibroin was 24% by mass, and mixed for 3 hours to be dissolved using a shaker. Then, insoluble bodies and bubbles were removed, whereby a modified fibroin solution (spinning stock solution) was obtained.

The obtained spinning stock solution was heated to 90°C, filtered with a metal filter having a mesh size of 5 $\mu$m, and then allowed to be left in a 30 mL stainless steel syringe to be defoamed, and then discharged from a solid nozzle having a needle diameter of 0.2 mm into 100% by mass of methanol in the coagulation bath. The discharge temperature was 90°C. After coagulation, the obtained raw yarn was wound and naturally dried to obtain a modified fibroin fiber (raw material fiber).

A knitted fabric (thickness: 180 denier, gauge number: 18) was manufactured by circular knitting using a circular knitting machine, using twisted yarn in which the raw material fibers were twisted with each other. 20 g of the obtained knitted fabric was cut out and used as a test piece.

The combustibility test was performed in accordance with the "testing method for powdery or low melting point synthetic resin" described in "Fire and Disaster No. 50 (on May 31th, 1995)". The test was performed under the conditions of a temperature of 22°C, a relative humidity of 45%, and an atmospheric pressure of 1,021 hPa. Table 9 shows the measurement results (oxygen concentration (%), combustion rate (%), and converted combustion rate (%)).

[Table 9]

| Oxygen concentration (%) | Combustion rate (%) | Converted combustion rate (%) |
|---|---|---|
| 20.0 | 39.1 | 40.1 |
| 27.0 | 48.1 | 49.3 |
| 28.0 | 51.9 | 53.2 |
| 30.0 | 53.6 | 54.9 |
| 50.0 | 61.2 | 62.7 |
| 70.0 | 91.1 | 93.3 |
| 100.0 | 97.6 | 100.0 |

As a result of the combustibility test, the knitted fabric knitted with the modified fibroin (PRT799) fiber had a limiting oxygen index (LOI) value of 27.2. It is generally known that in a case where an LOI value is 26 or more, it is flame retardant. It can be seen that the modified fibroin has excellent flame retardancy.

Reference Example 2: Evaluation of hygroscopic heat generating property of modified fibroin

A freeze-dried powder of the modified fibroin was added to a dimethyl sulfoxide solution of lithium chloride (concentration: 4.0% by mass) such that the concentration of the modified fibroin was 24% by mass, and mixed for 3 hours to be dissolved using a shaker. Then, insoluble bodies and bubbles were removed, whereby a modified fibroin solution (spinning stock solution) was obtained.

The obtained spinning stock solution was heated to 60°C, filtered with a metal filter having a mesh size of 5 μm, and then allowed to be left in a 30 mL stainless steel syringe to be defoamed, and then discharged from a solid nozzle having a needle diameter of 0.2 mm into 100% by mass of methanol in the coagulation bath. The discharge temperature was 60°C. After coagulation, the obtained raw yarn was wound and naturally dried to obtain a modified fibroin fiber (raw material fiber).

For comparison, a wool fiber, a cotton fiber, TENCEL fiber, a rayon fiber, and a polyester fiber, which are commercially available, were prepared as raw material fibers.

Each raw material fiber was used to manufacture a knitted fabric by flat knitting using a flat knitting machine. Table 10 shows the thickness and the gauge number of the knitted fabric obtained by using the PRT918 fiber or the PRT799 fiber. The thickness and gauge number of the knitted fabrics using other raw material fibers were adjusted such that the cover factor was almost the same as that of the knitted fabric of the modified fibroin fiber, which is specifically described as follows.

[Table 10]

| Raw material fiber | Thickness [N] | Gauge number [GG] |
|---|---|---|
| PRT918 | 1/30 (wool count number single yarn) | 18 |
| PRT799 | 1/30 (wool count number single yarn) | 16 |
| Wool | 2/30 (two folded yarn) | 14 |
| Cotton | 2/34 (two folded yarn) | 14 |
| TENCEL | 2/30 (two folded yarn) | 15 |
| Rayon | 1/38 (single yarn) | 14 |
| Polyester | 1/60 (single yarn) | 14 |

Two pieces of knitted fabric cut into 10 cm × 10 cm were overlapped together and the four sides were sewn together to obtain a test piece (sample). After leaving the test piece in a low humidity environment (temperature 20°C ± 2°C, relative humidity 40% ± 5%) for 4 hours or more, the test piece was transferred to a high humidity environment (temperature 20°C ± 2°C, relative humidity 90% ± 5%), and the temperature was measured at an interval of 1 minute for 30 minutes by a temperature sensor attached in the center of the inside of the test piece.

From the measurement results, the highest hygroscopic heat generation degree was determined according to Expression A.

Expression A: highest hygroscopic heat generation degree = {(highest temperature of a sample when the sample has been transferred to a high humidity environment after being placed in a low humidity environment until a temperature of the sample reaches equilibrium) - (temperature of the sample when the sample is being transferred to the high humidity environment after being placed in the low humidity environment until the temperature of the sample reaches equilibrium)} (°C)/sample weight (g)

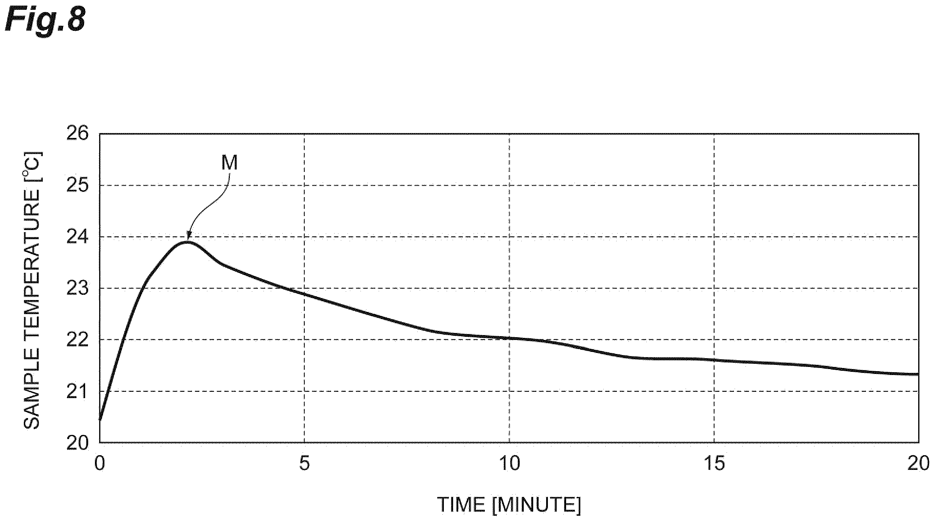

Fig. 8 is a graph showing an example of a result of a hygroscopic heat generating property test. The horizontal axis of the graph indicates the time (minutes) left in the high humidity environment, where 0 is the time when the sample was transferred from the low humidity environment to the high humidity environment. The vertical axis of the graph indicates the temperature (sample temperature) measured by the temperature sensor. In the graph shown in Fig. 8, the point indicated by M corresponds to the maximum value of the sample temperature.

Table 11 shows the calculation results of the highest hygroscopic heat generation degree of each knitted fabric.

[Table 11]

| Raw material fiber | Highest hygroscopic heat generation degree (°C/g) |
|---|---|
| PRT918 | 0.040 |
| PRT799 | 0.031 |
| Wool | 0.020 |
| Cotton | 0.021 |
| TENCEL | 0.018 |
| Rayon | 0.025 |
| Polyester | 0.010 |

As shown in Table 11, it can be seen that the modified fibroins (PRT918 and PRT799) have a higher highest hygroscopic heat generation degree and a more excellent hygroscopic heat generating property than existing materials.

Reference Example 3: Evaluation of heat retaining property of modified fibroin

A freeze-dried powder of the modified fibroin was added to a dimethyl sulfoxide solution of lithium chloride (concentration: 4.0% by mass) such that the concentration of the modified fibroin was 24% by mass, and mixed for 3 hours to be dissolved using a shaker. Then, insoluble bodies and bubbles were removed, whereby a modified fibroin solution (spinning stock solution) was obtained.

The obtained spinning stock solution was heated to 60°C, filtered with a metal filter having a mesh size of 5 $\mu$m, and then allowed to be left in a 30 mL stainless steel syringe to be defoamed, and then discharged from a solid nozzle having a needle diameter of 0.2 mm into 100% by mass of methanol in the coagulation bath. The discharge temperature was 60°C. After coagulation, the obtained raw yarn was wound and naturally dried to obtain a modified fibroin fiber (raw material fiber).

For comparison, a wool fiber, a silk fiber, a cotton fiber, a rayon fiber, and a polyester fiber, which are commercially available, were prepared as raw material fibers.

Each raw material fiber was used to manufacture a knitted fabric by flat knitting using a flat knitting machine. Table 12 shows the count number, number of twisted fibers, gauge number, and basis weight of the knitted fabric using the PRT966 fiber or the PRT799 fiber. The knitted fabrics using other raw material fibers were adjusted such that the cover factor was almost the same as that of the knitted fabric of the modified fibroin fiber, which is specifically described as follows.

[Table 12]

| Raw material fiber | Count number [Nm] | Number of twisted fibers | Gauge number [GG] | Basis weight [g/m$^2$] |
|---|---|---|---|---|
| PRT966 | 30 | 1 | 18 | 90. 1 |
| PRT799 | 30 | 1 | 16 | 111.0 |
| Wool | 30 | 2 | 14 | 242.6 |
| Silk | 60 | 2 | 14 | 225.2 |
| Cotton | 34 | 2 | 14 | 194.1 |
| Rayon | 38 | 1 | 14 | 181.8 |
| Polyester | 60 | 1 | 14 | 184.7 |

The heat retaining property was evaluated using Thermolab II tester KES-F7 manufactured by Kato Tech Co.,

Ltd., using a dry contact method (a method performed on assumption that skin and clothes are in direct contact with each other in a dry state). One piece of knitted fabric cut into a square of 20 cm × 20 cm was used as a test piece (sample). The test piece was set on a hot plate set to a constant temperature (30°C), and a heat quantity (a) dissipated through the test piece was obtained under the condition of the wind velocity in the wind tunnel of 30 cm/sec. A heat quantity (b) dissipated under the same condition described above was obtained without setting the test piece, and the heat retention rate (%) was calculated according to Expression B.

$$\text{Expression B: Heat retention rate (\%)} = (1\text{-}a/b) \times 100$$

From the measurement result, the heat retaining property index was determined according to Expression C.

$$\text{Formula C: Heat retaining property index} = \text{heat retention rate}$$
$$(\%)/\text{weight of sample } (g/m^2).$$

Table 13 shows the calculation results of the heat retaining property index. It can be evaluated that the higher the heat retaining property index is, the more excellent the heat retaining property.

[Table 13]

| Raw material fiber | Heat retaining property index |
|---|---|
| PRT966 | 0.33 |
| PRT799 | 0.22 |
| Wool | 0.16 |
| Silk | 0.11 |
| Cotton | 0.13 |
| Rayon | 0.02 |
| Polyester | 0.18 |

As shown in Table 13, it can be seen that the modified fibroins (PRT966 and PRT799) have a higher heat retaining property index and a more excellent heat retaining property than existing materials.

Reference Example 4: Manufacturing of raw material fiber

A freeze-dried powder of the modified fibroin (PRT380, PRT410, PRT399, or PRT799) was added to a dimethyl sulfoxide solution of lithium chloride (concentration: 4.0% by mass) such that the concentration of the modified fibroin was 18% or 24% by mass (see Table 14), and mixed for 3 hours to be dissolved using a shaker. Then, insoluble bodies and bubbles were removed, whereby a modified fibroin solution (spinning stock solution) was obtained.
From the obtained spinning stock solution, raw material fibers which were spun and drawn were manufactured by a dry-wet type spinning method using a spinning device based on the spinning device 10 shown in Fig. 6. The spinning device used is a spinning device further having an additional second undrawn yarn manufacturing device (second bath) between the undrawn yarn manufacturing device 2 (first bath) and a wet heat drawing device 3 (third bath) in the spinning device 10 shown in Fig. 6. The conditions of the dry-wet type spinning method are as follows.
Extrusion nozzle diameter: 0.2 mm
Liquid and temperature in the first to third baths: see Table 14
Total drawing rate: see Table 14
Drying temperature: 60°C

[Table 14]

| | Doping liquid | | First bath | | Second bath | | Third bath | | Total drawing rate (times) |
|---|---|---|---|---|---|---|---|---|---|
| | Modified fibroin | Concentration (% by mass) | Liquid | Temp. (°C) | Liquid | Temp. (°C) | Liquid | Temp. (°C) | |
| Manufacture Example 1 | PRT799 | 24 | | -5 | | 16 | | | 1 |
| Manufacture Example 2 | | | | | | | | | 2 |
| Manufacture Example 3 | | | | | | | | | 3 |
| Manufacture Example 4 | | | | | | | | | 4 |
| Manufacture Example 5 | | 18 | | | | | | | 1 |
| Manufacture Example 6 | | | | | | | | | 2 |
| Manufacture Example 7 | | | | | | | | | 3 |
| Manufacture Example 8 | | | | | | | | | 4 |

(continued)

| | Doping liquid | | First bath | | Second bath | | Third bath | | Total drawing rate (times) |
|---|---|---|---|---|---|---|---|---|---|
| | Modified fibroin | Concentration (% by mass) | Liquid | Temp. (°C) | Liquid | Temp. (°C) | Liquid | Temp. (°C) | |
| Manufacture Example 9 | PRT410 | 24 | 100% Methanol | -11 | 100% Methanol | 14 | Water | 17 | 1 |
| Manufacture Example 10 | | | | | | | | | 2 |
| Manufacture Example 11 | | | | | | | | | 3 |
| Manufacture Example 12 | | | | | | | | | 4 |
| Manufacture Example 13 | PRT399 | | | | | | | | 1 |
| Manufacture Example 14 | | | | | | | | | 2 |
| Manufacture Example 15 | | | | | | | | | 3 |
| Manufacture Example 16 | PRT380 | | | | | 11 | | | 1 |
| Manufacture Example 17 | | | | | | | | | 2 |
| Manufacture Example 18 | | | | | | | | | 3 |
| Manufacture Example 19 | | | | | | | | | 4 |

(3) Manufacturing of modified fibroin fiber and evaluation of shrinkage rate A and shrinkage rate B

The modified fibroin fiber (artificial fibroin fiber) was manufactured by subjecting each raw material fiber obtained in Manufacture Examples 1 to 19 to the contacting step of bringing the raw material fiber into contact with water, or the drying step of drying the raw material fiber at room temperature after completion of the contacting step.

<Evaluation of shrinkage rate A in contacting step>

A plurality of raw material fiber threads having a length of 30 cm were cut from each of the wound raw material fibers obtained in Manufacture Examples 1 to 19. The plurality of raw material fiber threads were bundled to form a raw material fiber bundle having a fineness of 150 denier. With 0.8 g of lead weight being attached to each raw material fiber bundle, each raw material fiber bundle was immersed in water for 10 minutes at a temperature shown in Tables 15 to 18 (contacting step). Thereafter, the length of each raw material fiber bundle was measured in water. The length measurement of the raw material fiber bundle in water was carried out with 0.8 g lead weight being attached to the raw material fiber bundle in order to eliminate the curling of the raw material fiber bundle. Next, the shrinkage rate A (%) of each raw material fiber was calculated according to Expression (D). In Expression D, LO represents the length of the fiber before contact with water and after spinning, and here LO is 30 cm. Similarly, in Expression, Lw represents the length of the fiber irreversibly shrunk due to contact with water after spinning, and here LO is the length of each raw material fiber bundle measured in water.

$$\text{Expression D: Shrinkage rate A (\%)} = \{1 - (Lw/LO)\} \times 100$$

<Evaluation of shrinkage rate B in drying step>

After completion of the contacting step, the raw material fiber bundle was taken out of water. The raw material fiber bundle taken out was dried at room temperature for 2 hours with 0.8 g lead weight being attached (drying step) to obtain the modified fibroin fiber. After drying, the length of each of the modified fibroin fiber bundles was measured. Next, the shrinkage rate B (%) of each of the modified fibroin fibers was calculated according to Expression E. In Expression E, LO represents the length of the fiber before contact with water and after spinning, and here LO is 30 cm. Similarly, in Expression E, Lwd represents the length of the fiber irreversibly shrunk due to contact with water after spinning and then further shrunk by drying, and here LO is the length of each of the modified fibroin fiber bundle measured after drying.

$$\text{Expression E: Shrinkage rate B} = \{1 - (Lwd/LO)\} \times 100 \ (\%)$$

The results are shown in Tables 15 to 18.

[Table 15]

| Raw material fiber/artificial fibroin fiber | | Water temperature (°C) | Shrinkage rate A (%) | Shrinkage rate B (%) |
|---|---|---|---|---|
| Manufacture Example 1 | 24wt% PRT799 x1 | | 0.0 | 7.8 |
| Manufacture Example 2 | 24wt% PRT799 x2 | | -1.2 | 10.3 |
| Manufacture Example 3 | 24wt% PRT799 x3 | | 7.2 | 21.2 |
| Manufacture Example 4 | 24wt% PRT799 x4 | 20 | 13.5 | 26.3 |
| Manufacture Example 6 | 18wt% PRT799 x2 | | -2.3 | 9.5 |
| Manufacture Example 7 | 18wt% PRT799 x3 | | 6.0 | 19.7 |
| Manufacture Example 8 | 18wt% PRT799 x4 | | 14.3 | 27.5 |

(continued)

| Raw material fiber/artificial fibroin fiber | | Water temperature (°C) | Shrinkage rate A (%) | Shrinkage rate B (%) |
|---|---|---|---|---|
| Manufacture Example 2 | 24wt% PRT799 x2 | 40 | -5.3 | 7.2 |
| Manufacture Example 3 | 24wt% PRT799 x3 | | 8.7 | 21.3 |
| Manufacture Example 4 | 24wt% PRT799 x4 | | 14.5 | 26.0 |
| Manufacture Example 6 | 18wt% PRT799 x2 | | -4.3 | 7.3 |
| Manufacture Example 7 | 18wt% PRT799 x3 | | 6.2 | 18.3 |
| Manufacture Example 8 | 18wt% PRT799 x4 | | 16.0 | 28.7 |
| Manufacture Example 3 | 24wt% PRT799 x3 | 60 | 6.8 | 21.0 |
| Manufacture Example 4 | 24wt% PRT799 x4 | | 15.0 | 27.5 |
| Manufacture Example 6 | 18wt% PRT799 x2 | | -1.5 | 10.7 |
| Manufacture Example 7 | 18wt% PRT799 x3 | | 3.3 | 18.2 |
| Manufacture Example 8 | 18wt% PRT799 x4 | | 16.2 | 29.0 |

[Table 16]

| Raw material fiber/artificial fibroin fiber | | Water temperature (°C) | Shrinkage rate A (%) | Shrinkage rate B (%) |
|---|---|---|---|---|
| Manufacture Example 10 | 24wt% PRT410 x2 | 20 | -2.3 | 8.7 |
| Manufacture Example 11 | 24wt% PRT410 x3 | | 4.7 | 16.7 |
| Manufacture Example 12 | 24wt% PRT410 x4 | | 10.3 | 22.3 |
| Manufacture Example 11 | 24wt% PRT410 x3 | 40 | 4.7 | 17.5 |
| Manufacture Example 12 | 24wt% PRT410 x4 | | 11.5 | 24.0 |
| Manufacture Example 11 | 24wt% PRT410 x3 | 60 | 2.0 | 16.5 |
| Manufacture Example 12 | 24wt% PRT410 x4 | | 10.8 | 25.0 |

[Table 17]

| Raw material fiber/artificial fibroin fiber | | Water temperature (°C) | Shrinkage rate A (%) | Shrinkage rate B (%) |
|---|---|---|---|---|
| Manufacture Example 13 | 24wt% PRT399 x1 | 20 | -3.5 | 7.6 |
| Manufacture Example 14 | 24wt% PRT399 x2 | | 3.7 | 12.5 |
| Manufacture Example 15 | 24wt% PRT399 x3 | | 7.0 | 16.8 |
| Manufacture Example 14 | 24wt% PRT399 x2 | 40 | 3.0 | 12.7 |
| Manufacture Example 15 | 24wt% PRT399 x3 | | 7.3 | 16.7 |
| Manufacture Example 14 | 24wt% PRT399 x2 | 60 | 3.3 | 9.3 |
| Manufacture Example 15 | 24wt% PRT399 x3 | | 6.8 | 14.2 |

[Table 18]

| Raw material fiber/artificial fibroin fiber | | Water temperature (°C) | Shrinkage rate A (%) | Shrinkage rate B (%) |
|---|---|---|---|---|
| Manufacture Example 16 | 24wt% PRT380 x1 | 20 | -1.1 | 9.4 |
| Manufacture Example 17 | 24wt% PRT380 x2 | | 2.7 | 13.3 |
| Manufacture Example 18 | 24wt% PRT380 x3 | | 7.0 | 17.7 |
| Manufacture Example 19 | 24wt% PRT380 x4 | | 10.0 | 20.2 |
| Manufacture Example 17 | 24wt% PRT380 x2 | 40 | 3.3 | 14.2 |
| Manufacture Example 18 | 24wt% PRT380 x3 | | 7.7 | 19.0 |
| Manufacture Example 19 | 24wt% PRT380 x4 | | 12.0 | 22.0 |
| Manufacture Example 17 | 24wt% PRT380 x2 | 60 | 2.7 | 14.3 |
| Manufacture Example 18 | 24wt% PRT380 x3 | | 8.2 | 20.3 |
| Manufacture Example 19 | 24wt% PRT380 x4 | | 12.0 | 23.2 |

As shown in Tables 15 to 18, it can be seen that the modified fibroin (PRT380, PRT410, PRT399, or PRT799) fiber has excellent shrinkability with respect to moisture and has an excellent latent crimping ability. In a case where a side-by-side type composite fiber is formed using the modified fibroin, a composite fiber excellent in the crimping ability, bulkiness, and elasticity can be obtained.

Reference Signs List

1     extrusion device

2     undrawn yarn manufacturing device

3·    wet heat drawing device

4     drying device

6     doping liquid

9     spinneret

10    spinning device

11    coagulation liquid

20    coagulation liquid bath

21    drawing bath

36    composite fiber

SEQUENCE LISTING

<110> Spiber Inc.

<120> CONJUGATED FIBER AND METHOD FOR PRODUCING THE SAME

<130> FP19-0317-00

<160> 47

<170> PatentIn version 3.5

<210> 1
<211> 50
<212> PRT
<213> Araneus diadematus

<400> 1

Ser Gly Cys Asp Val Leu Val Gln Ala Leu Leu Glu Val Val Ser Ala
1               5                   10                  15

Leu Val Ser Ile Leu Gly Ser Ser Ser Ile Gly Gln Ile Asn Tyr Gly
                20                  25                  30

Ala Ser Ala Gln Tyr Thr Gln Met Val Gly Gln Ser Val Ala Gln Ala
            35                  40                  45

Leu Ala
    50

<210> 2
<211> 30
<212> PRT
<213> Araneus diadematus

<400> 2

Ser Gly Cys Asp Val Leu Val Gln Ala Leu Leu Glu Val Val Ser Ala
1               5                   10                  15

Leu Val Ser Ile Leu Gly Ser Ser Ser Ile Gly Gln Ile Asn
                20                  25                  30

<210> 3
<211> 21
<212> PRT
<213> Araneus diadematus

<400> 3

Ser Gly Cys Asp Val Leu Val Gln Ala Leu Leu Glu Val Val Ser Ala
1               5                   10                  15

Leu Val Ser Ile Leu
                20

```
<210>   4
<211>   1154
<212>   PRT
<213>   Artificial Sequence

<220>
<223>   recombinant spider silk protein ADF3KaiLargeNRSH1

<400>   4

Met His His His His His His His His His Ser Ser Gly Ser Ser
1               5                   10                  15


Leu Glu Val Leu Phe Gln Gly Pro Ala Arg Ala Gly Ser Gly Gln Gln
            20                  25                  30


Gly Pro Gly Gln Gln Gly Pro Gly Gln Gln Gly Pro Gly Gln Gln Gly
            35                  40                  45


Pro Tyr Gly Pro Gly Ala Ser Ala Ala Ala Ala Ala Ala Gly Gly Tyr
    50                  55                  60


Gly Pro Gly Ser Gly Gln Gln Gly Pro Ser Gln Gln Gly Pro Gly Gln
65                  70                  75                  80


Gln Gly Pro Gly Gly Gln Gly Pro Tyr Gly Pro Gly Ala Ser Ala Ala
                85                  90                  95


Ala Ala Ala Ala Gly Gly Tyr Gly Pro Gly Ser Gly Gln Gln Gly Pro
            100                 105                 110


Gly Gly Gln Gly Pro Tyr Gly Pro Gly Ser Ser Ala Ala Ala Ala Ala
            115                 120                 125


Ala Gly Gly Asn Gly Pro Gly Ser Gly Gln Gln Gly Ala Gly Gln Gln
    130                 135                 140


Gly Pro Gly Gln Gln Gly Pro Gly Ala Ser Ala Ala Ala Ala Ala Ala
145                 150                 155                 160


Gly Gly Tyr Gly Pro Gly Ser Gly Gln Gln Gly Pro Gly Gln Gln Gly
            165                 170                 175


Pro Gly Gly Gln Gly Pro Tyr Gly Pro Gly Ala Ser Ala Ala Ala Ala
            180                 185                 190


Ala Ala Gly Gly Tyr Gly Pro Gly Ser Gly Gln Gly Pro Gly Gln Gln
    195                 200                 205
```

```
Gly Pro Gly Gly Gln Gly Pro Tyr Gly Pro Gly Ala Ser Ala Ala Ala
    210                 215             220

Ala Ala Ala Gly Gly Tyr Gly Pro Gly Ser Gly Gln Gln Gly Pro Gly
    225             230             235                     240

Gln Gln Gly Pro Gly Gln Gln Gly Pro Gly Gly Gln Gly Pro Tyr Gly
                245             250                 255

Pro Gly Ala Ser Ala Ala Ala Ala Ala Ala Gly Gly Tyr Gly Pro Gly
            260             265             270

Tyr Gly Gln Gln Gly Pro Gly Gln Gln Gly Pro Gly Gly Gln Gly Pro
    275             280             285

Tyr Gly Pro Gly Ala Ser Ala Ala Ser Ala Ala Ser Gly Gly Tyr Gly
    290             295             300

Pro Gly Ser Gly Gln Gln Gly Pro Gly Gln Gln Gly Pro Gly Gly Gln
305             310             315                     320

Gly Pro Tyr Gly Pro Gly Ala Ser Ala Ala Ala Ala Ala Gly Gly
            325             330             335

Tyr Gly Pro Gly Ser Gly Gln Gln Gly Pro Gly Gln Gln Gly Pro Gly
        340             345                 350

Gln Gln Gly Pro Gly Gln Gln Gly Pro Gly Gly Gln Gly Pro Tyr Gly
        355             360             365

Pro Gly Ala Ser Ala Ala Ala Ala Ala Ala Gly Gly Tyr Gly Pro Gly
    370             375             380

Ser Gly Gln Gln Gly Pro Gly Gln Gln Gly Pro Gly Gln Gln Gly Pro
385             390             395                     400

Gly Gln Gln Gly Pro Gly Gln Gln Gly Pro Gly Gln Gln Gly Pro Gly
            405             410                 415

Gln Gln Gly Pro Gly Gln Gln Gly Pro Gly Gln Gln Gly Pro Gly Gly
            420             425                 430

Gln Gly Ala Tyr Gly Pro Gly Ala Ser Ala Ala Ala Gly Ala Ala Gly
        435             440                 445

Gly Tyr Gly Pro Gly Ser Gly Gln Gln Gly Pro Gly Gln Gln Gly Pro
    450             455             460
```

Gly Gln Gln Gly Pro Gly Gln Gln Gly Pro Gly Gln Gln Gly Pro Gly
465                 470                 475                 480

Gln Gln Gly Pro Gly Gln Gln Gly Pro Gly Gln Gln Gly Pro Tyr Gly
                485                 490                 495

Pro Gly Ala Ser Ala Ala Ala Ala Ala Ala Gly Gly Tyr Gly Pro Gly
            500                 505                 510

Ser Gly Gln Gln Gly Pro Gly Gln Gln Gly Pro Gly Gln Gln Gly Pro
        515                 520                 525

Gly Gly Gln Gly Pro Tyr Gly Pro Gly Ala Ala Ser Ala Ala Val Ser
        530                 535                 540

Val Ser Arg Ala Arg Ala Gly Ser Gly Gln Gln Gly Pro Gly Gln Gln
545                 550                 555                 560

Gly Pro Gly Gln Gln Gly Pro Gly Gln Gln Gly Pro Tyr Gly Pro Gly
                565                 570                 575

Ala Ser Ala Ala Ala Ala Ala Ala Gly Gly Tyr Gly Pro Gly Ser Gly
            580                 585                 590

Gln Gln Gly Pro Ser Gln Gln Gly Pro Gly Gln Gln Gly Pro Gly Gly
        595                 600                 605

Gln Gly Pro Tyr Gly Pro Gly Ala Ser Ala Ala Ala Ala Ala Ala Gly
    610                 615                 620

Gly Tyr Gly Pro Gly Ser Gly Gln Gln Gly Pro Gly Gly Gln Gly Pro
625                 630                 635                 640

Tyr Gly Pro Gly Ser Ser Ala Ala Ala Ala Ala Ala Gly Gly Asn Gly
            645                 650                 655

Pro Gly Ser Gly Gln Gln Gly Ala Gly Gln Gln Gly Pro Gly Gln Gln
            660                 665                 670

Gly Pro Gly Ala Ser Ala Ala Ala Ala Ala Ala Gly Gly Tyr Gly Pro
        675                 680                 685

Gly Ser Gly Gln Gln Gly Pro Gly Gln Gln Gly Pro Gly Gly Gln Gly
        690                 695                 700

Pro Tyr Gly Pro Gly Ala Ser Ala Ala Ala Ala Ala Ala Gly Gly Tyr

705                  710                  715                  720

Gly Pro Gly Ser Gly Gln Gly Pro Gly Gln Gln Gly Pro Gly Gly Gln
            725                  730                  735

Gly Pro Tyr Gly Pro Gly Ala Ser Ala Ala Ala Ala Ala Ala Gly Gly
            740                  745                  750

Tyr Gly Pro Gly Ser Gly Gln Gln Gly Pro Gly Gln Gln Gly Pro Gly
            755                  760                  765

Gln Gln Gly Pro Gly Gly Gln Gly Pro Tyr Gly Pro Gly Ala Ser Ala
    770                  775                  780

Ala Ala Ala Ala Ala Gly Gly Tyr Gly Pro Gly Tyr Gly Gln Gln Gly
785                  790                  795                  800

Pro Gly Gln Gln Gly Pro Gly Gly Gln Gly Pro Tyr Gly Pro Gly Ala
            805                  810                  815

Ser Ala Ala Ser Ala Ala Ser Gly Gly Tyr Gly Pro Gly Ser Gly Gln
            820                  825                  830

Gln Gly Pro Gly Gln Gln Gly Pro Gly Gly Gln Gly Pro Tyr Gly Pro
            835                  840                  845

Gly Ala Ser Ala Ala Ala Ala Ala Ala Gly Gly Tyr Gly Pro Gly Ser
    850                  855                  860

Gly Gln Gln Gly Pro Gly Gln Gln Gly Pro Gly Gln Gln Gly Pro Gly
865                  870                  875                  880

Gln Gln Gly Pro Gly Gly Gln Gly Pro Tyr Gly Pro Gly Ala Ser Ala
            885                  890                  895

Ala Ala Ala Ala Gly Gly Tyr Gly Pro Gly Ser Gly Gln Gln Gly
            900                  905                  910

Pro Gly Gln Gln Gly Pro Gly Gln Gln Gly Pro Gly Gln Gln Gly Pro
            915                  920                  925

Gly Gln Gln Gly Pro Gly Gln Gln Gly Pro Gly Gln Gln Gly Pro Gly
    930                  935                  940

Gln Gln Gly Pro Gly Gln Gln Gly Pro Gly Gly Gln Gly Ala Tyr Gly
945                  950                  955                  960

```
Pro Gly Ala Ser Ala Ala Ala Gly Ala Ala Gly Gly Tyr Gly Pro Gly
             965                 970                 975


Ser Gly Gln Gln Gly Pro Gly Gln Gln Gly Pro Gly Gln Gln Gly Pro
             980                 985                 990


Gly Gln Gln Gly Pro Gly Gln Gln  Gly Pro Gly Gln Gln  Gly Pro Gly
         995                 1000                 1005


Gln Gln  Gly Pro Gly Gln Gln  Gly Pro Tyr Gly Pro  Gly Ala Ser
    1010                 1015                 1020


Ala Ala  Ala Ala Ala Ala Gly  Gly Tyr Gly Pro Gly  Ser Gly Gln
    1025                 1030                 1035


Gln Gly  Pro Gly Gln Gln Gly  Pro Gly Gln Gln Gly  Pro Gly Gly
    1040                 1045                 1050


Gln Gly  Pro Tyr Gly Pro Gly  Ala Ala Ser Ala Ala  Val Ser Val
    1055                 1060                 1065


Gly Gly  Tyr Gly Pro Gln Ser  Ser Ser Val Pro Val  Ala Ser Ala
    1070                 1075                 1080


Val Ala  Ser Arg Leu Ser Ser  Pro Ala Ala Ser Ser  Arg Val Ser
    1085                 1090                 1095


Ser Ala  Val Ser Ser Leu Val  Ser Ser Gly Pro Thr  Lys His Ala
    1100                 1105                 1110


Ala Leu  Ser Asn Thr Ile Ser  Ser Val Val Ser Gln  Val Ser Ala
    1115                 1120                 1125


Ser Asn  Pro Gly Leu Ser Gly  Cys Asp Val Leu Val  Gln Ala Leu
    1130                 1135                 1140


Leu Glu  Val Val Ser Ala Leu  Val Ser Ile Leu
    1145                 1150
```

```
<210>  5
<211>  24
<212>  PRT
<213>  Artificial Sequence

<220>
<223>  His tag and start codon

<400>  5
```

```
Met His His His His His His His His His Ser Ser Gly Ser Ser
```

Leu Glu Val Leu Phe Gln Gly Pro
            20


<210>   6
<211>   597
<212>   PRT
<213>   Artificial Sequence

<220>
<223>   Met-PRT380

<400>   6

Met Gly Pro Gly Gln Gln Gly Pro Tyr Gly Pro Gly Ala Ser Ala Ala
1                   5                   10                  15


Ala Ala Ala Gly Gln Asn Gly Pro Gly Ser Gly Gln Gln Gly Pro Gly
            20                  25                  30


Gln Ser Ala Ala Ala Ala Ala Gly Gln Tyr Gly Pro Gly Gln Gln Gly
            35                  40                  45


Pro Gly Gln Gln Gly Pro Gly Ser Ser Ala Ala Ala Ala Ala Gly Pro
    50                  55                  60


Gly Gln Tyr Gly Pro Gly Gln Gln Gly Pro Ser Ala Ser Ala Ala Ala
65                  70                  75                  80


Ala Ala Gly Pro Gly Ser Gly Gln Gln Gly Pro Gly Ala Ser Ala Ala
                85                  90                  95


Ala Ala Ala Gly Gln Tyr Gly Pro Gly Gln Gln Gly Pro Gly Gln Gln
            100                 105                 110


Gly Pro Gly Ser Ser Ala Ala Ala Ala Ala Gly Gln Tyr Gly Ser Gly
            115                 120                 125


Pro Gly Gln Gln Gly Pro Tyr Gly Ser Ala Ala Ala Ala Ala Gly Pro
    130                 135                 140


Gly Ser Gly Gln Tyr Gly Gln Gly Pro Tyr Gly Pro Gly Ala Ser Ala
145                 150                 155                 160


Ala Ala Ala Gly Pro Gly Gln Tyr Gly Pro Gly Gln Gln Gly Pro
                165                 170                 175


Ser Ala Ser Ala Ala Ala Ala Ala Gly Ser Gly Gln Gln Gly Pro Gly
            180                 185                 190

51

```
Gln Tyr Gly Pro Tyr Ala Ser Ala Ala Ala Ala Gly Gln Tyr Gly
        195                 200             205

Ser Gly Pro Gly Gln Gln Gly Pro Tyr Gly Pro Gly Gln Ser Ala Ala
        210             215                 220

Ala Ala Ala Gly Ser Gly Gln Gln Gly Pro Gly Gln Gln Gly Pro Tyr
225             230                 235                 240

Ala Ser Ala Ala Ala Ala Ala Gly Pro Gly Gln Gln Gly Pro Tyr Gly
            245             250                 255

Pro Gly Ser Ser Ala Ala Ala Ala Gly Gln Tyr Gly Tyr Gly Pro
        260             265                 270

Gly Gln Gln Gly Pro Tyr Gly Pro Gly Ala Ser Ala Ala Ala Ala Ala
        275             280                 285

Gly Gln Asn Gly Pro Gly Ser Gly Gln Tyr Gly Pro Gly Gln Gln Gly
        290             295                 300

Pro Gly Gln Ser Ala Ala Ala Ala Gly Pro Gly Gln Gln Gly Pro
305             310                 315                 320

Tyr Gly Pro Gly Ala Ser Ala Ala Ala Ala Gly Gln Tyr Gly Pro
            325             330                 335

Gly Gln Gln Gly Pro Gly Gln Tyr Gly Pro Gly Ser Ser Ala Ala Ala
        340             345                 350

Ala Ala Gly Pro Gly Gln Gln Gly Pro Tyr Gly Pro Gly Ser Ser Ala
        355             360                 365

Ala Ala Ala Gly Gln Tyr Gly Pro Gly Gln Gln Gly Pro Tyr Gly
370             375                 380

Pro Gly Gln Ser Ala Ala Ala Ala Gly Gln Tyr Gln Gln Gly Pro
385             390                 395                 400

Gly Gln Gln Gly Pro Tyr Gly Pro Gly Ala Ser Ala Ala Ala Ala Ala
            405                 410                 415

Gly Pro Gly Gln Gln Gly Pro Tyr Gly Pro Gly Ala Ser Ala Ala Ala
            420                 425                 430

Ala Ala Gly Pro Gly Gln Tyr Gly Pro Gly Gln Gln Gly Pro Ser Ala
```

```
                    435                   440                   445


        Ser Ala Ala Ala Ala Ala Gly Gln Tyr Gly Ser Gly Pro Gly Gln Tyr
            450                   455                   460


        Gly Pro Tyr Gly Pro Gly Gln Ser Ala Ala Ala Ala Gly Pro Gly
        465                   470                   475                   480


        Ser Gly Gln Gln Gly Gln Gly Pro Tyr Gly Pro Gly Ala Ser Ala Ala
                            485                   490                   495


        Ala Ala Ala Gly Gln Tyr Gly Pro Gly Gln Gln Gly Pro Tyr Gly Pro
                        500                   505                   510


        Gly Gln Ser Ala Ala Ala Ala Ala Gly Pro Gly Ser Gly Gln Tyr Gly
                    515                   520                   525


        Pro Gly Ala Ser Ala Ala Ala Ala Ala Gly Gln Asn Gly Pro Gly Ser
                    530                   535                   540


        Gly Gln Tyr Gly Pro Gly Gln Gln Gly Pro Gly Gln Ser Ala Ala Ala
        545                   550                   555                   560


        Ala Ala Gly Gln Tyr Gln Gln Gly Pro Gly Gln Gln Gly Pro Tyr Gly
                            565                   570                   575


        Pro Gly Ala Ser Ala Ala Ala Ala Ala Gly Pro Gly Ser Gly Gln Gln
                    580                   585                   590


        Gly Pro Gly Ala Ser
                595


        <210>   7
        <211>   590
        <212>   PRT
        <213>   Artificial Sequence

        <220>
        <223>   Met-PRT410

        <400>   7

        Met Gly Pro Gly Gln Gln Gly Pro Tyr Gly Pro Gly Ala Ser Ala Ala
        1                   5                   10                  15


        Ala Ala Ala Gly Gln Asn Gly Pro Gly Ser Gly Gln Gln Gly Pro Gly
                    20                  25                  30


        Gln Ser Gly Gln Tyr Gly Pro Gly Gln Gln Gly Pro Gly Gln Gln Gly
                35                  40                  45
```

Pro Gly Ser Ser Ala Ala Ala Ala Ala Gly Pro Gly Gln Tyr Gly Pro
        50                  55                  60

Gly Gln Gln Gly Pro Ser Ala Ser Ala Ala Ala Ala Gly Pro Gly
65              70                  75                  80

Ser Gly Gln Gln Gly Pro Gly Ala Ser Gly Gln Tyr Gly Pro Gly Gln
                85                  90                  95

Gln Gly Pro Gly Gln Gln Gly Pro Gly Ser Ser Ala Ala Ala Ala Ala
            100                 105                 110

Gly Gln Tyr Gly Ser Gly Pro Gly Gln Gln Gly Pro Tyr Gly Ser Ala
        115                 120                 125

Ala Ala Ala Ala Gly Pro Gly Ser Gly Gln Tyr Gly Gln Gly Pro Tyr
    130                 135                 140

Gly Pro Gly Ala Ser Gly Pro Gly Gln Tyr Gly Pro Gly Gln Gln Gly
145                 150                 155                 160

Pro Ser Ala Ser Ala Ala Ala Ala Ala Gly Ser Gly Gln Gln Gly Pro
            165                 170                 175

Gly Gln Tyr Gly Pro Tyr Ala Ser Ala Ala Ala Ala Gly Gln Tyr
        180                 185                 190

Gly Ser Gly Pro Gly Gln Gln Gly Pro Tyr Gly Pro Gly Gln Ser Gly
        195                 200                 205

Ser Gly Gln Gln Gly Pro Gly Gln Gln Gly Pro Tyr Ala Ser Ala Ala
    210                 215                 220

Ala Ala Ala Gly Pro Gly Gln Gln Gly Pro Tyr Gly Pro Gly Ser Ser
225                 230                 235                 240

Ala Ala Ala Ala Ala Gly Gln Tyr Gly Tyr Gly Pro Gly Gln Gln Gly
                245                 250                 255

Pro Tyr Gly Pro Gly Ala Ser Gly Gln Asn Gly Pro Gly Ser Gly Gln
        260                 265                 270

Tyr Gly Pro Gly Gln Gln Gly Pro Gly Gln Ser Ala Ala Ala Ala Ala
        275                 280                 285

Gly Pro Gly Gln Gln Gly Pro Tyr Gly Pro Gly Ala Ser Ala Ala Ala

```
                  290                        295                        300


         Ala Ala Gly Gln Tyr Gly Pro Gly Gln Gln Gly Pro Gly Gln Tyr Gly
         305                 310                 315                 320


         Pro Gly Ser Ser Gly Pro Gly Gln Gln Gly Pro Tyr Gly Pro Gly Ser
                         325                 330                 335


         Ser Ala Ala Ala Ala Ala Gly Gln Tyr Gly Pro Gly Gln Gln Gly Pro
                     340                 345                 350


         Tyr Gly Pro Gly Gln Ser Ala Ala Ala Ala Ala Gly Gln Tyr Gln Gln
                     355                 360                 365


         Gly Pro Gly Gln Gln Gly Pro Tyr Gly Pro Gly Ala Ser Gly Pro Gly
                     370                 375                 380


         Gln Gln Gly Pro Tyr Gly Pro Gly Ala Ser Ala Ala Ala Ala Ala Gly
         385                 390                 395                 400


         Pro Gly Gln Tyr Gly Pro Gly Gln Gln Gly Pro Ser Ala Ser Ala Ala
                     405                 410                 415


         Ala Ala Ala Gly Gln Tyr Gly Ser Gly Pro Gly Gln Tyr Gly Pro Tyr
                     420                 425                 430


         Gly Pro Gly Gln Ser Gly Pro Gly Ser Gly Gln Gln Gly Gln Gly Pro
                     435                 440                 445


         Tyr Gly Pro Gly Ala Ser Ala Ala Ala Ala Gly Gln Tyr Gly Pro
                     450                 455                 460


         Gly Gln Gln Gly Pro Tyr Gly Pro Gly Gln Ser Ala Ala Ala Ala Ala
         465                 470                 475                 480


         Gly Pro Gly Ser Gly Gln Tyr Gly Pro Gly Ala Ser Gly Gln Asn Gly
                     485                 490                 495


         Pro Gly Ser Gly Gln Tyr Gly Pro Gly Gln Gln Gly Pro Gly Gln Ser
                     500                 505                 510


         Ala Ala Ala Ala Ala Gly Gln Tyr Gln Gln Gly Pro Gly Gln Gln Gly
                     515                 520                 525


         Pro Tyr Gly Pro Gly Ala Ser Ala Ala Ala Ala Ala Gly Gln Tyr Gly
                     530                 535                 540
```

55

```
Ser Gly Pro Gly Gln Gln Gly Pro Tyr Gly Pro Gly Gln Ser Gly Ser
545                 550                 555                 560


Gly Gln Gln Gly Pro Gly Gln Gln Gly Pro Tyr Ala Ser Ala Ala Ala
                565                 570                 575


Ala Ala Gly Pro Gly Ser Gly Gln Gln Gly Pro Gly Ala Ser
            580                 585                 590
```

```
<210>  8
<211>  565
<212>  PRT
<213>  Artificial Sequence

<220>
<223>  Met-PRT525

<400>  8
```

```
Met Gly Pro Gly Gln Gln Gly Pro Tyr Gly Pro Gly Ala Ser Ala Ala
1                   5                   10                  15


Ala Ala Ala Ala Ala Gly Ser Asn Gly Pro Gly Ser Gly Gln Gln Gly
            20                  25                  30


Pro Gly Gln Ser Gly Gln Tyr Gly Pro Gly Gln Gln Gly Pro Gly Gln
        35                  40                  45


Gln Gly Pro Gly Ser Ser Ala Ala Ala Ala Ala Ala Gly Pro Gly
        50                  55                  60


Gln Tyr Gly Pro Gly Gln Gln Gly Pro Ser Ala Ser Ala Ala Ala Ala
65                  70                  75                  80


Ala Ala Ala Gly Pro Gly Ser Gly Gln Gln Gly Pro Gly Ala Ser Gly
            85                  90                  95


Gln Tyr Gly Pro Gly Gln Gln Gly Pro Gly Gln Gln Gly Pro Gly Ser
        100                 105                 110


Ser Ala Ala Ala Ala Ala Ala Ala Gly Ser Tyr Gly Ser Gly Pro Gly
        115                 120                 125


Gln Gln Gly Pro Tyr Gly Ser Ala Ala Ala Ala Ala Ala Gly Pro
        130                 135                 140


Gly Ser Gly Gln Tyr Gly Gln Gly Pro Tyr Gly Pro Gly Ala Ser Gly
145                 150                 155                 160


Pro Gly Gln Tyr Gly Pro Gly Gln Gln Gly Pro Ser Ala Ser Ala Ala
```

<div align="center">165              170              175</div>

```
Ala Ala Ala Ala Ala Gly Ser Gly Gln Gln Gly Pro Gly Gln Tyr Gly
            180                 185                 190

Pro Tyr Ala Ser Ala Ala Ala Ala Ala Ala Ala Gly Ser Tyr Gly Ser
            195                 200                 205

Gly Pro Gly Gln Gln Gly Pro Tyr Gly Pro Gly Gln Ser Gly Ser Gly
        210                 215                 220

Gln Gln Gly Pro Gly Gln Gln Gly Pro Tyr Ala Ser Ala Ala Ala Ala
225                 230                 235                 240

Ala Ala Ala Gly Pro Gly Gln Gln Gly Pro Tyr Gly Pro Gly Ser Ser
            245                 250                 255

Ala Ala Ala Ala Ala Ala Ala Gly Ser Tyr Gly Tyr Gly Pro Gly Gln
            260                 265                 270

Gln Gly Pro Tyr Gly Pro Gly Ala Ser Gly Gln Asn Gly Pro Gly Ser
        275                 280                 285

Gly Gln Tyr Gly Pro Gly Gln Gln Gly Pro Gly Pro Ser Ala Ala Ala
        290                 295                 300

Ala Ala Ala Ala Gly Pro Gly Gln Gln Gly Pro Tyr Gly Pro Gly Ala
305                 310                 315                 320

Ser Ala Ala Ala Ala Ala Ala Ala Gly Ser Tyr Gly Pro Gly Gln Gln
            325                 330                 335

Gly Pro Gly Gln Tyr Gly Pro Gly Ser Ser Gly Pro Gly Gln Gln Gly
        340                 345                 350

Pro Tyr Gly Pro Gly Ser Ser Ala Ala Ala Ala Ala Ala Gly Ser
        355                 360                 365

Tyr Gly Pro Gly Gln Gln Gly Pro Tyr Gly Pro Gly Pro Ser Ala Ala
        370                 375                 380

Ala Ala Ala Ala Ala Gly Ser Tyr Gln Gln Gly Pro Gly Gln Gln Gly
385                 390                 395                 400

Pro Tyr Gly Pro Gly Ala Ser Gly Pro Gly Gln Gln Gly Pro Tyr Gly
        405                 410                 415
```

Pro Gly Ala Ser Ala Ala Ala Ala Ala Ala Ala Gly Pro Gly Gln Tyr
420 425 430

Gly Pro Gly Gln Gln Gly Pro Ser Ala Ser Ala Ala Ala Ala Ala Ala
435 440 445

Ala Gly Ser Tyr Gly Ser Gly Pro Gly Gln Tyr Gly Pro Tyr Gly Pro
450 455 460

Gly Gln Ser Gly Pro Gly Ser Gly Gln Gln Gly Gln Gly Pro Tyr Gly
465 470 475 480

Pro Gly Ala Ser Ala Ala Ala Ala Ala Ala Gly Ser Tyr Gly Pro
485 490 495

Gly Gln Gln Gly Pro Tyr Gly Pro Gly Pro Ser Ala Ala Ala Ala Ala
500 505 510

Ala Ala Gly Pro Gly Ser Gly Gln Tyr Gly Pro Gly Ala Ser Gly Gln
515 520 525

Asn Gly Pro Gly Ser Gly Gln Tyr Gly Pro Gly Gln Gln Gly Pro Gly
530 535 540

Pro Ser Ala Ala Ala Ala Ala Ala Ala Gly Pro Gly Ser Gly Gln Gln
545 550 555 560

Gly Pro Gly Ala Ser
565

<210> 9
<211> 2364
<212> PRT
<213> Artificial Sequence

<220>
<223> Met-PRT799

<400> 9

Met Gly Pro Gly Gln Gln Gly Pro Tyr Gly Pro Gly Ala Ser Ala Ala
1 5 10 15

Ala Ala Ala Gly Gln Asn Gly Pro Gly Ser Gly Gln Gln Gly Pro Gly
20 25 30

Gln Ser Gly Gln Tyr Gly Pro Gly Gln Gln Gly Pro Gly Gln Gln Gly
35 40 45

Pro Gly Ser Ser Ala Ala Ala Ala Ala Gly Pro Gly Gln Tyr Gly Pro

```
              50                        55                        60

    Gly Gln Gln Gly Pro Ser Ala Ser Ala Ala Ala Ala Ala Gly Pro Gly
    65                  70              75                      80


    Ser Gly Gln Gln Gly Pro Gly Ala Ser Gly Gln Tyr Gly Pro Gly Gln
                    85              90                  95


    Gln Gly Pro Gly Gln Gln Gly Pro Gly Ser Ser Ala Ala Ala Ala Ala
                100             105             110


    Gly Gln Tyr Gly Ser Gly Pro Gly Gln Gln Gly Pro Tyr Gly Ser Ala
                115             120             125


    Ala Ala Ala Gly Pro Gly Ser Gly Gln Tyr Gly Gln Gly Pro Tyr
        130             135             140


    Gly Pro Gly Ala Ser Gly Pro Gly Gln Tyr Gly Pro Gly Gln Gln Gly
    145             150             155                     160


    Pro Ser Ala Ser Ala Ala Ala Ala Ala Gly Ser Gly Gln Gln Gly Pro
                165             170                     175


    Gly Gln Tyr Gly Pro Tyr Ala Ser Ala Ala Ala Ala Gly Gln Tyr
                180             185                 190


    Gly Ser Gly Pro Gly Gln Gln Gly Pro Tyr Gly Pro Gly Gln Ser Gly
                195             200             205


    Ser Gly Gln Gln Gly Pro Gly Gln Gln Gly Pro Tyr Ala Ser Ala Ala
                210             215             220


    Ala Ala Ala Gly Pro Gly Gln Gln Gly Pro Tyr Gly Pro Gly Ser Ser
    225             230             235                     240


    Ala Ala Ala Ala Ala Gly Gln Tyr Gly Tyr Gly Pro Gly Gln Gln Gly
                245             250                     255


    Pro Tyr Gly Pro Gly Ala Ser Gly Gln Asn Gly Pro Gly Ser Gly Gln
                260             265                 270


    Tyr Gly Pro Gly Gln Gln Gly Pro Gly Gln Ser Ala Ala Ala Ala Ala
                275             280                 285


    Gly Pro Gly Gln Gln Gly Pro Tyr Gly Pro Gly Ala Ser Ala Ala Ala
                290             295                 300
```

59

```
Ala Ala Gly Gln Tyr Gly Pro Gly Gln Gln Gly Pro Gly Gln Tyr Gly
305             310             315             320

Pro Gly Ser Ser Gly Pro Gly Gln Gln Gly Pro Tyr Gly Pro Gly Ser
            325             330             335

Ser Ala Ala Ala Ala Ala Gly Gln Tyr Gly Pro Gly Gln Gln Gly Pro
        340             345             350

Tyr Gly Pro Gly Gln Ser Ala Ala Ala Ala Gly Gln Tyr Gln Gln
        355             360             365

Gly Pro Gly Gln Gln Gly Pro Tyr Gly Pro Gly Ala Ser Gly Pro Gly
        370             375             380

Gln Gln Gly Pro Tyr Gly Pro Gly Ala Ser Ala Ala Ala Ala Gly
385             390             395             400

Pro Gly Gln Tyr Gly Pro Gly Gln Gln Gly Pro Ser Ala Ser Ala Ala
            405             410             415

Ala Ala Ala Gly Gln Tyr Gly Ser Gly Pro Gly Gln Tyr Gly Pro Tyr
        420             425             430

Gly Pro Gly Gln Ser Gly Pro Gly Ser Gly Gln Gln Gly Gln Gly Pro
        435             440             445

Tyr Gly Pro Gly Ala Ser Ala Ala Ala Ala Gly Gln Tyr Gly Pro
        450             455             460

Gly Gln Gln Gly Pro Tyr Gly Pro Gly Gln Ser Ala Ala Ala Ala Ala
465             470             475             480

Gly Pro Gly Ser Gly Gln Tyr Gly Pro Gly Ala Ser Gly Gln Asn Gly
            485             490             495

Pro Gly Ser Gly Gln Tyr Gly Pro Gly Gln Gln Gly Pro Gly Gln Ser
            500             505             510

Ala Ala Ala Ala Ala Gly Gln Tyr Gln Gln Gly Pro Gly Gln Gln Gly
        515             520             525

Pro Tyr Gly Pro Gly Ala Ser Ala Ala Ala Ala Ala Gly Gln Tyr Gly
        530             535             540

Ser Gly Pro Gly Gln Gln Gly Pro Tyr Gly Pro Gly Gln Ser Gly Ser
545             550             555             560
```

60

Gly Gln Gln Gly Pro Gly Gln Gln Gly Pro Tyr Ala Ser Ala Ala Ala
                565             570             575

Ala Ala Gly Pro Gly Ser Gly Gln Gln Gly Pro Gly Ala Ser Gly Gln
            580             585             590

Gln Gly Pro Tyr Gly Pro Gly Ala Ser Ala Ala Ala Ala Ala Gly Gln
        595             600             605

Asn Gly Pro Gly Ser Gly Gln Gln Gly Pro Gly Gln Ser Gly Gln Tyr
    610             615             620

Gly Pro Gly Gln Gln Gly Pro Gly Gln Gln Gly Pro Gly Ser Ser Ala
625             630             635             640

Ala Ala Ala Ala Gly Pro Gly Gln Tyr Gly Pro Gly Gln Gln Gly Pro
            645             650             655

Ser Ala Ser Ala Ala Ala Ala Ala Gly Pro Gly Ser Gly Gln Gln Gly
        660             665             670

Pro Gly Ala Ser Gly Gln Tyr Gly Pro Gly Gln Gln Gly Pro Gly Gln
        675             680             685

Gln Gly Pro Gly Ser Ser Ala Ala Ala Ala Gly Gln Tyr Gly Ser
    690             695             700

Gly Pro Gly Gln Gln Gly Pro Tyr Gly Ser Ala Ala Ala Ala Gly
705             710             715             720

Pro Gly Ser Gly Gln Tyr Gly Gln Gly Pro Tyr Gly Pro Gly Ala Ser
            725             730             735

Gly Pro Gly Gln Tyr Gly Pro Gly Gln Gln Gly Pro Ser Ala Ser Ala
            740             745             750

Ala Ala Ala Ala Gly Ser Gly Gln Gln Gly Pro Gly Gln Tyr Gly Pro
    755             760             765

Tyr Ala Ser Ala Ala Ala Ala Ala Gly Gln Tyr Gly Ser Gly Pro Gly
    770             775             780

Gln Gln Gly Pro Tyr Gly Pro Gly Gln Ser Gly Ser Gly Gln Gln Gly
785             790             795             800

Pro Gly Gln Gln Gly Pro Tyr Ala Ser Ala Ala Ala Ala Gly Pro
            805             810             815

61

Gly Gln Gln Gly Pro Tyr Gly Pro Gly Ser Ser Ala Ala Ala Ala Ala
            820              825              830

Gly Gln Tyr Gly Tyr Gly Pro Gly Gln Gln Gly Pro Tyr Gly Pro Gly
            835              840              845

Ala Ser Gly Gln Asn Gly Pro Gly Ser Gly Gln Tyr Gly Pro Gly Gln
    850              855              860

Gln Gly Pro Gly Gln Ser Ala Ala Ala Ala Gly Pro Gly Gln Gln
865              870              875              880

Gly Pro Tyr Gly Pro Gly Ala Ser Ala Ala Ala Ala Gly Gln Tyr
            885              890              895

Gly Pro Gly Gln Gln Gly Pro Gly Gln Tyr Gly Pro Gly Ser Ser Gly
            900              905              910

Pro Gly Gln Gln Gly Pro Tyr Gly Pro Gly Ser Ser Ala Ala Ala Ala
            915              920              925

Ala Gly Gln Tyr Gly Pro Gly Gln Gln Gly Pro Tyr Gly Pro Gly Gln
    930              935              940

Ser Ala Ala Ala Ala Ala Gly Gln Tyr Gln Gln Gly Pro Gly Gln Gln
945              950              955              960

Gly Pro Tyr Gly Pro Gly Ala Ser Gly Pro Gly Gln Gln Gly Pro Tyr
            965              970              975

Gly Pro Gly Ala Ser Ala Ala Ala Ala Ala Gly Pro Gly Gln Tyr Gly
            980              985              990

Pro Gly Gln Gln Gly Pro Ser Ala Ser Ala Ala Ala Ala Ala Gly Gln
            995              1000             1005

Tyr Gly Ser Gly Pro Gly Gln Tyr Gly Pro Tyr Gly Pro Gly Gln
    1010             1015             1020

Ser Gly Pro Gly Ser Gly Gln Gln Gly Gln Gly Pro Tyr Gly Pro
    1025             1030             1035

Gly Ala Ser Ala Ala Ala Ala Ala Gly Gln Tyr Gly Pro Gly Gln
    1040             1045             1050

Gln Gly Pro Tyr Gly Pro Gly Gln Ser Ala Ala Ala Ala Ala Gly

```
        1055                      1060                            1065


        Pro Gly  Ser Gly Gln Tyr Gly  Pro Gly Ala Ser Gly  Gln Asn Gly
             1070                 1075                 1080


        Pro Gly  Ser Gly Gln Tyr Gly  Pro Gly Gln Gln Gly  Pro Gly Gln
             1085                 1090                 1095


        Ser Ala  Ala Ala Ala Ala Gly  Gln Tyr Gln Gln Gly  Pro Gly Gln
             1100                 1105                 1110


        Gln Gly  Pro Tyr Gly Pro Gly  Ala Ser Ala Ala Ala  Ala Ala Gly
             1115                 1120                 1125


        Gln Tyr  Gly Ser Gly Pro Gly  Gln Gln Gly Pro Tyr  Gly Pro Gly
             1130                 1135                 1140


        Gln Ser  Gly Ser Gly Gln Gln  Gly Pro Gly Gln Gln  Gly Pro Tyr
             1145                 1150                 1155


        Ala Ser  Ala Ala Ala Ala Ala  Gly Pro Gly Ser Gly  Gln Gln Gly
             1160                 1165                 1170


        Pro Gly  Ala Ser Gly Gln Gln  Gly Pro Tyr Gly Pro  Gly Ala Ser
             1175                 1180                 1185


        Ala Ala  Ala Ala Ala Gly Gln  Asn Gly Pro Gly Ser  Gly Gln Gln
             1190                 1195                 1200


        Gly Pro  Gly Gln Ser Gly Gln  Tyr Gly Pro Gly Gln  Gln Gly Pro
             1205                 1210                 1215


        Gly Gln  Gln Gly Pro Gly Ser  Ser Ala Ala Ala Ala  Ala Gly Pro
             1220                 1225                 1230


        Gly Gln  Tyr Gly Pro Gly Gln  Gln Gly Pro Ser Ala  Ser Ala Ala
             1235                 1240                 1245


        Ala Ala  Ala Gly Pro Gly Ser  Gly Gln Gln Gly Pro  Gly Ala Ser
             1250                 1255                 1260


        Gly Gln  Tyr Gly Pro Gly Gln  Gln Gly Pro Gly Gln  Gln Gly Pro
             1265                 1270                 1275


        Gly Ser  Ser Ala Ala Ala Ala  Ala Gly Gln Tyr Gly  Ser Gly Pro
             1280                 1285                 1290
```

Gly Gln Gln Gly Pro Tyr Gly Ser Ala Ala Ala Ala Ala Gly Pro
1295 1300 1305

Gly Ser Gly Gln Tyr Gly Gln Gly Pro Tyr Gly Pro Gly Ala Ser
1310 1315 1320

Gly Pro Gly Gln Tyr Gly Pro Gly Gln Gln Gly Pro Ser Ala Ser
1325 1330 1335

Ala Ala Ala Ala Ala Gly Ser Gly Gln Gln Gly Pro Gly Gln Tyr
1340 1345 1350

Gly Pro Tyr Ala Ser Ala Ala Ala Ala Ala Gly Gln Tyr Gly Ser
1355 1360 1365

Gly Pro Gly Gln Gln Gly Pro Tyr Gly Pro Gly Gln Ser Gly Ser
1370 1375 1380

Gly Gln Gln Gly Pro Gly Gln Gln Gly Pro Tyr Ala Ser Ala Ala
1385 1390 1395

Ala Ala Ala Gly Pro Gly Gln Gln Gly Pro Tyr Gly Pro Gly Ser
1400 1405 1410

Ser Ala Ala Ala Ala Ala Gly Gln Tyr Gly Tyr Gly Pro Gly Gln
1415 1420 1425

Gln Gly Pro Tyr Gly Pro Gly Ala Ser Gly Gln Asn Gly Pro Gly
1430 1435 1440

Ser Gly Gln Tyr Gly Pro Gly Gln Gln Gly Pro Gly Gln Ser Ala
1445 1450 1455

Ala Ala Ala Ala Gly Pro Gly Gln Gln Gly Pro Tyr Gly Pro Gly
1460 1465 1470

Ala Ser Ala Ala Ala Ala Ala Gly Gln Tyr Gly Pro Gly Gln Gln
1475 1480 1485

Gly Pro Gly Gln Tyr Gly Pro Gly Ser Ser Gly Pro Gly Gln Gln
1490 1495 1500

Gly Pro Tyr Gly Pro Gly Ser Ser Ala Ala Ala Ala Ala Gly Gln
1505 1510 1515

Tyr Gly Pro Gly Gln Gln Gly Pro Tyr Gly Pro Gly Gln Ser Ala
1520 1525 1530

```
Ala Ala   Ala Ala Gly Gln Tyr   Gln Gln Gly Pro Gly   Gln Gln Gly
    1535                  1540                  1545

Pro Tyr   Gly Pro Gly Ala Ser   Gly Pro Gly Gln Gln   Gly Pro Tyr
    1550                  1555                  1560

Gly Pro   Gly Ala Ser Ala Ala   Ala Ala Ala Gly Pro   Gly Gln Tyr
    1565                  1570                  1575

Gly Pro   Gly Gln Gln Gly Pro   Ser Ala Ser Ala Ala   Ala Ala Ala
    1580                  1585                  1590

Gly Gln   Tyr Gly Ser Gly Pro   Gly Gln Tyr Gly Pro   Tyr Gly Pro
    1595                  1600                  1605

Gly Gln   Ser Gly Pro Gly Ser   Gly Gln Gln Gly Gln   Gly Pro Tyr
    1610                  1615                  1620

Gly Pro   Gly Ala Ser Ala Ala   Ala Ala Ala Gly Gln   Tyr Gly Pro
    1625                  1630                  1635

Gly Gln   Gln Gly Pro Tyr Gly   Pro Gly Gln Ser Ala   Ala Ala Ala
    1640                  1645                  1650

Ala Gly   Pro Gly Ser Gly Gln   Tyr Gly Pro Gly Ala   Ser Gly Gln
    1655                  1660                  1665

Asn Gly   Pro Gly Ser Gly Gln   Tyr Gly Pro Gly Gln   Gln Gly Pro
    1670                  1675                  1680

Gly Gln   Ser Ala Ala Ala Ala   Ala Gly Gln Tyr Gln   Gln Gly Pro
    1685                  1690                  1695

Gly Gln   Gln Gly Pro Tyr Gly   Pro Gly Ala Ser Ala   Ala Ala Ala
    1700                  1705                  1710

Ala Gly   Gln Tyr Gly Ser Gly   Pro Gly Gln Gln Gly   Pro Tyr Gly
    1715                  1720                  1725

Pro Gly   Gln Ser Gly Ser Gly   Gln Gln Gly Pro Gly   Gln Gln Gly
    1730                  1735                  1740

Pro Tyr   Ala Ser Ala Ala Ala   Ala Ala Gly Pro Gly   Ser Gly Gln
    1745                  1750                  1755

Gln Gly   Pro Gly Ala Ser Gly   Gln Gln Gly Pro Tyr   Gly Pro Gly
    1760                  1765                  1770
```

```
Ala Ser  Ala Ala Ala Ala Ala  Gly Gln Asn Gly Pro  Gly Ser Gly
    1775                1780                1785


Gln Gln  Gly Pro Gly Gln Ser  Gly Gln Tyr Gly Pro  Gly Gln Gln
    1790                1795                1800


Gly Pro  Gly Gln Gln Gly Pro  Gly Ser Ser Ala Ala  Ala Ala Ala
    1805                1810                1815


Gly Pro  Gly Gln Tyr Gly Pro  Gly Gln Gln Gly Pro  Ser Ala Ser
    1820                1825                1830


Ala Ala  Ala Ala Ala Gly Pro  Gly Ser Gly Gln Gln  Gly Pro Gly
    1835                1840                1845


Ala Ser  Gly Gln Tyr Gly Pro  Gly Gln Gln Gly Pro  Gly Gln Gln
    1850                1855                1860


Gly Pro  Gly Ser Ser Ala Ala  Ala Ala Ala Gly Gln  Tyr Gly Ser
    1865                1870                1875


Gly Pro  Gly Gln Gln Gly Pro  Tyr Gly Ser Ala Ala  Ala Ala Ala
    1880                1885                1890


Gly Pro  Gly Ser Gly Gln Tyr  Gly Gln Gly Pro Tyr  Gly Pro Gly
    1895                1900                1905


Ala Ser  Gly Pro Gly Gln Tyr  Gly Pro Gly Gln Gln  Gly Pro Ser
    1910                1915                1920


Ala Ser  Ala Ala Ala Ala Ala  Gly Ser Gly Gln Gln  Gly Pro Gly
    1925                1930                1935


Gln Tyr  Gly Pro Tyr Ala Ser  Ala Ala Ala Ala Ala  Gly Gln Tyr
    1940                1945                1950


Gly Ser  Gly Pro Gly Gln Gln  Gly Pro Tyr Gly Pro  Gly Gln Ser
    1955                1960                1965


Gly Ser  Gly Gln Gln Gly Pro  Gly Gln Gln Gly Pro  Tyr Ala Ser
    1970                1975                1980


Ala Ala  Ala Ala Ala Gly Pro  Gly Gln Gln Gly Pro  Tyr Gly Pro
    1985                1990                1995


Gly Ser  Ser Ala Ala Ala Ala  Ala Gly Gln Tyr Gly  Tyr Gly Pro
```

66

```
                2000                        2005                         2010


        Gly Gln   Gln Gly Pro Tyr Gly   Pro Gly Ala Ser Gly   Gln Asn Gly
            2015                2020                2025


        Pro Gly   Ser Gly Gln Tyr Gly   Pro Gly Gln Gln Gly   Pro Gly Gln
            2030                2035                2040


        Ser Ala   Ala Ala Ala Ala Gly   Pro Gly Gln Gln Gly   Pro Tyr Gly
            2045                2050                2055


        Pro Gly   Ala Ser Ala Ala Ala   Ala Ala Gly Gln Tyr   Gly Pro Gly
            2060                2065                2070


        Gln Gln   Gly Pro Gly Gln Tyr   Gly Pro Gly Ser Ser   Gly Pro Gly
            2075                2080                2085


        Gln Gln   Gly Pro Tyr Gly Pro   Gly Ser Ser Ala Ala   Ala Ala Ala
            2090                2095                2100


        Gly Gln   Tyr Gly Pro Gly Gln   Gln Gly Pro Tyr Gly   Pro Gly Gln
            2105                2110                2115


        Ser Ala   Ala Ala Ala Ala Gly   Gln Tyr Gln Gln Gly   Pro Gly Gln
            2120                2125                2130


        Gln Gly   Pro Tyr Gly Pro Gly   Ala Ser Gly Pro Gly   Gln Gln Gly
            2135                2140                2145


        Pro Tyr   Gly Pro Gly Ala Ser   Ala Ala Ala Ala Ala   Gly Pro Gly
            2150                2155                2160


        Gln Tyr   Gly Pro Gly Gln Gln   Gly Pro Ser Ala Ser   Ala Ala Ala
            2165                2170                2175


        Ala Ala   Gly Gln Tyr Gly Ser   Gly Pro Gly Gln Tyr   Gly Pro Tyr
            2180                2185                2190


        Gly Pro   Gly Gln Ser Gly Pro   Gly Ser Gly Gln Gln   Gly Gln Gly
            2195                2200                2205


        Pro Tyr   Gly Pro Gly Ala Ser   Ala Ala Ala Ala Ala   Gly Gln Tyr
            2210                2215                2220


        Gly Pro   Gly Gln Gln Gly Pro   Tyr Gly Pro Gly Gln   Ser Ala Ala
            2225                2230                2235
```

```
Ala Ala  Ala Gly Pro Gly Ser  Gly Gln Tyr Gly Pro  Gly Ala Ser
    2240                 2245                 2250


Gly Gln  Asn Gly Pro Gly Ser  Gly Gln Tyr Gly Pro  Gly Gln Gln
    2255                 2260                 2265


Gly Pro  Gly Gln Ser Ala Ala  Ala Ala Ala Gly Gln  Tyr Gln Gln
    2270                 2275                 2280


Gly Pro  Gly Gln Gln Gly Pro  Tyr Gly Pro Gly Ala  Ser Ala Ala
    2285                 2290                 2295


Ala Ala  Ala Gly Gln Tyr Gly  Ser Gly Pro Gly Gln  Gln Gly Pro
    2300                 2305                 2310


Tyr Gly  Pro Gly Gln Ser Gly  Ser Gly Gln Gln Gly  Pro Gly Gln
    2315                 2320                 2325


Gln Gly  Pro Tyr Ala Ser Ala  Ala Ala Ala Gly  Pro Gly Ser
    2330                 2335                 2340


Gly Gln  Gln Gly Ser Ser Val  Asp Lys Leu Ala Ala  Ala Leu Glu
    2345                 2350                 2355


His His  His His His His
    2360


<210>  10
<211>  597
<212>  PRT
<213>  Artificial Sequence

<220>
<223>  Met-PRT313

<400>  10

Met Gly Pro Gly Gly Gln Gly Pro Tyr Gly Pro Gly Ala Ser Ala Ala
1                   5                   10                  15


Ala Ala Ala Gly Gly Asn Gly Pro Gly Ser Gly Gln Gln Gly Pro Gly
            20                  25                  30


Gly Ser Ala Ala Ala Ala Ala Gly Gly Tyr Gly Pro Gly Gly Gln Gly
            35                  40                  45


Pro Gly Gln Gln Gly Pro Gly Ser Ser Ala Ala Ala Ala Ala Gly Pro
    50                  55                  60


Gly Gly Tyr Gly Pro Gly Gly Gln Gly Pro Ser Ala Ser Ala Ala Ala
```

68

|  | 65 |  |  | 70 |  |  | 75 |  |  | 80 |
|---|---|---|---|---|---|---|---|---|---|---|

Ala Ala Gly Pro Gly Ser Gly Gln Gln Gly Pro Gly Ala Ser Ala Ala
85                          90                          95

Ala Ala Ala Gly Gly Tyr Gly Pro Gly Gly Gln Gly Pro Gly Gln Gln
100                        105                        110

Gly Pro Gly Ser Ser Ala Ala Ala Ala Ala Gly Gly Tyr Gly Ser Gly
115                        120                        125

Pro Gly Gln Gln Gly Pro Tyr Gly Ser Ala Ala Ala Ala Ala Gly Pro
130                        135                        140

Gly Ser Gly Gly Tyr Gly Gln Gly Pro Tyr Gly Pro Gly Ala Ser Ala
145                        150                        155                        160

Ala Ala Ala Ala Gly Pro Gly Gly Tyr Gly Pro Gly Gly Gln Gly Pro
165                        170                        175

Ser Ala Ser Ala Ala Ala Ala Ala Gly Ser Gly Gln Gln Gly Pro Gly
180                        185                        190

Gly Tyr Gly Pro Tyr Ala Ser Ala Ala Ala Ala Gly Gly Tyr Gly
195                        200                        205

Ser Gly Pro Gly Gln Gln Gly Pro Tyr Gly Pro Gly Gly Ser Ala Ala
210                        215                        220

Ala Ala Ala Gly Ser Gly Gln Gln Gly Pro Gly Gln Gln Gly Pro Tyr
225                        230                        235                        240

Ala Ser Ala Ala Ala Ala Ala Gly Pro Gly Gly Gln Gly Pro Tyr Gly
245                        250                        255

Pro Gly Ser Ser Ala Ala Ala Ala Ala Gly Gly Tyr Gly Tyr Gly Pro
260                        265                        270

Gly Gly Gln Gly Pro Tyr Gly Pro Gly Ala Ser Ala Ala Ala Ala Ala
275                        280                        285

Gly Gly Asn Gly Pro Gly Ser Gly Gly Tyr Gly Pro Gly Gln Gln Gly
290                        295                        300

Pro Gly Gly Ser Ala Ala Ala Ala Ala Gly Pro Gly Gly Gln Gly Pro
305                        310                        315                        320

```
Tyr Gly Pro Gly Ala Ser Ala Ala Ala Ala Ala Gly Gly Tyr Gly Pro
            325             330             335

Gly Gly Gln Gly Pro Gly Gly Tyr Gly Pro Gly Ser Ser Ala Ala Ala
            340             345             350

Ala Ala Gly Pro Gly Gly Gln Gly Pro Tyr Gly Pro Gly Ser Ser Ala
            355             360             365

Ala Ala Ala Gly Gly Tyr Gly Pro Gly Gln Gln Gly Pro Tyr Gly
370             375             380

Pro Gly Gly Ser Ala Ala Ala Ala Ala Gly Gly Tyr Gln Gln Gly Pro
385             390             395             400

Gly Gly Gln Gly Pro Tyr Gly Pro Gly Ala Ser Ala Ala Ala Ala Ala
            405             410             415

Gly Pro Gly Gly Gln Gly Pro Tyr Gly Pro Gly Ala Ser Ala Ala Ala
            420             425             430

Ala Ala Gly Pro Gly Gly Tyr Gly Pro Gly Gly Gln Gly Pro Ser Ala
            435             440             445

Ser Ala Ala Ala Ala Ala Gly Gly Tyr Gly Ser Gly Pro Gly Gly Tyr
    450             455             460

Gly Pro Tyr Gly Pro Gly Gly Ser Ala Ala Ala Ala Ala Gly Pro Gly
465             470             475             480

Ser Gly Gln Gln Gly Gln Gly Pro Tyr Gly Pro Gly Ala Ser Ala Ala
            485             490             495

Ala Ala Ala Gly Gly Tyr Gly Pro Gly Gln Gln Gly Pro Tyr Gly Pro
            500             505             510

Gly Gly Ser Ala Ala Ala Ala Ala Gly Pro Gly Ser Gly Gly Tyr Gly
            515             520             525

Pro Gly Ala Ser Ala Ala Ala Ala Gly Gly Asn Gly Pro Gly Ser
530             535             540

Gly Gly Tyr Gly Pro Gly Gln Gln Gly Pro Gly Gly Ser Ala Ala Ala
545             550             555             560

Ala Ala Gly Gly Tyr Gln Gln Gly Pro Gly Gly Gln Gly Pro Tyr Gly
            565             570             575
```

```
Pro Gly Ala Ser Ala Ala Ala Ala Ala Gly Pro Gly Ser Gly Gln Gln
            580                 585                 590

Gly Pro Gly Ala Ser
            595


<210>   11
<211>   12
<212>   PRT
<213>   Artificial Sequence

<220>
<223>   HisTag

<400>   11

Met His His His His His His Ser Ser Gly Ser Ser
1                   5                   10


<210>   12
<211>   608
<212>   PRT
<213>   Artificial Sequence

<220>
<223>   PRT380

<400>   12

Met His His His His His His Ser Ser Gly Ser Ser Gly Pro Gly Gln
1                   5                   10                  15

Gln Gly Pro Tyr Gly Pro Gly Ala Ser Ala Ala Ala Ala Ala Gly Gln
                20                  25                  30

Asn Gly Pro Gly Ser Gly Gln Gln Gly Pro Gly Gln Ser Ala Ala Ala
            35                  40                  45

Ala Ala Gly Gln Tyr Gly Pro Gly Gln Gln Gly Pro Gly Gln Gln Gly
        50                  55                  60

Pro Gly Ser Ser Ala Ala Ala Ala Ala Gly Pro Gly Gln Tyr Gly Pro
65                  70                  75                  80

Gly Gln Gln Gly Pro Ser Ala Ser Ala Ala Ala Ala Gly Pro Gly
                85                  90                  95

Ser Gly Gln Gln Gly Pro Gly Ala Ser Ala Ala Ala Ala Ala Gly Gln
            100                 105                 110

Tyr Gly Pro Gly Gln Gln Gly Pro Gly Gln Gln Gly Pro Gly Ser Ser
            115                 120                 125
```

```
Ala Ala Ala Ala Ala Gly Gln Tyr Gly Ser Gly Pro Gly Gln Gln Gly
    130                 135                 140

Pro Tyr Gly Ser Ala Ala Ala Ala Ala Gly Pro Gly Ser Gly Gln Tyr
145                 150                 155                 160

Gly Gln Gly Pro Tyr Gly Pro Gly Ala Ser Ala Ala Ala Ala Ala Gly
                165                 170                 175

Pro Gly Gln Tyr Gly Pro Gly Gln Gln Gly Pro Ser Ala Ser Ala Ala
            180                 185                 190

Ala Ala Ala Gly Ser Gly Gln Gln Gly Pro Gly Gln Tyr Gly Pro Tyr
            195                 200                 205

Ala Ser Ala Ala Ala Ala Ala Gly Gln Tyr Gly Ser Gly Pro Gly Gln
    210                 215                 220

Gln Gly Pro Tyr Gly Pro Gly Gln Ser Ala Ala Ala Ala Ala Gly Ser
225                 230                 235                 240

Gly Gln Gln Gly Pro Gly Gln Gln Gly Pro Tyr Ala Ser Ala Ala Ala
                245                 250                 255

Ala Ala Gly Pro Gly Gln Gln Gly Pro Tyr Gly Pro Gly Ser Ser Ala
            260                 265                 270

Ala Ala Ala Ala Gly Gln Tyr Gly Tyr Gly Pro Gly Gln Gln Gly Pro
    275                 280                 285

Tyr Gly Pro Gly Ala Ser Ala Ala Ala Ala Gly Gln Asn Gly Pro
    290                 295                 300

Gly Ser Gly Gln Tyr Gly Pro Gly Gln Gln Gly Pro Gly Gln Ser Ala
305                 310                 315                 320

Ala Ala Ala Gly Pro Gly Gln Gln Gly Pro Tyr Gly Pro Gly Ala
            325                 330                 335

Ser Ala Ala Ala Ala Ala Gly Gln Tyr Gly Pro Gly Gln Gln Gly Pro
        340                 345                 350

Gly Gln Tyr Gly Pro Gly Ser Ser Ala Ala Ala Ala Ala Gly Pro Gly
    355                 360                 365

Gln Gln Gly Pro Tyr Gly Pro Gly Ser Ser Ala Ala Ala Ala Ala Gly
```

370                              375                              380

Gln Tyr Gly Pro Gly Gln Gln Gly Pro Tyr Gly Pro Gly Gln Ser Ala
385               390               395               400

Ala Ala Ala Ala Gly Gln Tyr Gln Gln Gly Pro Gly Gln Gln Gly Pro
          405               410               415

Tyr Gly Pro Gly Ala Ser Ala Ala Ala Ala Ala Gly Pro Gly Gln Gln
          420               425               430

Gly Pro Tyr Gly Pro Gly Ala Ser Ala Ala Ala Ala Ala Gly Pro Gly
          435               440               445

Gln Tyr Gly Pro Gly Gln Gln Gly Pro Ser Ala Ser Ala Ala Ala Ala
          450               455               460

Ala Gly Gln Tyr Gly Ser Gly Pro Gly Gln Tyr Gly Pro Tyr Gly Pro
465               470               475               480

Gly Gln Ser Ala Ala Ala Ala Ala Gly Pro Gly Ser Gly Gln Gln Gly
          485               490               495

Gln Gly Pro Tyr Gly Pro Gly Ala Ser Ala Ala Ala Ala Ala Gly Gln
          500               505               510

Tyr Gly Pro Gly Gln Gln Gly Pro Tyr Gly Pro Gly Gln Ser Ala Ala
          515               520               525

Ala Ala Ala Gly Pro Gly Ser Gly Gln Tyr Gly Pro Gly Ala Ser Ala
          530               535               540

Ala Ala Ala Gly Gln Asn Gly Pro Gly Ser Gly Gln Tyr Gly Pro
545               550               555               560

Gly Gln Gln Gly Pro Gly Gln Ser Ala Ala Ala Ala Ala Gly Gln Tyr
          565               570               575

Gln Gln Gly Pro Gly Gln Gln Gly Pro Tyr Gly Pro Gly Ala Ser Ala
          580               585               590

Ala Ala Ala Gly Pro Gly Ser Gly Gln Gln Gly Pro Gly Ala Ser
          595               600               605

<210>   13
<211>   601
<212>   PRT
<213>   Artificial Sequence

<220>
<223>  PRT410

<400>  13

Met His His His His His His Ser Ser Gly Ser Ser Gly Pro Gly Gln
1                       5                   10                  15

Gln Gly Pro Tyr Gly Pro Gly Ala Ser Ala Ala Ala Ala Ala Gly Gln
                20                  25                  30

Asn Gly Pro Gly Ser Gly Gln Gln Gly Pro Gly Gln Ser Gly Gln Tyr
            35                  40                  45

Gly Pro Gly Gln Gln Gly Pro Gly Gln Gln Gly Pro Gly Ser Ser Ala
        50                  55                  60

Ala Ala Ala Ala Gly Pro Gly Gln Tyr Gly Pro Gly Gln Gln Gly Pro
65                  70                  75                  80

Ser Ala Ser Ala Ala Ala Ala Ala Gly Pro Gly Ser Gly Gln Gln Gly
                85                  90                  95

Pro Gly Ala Ser Gly Gln Tyr Gly Pro Gly Gln Gln Gly Pro Gly Gln
            100                 105                 110

Gln Gly Pro Gly Ser Ser Ala Ala Ala Ala Ala Gly Gln Tyr Gly Ser
            115                 120                 125

Gly Pro Gly Gln Gln Gly Pro Tyr Gly Ser Ala Ala Ala Ala Ala Gly
        130                 135                 140

Pro Gly Ser Gly Gln Tyr Gly Gln Gly Pro Tyr Gly Pro Gly Ala Ser
145                 150                 155                 160

Gly Pro Gly Gln Tyr Gly Pro Gly Gln Gln Gly Pro Ser Ala Ser Ala
            165                 170                 175

Ala Ala Ala Gly Ser Gly Gln Gln Gly Pro Gly Gln Tyr Gly Pro
            180                 185                 190

Tyr Ala Ser Ala Ala Ala Ala Ala Gly Gln Tyr Gly Ser Gly Pro Gly
            195                 200                 205

Gln Gln Gly Pro Tyr Gly Pro Gly Gln Ser Gly Ser Gly Gln Gln Gly
        210                 215                 220

Pro Gly Gln Gln Gly Pro Tyr Ala Ser Ala Ala Ala Ala Ala Gly Pro

```
          225                    230                    235                    240


          Gly Gln Gln Gly Pro Tyr Gly Pro Gly Ser Ser Ala Ala Ala Ala Ala
                          245                    250                    255


          Gly Gln Tyr Gly Tyr Gly Pro Gly Gln Gln Gly Pro Tyr Gly Pro Gly
                          260                    265                    270


          Ala Ser Gly Gln Asn Gly Pro Gly Ser Gly Gln Tyr Gly Pro Gly Gln
                          275                    280                    285


          Gln Gly Pro Gly Gln Ser Ala Ala Ala Ala Gly Pro Gly Gln Gln
                  290                    295                    300


          Gly Pro Tyr Gly Pro Gly Ala Ser Ala Ala Ala Ala Gly Gln Tyr
          305                    310                    315                    320


          Gly Pro Gly Gln Gln Gly Pro Gly Gln Tyr Gly Pro Gly Ser Ser Gly
                          325                    330                    335


          Pro Gly Gln Gln Gly Pro Tyr Gly Pro Gly Ser Ser Ala Ala Ala Ala
                          340                    345                    350


          Ala Gly Gln Tyr Gly Pro Gly Gln Gln Gly Pro Tyr Gly Pro Gly Gln
                  355                    360                    365


          Ser Ala Ala Ala Ala Ala Gly Gln Tyr Gln Gln Gly Pro Gly Gln Gln
                  370                    375                    380


          Gly Pro Tyr Gly Pro Gly Ala Ser Gly Pro Gly Gln Gln Gly Pro Tyr
          385                    390                    395                    400


          Gly Pro Gly Ala Ser Ala Ala Ala Ala Ala Gly Pro Gly Gln Tyr Gly
                          405                    410                    415


          Pro Gly Gln Gln Gly Pro Ser Ala Ser Ala Ala Ala Ala Ala Gly Gln
                  420                    425                    430


          Tyr Gly Ser Gly Pro Gly Gln Tyr Gly Pro Tyr Gly Pro Gly Gln Ser
                  435                    440                    445


          Gly Pro Gly Ser Gly Gln Gln Gly Gln Gly Pro Tyr Gly Pro Gly Ala
                  450                    455                    460


          Ser Ala Ala Ala Ala Ala Gly Gln Tyr Gly Pro Gly Gln Gln Gly Pro
          465                    470                    475                    480
```

```
Tyr Gly Pro Gly Gln Ser Ala Ala Ala Ala Gly Pro Gly Ser Gly
                485                 490                 495


Gln Tyr Gly Pro Gly Ala Ser Gly Gln Asn Gly Pro Gly Ser Gly Gln
                500                 505                 510


Tyr Gly Pro Gly Gln Gln Gly Pro Gly Gln Ser Ala Ala Ala Ala Ala
            515                 520                 525


Gly Gln Tyr Gln Gln Gly Pro Gly Gln Gln Gly Pro Tyr Gly Pro Gly
        530                 535                 540


Ala Ser Ala Ala Ala Ala Ala Gly Gln Tyr Gly Ser Gly Pro Gly Gln
545                 550                 555                 560


Gln Gly Pro Tyr Gly Pro Gly Gln Ser Gly Ser Gly Gln Gln Gly Pro
                565                 570                 575


Gly Gln Gln Gly Pro Tyr Ala Ser Ala Ala Ala Ala Ala Gly Pro Gly
            580                 585                 590


Ser Gly Gln Gln Gly Pro Gly Ala Ser
        595                 600


<210>  14
<211>  576
<212>  PRT
<213>  Artificial Sequence

<220>
<223>  PRT525

<400>  14

Met His His His His His His Ser Ser Gly Ser Ser Gly Pro Gly Gln
1                   5                   10                  15


Gln Gly Pro Tyr Gly Pro Gly Ala Ser Ala Ala Ala Ala Ala Ala Ala
                20                  25                  30


Gly Ser Asn Gly Pro Gly Ser Gly Gln Gln Gly Pro Gly Gln Ser Gly
            35                  40                  45


Gln Tyr Gly Pro Gly Gln Gln Gly Pro Gly Gln Gln Gly Pro Gly Ser
        50                  55                  60


Ser Ala Ala Ala Ala Ala Ala Ala Gly Pro Gly Gln Tyr Gly Pro Gly
65                  70                  75                  80


Gln Gln Gly Pro Ser Ala Ser Ala Ala Ala Ala Ala Ala Ala Gly Pro
```

|  |  |  | 85 |  |  |  | 90 |  |  |  |  | 95 |  |  |
|---|---|---|---|---|---|---|---|---|---|---|---|---|---|---|

Gly Ser Gly Gln Gln Gly Pro Gly Ala Ser Gly Gln Tyr Gly Pro Gly
          100                105               110

Gln Gln Gly Pro Gly Gln Gln Gly Pro Gly Ser Ser Ala Ala Ala Ala
          115                120               125

Ala Ala Ala Gly Ser Tyr Gly Ser Gly Pro Gly Gln Gln Gly Pro Tyr
          130                135               140

Gly Ser Ala Ala Ala Ala Ala Ala Ala Gly Pro Gly Ser Gly Gln Tyr
145                150              155              160

Gly Gln Gly Pro Tyr Gly Pro Gly Ala Ser Gly Pro Gly Gln Tyr Gly
              165              170              175

Pro Gly Gln Gln Gly Pro Ser Ala Ser Ala Ala Ala Ala Ala Ala Ala
          180              185              190

Gly Ser Gly Gln Gln Gly Pro Gly Gln Tyr Gly Pro Tyr Ala Ser Ala
          195              200              205

Ala Ala Ala Ala Ala Ala Gly Ser Tyr Gly Ser Gly Pro Gly Gln Gln
          210              215              220

Gly Pro Tyr Gly Pro Gly Gln Ser Gly Ser Gly Gln Gln Gly Pro Gly
225              230              235              240

Gln Gln Gly Pro Tyr Ala Ser Ala Ala Ala Ala Ala Ala Ala Gly Pro
          245              250              255

Gly Gln Gln Gly Pro Tyr Gly Pro Gly Ser Ser Ala Ala Ala Ala Ala
          260              265              270

Ala Ala Gly Ser Tyr Gly Tyr Gly Pro Gly Gln Gln Gly Pro Tyr Gly
          275              280              285

Pro Gly Ala Ser Gly Gln Asn Gly Pro Gly Ser Gly Gln Tyr Gly Pro
          290              295              300

Gly Gln Gln Gly Pro Gly Pro Ser Ala Ala Ala Ala Ala Ala Ala Gly
305              310              315              320

Pro Gly Gln Gln Gly Pro Tyr Gly Pro Gly Ala Ser Ala Ala Ala Ala
          325              330              335

```
        Ala Ala Ala Gly Ser Tyr Gly Pro Gly Gln Gln Gly Pro Gly Gln Tyr
                    340                 345                 350

        Gly Pro Gly Ser Ser Gly Pro Gly Gln Gln Gly Pro Tyr Gly Pro Gly
                    355                 360                 365

        Ser Ser Ala Ala Ala Ala Ala Ala Ala Gly Ser Tyr Gly Pro Gly Gln
                    370                 375                 380

        Gln Gly Pro Tyr Gly Pro Gly Pro Ser Ala Ala Ala Ala Ala Ala Ala
        385                 390                 395                 400

        Gly Ser Tyr Gln Gln Gly Pro Gly Gln Gln Gly Pro Tyr Gly Pro Gly
                        405                 410                 415

        Ala Ser Gly Pro Gly Gln Gln Gly Pro Tyr Gly Pro Gly Ala Ser Ala
                    420                 425                 430

        Ala Ala Ala Ala Ala Ala Gly Pro Gly Gln Tyr Gly Pro Gly Gln Gln
                    435                 440                 445

        Gly Pro Ser Ala Ser Ala Ala Ala Ala Ala Ala Ala Gly Ser Tyr Gly
                    450                 455                 460

        Ser Gly Pro Gly Gln Tyr Gly Pro Tyr Gly Pro Gly Gln Ser Gly Pro
        465                 470                 475                 480

        Gly Ser Gly Gln Gln Gly Gln Gly Pro Tyr Gly Pro Gly Ala Ser Ala
                        485                 490                 495

        Ala Ala Ala Ala Ala Ala Gly Ser Tyr Gly Pro Gly Gln Gln Gly Pro
                    500                 505                 510

        Tyr Gly Pro Gly Pro Ser Ala Ala Ala Ala Ala Ala Ala Gly Pro Gly
                    515                 520                 525

        Ser Gly Gln Tyr Gly Pro Gly Ala Ser Gly Gln Asn Gly Pro Gly Ser
                    530                 535                 540

        Gly Gln Tyr Gly Pro Gly Gln Gln Gly Pro Gly Pro Ser Ala Ala Ala
        545                 550                 555                 560

        Ala Ala Ala Ala Gly Pro Gly Ser Gly Gln Gln Gly Pro Gly Ala Ser
                    565                 570                 575


        <210>   15
        <211>   2375
        <212>   PRT
```

<213> Artificial Sequence

<220>
<223> PRT799

<400> 15

Met His His His His His His Ser Ser Gly Ser Ser Gly Pro Gly Gln
1                   5                   10                  15

Gln Gly Pro Tyr Gly Pro Gly Ala Ser Ala Ala Ala Ala Ala Gly Gln
                20                  25                  30

Asn Gly Pro Gly Ser Gly Gln Gln Gly Pro Gly Gln Ser Gly Gln Tyr
            35                  40                  45

Gly Pro Gly Gln Gln Gly Pro Gly Gln Gln Gly Pro Gly Ser Ser Ala
        50                  55                  60

Ala Ala Ala Ala Gly Pro Gly Gln Tyr Gly Pro Gly Gln Gln Gly Pro
65                  70                  75                  80

Ser Ala Ser Ala Ala Ala Ala Ala Gly Pro Gly Ser Gly Gln Gln Gly
                85                  90                  95

Pro Gly Ala Ser Gly Gln Tyr Gly Pro Gly Gln Gln Gly Pro Gly Gln
            100                 105                 110

Gln Gly Pro Gly Ser Ser Ala Ala Ala Ala Ala Gly Gln Tyr Gly Ser
        115                 120                 125

Gly Pro Gly Gln Gln Gly Pro Tyr Gly Ser Ala Ala Ala Ala Ala Gly
        130                 135                 140

Pro Gly Ser Gly Gln Tyr Gly Gln Gly Pro Tyr Gly Pro Gly Ala Ser
145                 150                 155                 160

Gly Pro Gly Gln Tyr Gly Pro Gly Gln Gln Gly Pro Ser Ala Ser Ala
            165                 170                 175

Ala Ala Ala Gly Ser Gly Gln Gln Gly Pro Gly Gln Tyr Gly Pro
            180                 185                 190

Tyr Ala Ser Ala Ala Ala Ala Ala Gly Gln Tyr Gly Ser Gly Pro Gly
            195                 200                 205

Gln Gln Gly Pro Tyr Gly Pro Gly Gln Ser Gly Ser Gly Gln Gln Gly
        210                 215                 220

Pro Gly Gln Gln Gly Pro Tyr Ala Ser Ala Ala Ala Ala Ala Gly Pro
225                 230                 235                     240

Gly Gln Gln Gly Pro Tyr Gly Pro Gly Ser Ser Ala Ala Ala Ala Ala
                245                 250                     255

Gly Gln Tyr Gly Tyr Gly Pro Gly Gln Gln Gly Pro Tyr Gly Pro Gly
            260                 265                 270

Ala Ser Gly Gln Asn Gly Pro Gly Ser Gly Gln Tyr Gly Pro Gly Gln
        275                 280                 285

Gln Gly Pro Gly Gln Ser Ala Ala Ala Ala Gly Pro Gly Gln Gln
    290                 295                 300

Gly Pro Tyr Gly Pro Gly Ala Ser Ala Ala Ala Ala Ala Gly Gln Tyr
305                 310                 315                     320

Gly Pro Gly Gln Gln Gly Pro Gly Gln Tyr Gly Pro Gly Ser Ser Gly
            325                 330                 335

Pro Gly Gln Gln Gly Pro Tyr Gly Pro Gly Ser Ser Ala Ala Ala Ala
            340                 345                 350

Ala Gly Gln Tyr Gly Pro Gly Gln Gln Gly Pro Tyr Gly Pro Gly Gln
    355                 360                 365

Ser Ala Ala Ala Ala Ala Gly Gln Tyr Gln Gln Gly Pro Gly Gln Gln
    370                 375                 380

Gly Pro Tyr Gly Pro Gly Ala Ser Gly Pro Gly Gln Gln Gly Pro Tyr
385                 390                 395                     400

Gly Pro Gly Ala Ser Ala Ala Ala Ala Ala Gly Pro Gly Gln Tyr Gly
            405                 410                 415

Pro Gly Gln Gln Gly Pro Ser Ala Ser Ala Ala Ala Ala Gly Gln
            420                 425                 430

Tyr Gly Ser Gly Pro Gly Gln Tyr Gly Pro Tyr Gly Pro Gly Gln Ser
        435                 440                 445

Gly Pro Gly Ser Gly Gln Gln Gly Gln Gly Pro Tyr Gly Pro Gly Ala
    450                 455                 460

Ser Ala Ala Ala Ala Ala Gly Gln Tyr Gly Pro Gly Gln Gln Gly Pro
465                 470                 475                     480

```
Tyr Gly Pro Gly Gln Ser Ala Ala Ala Ala Ala Gly Pro Gly Ser Gly
                485             490             495

Gln Tyr Gly Pro Gly Ala Ser Gly Gln Asn Gly Pro Gly Ser Gly Gln
            500             505             510

Tyr Gly Pro Gly Gln Gln Gly Pro Gly Gln Ser Ala Ala Ala Ala Ala
        515             520             525

Gly Gln Tyr Gln Gln Gly Pro Gly Gln Gln Gly Pro Tyr Gly Pro Gly
    530             535             540

Ala Ser Ala Ala Ala Ala Ala Gly Gln Tyr Gly Ser Gly Pro Gly Gln
545             550             555             560

Gln Gly Pro Tyr Gly Pro Gly Gln Ser Gly Ser Gly Gln Gln Gly Pro
            565             570             575

Gly Gln Gln Gly Pro Tyr Ala Ser Ala Ala Ala Ala Gly Pro Gly
            580             585             590

Ser Gly Gln Gln Gly Pro Gly Ala Ser Gly Gln Gln Gly Pro Tyr Gly
            595             600             605

Pro Gly Ala Ser Ala Ala Ala Ala Gly Gln Asn Gly Pro Gly Ser
    610             615             620

Gly Gln Gln Gly Pro Gly Gln Ser Gly Gln Tyr Gly Pro Gly Gln Gln
625             630             635             640

Gly Pro Gly Gln Gln Gly Pro Gly Ser Ser Ala Ala Ala Ala Gly
            645             650             655

Pro Gly Gln Tyr Gly Pro Gly Gln Gln Gly Pro Ser Ala Ser Ala Ala
        660             665             670

Ala Ala Ala Gly Pro Gly Ser Gly Gln Gln Gly Pro Gly Ala Ser Gly
    675             680             685

Gln Tyr Gly Pro Gly Gln Gln Gly Pro Gly Gln Gln Gly Pro Gly Ser
    690             695             700

Ser Ala Ala Ala Ala Ala Gly Gln Tyr Gly Ser Gly Pro Gly Gln Gln
705             710             715             720

Gly Pro Tyr Gly Ser Ala Ala Ala Ala Ala Gly Pro Gly Ser Gly Gln
            725             730             735
```

Tyr Gly Gln Gly Pro Tyr Gly Pro Gly Ala Ser Gly Pro Gly Gln Tyr
        740             745             750

Gly Pro Gly Gln Gln Gly Pro Ser Ala Ser Ala Ala Ala Ala Ala Gly
        755             760             765

Ser Gly Gln Gln Gly Pro Gly Gln Tyr Gly Pro Tyr Ala Ser Ala Ala
        770             775             780

Ala Ala Ala Gly Gln Tyr Gly Ser Gly Pro Gly Gln Gln Gly Pro Tyr
785             790             795             800

Gly Pro Gly Gln Ser Gly Ser Gly Gln Gln Gly Pro Gly Gln Gln Gly
                805             810             815

Pro Tyr Ala Ser Ala Ala Ala Ala Ala Gly Pro Gly Gln Gln Gly Pro
        820             825             830

Tyr Gly Pro Gly Ser Ser Ala Ala Ala Ala Gly Gln Tyr Gly Tyr
        835             840             845

Gly Pro Gly Gln Gln Gly Pro Tyr Gly Pro Gly Ala Ser Gly Gln Asn
        850             855             860

Gly Pro Gly Ser Gly Gln Tyr Gly Pro Gly Gln Gln Gly Pro Gly Gln
865             870             875             880

Ser Ala Ala Ala Ala Ala Gly Pro Gly Gln Gln Gly Pro Tyr Gly Pro
                885             890             895

Gly Ala Ser Ala Ala Ala Ala Ala Gly Gln Tyr Gly Pro Gly Gln Gln
        900             905             910

Gly Pro Gly Gln Tyr Gly Pro Gly Ser Ser Gly Pro Gly Gln Gln Gly
        915             920             925

Pro Tyr Gly Pro Gly Ser Ser Ala Ala Ala Ala Gly Gln Tyr Gly
        930             935             940

Pro Gly Gln Gln Gly Pro Tyr Gly Pro Gly Gln Ser Ala Ala Ala Ala
945             950             955             960

Ala Gly Gln Tyr Gln Gln Gly Pro Gly Gln Gly Pro Tyr Gly Pro
                965             970             975

Gly Ala Ser Gly Pro Gly Gln Gln Gly Pro Tyr Gly Pro Gly Ala Ser

82

                    980                          985                          990


Ala Ala Ala Ala Ala Gly Pro Gly  Gln Tyr Gly Pro Gly  Gln Gln Gly
        995                     1000                     1005


Pro Ser  Ala Ser Ala Ala Ala  Ala Ala Gly Gln Tyr  Gly Ser Gly
    1010                    1015                    1020


Pro Gly  Gln Tyr Gly Pro Tyr  Gly Pro Gly Gln Ser  Gly Pro Gly
    1025                    1030                    1035


Ser Gly  Gln Gln Gly Gln Gly  Pro Tyr Gly Pro Gly  Ala Ser Ala
    1040                    1045                    1050


Ala Ala  Ala Ala Gly Gln Tyr  Gly Pro Gly Gln Gln  Gly Pro Tyr
    1055                    1060                    1065


Gly Pro  Gly Gln Ser Ala Ala  Ala Ala Ala Gly Pro  Gly Ser Gly
    1070                    1075                    1080


Gln Tyr  Gly Pro Gly Ala Ser  Gly Gln Asn Gly Pro  Gly Ser Gly
    1085                    1090                    1095


Gln Tyr  Gly Pro Gly Gln Gln  Gly Pro Gly Gln Ser  Ala Ala Ala
    1100                    1105                    1110


Ala Ala  Gly Gln Tyr Gln Gln  Gly Pro Gly Gln Gln  Gly Pro Tyr
    1115                    1120                    1125


Gly Pro  Gly Ala Ser Ala Ala  Ala Ala Ala Gly Gln  Tyr Gly Ser
    1130                    1135                    1140


Gly Pro  Gly Gln Gln Gly Pro  Tyr Gly Pro Gly Gln  Ser Gly Ser
    1145                    1150                    1155


Gly Gln  Gln Gly Pro Gly Gln  Gln Gly Pro Tyr Ala  Ser Ala Ala
    1160                    1165                    1170


Ala Ala  Ala Gly Pro Gly Ser  Gly Gln Gln Gly Pro  Gly Ala Ser
    1175                    1180                    1185


Gly Gln  Gln Gly Pro Tyr Gly  Pro Gly Ala Ser Ala  Ala Ala Ala
    1190                    1195                    1200


Ala Gly  Gln Asn Gly Pro Gly  Ser Gly Gln Gln Gly  Pro Gly Gln
    1205                    1210                    1215

```
Ser Gly  Gln Tyr Gly Pro Gly  Gln Gln Gly Pro Gly  Gln Gln Gly
    1220              1225                1230

Pro Gly  Ser Ser Ala Ala Ala  Ala Ala Gly Pro Gly  Gln Tyr Gly
    1235              1240                1245

Pro Gly  Gln Gln Gly Pro Ser  Ala Ser Ala Ala Ala  Ala Ala Gly
    1250              1255                1260

Pro Gly  Ser Gly Gln Gln Gly  Pro Gly Ala Ser Gly  Gln Tyr Gly
    1265              1270                1275

Pro Gly  Gln Gln Gly Pro Gly  Gln Gln Gly Pro Gly  Ser Ser Ala
    1280              1285                1290

Ala Ala  Ala Ala Gly Gln Tyr  Gly Ser Gly Pro Gly  Gln Gln Gly
    1295              1300                1305

Pro Tyr  Gly Ser Ala Ala Ala  Ala Ala Gly Pro Gly  Ser Gly Gln
    1310              1315                1320

Tyr Gly  Gln Gly Pro Tyr Gly  Pro Gly Ala Ser Gly  Pro Gly Gln
    1325              1330                1335

Tyr Gly  Pro Gly Gln Gln Gly  Pro Ser Ala Ser Ala  Ala Ala Ala
    1340              1345                1350

Ala Gly  Ser Gly Gln Gln Gly  Pro Gly Gln Tyr Gly  Pro Tyr Ala
    1355              1360                1365

Ser Ala  Ala Ala Ala Ala Gly  Gln Tyr Gly Ser Gly  Pro Gly Gln
    1370              1375                1380

Gln Gly  Pro Tyr Gly Pro Gly  Gln Ser Gly Ser Gly  Gln Gln Gly
    1385              1390                1395

Pro Gly  Gln Gln Gly Pro Tyr  Ala Ser Ala Ala Ala  Ala Ala Gly
    1400              1405                1410

Pro Gly  Gln Gln Gly Pro Tyr  Gly Pro Gly Ser Ser  Ala Ala Ala
    1415              1420                1425

Ala Ala  Gly Gln Tyr Gly Tyr  Gly Pro Gly Gln Gln  Gly Pro Tyr
    1430              1435                1440

Gly Pro  Gly Ala Ser Gly Gln  Asn Gly Pro Gly Ser  Gly Gln Tyr
    1445              1450                1455
```

84

```
Gly Pro  Gly Gln Gln Gly Pro  Gly Gln Ser Ala Ala  Ala Ala Ala
    1460             1465             1470

Gly Pro  Gly Gln Gln Gly Pro  Tyr Gly Pro Gly Ala  Ser Ala Ala
    1475             1480             1485

Ala Ala  Ala Gly Gln Tyr Gly  Pro Gly Gln Gln Gly  Pro Gly Gln
    1490             1495             1500

Tyr Gly  Pro Gly Ser Ser Gly  Pro Gly Gln Gln Gly  Pro Tyr Gly
    1505             1510             1515

Pro Gly  Ser Ser Ala Ala Ala  Ala Ala Gly Gln Tyr  Gly Pro Gly
    1520             1525             1530

Gln Gln  Gly Pro Tyr Gly Pro  Gly Gln Ser Ala Ala  Ala Ala Ala
    1535             1540             1545

Gly Gln  Tyr Gln Gln Gly Pro  Gly Gln Gln Gly Pro  Tyr Gly Pro
    1550             1555             1560

Gly Ala  Ser Gly Pro Gly Gln  Gln Gly Pro Tyr Gly  Pro Gly Ala
    1565             1570             1575

Ser Ala  Ala Ala Ala Ala Gly  Pro Gly Gln Tyr Gly  Pro Gly Gln
    1580             1585             1590

Gln Gly  Pro Ser Ala Ser Ala  Ala Ala Ala Ala Gly  Gln Tyr Gly
    1595             1600             1605

Ser Gly  Pro Gly Gln Tyr Gly  Pro Tyr Gly Pro Gly  Gln Ser Gly
    1610             1615             1620

Pro Gly  Ser Gly Gln Gln Gly  Gln Gly Pro Tyr Gly  Pro Gly Ala
    1625             1630             1635

Ser Ala  Ala Ala Ala Ala Gly  Gln Tyr Gly Pro Gly  Gln Gln Gly
    1640             1645             1650

Pro Tyr  Gly Pro Gly Gln Ser  Ala Ala Ala Ala Ala  Gly Pro Gly
    1655             1660             1665

Ser Gly  Gln Tyr Gly Pro Gly  Ala Ser Gly Gln Asn  Gly Pro Gly
    1670             1675             1680

Ser Gly  Gln Tyr Gly Pro Gly  Gln Gln Gly Pro Gly  Gln Ser Ala
    1685             1690             1695
```

```
Ala Ala  Ala Ala Gly Gln Tyr  Gln Gln Gly Pro Gly  Gln Gln Gly
1700             1705                 1710

Pro Tyr  Gly Pro Gly Ala Ser  Ala Ala Ala Ala Ala  Gly Gln Tyr
1715             1720                 1725

Gly Ser  Gly Pro Gly Gln Gln  Gly Pro Tyr Gly Pro  Gly Gln Ser
1730             1735                 1740

Gly Ser  Gly Gln Gln Gly Pro  Gly Gln Gln Gly Pro  Tyr Ala Ser
1745             1750                 1755

Ala Ala  Ala Ala Ala Gly Pro  Gly Ser Gly Gln Gln  Gly Pro Gly
1760             1765                 1770

Ala Ser  Gly Gln Gln Gly Pro  Tyr Gly Pro Gly Ala  Ser Ala Ala
1775             1780                 1785

Ala Ala  Ala Gly Gln Asn Gly  Pro Gly Ser Gly Gln  Gln Gly Pro
1790             1795                 1800

Gly Gln  Ser Gly Gln Tyr Gly  Pro Gly Gln Gln Gly  Pro Gly Gln
1805             1810                 1815

Gln Gly  Pro Gly Ser Ser Ala  Ala Ala Ala Ala Gly  Pro Gly Gln
1820             1825                 1830

Tyr Gly  Pro Gly Gln Gln Gly  Pro Ser Ala Ser Ala  Ala Ala Ala
1835             1840                 1845

Ala Gly  Pro Gly Ser Gly Gln  Gln Gly Pro Gly Ala  Ser Gly Gln
1850             1855                 1860

Tyr Gly  Pro Gly Gln Gln Gly  Pro Gly Gln Gln Gly  Pro Gly Ser
1865             1870                 1875

Ser Ala  Ala Ala Ala Ala Gly  Gln Tyr Gly Ser Gly  Pro Gly Gln
1880             1885                 1890

Gln Gly  Pro Tyr Gly Ser Ala  Ala Ala Ala Ala Gly  Pro Gly Ser
1895             1900                 1905

Gly Gln  Tyr Gly Gln Gly Pro  Tyr Gly Pro Gly Ala  Ser Gly Pro
1910             1915                 1920

Gly Gln  Tyr Gly Pro Gly Gln  Gln Gly Pro Ser Ala  Ser Ala Ala
```

86

```
                1925                        1930                        1935


        Ala Ala  Ala Gly Ser Gly Gln  Gln Gly Pro Gly Gln  Tyr Gly Pro
            1940                1945                1950


        Tyr Ala  Ser Ala Ala Ala Ala  Ala Gly Gln Tyr Gly  Ser Gly Pro
            1955                1960                1965


        Gly Gln  Gln Gly Pro Tyr Gly  Pro Gly Gln Ser Gly  Ser Gly Gln
            1970                1975                1980


        Gln Gly  Pro Gly Gln Gln Gly  Pro Tyr Ala Ser Ala  Ala Ala Ala
            1985                1990                1995


        Ala Gly  Pro Gly Gln Gln Gly  Pro Tyr Gly Pro Gly  Ser Ser Ala
            2000                2005                2010


        Ala Ala  Ala Ala Gly Gln Tyr  Gly Tyr Gly Pro Gly  Gln Gln Gly
            2015                2020                2025


        Pro Tyr  Gly Pro Gly Ala Ser  Gly Gln Asn Gly Pro  Gly Ser Gly
            2030                2035                2040


        Gln Tyr  Gly Pro Gly Gln Gln  Gly Pro Gly Gln Ser  Ala Ala Ala
            2045                2050                2055


        Ala Ala  Gly Pro Gly Gln Gln  Gly Pro Tyr Gly Pro  Gly Ala Ser
            2060                2065                2070


        Ala Ala  Ala Ala Ala Gly Gln  Tyr Gly Pro Gly Gln  Gln Gly Pro
            2075                2080                2085


        Gly Gln  Tyr Gly Pro Gly Ser  Ser Gly Pro Gly Gln  Gln Gly Pro
            2090                2095                2100


        Tyr Gly  Pro Gly Ser Ser Ala  Ala Ala Ala Ala Gly  Gln Tyr Gly
            2105                2110                2115


        Pro Gly  Gln Gln Gly Pro Tyr  Gly Pro Gly Gln Ser  Ala Ala Ala
            2120                2125                2130


        Ala Ala  Gly Gln Tyr Gln Gln  Gly Pro Gly Gln Gln  Gly Pro Tyr
            2135                2140                2145


        Gly Pro  Gly Ala Ser Gly Pro  Gly Gln Gln Gly Pro  Tyr Gly Pro
            2150                2155                2160
```

```
Gly Ala  Ser Ala Ala Ala Ala  Ala Gly Pro Gly Gln  Tyr Gly Pro
    2165             2170                 2175

Gly Gln  Gln Gly Pro Ser Ala  Ser Ala Ala Ala Ala  Ala Gly Gln
    2180             2185                 2190

Tyr Gly  Ser Gly Pro Gly Gln  Tyr Gly Pro Tyr Gly  Pro Gly Gln
    2195             2200                 2205

Ser Gly  Pro Gly Ser Gly Gln  Gln Gly Gln Gly Pro  Tyr Gly Pro
    2210             2215                 2220

Gly Ala  Ser Ala Ala Ala Ala  Ala Gly Gln Tyr Gly  Pro Gly Gln
    2225             2230                 2235

Gln Gly  Pro Tyr Gly Pro Gly  Gln Ser Ala Ala Ala  Ala Ala Gly
    2240             2245                 2250

Pro Gly  Ser Gly Gln Tyr Gly  Pro Gly Ala Ser Gly  Gln Asn Gly
    2255             2260                 2265

Pro Gly  Ser Gly Gln Tyr Gly  Pro Gly Gln Gln Gly  Pro Gly Gln
    2270             2275                 2280

Ser Ala  Ala Ala Ala Ala Gly  Gln Tyr Gln Gln Gly  Pro Gly Gln
    2285             2290                 2295

Gln Gly  Pro Tyr Gly Pro Gly  Ala Ser Ala Ala Ala  Ala Ala Gly
    2300             2305                 2310

Gln Tyr  Gly Ser Gly Pro Gly  Gln Gln Gly Pro Tyr  Gly Pro Gly
    2315             2320                 2325

Gln Ser  Gly Ser Gly Gln Gln  Gly Pro Gly Gln Gln  Gly Pro Tyr
    2330             2335                 2340

Ala Ser  Ala Ala Ala Ala Ala  Gly Pro Gly Ser Gly  Gln Gln Gly
    2345             2350                 2355

Ser Ser  Val Asp Lys Leu Ala  Ala Ala Leu Glu His  His His His
    2360             2365                 2370

His His
    2375


<210>  16
<211>  608
<212>  PRT
```

<213>    Artificial Sequence

<220>
<223>    PRT313

<400>    16

Met His His His His His His Ser Ser Gly Ser Ser Gly Pro Gly Gly
1                   5                   10                  15

Gln Gly Pro Tyr Gly Pro Gly Ala Ser Ala Ala Ala Ala Ala Gly Gly
            20                  25                  30

Asn Gly Pro Gly Ser Gly Gln Gln Gly Pro Gly Gly Ser Ala Ala Ala
            35                  40                  45

Ala Ala Gly Gly Tyr Gly Pro Gly Gly Gln Gly Pro Gly Gln Gln Gly
    50                  55                  60

Pro Gly Ser Ser Ala Ala Ala Ala Ala Gly Pro Gly Gly Tyr Gly Pro
65                  70                  75                  80

Gly Gly Gln Gly Pro Ser Ala Ser Ala Ala Ala Ala Ala Gly Pro Gly
            85                  90                  95

Ser Gly Gln Gln Gly Pro Gly Ala Ser Ala Ala Ala Ala Ala Gly Gly
            100                 105                 110

Tyr Gly Pro Gly Gly Gln Gly Pro Gly Gln Gln Gly Pro Gly Ser Ser
            115                 120                 125

Ala Ala Ala Ala Ala Gly Gly Tyr Gly Ser Gly Pro Gly Gln Gln Gly
    130                 135                 140

Pro Tyr Gly Ser Ala Ala Ala Ala Ala Gly Pro Gly Ser Gly Gly Tyr
145                 150                 155                 160

Gly Gln Gly Pro Tyr Gly Pro Gly Ala Ser Ala Ala Ala Ala Ala Gly
            165                 170                 175

Pro Gly Gly Tyr Gly Pro Gly Gly Gln Gly Pro Ser Ala Ser Ala Ala
            180                 185                 190

Ala Ala Ala Gly Ser Gly Gln Gln Gly Pro Gly Gly Tyr Gly Pro Tyr
    195                 200                 205

Ala Ser Ala Ala Ala Ala Ala Gly Gly Tyr Gly Ser Gly Pro Gly Gln
    210                 215                 220

Gln Gly Pro Tyr Gly Pro Gly Gly Ser Ala Ala Ala Ala Gly Ser
225                 230             235                 240

Gly Gln Gln Gly Pro Gly Gln Gln Gly Pro Tyr Ala Ser Ala Ala Ala
                245             250                 255

Ala Ala Gly Pro Gly Gly Gln Gly Pro Tyr Gly Pro Gly Ser Ser Ala
            260             265             270

Ala Ala Ala Ala Gly Gly Tyr Gly Tyr Gly Pro Gly Gly Gln Gly Pro
        275             280             285

Tyr Gly Pro Gly Ala Ser Ala Ala Ala Ala Gly Gly Asn Gly Pro
    290             295             300

Gly Ser Gly Gly Tyr Gly Pro Gly Gln Gln Gly Pro Gly Gly Ser Ala
305             310             315                 320

Ala Ala Ala Gly Pro Gly Gly Gln Gly Pro Tyr Gly Pro Gly Ala
            325             330             335

Ser Ala Ala Ala Ala Ala Gly Gly Tyr Gly Pro Gly Gly Gln Gly Pro
        340             345             350

Gly Gly Tyr Gly Pro Gly Ser Ser Ala Ala Ala Ala Ala Gly Pro Gly
        355             360             365

Gly Gln Gly Pro Tyr Gly Pro Gly Ser Ser Ala Ala Ala Ala Ala Gly
    370             375             380

Gly Tyr Gly Pro Gly Gln Gln Gly Pro Tyr Gly Pro Gly Gly Ser Ala
385             390             395                 400

Ala Ala Ala Gly Gly Tyr Gln Gln Gly Pro Gly Gly Gln Gly Pro
            405             410                 415

Tyr Gly Pro Gly Ala Ser Ala Ala Ala Ala Gly Pro Gly Gly Gln
    420             425             430

Gly Pro Tyr Gly Pro Gly Ala Ser Ala Ala Ala Ala Gly Pro Gly
    435             440             445

Gly Tyr Gly Pro Gly Gly Gln Gly Pro Ser Ala Ser Ala Ala Ala Ala
    450             455             460

Ala Gly Gly Tyr Gly Ser Gly Pro Gly Gly Tyr Gly Pro Tyr Gly Pro
465             470             475             480

90

Gly Gly Ser Ala Ala Ala Ala Ala Gly Pro Gly Ser Gly Gln Gln Gly
           485              490            495

Gln Gly Pro Tyr Gly Pro Gly Ala Ser Ala Ala Ala Ala Gly Gly
          500            505            510

Tyr Gly Pro Gly Gln Gln Gly Pro Tyr Gly Pro Gly Gly Ser Ala Ala
          515            520            525

Ala Ala Ala Gly Pro Gly Ser Gly Gly Tyr Gly Pro Gly Ala Ser Ala
    530            535            540

Ala Ala Ala Ala Gly Gly Asn Gly Pro Gly Ser Gly Gly Tyr Gly Pro
545              550          555          560

Gly Gln Gln Gly Pro Gly Gly Ser Ala Ala Ala Ala Gly Gly Tyr
          565            570            575

Gln Gln Gly Pro Gly Gly Gln Gly Pro Tyr Gly Pro Gly Ala Ser Ala
          580            585          590

Ala Ala Ala Ala Gly Pro Gly Ser Gly Gln Gln Gly Pro Gly Ala Ser
    595            600          605

```
<210>  17
<211>  590
<212>  PRT
<213>  Artificial Sequence

<220>
<223>  Met-PRT399

<400>  17
```

Met Gly Pro Gly Gly Gln Gly Pro Tyr Gly Pro Gly Ala Ser Ala Ala
1              5            10          15

Ala Ala Gly Gly Asn Gly Pro Gly Ser Gly Gln Gln Gly Pro Gly
          20            25          30

Gly Ser Gly Gly Tyr Gly Pro Gly Gly Gln Gly Pro Gly Gln Gln Gly
          35            40          45

Pro Gly Ser Ser Ala Ala Ala Ala Ala Gly Pro Gly Gly Tyr Gly Pro
    50            55          60

Gly Gly Gln Gly Pro Ser Ala Ser Ala Ala Ala Ala Gly Pro Gly
65              70          75          80

```
Ser Gly Gln Gln Gly Pro Gly Ala Ser Gly Gly Tyr Gly Pro Gly Gly
                    85              90                  95

Gln Gly Pro Gly Gln Gln Gly Pro Gly Ser Ser Ala Ala Ala Ala Ala
            100             105             110

Gly Gly Tyr Gly Ser Gly Pro Gly Gln Gln Gly Pro Tyr Gly Ser Ala
            115             120             125

Ala Ala Ala Ala Gly Pro Gly Ser Gly Gly Tyr Gly Gln Gly Pro Tyr
    130             135             140

Gly Pro Gly Ala Ser Gly Pro Gly Gly Tyr Gly Pro Gly Gly Gln Gly
145             150             155             160

Pro Ser Ala Ser Ala Ala Ala Ala Ala Gly Ser Gly Gln Gln Gly Pro
            165             170             175

Gly Gly Tyr Gly Pro Tyr Ala Ser Ala Ala Ala Ala Gly Gly Tyr
            180             185             190

Gly Ser Gly Pro Gly Gln Gln Gly Pro Tyr Gly Pro Gly Gly Ser Gly
            195             200             205

Ser Gly Gln Gln Gly Pro Gly Gln Gln Gly Pro Tyr Ala Ser Ala Ala
    210             215             220

Ala Ala Ala Gly Pro Gly Gly Gln Gly Pro Tyr Gly Pro Gly Ser Ser
225             230             235             240

Ala Ala Ala Ala Ala Gly Gly Tyr Gly Tyr Gly Pro Gly Gly Gln Gly
            245             250             255

Pro Tyr Gly Pro Gly Ala Ser Gly Gly Asn Gly Pro Gly Ser Gly Gly
            260             265             270

Tyr Gly Pro Gly Gln Gln Gly Pro Gly Gly Ser Ala Ala Ala Ala Ala
            275             280             285

Gly Pro Gly Gly Gln Gly Pro Tyr Gly Pro Gly Ala Ser Ala Ala Ala
    290             295             300

Ala Ala Gly Gly Tyr Gly Pro Gly Gly Gln Gly Pro Gly Gly Tyr Gly
305             310             315             320

Pro Gly Ser Ser Gly Pro Gly Gly Gln Gly Pro Tyr Gly Pro Gly Ser
            325             330             335
```

Ser Ala Ala Ala Ala Ala Gly Gly Tyr Gly Pro Gly Gln Gln Gly Pro
    340             345             350

Tyr Gly Pro Gly Gly Ser Ala Ala Ala Ala Ala Gly Gly Tyr Gln Gln
    355             360             365

Gly Pro Gly Gly Gln Gly Pro Tyr Gly Pro Gly Ala Ser Gly Pro Gly
    370             375             380

Gly Gln Gly Pro Tyr Gly Pro Gly Ala Ser Ala Ala Ala Ala Ala Gly
385             390            395            400

Pro Gly Gly Tyr Gly Pro Gly Gly Gln Gly Pro Ser Ala Ser Ala Ala
        405            410            415

Ala Ala Ala Gly Gly Tyr Gly Ser Gly Pro Gly Gly Tyr Gly Pro Tyr
    420             425             430

Gly Pro Gly Gly Ser Gly Pro Gly Ser Gly Gln Gln Gly Gln Gly Pro
    435             440             445

Tyr Gly Pro Gly Ala Ser Ala Ala Ala Ala Gly Gly Tyr Gly Pro
    450             455            460

Gly Gln Gln Gly Pro Tyr Gly Pro Gly Gly Ser Ala Ala Ala Ala Ala
465             470           475            480

Gly Pro Gly Ser Gly Gly Tyr Gly Pro Gly Ala Ser Gly Gly Asn Gly
        485            490            495

Pro Gly Ser Gly Gly Tyr Gly Pro Gly Gln Gln Gly Pro Gly Gly Ser
        500            505            510

Ala Ala Ala Ala Ala Gly Gly Tyr Gln Gln Gly Pro Gly Gly Gln Gly
    515             520            525

Pro Tyr Gly Pro Gly Ala Ser Ala Ala Ala Ala Gly Gly Tyr Gly
    530             535            540

Ser Gly Pro Gly Gln Gln Gly Pro Tyr Gly Pro Gly Gly Ser Gly Ser
545             550           555            560

Gly Gln Gln Gly Pro Gly Gln Gln Gly Pro Tyr Ala Ser Ala Ala Ala
        565            570            575

Ala Ala Gly Pro Gly Ser Gly Gln Gln Gly Pro Gly Ala Ser
    580             585           590

<210> 18
<211> 601
<212> PRT
<213> Artificial Sequence

<220>
<223> PRT399

<400> 18

Met His His His His His His Ser Ser Gly Ser Ser Gly Pro Gly Gly
1               5                   10                  15

Gln Gly Pro Tyr Gly Pro Gly Ala Ser Ala Ala Ala Ala Ala Gly Gly
            20                  25                  30

Asn Gly Pro Gly Ser Gly Gln Gln Gly Pro Gly Gly Ser Gly Gly Tyr
            35                  40                  45

Gly Pro Gly Gly Gln Gly Pro Gly Gln Gln Gly Pro Gly Ser Ser Ala
        50                  55                  60

Ala Ala Ala Ala Gly Pro Gly Gly Tyr Gly Pro Gly Gly Gln Gly Pro
65                  70                  75                  80

Ser Ala Ser Ala Ala Ala Ala Ala Gly Pro Gly Ser Gly Gln Gln Gly
            85                  90                  95

Pro Gly Ala Ser Gly Gly Tyr Gly Pro Gly Gly Gln Gly Pro Gly Gln
            100                 105                 110

Gln Gly Pro Gly Ser Ser Ala Ala Ala Ala Ala Gly Gly Tyr Gly Ser
        115                 120                 125

Gly Pro Gly Gln Gln Gly Pro Tyr Gly Ser Ala Ala Ala Ala Ala Gly
    130                 135                 140

Pro Gly Ser Gly Gly Tyr Gly Gln Gly Pro Tyr Gly Pro Gly Ala Ser
145                 150                 155                 160

Gly Pro Gly Gly Tyr Gly Pro Gly Gly Gln Gly Pro Ser Ala Ser Ala
            165                 170                 175

Ala Ala Ala Gly Ser Gly Gln Gln Gly Pro Gly Gly Tyr Gly Pro
        180                 185                 190

Tyr Ala Ser Ala Ala Ala Ala Ala Gly Gly Tyr Gly Ser Gly Pro Gly
    195                 200                 205

```
Gln Gln Gly Pro Tyr Gly Pro Gly Gly Ser Gly Ser Gly Gln Gln Gly
    210             215             220

Pro Gly Gln Gln Gly Pro Tyr Ala Ser Ala Ala Ala Ala Ala Gly Pro
225             230             235             240

Gly Gly Gln Gly Pro Tyr Gly Pro Gly Ser Ser Ala Ala Ala Ala Ala
            245             250             255

Gly Gly Tyr Gly Tyr Gly Pro Gly Gly Gln Gly Pro Tyr Gly Pro Gly
        260             265             270

Ala Ser Gly Gly Asn Gly Pro Gly Ser Gly Gly Tyr Gly Pro Gly Gln
        275             280             285

Gln Gly Pro Gly Gly Ser Ala Ala Ala Ala Gly Pro Gly Gly Gln
    290             295             300

Gly Pro Tyr Gly Pro Gly Ala Ser Ala Ala Ala Ala Ala Gly Gly Tyr
305             310             315             320

Gly Pro Gly Gly Gln Gly Pro Gly Gly Tyr Gly Pro Gly Ser Ser Gly
            325             330             335

Pro Gly Gly Gln Gly Pro Tyr Gly Pro Gly Ser Ser Ala Ala Ala Ala
        340             345             350

Ala Gly Gly Tyr Gly Pro Gly Gln Gln Gly Pro Tyr Gly Pro Gly Gly
        355             360             365

Ser Ala Ala Ala Ala Ala Gly Gly Tyr Gln Gln Gly Pro Gly Gly Gln
370             375             380

Gly Pro Tyr Gly Pro Gly Ala Ser Gly Pro Gly Gly Gln Gly Pro Tyr
385             390             395             400

Gly Pro Gly Ala Ser Ala Ala Ala Ala Ala Gly Pro Gly Gly Tyr Gly
            405             410             415

Pro Gly Gly Gln Gly Pro Ser Ala Ser Ala Ala Ala Ala Ala Gly Gly
        420             425             430

Tyr Gly Ser Gly Pro Gly Gly Tyr Gly Pro Tyr Gly Pro Gly Gly Ser
        435             440             445

Gly Pro Gly Ser Gly Gln Gln Gly Gln Gly Pro Tyr Gly Pro Gly Ala
    450             455             460
```

95

```
Ser Ala Ala Ala Ala Ala Gly Gly Tyr Gly Pro Gly Gln Gln Gly Pro
465             470         475             480

Tyr Gly Pro Gly Gly Ser Ala Ala Ala Ala Ala Gly Pro Gly Ser Gly
            485             490             495

Gly Tyr Gly Pro Gly Ala Ser Gly Gly Asn Gly Pro Gly Ser Gly Gly
        500             505             510

Tyr Gly Pro Gly Gln Gln Gly Pro Gly Gly Ser Ala Ala Ala Ala Ala
        515             520             525

Gly Gly Tyr Gln Gln Gly Pro Gly Gly Gln Gly Pro Tyr Gly Pro Gly
    530             535             540

Ala Ser Ala Ala Ala Ala Ala Gly Gly Tyr Gly Ser Gly Pro Gly Gln
545             550             555             560

Gln Gly Pro Tyr Gly Pro Gly Gly Ser Gly Ser Gly Gln Gln Gly Pro
            565             570             575

Gly Gln Gln Gly Pro Tyr Ala Ser Ala Ala Ala Ala Ala Gly Pro Gly
        580             585             590

Ser Gly Gln Gln Gly Pro Gly Ala Ser
    595             600
```

```
<210>   19
<211>   612
<212>   PRT
<213>   Artificial Sequence

<220>
<223>   Met-PRT720

<400>   19
```

```
Met Gly Pro Gly Gln Gln Gly Pro Tyr Gly Pro Gly Ala Ser Ala Ala
1               5               10              15

Ala Ala Ala Gly Gln Asn Gly Pro Gly Ser Gly Gln Gln Gly Pro Gly
            20              25              30

Gln Ser Gly Gln Tyr Gly Pro Gly Gln Gln Gly Pro Gly Gln Gln Gly
        35              40              45

Pro Gly Ser Ser Ala Ala Ala Ala Ala Gly Pro Gly Gln Tyr Val Leu
    50              55              60
```

```
Ile Gly Pro Gly Gln Gln Val Leu Ile Gly Pro Ser Ala Ser Ala Ala
65                  70                  75                  80


Ala Ala Ala Gly Pro Gly Ser Gly Gln Gln Gly Pro Gly Ala Ser Gly
                85                  90                  95


Gln Tyr Gly Pro Gly Gln Gln Gly Pro Gly Gln Gln Gly Pro Gly Ser
            100                 105                 110


Ser Ala Ala Ala Ala Ala Gly Ser Tyr Gly Ser Val Leu Ile Gly Pro
        115                 120                 125


Gly Gln Gln Val Leu Ile Gly Pro Tyr Gly Ser Ala Ala Ala Ala Ala
        130                 135                 140


Gly Pro Gly Ser Gly Gln Tyr Gly Gln Gly Pro Tyr Gly Pro Gly Ala
145                 150                 155                 160


Ser Gly Pro Gly Gln Tyr Gly Pro Gly Gln Gln Gly Pro Ser Ala Ser
            165                 170                 175


Ala Ala Ala Ala Ala Gly Ser Gly Gln Gln Val Leu Ile Gly Pro Gly
        180                 185                 190


Gln Tyr Val Leu Ile Gly Pro Tyr Ala Ser Ala Ala Ala Ala Ala Gly
        195                 200                 205


Gln Tyr Gly Ser Gly Pro Gly Gln Gln Gly Pro Tyr Gly Pro Gly Gln
        210                 215                 220


Ser Gly Ser Gly Gln Gln Gly Pro Gly Gln Gln Gly Pro Tyr Ala Ser
225                 230                 235                 240


Ala Ala Ala Ala Ala Gly Pro Gly Gln Gln Val Leu Ile Gly Pro Tyr
        245                 250                 255


Val Leu Ile Gly Pro Gly Ser Ser Ala Ala Ala Ala Ala Gly Gln Tyr
        260                 265                 270


Gly Tyr Gly Pro Gly Gln Gln Gly Pro Tyr Gly Pro Gly Ala Ser Gly
        275                 280                 285


Gln Asn Gly Pro Gly Ser Gly Gln Tyr Gly Pro Gly Gln Gln Gly Pro
        290                 295                 300


Gly Gln Ser Ala Ala Ala Ala Ala Gly Pro Gly Gln Gln Val Leu Ile
305                 310                 315                 320
```

97

```
Gly Pro Tyr Val Leu Ile Gly Pro Gly Ala Ser Ala Ala Ala Ala Ala
            325                 330                 335

Gly Gln Tyr Gly Pro Gly Gln Gln Gly Pro Gly Gln Tyr Gly Pro Gly
            340                 345                 350

Ser Ser Gly Pro Gly Gln Gln Gly Pro Tyr Gly Pro Gly Ser Ser Ala
            355                 360                 365

Ala Ala Ala Ala Gly Ser Tyr Gly Pro Gly Gln Gln Val Leu Ile Gly
    370                 375                 380

Pro Tyr Val Leu Ile Gly Pro Gly Pro Ser Ala Ala Ala Ala Ala Gly
385                 390                 395                 400

Gln Tyr Gln Gln Gly Pro Gly Gln Gln Gly Pro Tyr Gly Pro Gly Ala
            405                 410                 415

Ser Gly Pro Gly Gln Gln Gly Pro Tyr Gly Pro Gly Ala Ser Ala Ala
            420                 425                 430

Ala Ala Ala Gly Pro Gly Gln Tyr Val Leu Ile Gly Pro Gly Gln Gln
        435                 440                 445

Val Leu Ile Gly Pro Ser Ala Ser Ala Ala Ala Ala Gly Gln Tyr
    450                 455                 460

Gly Ser Gly Pro Gly Gln Tyr Gly Pro Tyr Gly Pro Gly Gln Ser Gly
465                 470                 475                 480

Pro Gly Ser Gly Gln Gln Gly Gln Gly Pro Tyr Gly Pro Gly Ala Ser
            485                 490                 495

Ala Ala Ala Ala Ala Gly Ser Tyr Gly Pro Gly Gln Gln Val Leu Ile
        500                 505                 510

Gly Pro Tyr Val Leu Ile Gly Pro Gly Pro Ser Ala Ala Ala Ala Ala
        515                 520                 525

Gly Pro Gly Ser Gly Gln Tyr Gly Pro Gly Ala Ser Gly Gln Asn Gly
    530                 535                 540

Pro Gly Ser Gly Gln Tyr Gly Pro Gly Gln Gln Gly Pro Gly Gln Ser
545                 550                 555                 560

Ala Ala Ala Ala Ala Gly Gln Tyr Gln Gln Val Leu Ile Gly Pro Gly
```

                                    565                          570                          575


        Gln Gln Gly Pro Tyr Val Leu Ile Gly Pro Gly Ala Ser Ala Ala Ala
                    580                     585                 590


        Ala Ala Gly Pro Gly Ser Gly Gln Gln Val Leu Ile Gly Pro Gly Ala
                    595                     600                 605


        Ser Val Leu Ile
            610


        <210>   20
        <211>   592
        <212>   PRT
        <213>   Artificial Sequence

        <220>
        <223>   Met-PRT665

        <400>   20

        Met Gly Pro Gly Gln Gln Gly Pro Tyr Gly Pro Gly Ala Ser Ala Ala
        1                   5                   10                  15


        Ala Ala Ala Ala Ala Gly Ser Asn Gly Pro Gly Ser Gly Gln Gln Gly
                    20                      25                  30


        Pro Gly Gln Ser Gly Gln Tyr Gly Pro Gly Gln Gln Gly Pro Gly Gln
                    35                      40                  45


        Gln Gly Pro Gly Ser Ser Ala Ala Ala Ala Ala Ala Gly Pro Gly
                    50                      55                  60


        Gln Tyr Val Leu Ile Gly Pro Gly Gln Gln Gly Pro Ser Ala Ser Ala
        65                      70                  75                  80


        Ala Ala Ala Ala Ala Gly Pro Gly Ser Gly Gln Gln Gly Pro Gly
                    85                      90                  95


        Ala Ser Gly Gln Tyr Gly Pro Gly Gln Gln Gly Pro Gly Gln Gln Gly
                    100                     105                 110


        Pro Gly Ser Ser Ala Ala Ala Ala Ala Ala Ala Gly Ser Tyr Gly Ser
                    115                     120                 125


        Val Leu Ile Gly Pro Gly Gln Gln Gly Pro Tyr Gly Ser Ala Ala Ala
            130                     135                 140


        Ala Ala Ala Ala Gly Pro Gly Ser Gly Gln Tyr Gly Gln Gly Pro Tyr
        145                     150                 155                 160

```
Gly Pro Gly Ala Ser Gly Pro Gly Gln Tyr Gly Pro Gly Gln Gln Gly
              165                 170                 175

Pro Ser Ala Ser Ala Ala Ala Ala Ala Ala Ala Gly Ser Gly Gln Gln
              180                 185                 190

Val Leu Ile Gly Pro Gly Gln Tyr Gly Pro Tyr Ala Ser Ala Ala Ala
              195                 200                 205

Ala Ala Ala Ala Gly Ser Tyr Gly Ser Gly Pro Gly Gln Gln Gly Pro
              210                 215                 220

Tyr Gly Pro Gly Gln Ser Gly Ser Gly Gln Gln Gly Pro Gly Gln Gln
225                 230                 235                 240

Gly Pro Tyr Ala Ser Ala Ala Ala Ala Ala Ala Gly Pro Gly Gln
              245                 250                 255

Gln Val Leu Ile Gly Pro Tyr Gly Pro Gly Ser Ser Ala Ala Ala Ala
              260                 265                 270

Ala Ala Ala Gly Ser Tyr Gly Tyr Gly Pro Gly Gln Gln Gly Pro Tyr
              275                 280                 285

Gly Pro Gly Ala Ser Gly Gln Asn Gly Pro Gly Ser Gly Gln Tyr Gly
              290                 295                 300

Pro Gly Gln Gln Gly Pro Gly Pro Ser Ala Ala Ala Ala Ala Ala Ala
305                 310                 315                 320

Gly Pro Gly Gln Gln Val Leu Ile Gly Pro Tyr Gly Pro Gly Ala Ser
              325                 330                 335

Ala Ala Ala Ala Ala Ala Ala Gly Ser Tyr Gly Pro Gly Gln Gln Gly
              340                 345                 350

Pro Gly Gln Tyr Gly Pro Gly Ser Ser Gly Pro Gly Gln Gln Gly Pro
              355                 360                 365

Tyr Gly Pro Gly Ser Ser Ala Ala Ala Ala Ala Ala Gly Ser Tyr
              370                 375                 380

Gly Pro Gly Gln Gln Val Leu Ile Gly Pro Tyr Gly Pro Gly Pro Ser
385                 390                 395                 400

Ala Ala Ala Ala Ala Ala Ala Gly Ser Tyr Gln Gln Gly Pro Gly Gln
```

                    405                     410                     415

Gln Gly Pro Tyr Gly Pro Gly Ala Ser Gly Pro Gly Gln Gln Gly Pro
            420                 425                 430

Tyr Gly Pro Gly Ala Ser Ala Ala Ala Ala Ala Ala Ala Gly Pro Gly
            435                 440                 445

Gln Tyr Val Leu Ile Gly Pro Gly Gln Gln Gly Pro Ser Ala Ser Ala
        450                 455                 460

Ala Ala Ala Ala Ala Ala Gly Ser Tyr Gly Ser Gly Pro Gly Gln Tyr
465                 470                 475                 480

Gly Pro Tyr Gly Pro Gly Gln Ser Gly Pro Gly Ser Gly Gln Gln Gly
            485                 490                 495

Gln Gly Pro Tyr Gly Pro Gly Ala Ser Ala Ala Ala Ala Ala Ala Ala
            500                 505                 510

Gly Ser Tyr Gly Pro Gly Gln Gln Val Leu Ile Gly Pro Tyr Gly Pro
        515                 520                 525

Gly Pro Ser Ala Ala Ala Ala Ala Ala Ala Gly Pro Gly Ser Gly Gln
        530                 535                 540

Tyr Gly Pro Gly Ala Ser Gly Gln Asn Gly Pro Gly Ser Gly Gln Tyr
545                 550                 555                 560

Gly Pro Gly Gln Gln Gly Pro Gly Pro Ser Ala Ala Ala Ala Ala Ala
            565                 570                 575

Ala Gly Pro Gly Ser Gly Gln Gln Gly Pro Gly Ala Ser Val Leu Ile
            580                 585                 590

<210> 21
<211> 619
<212> PRT
<213> Artificial Sequence

<220>
<223> Met-PRT666

<400> 21

Met Gly Pro Gly Gln Gln Gly Pro Tyr Gly Pro Gly Ala Ser Ala Ala
1               5                   10                  15

Ala Ala Ala Ala Ala Gly Ser Asn Gly Pro Gly Ser Gly Gln Gln Gly
            20                  25                  30

Pro Gly Gln Ser Gly Gln Tyr Gly Pro Gly Gln Gln Gly Pro Gly Gln
            35                  40                  45

Gln Gly Pro Gly Ser Ser Ala Ala Ala Ala Ala Ala Gly Pro Gly
        50                  55                  60

Gln Tyr Val Leu Ile Gly Pro Gly Gln Gln Val Leu Ile Gly Pro Ser
65                  70                  75                  80

Ala Ser Ala Ala Ala Ala Ala Ala Ala Gly Pro Gly Ser Gly Gln Gln
                85                  90                  95

Gly Pro Gly Ala Ser Gly Gln Tyr Gly Pro Gly Gln Gln Gly Pro Gly
            100                 105                 110

Gln Gln Gly Pro Gly Ser Ser Ala Ala Ala Ala Ala Ala Gly Ser
        115                 120                 125

Tyr Gly Ser Val Leu Ile Gly Pro Gly Gln Gln Val Leu Ile Gly Pro
    130                 135                 140

Tyr Gly Ser Ala Ala Ala Ala Ala Ala Gly Pro Gly Ser Gly Gln
145                 150                 155                 160

Tyr Gly Gln Gly Pro Tyr Gly Pro Gly Ala Ser Gly Pro Gly Gln Tyr
            165                 170                 175

Gly Pro Gly Gln Gln Gly Pro Ser Ala Ser Ala Ala Ala Ala Ala Ala
            180                 185                 190

Ala Gly Ser Gly Gln Gln Val Leu Ile Gly Pro Gly Gln Tyr Val Leu
        195                 200                 205

Ile Gly Pro Tyr Ala Ser Ala Ala Ala Ala Ala Ala Gly Ser Tyr
    210                 215                 220

Gly Ser Gly Pro Gly Gln Gln Gly Pro Tyr Gly Pro Gly Gln Ser Gly
225                 230                 235                 240

Ser Gly Gln Gln Gly Pro Gly Gln Gln Gly Pro Tyr Ala Ser Ala Ala
            245                 250                 255

Ala Ala Ala Ala Ala Gly Pro Gly Gln Gln Val Leu Ile Gly Pro Tyr
        260                 265                 270

Val Leu Ile Gly Pro Gly Ser Ser Ala Ala Ala Ala Ala Ala Ala Gly

```
                 275                      280                      285

    Ser Tyr Gly Tyr Gly Pro Gly Gln Gln Gly Pro Tyr Gly Pro Gly Ala
        290                      295                      300

    Ser Gly Gln Asn Gly Pro Gly Ser Gly Gln Tyr Gly Pro Gly Gln Gln
    305                  310                      315                  320

    Gly Pro Gly Pro Ser Ala Ala Ala Ala Ala Ala Gly Pro Gly Gln
                     325                  330                      335

    Gln Val Leu Ile Gly Pro Tyr Val Leu Ile Gly Pro Gly Ala Ser Ala
                 340                      345                      350

    Ala Ala Ala Ala Ala Ala Gly Ser Tyr Gly Pro Gly Gln Gln Gly Pro
             355                      360                      365

    Gly Gln Tyr Gly Pro Gly Ser Ser Gly Pro Gly Gln Gln Gly Pro Tyr
        370                      375                      380

    Gly Pro Gly Ser Ser Ala Ala Ala Ala Ala Ala Gly Ser Tyr Gly
    385                      390                      395                  400

    Pro Gly Gln Gln Val Leu Ile Gly Pro Tyr Val Leu Ile Gly Pro Gly
                     405                      410                      415

    Pro Ser Ala Ala Ala Ala Ala Ala Ala Gly Ser Tyr Gln Gln Gly Pro
             420                      425                      430

    Gly Gln Gln Gly Pro Tyr Gly Pro Gly Ala Ser Gly Pro Gly Gln Gln
             435                      440                      445

    Gly Pro Tyr Gly Pro Gly Ala Ser Ala Ala Ala Ala Ala Ala Ala Gly
        450                      455                      460

    Pro Gly Gln Tyr Val Leu Ile Gly Pro Gly Gln Gln Val Leu Ile Gly
    465                  470                      475                  480

    Pro Ser Ala Ser Ala Ala Ala Ala Ala Ala Gly Ser Tyr Gly Ser
             485                      490                      495

    Gly Pro Gly Gln Tyr Gly Pro Tyr Gly Pro Gly Gln Ser Gly Pro Gly
             500                      505                      510

    Ser Gly Gln Gln Gly Gln Gly Pro Tyr Gly Pro Gly Ala Ser Ala Ala
        515                      520                      525
```

Ala Ala Ala Ala Ala Gly Ser Tyr Gly Pro Gly Gln Gln Val Leu Ile
530                 535                 540

Gly Pro Tyr Val Leu Ile Gly Pro Gly Pro Ser Ala Ala Ala Ala Ala
545                 550                 555                 560

Ala Ala Gly Pro Gly Ser Gly Gln Tyr Gly Pro Gly Ala Ser Gly Gln
                565                 570                 575

Asn Gly Pro Gly Ser Gly Gln Tyr Gly Pro Gly Gln Gln Gly Pro Gly
            580                 585                 590

Pro Ser Ala Ala Ala Ala Ala Ala Ala Gly Pro Gly Ser Gly Gln Gln
        595                 600                 605

Val Leu Ile Gly Pro Gly Ala Ser Val Leu Ile
    610                 615


<210>  22
<211>  623
<212>  PRT
<213>  Artificial Sequence

<220>
<223>  PRT720

<400>  22

Met His His His His His His Ser Ser Gly Ser Ser Gly Pro Gly Gln
1                   5                   10                  15

Gln Gly Pro Tyr Gly Pro Gly Ala Ser Ala Ala Ala Ala Ala Gly Gln
            20                  25                  30

Asn Gly Pro Gly Ser Gly Gln Gln Gly Pro Gly Gln Ser Gly Gln Tyr
            35                  40                  45

Gly Pro Gly Gln Gln Gly Pro Gly Gln Gln Gly Pro Gly Ser Ser Ala
    50                  55                  60

Ala Ala Ala Gly Pro Gly Gln Tyr Val Leu Ile Gly Pro Gly Gln
65                  70                  75                  80

Gln Val Leu Ile Gly Pro Ser Ala Ser Ala Ala Ala Ala Ala Gly Pro
                85                  90                  95

Gly Ser Gly Gln Gln Gly Pro Gly Ala Ser Gly Gln Tyr Gly Pro Gly
            100                 105                 110

Gln Gln Gly Pro Gly Gln Gln Gly Pro Gly Ser Ser Ala Ala Ala Ala

115 120 125

Ala Gly Ser Tyr Gly Ser Val Leu Ile Gly Pro Gly Gln Gln Val Leu
    130                 135                 140

Ile Gly Pro Tyr Gly Ser Ala Ala Ala Ala Ala Gly Pro Gly Ser Gly
145                 150                 155                 160

Gln Tyr Gly Gln Gly Pro Tyr Gly Pro Gly Ala Ser Gly Pro Gly Gln
                165                 170                 175

Tyr Gly Pro Gly Gln Gln Gly Pro Ser Ala Ser Ala Ala Ala Ala Ala
                180                 185                 190

Gly Ser Gly Gln Gln Val Leu Ile Gly Pro Gly Gln Tyr Val Leu Ile
                195                 200                 205

Gly Pro Tyr Ala Ser Ala Ala Ala Ala Ala Gly Gln Tyr Gly Ser Gly
    210                 215                 220

Pro Gly Gln Gln Gly Pro Tyr Gly Pro Gly Gln Ser Gly Ser Gly Gln
225                 230                 235                 240

Gln Gly Pro Gly Gln Gln Gly Pro Tyr Ala Ser Ala Ala Ala Ala Ala
                245                 250                 255

Gly Pro Gly Gln Gln Val Leu Ile Gly Pro Tyr Val Leu Ile Gly Pro
                260                 265                 270

Gly Ser Ser Ala Ala Ala Ala Ala Gly Gln Tyr Gly Tyr Gly Pro Gly
                275                 280                 285

Gln Gln Gly Pro Tyr Gly Pro Gly Ala Ser Gly Gln Asn Gly Pro Gly
    290                 295                 300

Ser Gly Gln Tyr Gly Pro Gly Gln Gln Gly Pro Gly Gln Ser Ala Ala
305                 310                 315                 320

Ala Ala Ala Gly Pro Gly Gln Gln Val Leu Ile Gly Pro Tyr Val Leu
                325                 330                 335

Ile Gly Pro Gly Ala Ser Ala Ala Ala Ala Ala Gly Gln Tyr Gly Pro
                340                 345                 350

Gly Gln Gln Gly Pro Gly Gln Tyr Gly Pro Gly Ser Ser Gly Pro Gly
    355                 360                 365

```
Gln Gln Gly Pro Tyr Gly Pro Gly Ser Ser Ala Ala Ala Ala Ala Gly
    370                 375             380

Ser Tyr Gly Pro Gly Gln Gln Val Leu Ile Gly Pro Tyr Val Leu Ile
    385             390             395                 400

Gly Pro Gly Pro Ser Ala Ala Ala Ala Ala Gly Gln Tyr Gln Gln Gly
                405             410                 415

Pro Gly Gln Gln Gly Pro Tyr Gly Pro Gly Ala Ser Gly Pro Gly Gln
            420             425             430

Gln Gly Pro Tyr Gly Pro Gly Ala Ser Ala Ala Ala Ala Ala Gly Pro
        435             440             445

Gly Gln Tyr Val Leu Ile Gly Pro Gly Gln Gln Val Leu Ile Gly Pro
    450             455             460

Ser Ala Ser Ala Ala Ala Ala Ala Gly Gln Tyr Gly Ser Gly Pro Gly
465             470             475                 480

Gln Tyr Gly Pro Tyr Gly Pro Gly Gln Ser Gly Pro Gly Ser Gly Gln
            485             490             495

Gln Gly Gln Gly Pro Tyr Gly Pro Gly Ala Ser Ala Ala Ala Ala Ala
            500             505             510

Gly Ser Tyr Gly Pro Gly Gln Gln Val Leu Ile Gly Pro Tyr Val Leu
        515             520             525

Ile Gly Pro Gly Pro Ser Ala Ala Ala Ala Gly Pro Gly Ser Gly
    530             535             540

Gln Tyr Gly Pro Gly Ala Ser Gly Gln Asn Gly Pro Gly Ser Gly Gln
545             550             555                 560

Tyr Gly Pro Gly Gln Gln Gly Pro Gly Gln Ser Ala Ala Ala Ala Ala
            565             570             575

Gly Gln Tyr Gln Gln Val Leu Ile Gly Pro Gly Gln Gln Gly Pro Tyr
        580             585             590

Val Leu Ile Gly Pro Gly Ala Ser Ala Ala Ala Ala Gly Pro Gly
    595             600             605

Ser Gly Gln Gln Val Leu Ile Gly Pro Gly Ala Ser Val Leu Ile
    610             615             620
```

<210> 23
<211> 603
<212> PRT
<213> Artificial Sequence

<220>
<223> PRT665

<400> 23

Met His His His His His His Ser Ser Gly Ser Ser Gly Pro Gly Gln
1               5                   10                  15

Gln Gly Pro Tyr Gly Pro Gly Ala Ser Ala Ala Ala Ala Ala Ala Ala
                20                  25                  30

Gly Ser Asn Gly Pro Gly Ser Gly Gln Gln Gly Pro Gly Gln Ser Gly
            35                  40                  45

Gln Tyr Gly Pro Gly Gln Gln Gly Pro Gly Gln Gln Gly Pro Gly Ser
        50                  55                  60

Ser Ala Ala Ala Ala Ala Ala Ala Gly Pro Gly Gln Tyr Val Leu Ile
65                  70                  75                  80

Gly Pro Gly Gln Gln Gly Pro Ser Ala Ser Ala Ala Ala Ala Ala Ala
                85                  90                  95

Ala Gly Pro Gly Ser Gly Gln Gln Gly Pro Gly Ala Ser Gly Gln Tyr
            100                 105                 110

Gly Pro Gly Gln Gln Gly Pro Gly Gln Gln Gly Pro Gly Ser Ser Ala
        115                 120                 125

Ala Ala Ala Ala Ala Ala Gly Ser Tyr Gly Ser Val Leu Ile Gly Pro
    130                 135                 140

Gly Gln Gln Gly Pro Tyr Gly Ser Ala Ala Ala Ala Ala Ala Ala Gly
145                 150                 155                 160

Pro Gly Ser Gly Gln Tyr Gly Gln Gly Pro Tyr Gly Pro Gly Ala Ser
            165                 170                 175

Gly Pro Gly Gln Tyr Gly Pro Gly Gln Gln Gly Pro Ser Ala Ser Ala
        180                 185                 190

Ala Ala Ala Ala Ala Gly Ser Gly Gln Gln Val Leu Ile Gly Pro
    195                 200                 205

Gly Gln Tyr Gly Pro Tyr Ala Ser Ala Ala Ala Ala Ala Ala Gly
    210                 215             220

Ser Tyr Gly Ser Gly Pro Gly Gln Gln Gly Pro Tyr Gly Pro Gly Gln
225             230                 235                         240

Ser Gly Ser Gly Gln Gln Gly Pro Gly Gln Gln Gly Pro Tyr Ala Ser
                245                 250                 255

Ala Ala Ala Ala Ala Ala Ala Gly Pro Gly Gln Gln Val Leu Ile Gly
            260                 265                 270

Pro Tyr Gly Pro Gly Ser Ser Ala Ala Ala Ala Ala Ala Gly Ser
        275                 280                 285

Tyr Gly Tyr Gly Pro Gly Gln Gln Gly Pro Tyr Gly Pro Gly Ala Ser
    290                 295                 300

Gly Gln Asn Gly Pro Gly Ser Gly Gln Tyr Gly Pro Gly Gln Gln Gly
305                 310                 315                     320

Pro Gly Pro Ser Ala Ala Ala Ala Ala Ala Gly Pro Gly Gln Gln
            325                 330                 335

Val Leu Ile Gly Pro Tyr Gly Pro Gly Ala Ser Ala Ala Ala Ala Ala
            340                 345                 350

Ala Ala Gly Ser Tyr Gly Pro Gly Gln Gln Gly Pro Gly Gln Tyr Gly
        355                 360                 365

Pro Gly Ser Ser Gly Pro Gly Gln Gln Gly Pro Tyr Gly Pro Gly Ser
    370                 375                 380

Ser Ala Ala Ala Ala Ala Ala Ala Gly Ser Tyr Gly Pro Gly Gln Gln
385                 390                 395                     400

Val Leu Ile Gly Pro Tyr Gly Pro Gly Pro Ser Ala Ala Ala Ala Ala
            405                 410                 415

Ala Ala Gly Ser Tyr Gln Gln Gly Pro Gly Gln Gln Gly Pro Tyr Gly
        420                 425                 430

Pro Gly Ala Ser Gly Pro Gly Gln Gln Gly Pro Tyr Gly Pro Gly Ala
        435                 440                 445

Ser Ala Ala Ala Ala Ala Ala Ala Gly Pro Gly Gln Tyr Val Leu Ile
    450                 455                 460

Gly Pro Gly Gln Gln Gly Pro Ser Ala Ser Ala Ala Ala Ala Ala Ala
465                 470             475                 480

Ala Gly Ser Tyr Gly Ser Gly Pro Gly Gln Tyr Gly Pro Tyr Gly Pro
                485             490                 495

Gly Gln Ser Gly Pro Gly Ser Gly Gln Gln Gly Gln Gly Pro Tyr Gly
            500             505             510

Pro Gly Ala Ser Ala Ala Ala Ala Ala Ala Gly Ser Tyr Gly Pro
            515             520             525

Gly Gln Gln Val Leu Ile Gly Pro Tyr Gly Pro Gly Pro Ser Ala Ala
        530             535             540

Ala Ala Ala Ala Ala Gly Pro Gly Ser Gly Gln Tyr Gly Pro Gly Ala
545             550             555                 560

Ser Gly Gln Asn Gly Pro Gly Ser Gly Gln Tyr Gly Pro Gly Gln Gln
            565             570             575

Gly Pro Gly Pro Ser Ala Ala Ala Ala Ala Ala Gly Pro Gly Ser
            580             585             590

Gly Gln Gln Gly Pro Gly Ala Ser Val Leu Ile
        595             600


<210> 24
<211> 630
<212> PRT
<213> Artificial Sequence

<220>
<223> PRT666

<400> 24

Met His His His His His His Ser Ser Gly Ser Ser Gly Pro Gly Gln
1               5               10              15

Gln Gly Pro Tyr Gly Pro Gly Ala Ser Ala Ala Ala Ala Ala Ala Ala
            20              25                  30

Gly Ser Asn Gly Pro Gly Ser Gly Gln Gln Gly Pro Gly Gln Ser Gly
        35              40                  45

Gln Tyr Gly Pro Gly Gln Gln Gly Pro Gly Gln Gln Gly Pro Gly Ser
    50              55                  60

Ser Ala Ala Ala Ala Ala Ala Ala Gly Pro Gly Gln Tyr Val Leu Ile
65                  70              75                  80

Gly Pro Gly Gln Gln Val Leu Ile Gly Pro Ser Ala Ser Ala Ala Ala
                85                  90                  95

Ala Ala Ala Ala Gly Pro Gly Ser Gly Gln Gln Gly Pro Gly Ala Ser
            100                 105                 110

Gly Gln Tyr Gly Pro Gly Gln Gln Gly Pro Gly Gln Gln Gly Pro Gly
        115                 120                 125

Ser Ser Ala Ala Ala Ala Ala Ala Gly Ser Tyr Gly Ser Val Leu
    130                 135                 140

Ile Gly Pro Gly Gln Gln Val Leu Ile Gly Pro Tyr Gly Ser Ala Ala
145                 150                 155                 160

Ala Ala Ala Ala Ala Gly Pro Gly Ser Gly Gln Tyr Gly Gln Gly Pro
            165                 170                 175

Tyr Gly Pro Gly Ala Ser Gly Pro Gly Gln Tyr Gly Pro Gly Gln Gln
        180                 185                 190

Gly Pro Ser Ala Ser Ala Ala Ala Ala Ala Ala Gly Ser Gly Gln
        195                 200                 205

Gln Val Leu Ile Gly Pro Gly Gln Tyr Val Leu Ile Gly Pro Tyr Ala
    210                 215                 220

Ser Ala Ala Ala Ala Ala Ala Gly Ser Tyr Gly Ser Gly Pro Gly
225                 230                 235                 240

Gln Gln Gly Pro Tyr Gly Pro Gly Gln Ser Gly Ser Gly Gln Gln Gly
            245                 250                 255

Pro Gly Gln Gln Gly Pro Tyr Ala Ser Ala Ala Ala Ala Ala Ala Ala
        260                 265                 270

Gly Pro Gly Gln Gln Val Leu Ile Gly Pro Tyr Val Leu Ile Gly Pro
        275                 280                 285

Gly Ser Ser Ala Ala Ala Ala Ala Ala Gly Ser Tyr Gly Tyr Gly
        290                 295                 300

Pro Gly Gln Gln Gly Pro Tyr Gly Pro Gly Ala Ser Gly Gln Asn Gly
305                 310                 315                 320

Pro Gly Ser Gly Gln Tyr Gly Pro Gly Gln Gln Gly Pro Gly Pro Ser
            325                 330                 335

Ala Ala Ala Ala Ala Ala Ala Gly Pro Gly Gln Gln Val Leu Ile Gly
            340                 345                 350

Pro Tyr Val Leu Ile Gly Pro Gly Ala Ser Ala Ala Ala Ala Ala Ala
        355                 360                 365

Ala Gly Ser Tyr Gly Pro Gly Gln Gln Gly Pro Gly Gln Tyr Gly Pro
    370                 375                 380

Gly Ser Ser Gly Pro Gly Gln Gln Gly Pro Tyr Gly Pro Gly Ser Ser
385                 390                 395                 400

Ala Ala Ala Ala Ala Ala Ala Gly Ser Tyr Gly Pro Gly Gln Gln Val
            405                 410                 415

Leu Ile Gly Pro Tyr Val Leu Ile Gly Pro Gly Pro Ser Ala Ala Ala
            420                 425                 430

Ala Ala Ala Ala Gly Ser Tyr Gln Gln Gly Pro Gly Gln Gln Gly Pro
            435                 440                 445

Tyr Gly Pro Gly Ala Ser Gly Pro Gly Gln Gln Gly Pro Tyr Gly Pro
    450                 455                 460

Gly Ala Ser Ala Ala Ala Ala Ala Ala Gly Pro Gly Gln Tyr Val
465                 470                 475                 480

Leu Ile Gly Pro Gly Gln Gln Val Leu Ile Gly Pro Ser Ala Ser Ala
            485                 490                 495

Ala Ala Ala Ala Ala Ala Gly Ser Tyr Gly Ser Gly Pro Gly Gln Tyr
            500                 505                 510

Gly Pro Tyr Gly Pro Gly Gln Ser Gly Pro Gly Ser Gly Gln Gln Gly
        515                 520                 525

Gln Gly Pro Tyr Gly Pro Gly Ala Ser Ala Ala Ala Ala Ala Ala Ala
    530                 535                 540

Gly Ser Tyr Gly Pro Gly Gln Gln Val Leu Ile Gly Pro Tyr Val Leu
545                 550                 555                 560

Ile Gly Pro Gly Pro Ser Ala Ala Ala Ala Ala Ala Ala Gly Pro Gly
            565                 570                 575

111

```
Ser Gly Gln Tyr Gly Pro Gly Ala Ser Gly Gln Asn Gly Pro Gly Ser
        580             585             590

Gly Gln Tyr Gly Pro Gly Gln Gln Gly Pro Gly Pro Ser Ala Ala Ala
        595             600             605

Ala Ala Ala Ala Gly Pro Gly Ser Gly Gln Gln Val Leu Ile Gly Pro
    610             615             620

Gly Ala Ser Val Leu Ile
625             630
```

```
<210>   25
<211>   593
<212>   PRT
<213>   Artificial Sequence

<220>
<223>   M_PRT888

<400>   25
```

```
Met Gly Ser Ser Gly Pro Gly Val Leu Gly Pro Tyr Gly Pro Gly Ala
1               5               10              15

Ser Ala Ala Ala Ala Ala Gly Gln Asn Gly Pro Gly Ser Gly Val Leu
        20              25              30

Gly Pro Gly Gln Ser Gly Gln Tyr Gly Pro Gly Val Leu Gly Pro Gly
        35              40              45

Val Leu Gly Pro Gly Ser Ser Ala Ala Ala Ala Ala Gly Pro Gly Gln
    50              55              60

Tyr Gly Pro Gly Val Leu Gly Pro Ser Ala Ser Ala Ala Ala Ala Ala
65              70              75              80

Gly Pro Gly Ser Gly Val Leu Gly Pro Gly Ala Ser Gly Gln Tyr Gly
            85              90              95

Pro Gly Val Leu Gly Pro Gly Val Leu Gly Pro Gly Ser Ser Ala Ala
        100             105             110

Ala Ala Ala Gly Gln Tyr Gly Ser Gly Pro Gly Val Leu Gly Pro Tyr
        115             120             125

Gly Ser Ala Ala Ala Ala Ala Gly Pro Gly Ser Gly Gln Tyr Gly Gln
    130             135             140
```

Gly Pro Tyr Gly Pro Gly Ala Ser Gly Pro Gly Gln Tyr Gly Pro Gly
145                 150                 155                 160

Val Leu Gly Pro Ser Ala Ser Ala Ala Ala Ala Ala Gly Ser Gly Val
                165                 170                 175

Leu Gly Pro Gly Gln Tyr Gly Pro Tyr Ala Ser Ala Ala Ala Ala Ala
                180                 185                 190

Gly Gln Tyr Gly Ser Gly Pro Gly Val Leu Gly Pro Tyr Gly Pro Gly
                195                 200                 205

Gln Ser Gly Ser Gly Val Leu Gly Pro Gly Val Leu Gly Pro Tyr Ala
    210                 215                 220

Ser Ala Ala Ala Ala Ala Gly Pro Gly Val Leu Gly Pro Tyr Gly Pro
225                 230                 235                 240

Gly Ser Ser Ala Ala Ala Ala Ala Gly Gln Tyr Gly Tyr Gly Pro Gly
                245                 250                 255

Val Leu Gly Pro Tyr Gly Pro Gly Ala Ser Gly Gln Asn Gly Pro Gly
                260                 265                 270

Ser Gly Gln Tyr Gly Pro Gly Val Leu Gly Pro Gly Gln Ser Ala Ala
                275                 280                 285

Ala Ala Ala Gly Pro Gly Val Leu Gly Pro Tyr Gly Pro Gly Ala Ser
    290                 295                 300

Ala Ala Ala Ala Ala Gly Gln Tyr Gly Pro Gly Val Leu Gly Pro Gly
305                 310                 315                 320

Gln Tyr Gly Pro Gly Ser Ser Gly Pro Gly Val Leu Gly Pro Tyr Gly
                325                 330                 335

Pro Gly Ser Ser Ala Ala Ala Ala Ala Gly Gln Tyr Gly Pro Gly Val
                340                 345                 350

Leu Gly Pro Tyr Gly Pro Gly Gln Ser Ala Ala Ala Ala Ala Gly Gln
                355                 360                 365

Tyr Val Leu Gly Pro Gly Val Leu Gly Pro Tyr Gly Pro Gly Ala Ser
    370                 375                 380

Gly Pro Gly Val Leu Gly Pro Tyr Gly Pro Gly Ala Ser Ala Ala Ala
385                 390                 395                 400

```
Ala Ala Gly Pro Gly Gln Tyr Gly Pro Gly Val Leu Gly Pro Ser Ala
            405             410                     415

Ser Ala Ala Ala Ala Ala Gly Gln Tyr Gly Ser Gly Pro Gly Gln Tyr
            420             425             430

Gly Pro Tyr Gly Pro Gly Gln Ser Gly Pro Gly Ser Gly Val Leu Gly
            435             440             445

Gln Gly Pro Tyr Gly Pro Gly Ala Ser Ala Ala Ala Ala Ala Gly Gln
        450             455             460

Tyr Gly Pro Gly Val Leu Gly Pro Tyr Gly Pro Gly Gln Ser Ala Ala
465             470             475                     480

Ala Ala Ala Gly Pro Gly Ser Gly Gln Tyr Gly Pro Gly Ala Ser Gly
            485             490             495

Gln Asn Gly Pro Gly Ser Gly Gln Tyr Gly Pro Gly Val Leu Gly Pro
            500             505             510

Gly Gln Ser Ala Ala Ala Ala Ala Gly Gln Tyr Val Leu Gly Pro Gly
        515             520             525

Val Leu Gly Pro Tyr Gly Pro Gly Ala Ser Ala Ala Ala Ala Ala Gly
        530             535             540

Gln Tyr Gly Ser Gly Pro Gly Val Leu Gly Pro Tyr Gly Pro Gly Gln
545             550             555             560

Ser Gly Ser Gly Val Leu Gly Pro Gly Val Leu Gly Pro Tyr Ala Ser
            565             570             575

Ala Ala Ala Ala Ala Gly Pro Gly Ser Gly Val Leu Gly Pro Gly Ala
            580             585             590

Ser
```

```
<210>  26
<211>  590
<212>  PRT
<213>  Artificial Sequence

<220>
<223>  M_PRT965

<400>  26
```

```
Met Gly Pro Gly Thr Ser Gly Pro Tyr Gly Pro Gly Ala Ser Ala Ala
1               5                   10              15

Ala Ala Ala Gly Ala Asn Gly Pro Gly Ser Gly Thr Ser Gly Pro Gly
            20                  25              30

Ala Ser Gly Ala Tyr Gly Pro Gly Thr Ser Gly Pro Gly Thr Ser Gly
            35                  40              45

Pro Gly Ser Ser Ala Ala Ala Ala Ala Gly Pro Gly Ala Tyr Gly Pro
        50              55              60

Gly Thr Ser Gly Pro Ser Ala Ser Ala Ala Ala Ala Gly Pro Gly
65                  70              75              80

Ser Gly Thr Ser Gly Pro Gly Ala Ser Gly Ala Tyr Gly Pro Gly Thr
                85              90              95

Ser Gly Pro Gly Thr Ser Gly Pro Gly Ser Ser Ala Ala Ala Ala Ala
            100             105             110

Gly Ala Tyr Gly Ser Gly Pro Gly Thr Ser Gly Pro Tyr Gly Ser Ala
    115                 120             125

Ala Ala Ala Ala Gly Pro Gly Ser Gly Ala Tyr Gly Ala Gly Pro Tyr
130             135             140

Gly Pro Gly Ala Ser Gly Pro Gly Ala Tyr Gly Pro Gly Thr Ser Gly
145             150             155             160

Pro Ser Ala Ser Ala Ala Ala Ala Ala Gly Ser Gly Thr Ser Gly Pro
            165             170             175

Gly Ala Tyr Gly Pro Tyr Ala Ser Ala Ala Ala Ala Gly Ala Tyr
    180             185             190

Gly Ser Gly Pro Gly Thr Ser Gly Pro Tyr Gly Pro Gly Ala Ser Gly
    195             200             205

Ser Gly Thr Ser Gly Pro Gly Thr Ser Gly Pro Tyr Ala Ser Ala Ala
    210             215             220

Ala Ala Ala Gly Pro Gly Thr Ser Gly Pro Tyr Gly Pro Gly Ser Ser
225             230             235             240

Ala Ala Ala Ala Ala Gly Ala Tyr Gly Tyr Gly Pro Gly Thr Ser Gly
            245             250             255
```

Pro Tyr Gly Pro Gly Ala Ser Gly Ala Asn Gly Pro Gly Ser Gly Ala
        260                 265                 270

Tyr Gly Pro Gly Thr Ser Gly Pro Gly Ala Ser Ala Ala Ala Ala Ala
        275                 280                 285

Gly Pro Gly Thr Ser Gly Pro Tyr Gly Pro Gly Ala Ser Ala Ala Ala
        290                 295                 300

Ala Ala Gly Ala Tyr Gly Pro Gly Thr Ser Gly Pro Gly Ala Tyr Gly
305                 310                 315                 320

Pro Gly Ser Ser Gly Pro Gly Thr Ser Gly Pro Tyr Gly Pro Gly Ser
                325                 330                 335

Ser Ala Ala Ala Ala Ala Gly Ala Tyr Gly Pro Gly Thr Ser Gly Pro
                340                 345                 350

Tyr Gly Pro Gly Ala Ser Ala Ala Ala Ala Ala Gly Ala Tyr Thr Ser
        355                 360                 365

Gly Pro Gly Thr Ser Gly Pro Tyr Gly Pro Gly Ala Ser Gly Pro Gly
        370                 375                 380

Thr Ser Gly Pro Tyr Gly Pro Gly Ala Ser Ala Ala Ala Ala Ala Gly
385                 390                 395                 400

Pro Gly Ala Tyr Gly Pro Gly Thr Ser Gly Pro Ser Ala Ser Ala Ala
                405                 410                 415

Ala Ala Ala Gly Ala Tyr Gly Ser Gly Pro Gly Ala Tyr Gly Pro Tyr
                420                 425                 430

Gly Pro Gly Ala Ser Gly Pro Gly Ser Gly Thr Ser Gly Ala Gly Pro
        435                 440                 445

Tyr Gly Pro Gly Ala Ser Ala Ala Ala Ala Gly Ala Tyr Gly Pro
        450                 455                 460

Gly Thr Ser Gly Pro Tyr Gly Pro Gly Ala Ser Ala Ala Ala Ala Ala
465                 470                 475                 480

Gly Pro Gly Ser Gly Ala Tyr Gly Pro Gly Ala Ser Gly Ala Asn Gly
                485                 490                 495

Pro Gly Ser Gly Ala Tyr Gly Pro Gly Thr Ser Gly Pro Gly Ala Ser

```
                  500                     505                     510


        Ala Ala Ala Ala Ala Gly Ala Tyr Thr Ser Gly Pro Gly Thr Ser Gly
                515                 520                 525


        Pro Tyr Gly Pro Gly Ala Ser Ala Ala Ala Ala Ala Gly Ala Tyr Gly
                530                 535                 540


        Ser Gly Pro Gly Thr Ser Gly Pro Tyr Gly Pro Gly Ala Ser Gly Ser
        545                 550                 555                 560


        Gly Thr Ser Gly Pro Gly Thr Ser Gly Pro Tyr Ala Ser Ala Ala Ala
                        565                 570                 575


        Ala Ala Gly Pro Gly Ser Gly Thr Ser Gly Pro Gly Ala Ser
                580                 585                 590


        <210>   27
        <211>   593
        <212>   PRT
        <213>   Artificial Sequence

        <220>
        <223>   M_PRT889

        <400>   27

        Met Gly Ser Ser Gly Pro Gly Val Leu Gly Pro Tyr Gly Pro Gly Ala
        1               5                   10                  15


        Ser Ala Ala Ala Ala Ala Gly Ile Asn Gly Pro Gly Ser Gly Val Leu
                20                  25                  30


        Gly Pro Gly Ile Ser Gly Ile Tyr Gly Pro Gly Val Leu Gly Pro Gly
                35                  40                  45


        Val Leu Gly Pro Gly Ser Ser Ala Ala Ala Ala Ala Gly Pro Gly Ile
                50                  55                  60


        Tyr Gly Pro Gly Val Leu Gly Pro Ser Ala Ser Ala Ala Ala Ala Ala
        65                  70                  75                  80


        Gly Pro Gly Ser Gly Val Leu Gly Pro Gly Ala Ser Gly Ile Tyr Gly
                        85                  90                  95


        Pro Gly Val Leu Gly Pro Gly Val Leu Gly Pro Gly Ser Ser Ala Ala
                100                 105                 110


        Ala Ala Ala Gly Ile Tyr Gly Ser Gly Pro Gly Val Leu Gly Pro Tyr
                115                 120                 125
```

```
Gly Ser Ala Ala Ala Ala Ala Gly Pro Gly Ser Gly Ile Tyr Gly Ile
    130                 135                 140

Gly Pro Tyr Gly Pro Gly Ala Ser Gly Pro Gly Ile Tyr Gly Pro Gly
145                 150                 155                 160

Val Leu Gly Pro Ser Ala Ser Ala Ala Ala Ala Gly Ser Gly Val
                165                 170                 175

Leu Gly Pro Gly Ile Tyr Gly Pro Tyr Ala Ser Ala Ala Ala Ala Ala
            180                 185                 190

Gly Ile Tyr Gly Ser Gly Pro Gly Val Leu Gly Pro Tyr Gly Pro Gly
            195                 200                 205

Ile Ser Gly Ser Gly Val Leu Gly Pro Gly Val Leu Gly Pro Tyr Ala
    210                 215                 220

Ser Ala Ala Ala Ala Ala Gly Pro Gly Val Leu Gly Pro Tyr Gly Pro
225                 230                 235                 240

Gly Ser Ser Ala Ala Ala Ala Ala Gly Ile Tyr Gly Tyr Gly Pro Gly
                245                 250                 255

Val Leu Gly Pro Tyr Gly Pro Gly Ala Ser Gly Ile Asn Gly Pro Gly
            260                 265                 270

Ser Gly Ile Tyr Gly Pro Gly Val Leu Gly Pro Gly Ile Ser Ala Ala
            275                 280                 285

Ala Ala Ala Gly Pro Gly Val Leu Gly Pro Tyr Gly Pro Gly Ala Ser
    290                 295                 300

Ala Ala Ala Ala Ala Gly Ile Tyr Gly Pro Gly Val Leu Gly Pro Gly
305                 310                 315                 320

Ile Tyr Gly Pro Gly Ser Ser Gly Pro Gly Val Leu Gly Pro Tyr Gly
            325                 330                 335

Pro Gly Ser Ser Ala Ala Ala Ala Ala Gly Ile Tyr Gly Pro Gly Val
            340                 345                 350

Leu Gly Pro Tyr Gly Pro Gly Ile Ser Ala Ala Ala Ala Ala Gly Ile
    355                 360                 365

Tyr Val Leu Gly Pro Gly Val Leu Gly Pro Tyr Gly Pro Gly Ala Ser
```

                    370                         375                          380


Gly Pro Gly Val Leu Gly Pro Tyr Gly Pro Gly Ala Ser Ala Ala Ala
385                 390                 395                 400


Ala Ala Gly Pro Gly Ile Tyr Gly Pro Gly Val Leu Gly Pro Ser Ala
            405                 410                 415


Ser Ala Ala Ala Ala Ala Gly Ile Tyr Gly Ser Gly Pro Gly Ile Tyr
        420                 425                 430


Gly Pro Tyr Gly Pro Gly Ile Ser Gly Pro Gly Ser Gly Val Leu Gly
        435                 440                 445


Ile Gly Pro Tyr Gly Pro Gly Ala Ser Ala Ala Ala Ala Gly Ile
    450                 455                 460


Tyr Gly Pro Gly Val Leu Gly Pro Tyr Gly Pro Gly Ile Ser Ala Ala
465                 470                 475                 480


Ala Ala Ala Gly Pro Gly Ser Gly Ile Tyr Gly Pro Gly Ala Ser Gly
            485                 490                 495


Ile Asn Gly Pro Gly Ser Gly Ile Tyr Gly Pro Gly Val Leu Gly Pro
            500                 505                 510


Gly Ile Ser Ala Ala Ala Ala Ala Gly Ile Tyr Val Leu Gly Pro Gly
        515                 520                 525


Val Leu Gly Pro Tyr Gly Pro Gly Ala Ser Ala Ala Ala Ala Ala Gly
        530                 535                 540


Ile Tyr Gly Ser Gly Pro Gly Val Leu Gly Pro Tyr Gly Pro Gly Ile
545                 550                 555                 560


Ser Gly Ser Gly Val Leu Gly Pro Gly Val Leu Gly Pro Tyr Ala Ser
                565                 570                 575


Ala Ala Ala Ala Ala Gly Pro Gly Ser Gly Val Leu Gly Pro Gly Ala
            580                 585                 590


Ser


<210>    28
<211>    590
<212>    PRT
<213>    Artificial Sequence

<220>
<223> M_PRT916

<400> 28

Met Gly Pro Gly Val Ile Gly Pro Tyr Gly Pro Gly Ala Ser Ala Ala
1               5                   10                  15

Ala Ala Ala Gly Leu Asn Gly Pro Gly Ser Gly Val Ile Gly Pro Gly
                20                  25                  30

Leu Ser Gly Leu Tyr Gly Pro Gly Val Ile Gly Pro Gly Val Ile Gly
            35                  40                  45

Pro Gly Ser Ser Ala Ala Ala Ala Ala Gly Pro Gly Leu Tyr Gly Pro
        50                  55                  60

Gly Val Ile Gly Pro Ser Ala Ser Ala Ala Ala Ala Gly Pro Gly
65                  70                  75                  80

Ser Gly Val Ile Gly Pro Gly Ala Ser Gly Leu Tyr Gly Pro Gly Val
                85                  90                  95

Ile Gly Pro Gly Val Ile Gly Pro Gly Ser Ser Ala Ala Ala Ala Ala
            100                 105                 110

Gly Leu Tyr Gly Ser Gly Pro Gly Val Ile Gly Pro Tyr Gly Ser Ala
        115                 120                 125

Ala Ala Ala Ala Gly Pro Gly Ser Gly Leu Tyr Gly Leu Gly Pro Tyr
        130                 135                 140

Gly Pro Gly Ala Ser Gly Pro Gly Leu Tyr Gly Pro Gly Val Ile Gly
145                 150                 155                 160

Pro Ser Ala Ser Ala Ala Ala Ala Gly Ser Gly Val Ile Gly Pro
                165                 170                 175

Gly Leu Tyr Gly Pro Tyr Ala Ser Ala Ala Ala Ala Gly Leu Tyr
        180                 185                 190

Gly Ser Gly Pro Gly Val Ile Gly Pro Tyr Gly Pro Gly Leu Ser Gly
        195                 200                 205

Ser Gly Val Ile Gly Pro Gly Val Ile Gly Pro Tyr Ala Ser Ala Ala
        210                 215                 220

Ala Ala Ala Gly Pro Gly Val Ile Gly Pro Tyr Gly Pro Gly Ser Ser

```
         225                    230                    235                    240


         Ala Ala Ala Ala Ala Gly Leu Tyr Gly Tyr Gly Pro Gly Val Ile Gly
                         245                250                255


         Pro Tyr Gly Pro Gly Ala Ser Gly Leu Asn Gly Pro Gly Ser Gly Leu
                         260                265                270


         Tyr Gly Pro Gly Val Ile Gly Pro Gly Leu Ser Ala Ala Ala Ala Ala
                         275                280                285


         Gly Pro Gly Val Ile Gly Pro Tyr Gly Pro Gly Ala Ser Ala Ala Ala
                         290                295                300


         Ala Ala Gly Leu Tyr Gly Pro Gly Val Ile Gly Pro Gly Leu Tyr Gly
         305                310                315                320


         Pro Gly Ser Ser Gly Pro Gly Val Ile Gly Pro Tyr Gly Pro Gly Ser
                         325                330                335


         Ser Ala Ala Ala Ala Ala Gly Leu Tyr Gly Pro Gly Val Ile Gly Pro
                         340                345                350


         Tyr Gly Pro Gly Leu Ser Ala Ala Ala Ala Ala Gly Leu Tyr Val Ile
                         355                360                365


         Gly Pro Gly Val Ile Gly Pro Tyr Gly Pro Gly Ala Ser Gly Pro Gly
                         370                375                380


         Val Ile Gly Pro Tyr Gly Pro Gly Ala Ser Ala Ala Ala Ala Ala Gly
         385                390                395                400


         Pro Gly Leu Tyr Gly Pro Gly Val Ile Gly Pro Ser Ala Ser Ala Ala
                         405                410                415


         Ala Ala Ala Gly Leu Tyr Gly Ser Gly Pro Gly Leu Tyr Gly Pro Tyr
                         420                425                430


         Gly Pro Gly Leu Ser Gly Pro Gly Ser Gly Val Ile Gly Leu Gly Pro
                         435                440                445


         Tyr Gly Pro Gly Ala Ser Ala Ala Ala Ala Gly Leu Tyr Gly Pro
                         450                455                460


         Gly Val Ile Gly Pro Tyr Gly Pro Gly Leu Ser Ala Ala Ala Ala Ala
         465                470                475                480
```

```
Gly Pro Gly Ser Gly Leu Tyr Gly Pro Gly Ala Ser Gly Leu Asn Gly
                485                 490                 495

Pro Gly Ser Gly Leu Tyr Gly Pro Gly Val Ile Gly Pro Gly Leu Ser
                500                 505                 510

Ala Ala Ala Ala Ala Gly Leu Tyr Val Ile Gly Pro Gly Val Ile Gly
            515                 520                 525

Pro Tyr Gly Pro Gly Ala Ser Ala Ala Ala Ala Ala Gly Leu Tyr Gly
        530                 535                 540

Ser Gly Pro Gly Val Ile Gly Pro Tyr Gly Pro Gly Leu Ser Gly Ser
545                 550                 555                 560

Gly Val Ile Gly Pro Gly Val Ile Gly Pro Tyr Ala Ser Ala Ala Ala
                565                 570                 575

Ala Ala Gly Pro Gly Ser Gly Val Ile Gly Pro Gly Ala Ser
            580                 585                 590
```

```
<210>   29
<211>   590
<212>   PRT
<213>   Artificial Sequence

<220>
<223>   M_ PRT918

<400>   29
```

```
Met Gly Pro Gly Val Phe Gly Pro Tyr Gly Pro Gly Ala Ser Ala Ala
1               5                   10                  15

Ala Ala Ala Gly Ile Asn Gly Pro Gly Ser Gly Val Phe Gly Pro Gly
            20                  25                  30

Ile Ser Gly Ile Tyr Gly Pro Gly Val Phe Gly Pro Gly Val Phe Gly
        35                  40                  45

Pro Gly Ser Ser Ala Ala Ala Ala Ala Gly Pro Gly Ile Tyr Gly Pro
        50                  55                  60

Gly Val Phe Gly Pro Ser Ala Ser Ala Ala Ala Ala Gly Pro Gly
65                  70                  75                  80

Ser Gly Val Phe Gly Pro Gly Ala Ser Gly Ile Tyr Gly Pro Gly Val
                85                  90                  95

Phe Gly Pro Gly Val Phe Gly Pro Gly Ser Ser Ala Ala Ala Ala Ala
```

```
                    100                      105                          110

      Gly Ile Tyr Gly Ser Gly Pro Gly Val Phe Gly Pro Tyr Gly Ser Ala
              115                 120                 125

      Ala Ala Ala Ala Gly Pro Gly Ser Gly Ile Tyr Gly Ile Gly Pro Tyr
          130                 135                 140

      Gly Pro Gly Ala Ser Gly Pro Gly Ile Tyr Gly Pro Gly Val Phe Gly
      145                 150                 155                 160

      Pro Ser Ala Ser Ala Ala Ala Ala Ala Gly Ser Gly Val Phe Gly Pro
                  165                 170                 175

      Gly Ile Tyr Gly Pro Tyr Ala Ser Ala Ala Ala Ala Gly Ile Tyr
                  180                 185                 190

      Gly Ser Gly Pro Gly Val Phe Gly Pro Tyr Gly Pro Gly Ile Ser Gly
                  195                 200                 205

      Ser Gly Val Phe Gly Pro Gly Val Phe Gly Pro Tyr Ala Ser Ala Ala
          210                 215                 220

      Ala Ala Ala Gly Pro Gly Val Phe Gly Pro Tyr Gly Pro Gly Ser Ser
      225                 230                 235                 240

      Ala Ala Ala Ala Ala Gly Ile Tyr Gly Tyr Gly Pro Gly Val Phe Gly
                  245                 250                 255

      Pro Tyr Gly Pro Gly Ala Ser Gly Ile Asn Gly Pro Gly Ser Gly Ile
                  260                 265                 270

      Tyr Gly Pro Gly Val Phe Gly Pro Gly Ile Ser Ala Ala Ala Ala Ala
                  275                 280                 285

      Gly Pro Gly Val Phe Gly Pro Tyr Gly Pro Gly Ala Ser Ala Ala Ala
                  290                 295                 300

      Ala Ala Gly Ile Tyr Gly Pro Gly Val Phe Gly Pro Gly Ile Tyr Gly
      305                 310                 315                 320

      Pro Gly Ser Ser Gly Pro Gly Val Phe Gly Pro Tyr Gly Pro Gly Ser
                  325                 330                 335

      Ser Ala Ala Ala Ala Ala Gly Ile Tyr Gly Pro Gly Val Phe Gly Pro
                  340                 345                 350
```

```
Tyr Gly Pro Gly Ile Ser Ala Ala Ala Ala Ala Gly Ile Tyr Val Phe
        355             360             365

Gly Pro Gly Val Phe Gly Pro Tyr Gly Pro Gly Ala Ser Gly Pro Gly
        370             375             380

Val Phe Gly Pro Tyr Gly Pro Gly Ala Ser Ala Ala Ala Ala Ala Gly
385             390             395             400

Pro Gly Ile Tyr Gly Pro Gly Val Phe Gly Pro Ser Ala Ser Ala Ala
                405             410             415

Ala Ala Ala Gly Ile Tyr Gly Ser Gly Pro Gly Ile Tyr Gly Pro Tyr
        420             425             430

Gly Pro Gly Ile Ser Gly Pro Gly Ser Gly Val Phe Gly Ile Gly Pro
        435             440             445

Tyr Gly Pro Gly Ala Ser Ala Ala Ala Ala Gly Ile Tyr Gly Pro
        450             455             460

Gly Val Phe Gly Pro Tyr Gly Pro Gly Ile Ser Ala Ala Ala Ala Ala
465             470             475             480

Gly Pro Gly Ser Gly Ile Tyr Gly Pro Gly Ala Ser Gly Ile Asn Gly
        485             490             495

Pro Gly Ser Gly Ile Tyr Gly Pro Gly Val Phe Gly Pro Gly Ile Ser
        500             505             510

Ala Ala Ala Ala Ala Gly Ile Tyr Val Phe Gly Pro Gly Val Phe Gly
        515             520             525

Pro Tyr Gly Pro Gly Ala Ser Ala Ala Ala Ala Ala Gly Ile Tyr Gly
        530             535             540

Ser Gly Pro Gly Val Phe Gly Pro Tyr Gly Pro Gly Ile Ser Gly Ser
545             550             555             560

Gly Val Phe Gly Pro Gly Val Phe Gly Pro Tyr Ala Ser Ala Ala Ala
                565             570             575

Ala Ala Gly Pro Gly Ser Gly Val Phe Gly Pro Gly Ala Ser
        580             585             590
```

```
<210>   30
<211>   565
<212>   PRT
```

<213>   Artificial Sequence

<220>
<223>   M_PRT699

<400>   30

Met Gly Pro Gly Val Leu Gly Pro Tyr Gly Pro Gly Ala Ser Ala Ala
1               5                   10                  15

Ala Ala Ala Ala Ala Gly Ser Asn Gly Pro Gly Ser Gly Val Leu Gly
            20                  25                  30

Pro Gly Gln Ser Gly Gln Tyr Gly Pro Gly Val Leu Gly Pro Gly Val
        35                  40                  45

Leu Gly Pro Gly Ser Ser Ala Ala Ala Ala Ala Ala Gly Pro Gly
        50                  55                  60

Gln Tyr Gly Pro Gly Val Leu Gly Pro Ser Ala Ser Ala Ala Ala Ala
65                  70                  75                  80

Ala Ala Ala Gly Pro Gly Ser Gly Val Leu Gly Pro Gly Ala Ser Gly
                85                  90                  95

Gln Tyr Gly Pro Gly Val Leu Gly Pro Gly Val Leu Gly Pro Gly Ser
            100                 105                 110

Ser Ala Ala Ala Ala Ala Ala Ala Gly Ser Tyr Gly Ser Gly Pro Gly
        115                 120                 125

Val Leu Gly Pro Tyr Gly Ser Ala Ala Ala Ala Ala Ala Gly Pro
        130                 135                 140

Gly Ser Gly Gln Tyr Gly Gln Gly Pro Tyr Gly Pro Gly Ala Ser Gly
145                 150                 155                 160

Pro Gly Gln Tyr Gly Pro Gly Val Leu Gly Pro Ser Ala Ser Ala Ala
            165                 170                 175

Ala Ala Ala Ala Ala Gly Ser Gly Val Leu Gly Pro Gly Gln Tyr Gly
        180                 185                 190

Pro Tyr Ala Ser Ala Ala Ala Ala Ala Ala Ala Gly Ser Tyr Gly Ser
        195                 200                 205

Gly Pro Gly Val Leu Gly Pro Tyr Gly Pro Gly Gln Ser Gly Ser Gly
        210                 215                 220

```
Val Leu Gly Pro Gly Val Leu Gly Pro Tyr Ala Ser Ala Ala Ala Ala
225                 230                 235                 240

Ala Ala Ala Gly Pro Gly Val Leu Gly Pro Tyr Gly Pro Gly Ser Ser
                245                 250                 255

Ala Ala Ala Ala Ala Ala Ala Gly Ser Tyr Gly Tyr Gly Pro Gly Val
            260                 265                 270

Leu Gly Pro Tyr Gly Pro Gly Ala Ser Gly Gln Asn Gly Pro Gly Ser
            275                 280                 285

Gly Gln Tyr Gly Pro Gly Val Leu Gly Pro Gly Pro Ser Ala Ala Ala
    290                 295                 300

Ala Ala Ala Ala Gly Pro Gly Val Leu Gly Pro Tyr Gly Pro Gly Ala
305                 310                 315                 320

Ser Ala Ala Ala Ala Ala Ala Ala Gly Ser Tyr Gly Pro Gly Val Leu
            325                 330                 335

Gly Pro Gly Gln Tyr Gly Pro Gly Ser Ser Gly Pro Gly Val Leu Gly
            340                 345                 350

Pro Tyr Gly Pro Gly Ser Ser Ala Ala Ala Ala Ala Ala Gly Ser
    355                 360                 365

Tyr Gly Pro Gly Val Leu Gly Pro Tyr Gly Pro Gly Pro Ser Ala Ala
    370                 375                 380

Ala Ala Ala Ala Ala Gly Ser Tyr Val Leu Gly Pro Gly Val Leu Gly
385                 390                 395                 400

Pro Tyr Gly Pro Gly Ala Ser Gly Pro Gly Val Leu Gly Pro Tyr Gly
            405                 410                 415

Pro Gly Ala Ser Ala Ala Ala Ala Ala Ala Gly Pro Gly Gln Tyr
            420                 425                 430

Gly Pro Gly Val Leu Gly Pro Ser Ala Ser Ala Ala Ala Ala Ala
            435                 440                 445

Ala Gly Ser Tyr Gly Ser Gly Pro Gly Gln Tyr Gly Pro Tyr Gly Pro
    450                 455                 460

Gly Gln Ser Gly Pro Gly Ser Gly Val Leu Gly Gln Gly Pro Tyr Gly
465                 470                 475                 480
```

126

Pro Gly Ala Ser Ala Ala Ala Ala Ala Ala Ala Gly Ser Tyr Gly Pro
                    485                 490                 495

Gly Val Leu Gly Pro Tyr Gly Pro Gly Pro Ser Ala Ala Ala Ala Ala
                500             505                 510

Ala Ala Gly Pro Gly Ser Gly Gln Tyr Gly Pro Gly Ala Ser Gly Gln
            515             520                 525

Asn Gly Pro Gly Ser Gly Gln Tyr Gly Pro Gly Val Leu Gly Pro Gly
    530             535                 540

Pro Ser Ala Ala Ala Ala Ala Ala Ala Gly Pro Gly Ser Gly Val Leu
545                 550                 555                 560

Gly Pro Gly Ala Ser
                565


<210>  31
<211>  565
<212>  PRT
<213>  Artificial Sequence

<220>
<223>  M_PRT698

<400>  31

Met Gly Pro Gly Val Leu Gly Pro Tyr Gly Pro Gly Ala Ser Ala Ala
1                   5                   10                  15

Ala Ala Ala Ala Ala Gly Ser Asn Gly Pro Gly Ser Gly Val Leu Gly
            20                  25                  30

Pro Gly Ile Ser Gly Ile Tyr Gly Pro Gly Val Leu Gly Pro Gly Val
            35                  40                  45

Leu Gly Pro Gly Ser Ser Ala Ala Ala Ala Ala Ala Gly Pro Gly
            50                  55                  60

Ile Tyr Gly Pro Gly Val Leu Gly Pro Ser Ala Ser Ala Ala Ala Ala
65                  70                  75                  80

Ala Ala Ala Gly Pro Gly Ser Gly Val Leu Gly Pro Gly Ala Ser Gly
                85                  90                  95

Ile Tyr Gly Pro Gly Val Leu Gly Pro Gly Val Leu Gly Pro Gly Ser
                100                 105                 110


127

Ser Ala Ala Ala Ala Ala Ala Ala Gly Ser Tyr Gly Ser Gly Pro Gly
    115                 120             125

Val Leu Gly Pro Tyr Gly Ser Ala Ala Ala Ala Ala Ala Ala Gly Pro
    130             135             140

Gly Ser Gly Ile Tyr Gly Ile Gly Pro Tyr Gly Pro Gly Ala Ser Gly
145             150             155             160

Pro Gly Ile Tyr Gly Pro Gly Val Leu Gly Pro Ser Ala Ser Ala Ala
            165             170             175

Ala Ala Ala Ala Ala Gly Ser Gly Val Leu Gly Pro Gly Ile Tyr Gly
        180             185             190

Pro Tyr Ala Ser Ala Ala Ala Ala Ala Ala Gly Ser Tyr Gly Ser
    195             200             205

Gly Pro Gly Val Leu Gly Pro Tyr Gly Pro Gly Ile Ser Gly Ser Gly
    210             215             220

Val Leu Gly Pro Gly Val Leu Gly Pro Tyr Ala Ser Ala Ala Ala Ala
225             230             235             240

Ala Ala Ala Gly Pro Gly Val Leu Gly Pro Tyr Gly Pro Gly Ser Ser
            245             250             255

Ala Ala Ala Ala Ala Ala Ala Gly Ser Tyr Gly Tyr Gly Pro Gly Val
        260             265             270

Leu Gly Pro Tyr Gly Pro Gly Ala Ser Gly Ile Asn Gly Pro Gly Ser
    275             280             285

Gly Ile Tyr Gly Pro Gly Val Leu Gly Pro Gly Pro Ser Ala Ala Ala
    290             295             300

Ala Ala Ala Ala Gly Pro Gly Val Leu Gly Pro Tyr Gly Pro Gly Ala
305             310             315             320

Ser Ala Ala Ala Ala Ala Ala Ala Gly Ser Tyr Gly Pro Gly Val Leu
        325             330             335

Gly Pro Gly Ile Tyr Gly Pro Gly Ser Ser Gly Pro Gly Val Leu Gly
            340             345             350

Pro Tyr Gly Pro Gly Ser Ser Ala Ala Ala Ala Ala Ala Ala Gly Ser
    355             360             365

```
Tyr Gly Pro Gly Val Leu Gly Pro Tyr Gly Pro Gly Pro Ser Ala Ala
    370             375             380

Ala Ala Ala Ala Ala Gly Ser Tyr Val Leu Gly Pro Gly Val Leu Gly
385             390             395             400

Pro Tyr Gly Pro Gly Ala Ser Gly Pro Gly Val Leu Gly Pro Tyr Gly
        405             410             415

Pro Gly Ala Ser Ala Ala Ala Ala Ala Ala Ala Gly Pro Gly Ile Tyr
        420             425             430

Gly Pro Gly Val Leu Gly Pro Ser Ala Ser Ala Ala Ala Ala Ala Ala
        435             440             445

Ala Gly Ser Tyr Gly Ser Gly Pro Gly Ile Tyr Gly Pro Tyr Gly Pro
    450             455             460

Gly Ile Ser Gly Pro Gly Ser Gly Val Leu Gly Ile Gly Pro Tyr Gly
465             470             475             480

Pro Gly Ala Ser Ala Ala Ala Ala Ala Ala Gly Ser Tyr Gly Pro
        485             490             495

Gly Val Leu Gly Pro Tyr Gly Pro Gly Pro Ser Ala Ala Ala Ala Ala
        500             505             510

Ala Ala Gly Pro Gly Ser Gly Ile Tyr Gly Pro Gly Ala Ser Gly Ile
    515             520             525

Asn Gly Pro Gly Ser Gly Ile Tyr Gly Pro Gly Val Leu Gly Pro Gly
    530             535             540

Pro Ser Ala Ala Ala Ala Ala Ala Ala Gly Pro Gly Ser Gly Val Leu
545             550             555             560

Gly Pro Gly Ala Ser
            565


<210>   32
<211>   2360
<212>   PRT
<213>   Artificial Sequence

<220>
<223>   Met-PRT1009

<400>   32
```

Gly Pro Gly Val Phe Gly Pro Tyr Gly Pro Gly Ala Ser Ala Ala Ala
1               5               10                  15

Ala Ala Gly Ile Asn Gly Pro Gly Ser Gly Val Phe Gly Pro Gly Ile
            20              25                  30

Ser Gly Ile Tyr Gly Pro Gly Val Phe Gly Pro Gly Val Phe Gly Pro
        35              40                  45

Gly Ser Ser Ala Ala Ala Ala Ala Gly Pro Gly Ile Tyr Gly Pro Gly
        50              55                  60

Val Phe Gly Pro Ser Ala Ser Ala Ala Ala Ala Gly Pro Gly Ser
65              70                  75                  80

Gly Val Phe Gly Pro Gly Ala Ser Gly Ile Tyr Gly Pro Gly Val Phe
            85                  90                  95

Gly Pro Gly Val Phe Gly Pro Gly Ser Ser Ala Ala Ala Ala Ala Gly
            100                 105                 110

Ile Tyr Gly Ser Gly Pro Gly Val Phe Gly Pro Tyr Gly Ser Ala Ala
        115                 120                 125

Ala Ala Ala Gly Pro Gly Ser Gly Ile Tyr Gly Ile Gly Pro Tyr Gly
    130                 135                 140

Pro Gly Ala Ser Gly Pro Gly Ile Tyr Gly Pro Gly Val Phe Gly Pro
145                 150                 155                 160

Ser Ala Ser Ala Ala Ala Ala Ala Gly Ser Gly Val Phe Gly Pro Gly
            165                 170                 175

Ile Tyr Gly Pro Tyr Ala Ser Ala Ala Ala Ala Ala Gly Ile Tyr Gly
        180                 185                 190

Ser Gly Pro Gly Val Phe Gly Pro Tyr Gly Pro Gly Ile Ser Gly Ser
        195                 200                 205

Gly Val Phe Gly Pro Gly Val Phe Gly Pro Tyr Ala Ser Ala Ala Ala
    210                 215                 220

Ala Ala Gly Pro Gly Val Phe Gly Pro Tyr Gly Pro Gly Ser Ser Ala
225                 230                 235                 240

Ala Ala Ala Gly Ile Tyr Gly Tyr Gly Pro Gly Val Phe Gly Pro
            245                 250                 255

Tyr Gly Pro Gly Ala Ser Gly Ile Asn Gly Pro Gly Ser Gly Ile Tyr
260 265 270

Gly Pro Gly Val Phe Gly Pro Gly Ile Ser Ala Ala Ala Ala Ala Gly
275 280 285

Pro Gly Val Phe Gly Pro Tyr Gly Pro Gly Ala Ser Ala Ala Ala Ala
290 295 300

Ala Gly Ile Tyr Gly Pro Gly Val Phe Gly Pro Gly Ile Tyr Gly Pro
305 310 315 320

Gly Ser Ser Gly Pro Gly Val Phe Gly Pro Tyr Gly Pro Gly Ser Ser
325 330 335

Ala Ala Ala Ala Ala Gly Ile Tyr Gly Pro Gly Val Phe Gly Pro Tyr
340 345 350

Gly Pro Gly Ile Ser Ala Ala Ala Ala Gly Ile Tyr Val Phe Gly
355 360 365

Pro Gly Val Phe Gly Pro Tyr Gly Pro Gly Ala Ser Gly Pro Gly Val
370 375 380

Phe Gly Pro Tyr Gly Pro Gly Ala Ser Ala Ala Ala Ala Gly Pro
385 390 395 400

Gly Ile Tyr Gly Pro Gly Val Phe Gly Pro Ser Ala Ser Ala Ala Ala
405 410 415

Ala Ala Gly Ile Tyr Gly Ser Gly Pro Gly Ile Tyr Gly Pro Tyr Gly
420 425 430

Pro Gly Ile Ser Gly Pro Gly Ser Gly Val Phe Gly Ile Gly Pro Tyr
435 440 445

Gly Pro Gly Ala Ser Ala Ala Ala Ala Gly Ile Tyr Gly Pro Gly
450 455 460

Val Phe Gly Pro Tyr Gly Pro Gly Ile Ser Ala Ala Ala Ala Ala Gly
465 470 475 480

Pro Gly Ser Gly Ile Tyr Gly Pro Gly Ala Ser Gly Ile Asn Gly Pro
485 490 495

Gly Ser Gly Ile Tyr Gly Pro Gly Val Phe Gly Pro Gly Ile Ser Ala
500 505 510

```
Ala Ala Ala Ala Gly Ile Tyr Val Phe Gly Pro Gly Val Phe Gly Pro
        515                 520                 525

Tyr Gly Pro Gly Ala Ser Ala Ala Ala Ala Ala Gly Ile Tyr Gly Ser
    530                 535                 540

Gly Pro Gly Val Phe Gly Pro Tyr Gly Pro Gly Ile Ser Gly Ser Gly
545                 550                 555                 560

Val Phe Gly Pro Gly Val Phe Gly Pro Tyr Ala Ser Ala Ala Ala Ala
                565                 570                 575

Ala Gly Pro Gly Ser Gly Val Phe Gly Pro Gly Ala Ser Gly Pro Gly
            580                 585                 590

Val Phe Gly Pro Tyr Gly Pro Gly Ala Ser Ala Ala Ala Ala Ala Gly
            595                 600                 605

Ile Asn Gly Pro Gly Ser Gly Val Phe Gly Pro Gly Ile Ser Gly Ile
    610                 615                 620

Tyr Gly Pro Gly Val Phe Gly Pro Gly Val Phe Gly Pro Gly Ser Ser
625                 630                 635                 640

Ala Ala Ala Ala Ala Gly Pro Gly Ile Tyr Gly Pro Gly Val Phe Gly
            645                 650                 655

Pro Ser Ala Ser Ala Ala Ala Ala Ala Gly Pro Gly Ser Gly Val Phe
        660                 665                 670

Gly Pro Gly Ala Ser Gly Ile Tyr Gly Pro Gly Val Phe Gly Pro Gly
        675                 680                 685

Val Phe Gly Pro Gly Ser Ser Ala Ala Ala Ala Ala Gly Ile Tyr Gly
    690                 695                 700

Ser Gly Pro Gly Val Phe Gly Pro Tyr Gly Ser Ala Ala Ala Ala Ala
705                 710                 715                 720

Gly Pro Gly Ser Gly Ile Tyr Gly Ile Gly Pro Tyr Gly Pro Gly Ala
            725                 730                 735

Ser Gly Pro Gly Ile Tyr Gly Pro Gly Val Phe Gly Pro Ser Ala Ser
        740                 745                 750

Ala Ala Ala Ala Ala Gly Ser Gly Val Phe Gly Pro Gly Ile Tyr Gly
```

```
                     755                     760                     765


          Pro Tyr Ala Ser Ala Ala Ala Ala Ala Gly Ile Tyr Gly Ser Gly Pro
              770             775             780


          Gly Val Phe Gly Pro Tyr Gly Pro Gly Ile Ser Gly Ser Gly Val Phe
              785             790             795             800


          Gly Pro Gly Val Phe Gly Pro Tyr Ala Ser Ala Ala Ala Ala Ala Gly
                  805             810             815


          Pro Gly Val Phe Gly Pro Tyr Gly Pro Gly Ser Ser Ala Ala Ala Ala
                  820             825             830


          Ala Gly Ile Tyr Gly Tyr Gly Pro Gly Val Phe Gly Pro Tyr Gly Pro
                  835             840             845


          Gly Ala Ser Gly Ile Asn Gly Pro Gly Ser Gly Ile Tyr Gly Pro Gly
              850             855             860


          Val Phe Gly Pro Gly Ile Ser Ala Ala Ala Ala Gly Pro Gly Val
              865             870             875             880


          Phe Gly Pro Tyr Gly Pro Gly Ala Ser Ala Ala Ala Ala Ala Gly Ile
                  885             890             895


          Tyr Gly Pro Gly Val Phe Gly Pro Gly Ile Tyr Gly Pro Gly Ser Ser
                  900             905             910


          Gly Pro Gly Val Phe Gly Pro Tyr Gly Pro Gly Ser Ser Ala Ala Ala
                  915             920             925


          Ala Ala Gly Ile Tyr Gly Pro Gly Val Phe Gly Pro Tyr Gly Pro Gly
                  930             935             940


          Ile Ser Ala Ala Ala Ala Ala Gly Ile Tyr Val Phe Gly Pro Gly Val
              945             950             955             960


          Phe Gly Pro Tyr Gly Pro Gly Ala Ser Gly Pro Gly Val Phe Gly Pro
                  965             970             975


          Tyr Gly Pro Gly Ala Ser Ala Ala Ala Ala Gly Pro Gly Ile Tyr
                  980             985             990


          Gly Pro Gly Val Phe Gly Pro Ser  Ala Ser Ala Ala Ala  Ala Ala Gly
                  995             1000            1005
```

```
Ile Tyr  Gly Ser Gly Pro Gly  Ile Tyr Gly Pro Tyr  Gly Pro Gly
    1010              1015              1020

Ile Ser  Gly Pro Gly Ser Gly  Val Phe Gly Ile Gly  Pro Tyr Gly
    1025              1030              1035

Pro Gly  Ala Ser Ala Ala Ala  Ala Ala Gly Ile Tyr  Gly Pro Gly
    1040              1045              1050

Val Phe  Gly Pro Tyr Gly Pro  Gly Ile Ser Ala Ala  Ala Ala Ala
    1055              1060              1065

Gly Pro  Gly Ser Gly Ile Tyr  Gly Pro Gly Ala Ser  Gly Ile Asn
    1070              1075              1080

Gly Pro  Gly Ser Gly Ile Tyr  Gly Pro Gly Val Phe  Gly Pro Gly
    1085              1090              1095

Ile Ser  Ala Ala Ala Ala Ala  Gly Ile Tyr Val Phe  Gly Pro Gly
    1100              1105              1110

Val Phe  Gly Pro Tyr Gly Pro  Gly Ala Ser Ala Ala  Ala Ala Ala
    1115              1120              1125

Gly Ile  Tyr Gly Ser Gly Pro  Gly Val Phe Gly Pro  Tyr Gly Pro
    1130              1135              1140

Gly Ile  Ser Gly Ser Gly Val  Phe Gly Pro Gly Val  Phe Gly Pro
    1145              1150              1155

Tyr Ala  Ser Ala Ala Ala Ala  Ala Gly Pro Gly Ser  Gly Val Phe
    1160              1165              1170

Gly Pro  Gly Ala Ser Glu Phe  Gly Pro Gly Val Phe  Gly Pro Tyr
    1175              1180              1185

Gly Pro  Gly Ala Ser Ala Ala  Ala Ala Ala Gly Ile  Asn Gly Pro
    1190              1195              1200

Gly Ser  Gly Val Phe Gly Pro  Gly Ile Ser Gly Ile  Tyr Gly Pro
    1205              1210              1215

Gly Val  Phe Gly Pro Gly Val  Phe Gly Pro Gly Ser  Ser Ala Ala
    1220              1225              1230

Ala Ala  Ala Gly Pro Gly Ile  Tyr Gly Pro Gly Val  Phe Gly Pro
    1235              1240              1245
```

134

Ser Ala  Ser Ala Ala Ala Ala  Ala Gly Pro Gly Ser  Gly Val Phe
    1250            1255            1260

Gly Pro  Gly Ala Ser Gly Ile  Tyr Gly Pro Gly Val  Phe Gly Pro
    1265            1270            1275

Gly Val  Phe Gly Pro Gly Ser  Ser Ala Ala Ala Ala  Ala Gly Ile
    1280            1285            1290

Tyr Gly  Ser Gly Pro Gly Val  Phe Gly Pro Tyr Gly  Ser Ala Ala
    1295            1300            1305

Ala Ala  Ala Gly Pro Gly Ser  Gly Ile Tyr Gly Ile  Gly Pro Tyr
    1310            1315            1320

Gly Pro  Gly Ala Ser Gly Pro  Gly Ile Tyr Gly Pro  Gly Val Phe
    1325            1330            1335

Gly Pro  Ser Ala Ser Ala Ala  Ala Ala Ala Gly Ser  Gly Val Phe
    1340            1345            1350

Gly Pro  Gly Ile Tyr Gly Pro  Tyr Ala Ser Ala Ala  Ala Ala Ala
    1355            1360            1365

Gly Ile  Tyr Gly Ser Gly Pro  Gly Val Phe Gly Pro  Tyr Gly Pro
    1370            1375            1380

Gly Ile  Ser Gly Ser Gly Val  Phe Gly Pro Gly Val  Phe Gly Pro
    1385            1390            1395

Tyr Ala  Ser Ala Ala Ala Ala  Ala Gly Pro Gly Val  Phe Gly Pro
    1400            1405            1410

Tyr Gly  Pro Gly Ser Ser Ala  Ala Ala Ala Ala Gly  Ile Tyr Gly
    1415            1420            1425

Tyr Gly  Pro Gly Val Phe Gly  Pro Tyr Gly Pro Gly  Ala Ser Gly
    1430            1435            1440

Ile Asn  Gly Pro Gly Ser Gly  Ile Tyr Gly Pro Gly  Val Phe Gly
    1445            1450            1455

Pro Gly  Ile Ser Ala Ala Ala  Ala Ala Gly Pro Gly  Val Phe Gly
    1460            1465            1470

Pro Tyr  Gly Pro Gly Ala Ser  Ala Ala Ala Ala Ala  Gly Ile Tyr
    1475            1480            1485

Gly Pro Gly Val Phe Gly Pro Gly Ile Tyr Gly Pro Gly Ser Ser
1490          1495          1500

Gly Pro Gly Val Phe Gly Pro Tyr Gly Pro Gly Ser Ser Ala Ala
1505          1510          1515

Ala Ala Ala Gly Ile Tyr Gly Pro Gly Val Phe Gly Pro Tyr Gly
1520          1525          1530

Pro Gly Ile Ser Ala Ala Ala Ala Ala Gly Ile Tyr Val Phe Gly
1535          1540          1545

Pro Gly Val Phe Gly Pro Tyr Gly Pro Gly Ala Ser Gly Pro Gly
1550          1555          1560

Val Phe Gly Pro Tyr Gly Pro Gly Ala Ser Ala Ala Ala Ala Ala
1565          1570          1575

Gly Pro Gly Ile Tyr Gly Pro Gly Val Phe Gly Pro Ser Ala Ser
1580          1585          1590

Ala Ala Ala Ala Ala Gly Ile Tyr Gly Ser Gly Pro Gly Ile Tyr
1595          1600          1605

Gly Pro Tyr Gly Pro Gly Ile Ser Gly Pro Gly Ser Gly Val Phe
1610          1615          1620

Gly Ile Gly Pro Tyr Gly Pro Gly Ala Ser Ala Ala Ala Ala Ala
1625          1630          1635

Gly Ile Tyr Gly Pro Gly Val Phe Gly Pro Tyr Gly Pro Gly Ile
1640          1645          1650

Ser Ala Ala Ala Ala Ala Gly Pro Gly Ser Gly Ile Tyr Gly Pro
1655          1660          1665

Gly Ala Ser Gly Ile Asn Gly Pro Gly Ser Gly Ile Tyr Gly Pro
1670          1675          1680

Gly Val Phe Gly Pro Gly Ile Ser Ala Ala Ala Ala Ala Gly Ile
1685          1690          1695

Tyr Val Phe Gly Pro Gly Val Phe Gly Pro Tyr Gly Pro Gly Ala
1700          1705          1710

Ser Ala Ala Ala Ala Ala Gly Ile Tyr Gly Ser Gly Pro Gly Val

```
                   1715                        1720                        1725

    Phe Gly  Pro Tyr Gly Pro Gly  Ile Ser Gly Ser Gly  Val Phe Gly
             1730                  1735                  1740

    Pro Gly  Val Phe Gly Pro Tyr  Ala Ser Ala Ala Ala  Ala Ala Gly
             1745                  1750                  1755

    Pro Gly  Ser Gly Val Phe Gly  Pro Gly Ala Ser Glu  Leu Gly Pro
             1760                  1765                  1770

    Gly Val  Phe Gly Pro Tyr Gly  Pro Gly Ala Ser Ala  Ala Ala Ala
             1775                  1780                  1785

    Ala Gly  Ile Asn Gly Pro Gly  Ser Gly Val Phe Gly  Pro Gly Ile
             1790                  1795                  1800

    Ser Gly  Ile Tyr Gly Pro Gly  Val Phe Gly Pro Gly  Val Phe Gly
             1805                  1810                  1815

    Pro Gly  Ser Ser Ala Ala Ala  Ala Ala Gly Pro Gly  Ile Tyr Gly
             1820                  1825                  1830

    Pro Gly  Val Phe Gly Pro Ser  Ala Ser Ala Ala Ala  Ala Ala Gly
             1835                  1840                  1845

    Pro Gly  Ser Gly Val Phe Gly  Pro Gly Ala Ser Gly  Ile Tyr Gly
             1850                  1855                  1860

    Pro Gly  Val Phe Gly Pro Gly  Val Phe Gly Pro Gly  Ser Ser Ala
             1865                  1870                  1875

    Ala Ala  Ala Ala Gly Ile Tyr  Gly Ser Gly Pro Gly  Val Phe Gly
             1880                  1885                  1890

    Pro Tyr  Gly Ser Ala Ala Ala  Ala Ala Gly Pro Gly  Ser Gly Ile
             1895                  1900                  1905

    Tyr Gly  Ile Gly Pro Tyr Gly  Pro Gly Ala Ser Gly  Pro Gly Ile
             1910                  1915                  1920

    Tyr Gly  Pro Gly Val Phe Gly  Pro Ser Ala Ser Ala  Ala Ala Ala
             1925                  1930                  1935

    Ala Gly  Ser Gly Val Phe Gly  Pro Gly Ile Tyr Gly  Pro Tyr Ala
             1940                  1945                  1950
```

```
Ser Ala  Ala Ala Ala Ala Gly  Ile Tyr Gly Ser Gly  Pro Gly Val
    1955                  1960                 1965


Phe Gly  Pro Tyr Gly Pro Gly  Ile Ser Gly Ser Gly  Val Phe Gly
    1970                  1975                 1980


Pro Gly  Val Phe Gly Pro Tyr  Ala Ser Ala Ala Ala  Ala Ala Gly
    1985                  1990                 1995


Pro Gly  Val Phe Gly Pro Tyr  Gly Pro Gly Ser Ser  Ala Ala Ala
    2000                  2005                 2010


Ala Ala  Gly Ile Tyr Gly Tyr  Gly Pro Gly Val Phe  Gly Pro Tyr
    2015                  2020                 2025


Gly Pro  Gly Ala Ser Gly Ile  Asn Gly Pro Gly Ser  Gly Ile Tyr
    2030                  2035                 2040


Gly Pro  Gly Val Phe Gly Pro  Gly Ile Ser Ala Ala  Ala Ala Ala
    2045                  2050                 2055


Gly Pro  Gly Val Phe Gly Pro  Tyr Gly Pro Gly Ala  Ser Ala Ala
    2060                  2065                 2070


Ala Ala  Ala Gly Ile Tyr Gly  Pro Gly Val Phe Gly  Pro Gly Ile
    2075                  2080                 2085


Tyr Gly  Pro Gly Ser Ser Gly  Pro Gly Val Phe Gly  Pro Tyr Gly
    2090                  2095                 2100


Pro Gly  Ser Ser Ala Ala Ala  Ala Ala Gly Ile Tyr  Gly Pro Gly
    2105                  2110                 2115


Val Phe  Gly Pro Tyr Gly Pro  Gly Ile Ser Ala Ala  Ala Ala Ala
    2120                  2125                 2130


Gly Ile  Tyr Val Phe Gly Pro  Gly Val Phe Gly Pro  Tyr Gly Pro
    2135                  2140                 2145


Gly Ala  Ser Gly Pro Gly Val  Phe Gly Pro Tyr Gly  Pro Gly Ala
    2150                  2155                 2160


Ser Ala  Ala Ala Ala Ala Gly  Pro Gly Ile Tyr Gly  Pro Gly Val
    2165                  2170                 2175


Phe Gly  Pro Ser Ala Ser Ala  Ala Ala Ala Ala Gly  Ile Tyr Gly
    2180                  2185                 2190
```

```
Ser Gly  Pro Gly Ile Tyr Gly  Pro Tyr Gly Pro Gly  Ile Ser Gly
    2195             2200             2205

Pro Gly  Ser Gly Val Phe Gly  Ile Gly Pro Tyr Gly  Pro Gly Ala
    2210             2215             2220

Ser Ala  Ala Ala Ala Ala Gly  Ile Tyr Gly Pro Gly  Val Phe Gly
    2225             2230             2235

Pro Tyr  Gly Pro Gly Ile Ser  Ala Ala Ala Ala Gly  Pro Gly
    2240             2245             2250

Ser Gly  Ile Tyr Gly Pro Gly  Ala Ser Gly Ile Asn  Gly Pro Gly
    2255             2260             2265

Ser Gly  Ile Tyr Gly Pro Gly  Val Phe Gly Pro Gly  Ile Ser Ala
    2270             2275             2280

Ala Ala  Ala Ala Gly Ile Tyr  Val Phe Gly Pro Gly  Val Phe Gly
    2285             2290             2295

Pro Tyr  Gly Pro Gly Ala Ser  Ala Ala Ala Ala Ala  Gly Ile Tyr
    2300             2305             2310

Gly Ser  Gly Pro Gly Val Phe  Gly Pro Tyr Gly Pro  Gly Ile Ser
    2315             2320             2325

Gly Ser  Gly Val Phe Gly Pro  Gly Val Phe Gly Pro  Tyr Ala Ser
    2330             2335             2340

Ala Ala  Ala Ala Ala Gly Pro  Gly Ser Gly Val Phe  Gly Pro Gly
    2345             2350             2355

Ala Ser
    2360
```

```
<210>  33
<211>  601
<212>  PRT
<213>  Artificial Sequence

<220>
<223>  PRT888

<400>  33

Met His His His His His His Ser Ser Gly Ser Ser Gly Pro Gly Val
1               5                   10                  15
```

Leu Gly Pro Tyr Gly Pro Gly Ala Ser Ala Ala Ala Ala Ala Gly Gln
            20              25              30

Asn Gly Pro Gly Ser Gly Val Leu Gly Pro Gly Gln Ser Gly Gln Tyr
            35              40              45

Gly Pro Gly Val Leu Gly Pro Gly Val Leu Gly Pro Gly Ser Ser Ala
        50              55              60

Ala Ala Ala Ala Gly Pro Gly Gln Tyr Gly Pro Gly Val Leu Gly Pro
65              70              75              80

Ser Ala Ser Ala Ala Ala Ala Ala Gly Pro Gly Ser Gly Val Leu Gly
                85              90              95

Pro Gly Ala Ser Gly Gln Tyr Gly Pro Gly Val Leu Gly Pro Gly Val
            100             105             110

Leu Gly Pro Gly Ser Ser Ala Ala Ala Ala Gly Gln Tyr Gly Ser
        115             120             125

Gly Pro Gly Val Leu Gly Pro Tyr Gly Ser Ala Ala Ala Ala Gly
        130             135             140

Pro Gly Ser Gly Gln Tyr Gly Gln Gly Pro Tyr Gly Pro Gly Ala Ser
145             150             155             160

Gly Pro Gly Gln Tyr Gly Pro Gly Val Leu Gly Pro Ser Ala Ser Ala
            165             170             175

Ala Ala Ala Gly Ser Gly Val Leu Gly Pro Gly Gln Tyr Gly Pro
        180             185             190

Tyr Ala Ser Ala Ala Ala Ala Ala Gly Gln Tyr Gly Ser Gly Pro Gly
        195             200             205

Val Leu Gly Pro Tyr Gly Pro Gly Gln Ser Gly Ser Gly Val Leu Gly
    210             215             220

Pro Gly Val Leu Gly Pro Tyr Ala Ser Ala Ala Ala Ala Gly Pro
225             230             235             240

Gly Val Leu Gly Pro Tyr Gly Pro Gly Ser Ser Ala Ala Ala Ala Ala
            245             250             255

Gly Gln Tyr Gly Tyr Gly Pro Gly Val Leu Gly Pro Tyr Gly Pro Gly
        260             265             270

```
Ala Ser Gly Gln Asn Gly Pro Gly Ser Gly Gln Tyr Gly Pro Gly Val
        275             280             285

Leu Gly Pro Gly Gln Ser Ala Ala Ala Ala Ala Gly Pro Gly Val Leu
        290             295             300

Gly Pro Tyr Gly Pro Gly Ala Ser Ala Ala Ala Ala Ala Gly Gln Tyr
305             310             315             320

Gly Pro Gly Val Leu Gly Pro Gly Gln Tyr Gly Pro Gly Ser Ser Gly
            325             330             335

Pro Gly Val Leu Gly Pro Tyr Gly Pro Gly Ser Ser Ala Ala Ala Ala
        340             345             350

Ala Gly Gln Tyr Gly Pro Gly Val Leu Gly Pro Tyr Gly Pro Gly Gln
        355             360             365

Ser Ala Ala Ala Ala Ala Gly Gln Tyr Val Leu Gly Pro Gly Val Leu
        370             375             380

Gly Pro Tyr Gly Pro Gly Ala Ser Gly Pro Gly Val Leu Gly Pro Tyr
385             390             395             400

Gly Pro Gly Ala Ser Ala Ala Ala Ala Ala Gly Pro Gly Gln Tyr Gly
            405             410             415

Pro Gly Val Leu Gly Pro Ser Ala Ser Ala Ala Ala Ala Gly Gln Gly
            420             425             430

Tyr Gly Ser Gly Pro Gly Gln Tyr Gly Pro Tyr Gly Pro Gly Gln Ser
        435             440             445

Gly Pro Gly Ser Gly Val Leu Gly Gln Gly Pro Tyr Gly Pro Gly Ala
        450             455             460

Ser Ala Ala Ala Ala Ala Gly Gln Tyr Gly Pro Gly Val Leu Gly Pro
465             470             475             480

Tyr Gly Pro Gly Gln Ser Ala Ala Ala Ala Gly Pro Gly Ser Gly
            485             490             495

Gln Tyr Gly Pro Gly Ala Ser Gly Gln Asn Gly Pro Gly Ser Gly Gln
        500             505             510

Tyr Gly Pro Gly Val Leu Gly Pro Gly Gln Ser Ala Ala Ala Ala Ala
        515             520             525
```

141

```
Gly Gln Tyr Val Leu Gly Pro Gly Val Leu Gly Pro Tyr Gly Pro Gly
    530             535             540

Ala Ser Ala Ala Ala Ala Ala Gly Gln Tyr Gly Ser Gly Pro Gly Val
545             550             555             560

Leu Gly Pro Tyr Gly Pro Gly Gln Ser Gly Ser Gly Val Leu Gly Pro
            565             570             575

Gly Val Leu Gly Pro Tyr Ala Ser Ala Ala Ala Ala Gly Pro Gly
            580             585             590

Ser Gly Val Leu Gly Pro Gly Ala Ser
    595             600
```

<210> 34
<211> 601
<212> PRT
<213> Artificial Sequence

<220>
<223> PRT965

<400> 34

```
Met His His His His His His Ser Ser Gly Ser Ser Gly Pro Gly Thr
1               5               10              15

Ser Gly Pro Tyr Gly Pro Gly Ala Ser Ala Ala Ala Ala Ala Gly Ala
            20              25              30

Asn Gly Pro Gly Ser Gly Thr Ser Gly Pro Gly Ala Ser Gly Ala Tyr
        35              40              45

Gly Pro Gly Thr Ser Gly Pro Gly Thr Ser Gly Pro Gly Ser Ser Ala
    50              55              60

Ala Ala Ala Ala Gly Pro Gly Ala Tyr Gly Pro Gly Thr Ser Gly Pro
65              70              75              80

Ser Ala Ser Ala Ala Ala Ala Ala Gly Pro Gly Ser Gly Thr Ser Gly
            85              90              95

Pro Gly Ala Ser Gly Ala Tyr Gly Pro Gly Thr Ser Gly Pro Gly Thr
        100             105             110

Ser Gly Pro Gly Ser Ser Ala Ala Ala Ala Ala Gly Ala Tyr Gly Ser
        115             120             125
```

Gly Pro Gly Thr Ser Gly Pro Tyr Gly Ser Ala Ala Ala Ala Ala Gly
    130                 135                 140

Pro Gly Ser Gly Ala Tyr Gly Ala Gly Pro Tyr Gly Pro Gly Ala Ser
145                 150                 155                 160

Gly Pro Gly Ala Tyr Gly Pro Gly Thr Ser Gly Pro Ser Ala Ser Ala
                165                 170                 175

Ala Ala Ala Ala Gly Ser Gly Thr Ser Gly Pro Gly Ala Tyr Gly Pro
        180                 185                 190

Tyr Ala Ser Ala Ala Ala Ala Ala Gly Ala Tyr Gly Ser Gly Pro Gly
        195                 200                 205

Thr Ser Gly Pro Tyr Gly Pro Gly Ala Ser Gly Ser Gly Thr Ser Gly
    210                 215                 220

Pro Gly Thr Ser Gly Pro Tyr Ala Ser Ala Ala Ala Ala Ala Gly Pro
225                 230                 235                 240

Gly Thr Ser Gly Pro Tyr Gly Pro Gly Ser Ser Ala Ala Ala Ala Ala
                245                 250                 255

Gly Ala Tyr Gly Tyr Gly Pro Gly Thr Ser Gly Pro Tyr Gly Pro Gly
        260                 265                 270

Ala Ser Gly Ala Asn Gly Pro Gly Ser Gly Ala Tyr Gly Pro Gly Thr
        275                 280                 285

Ser Gly Pro Gly Ala Ser Ala Ala Ala Ala Gly Pro Gly Thr Ser
    290                 295                 300

Gly Pro Tyr Gly Pro Gly Ala Ser Ala Ala Ala Ala Gly Ala Tyr
305                 310                 315                 320

Gly Pro Gly Thr Ser Gly Pro Gly Ala Tyr Gly Pro Gly Ser Ser Gly
                325                 330                 335

Pro Gly Thr Ser Gly Pro Tyr Gly Pro Gly Ser Ser Ala Ala Ala Ala
        340                 345                 350

Ala Gly Ala Tyr Gly Pro Gly Thr Ser Gly Pro Tyr Gly Pro Gly Ala
    355                 360                 365

Ser Ala Ala Ala Ala Ala Gly Ala Tyr Thr Ser Gly Pro Gly Thr Ser
370                 375                 380

```
Gly Pro Tyr Gly Pro Gly Ala Ser Gly Pro Gly Thr Ser Gly Pro Tyr
385                 390                 395                 400

Gly Pro Gly Ala Ser Ala Ala Ala Ala Ala Gly Pro Gly Ala Tyr Gly
                405                 410                 415

Pro Gly Thr Ser Gly Pro Ser Ala Ser Ala Ala Ala Ala Ala Gly Ala
            420                 425                 430

Tyr Gly Ser Gly Pro Gly Ala Tyr Gly Pro Tyr Gly Pro Gly Ala Ser
        435                 440                 445

Gly Pro Gly Ser Gly Thr Ser Gly Ala Gly Pro Tyr Gly Pro Gly Ala
    450                 455                 460

Ser Ala Ala Ala Ala Ala Gly Ala Tyr Gly Pro Gly Thr Ser Gly Pro
465                 470                 475                 480

Tyr Gly Pro Gly Ala Ser Ala Ala Ala Ala Gly Pro Gly Ser Gly
        485                 490                 495

Ala Tyr Gly Pro Gly Ala Ser Gly Ala Asn Gly Pro Gly Ser Gly Ala
        500                 505                 510

Tyr Gly Pro Gly Thr Ser Gly Pro Gly Ala Ser Ala Ala Ala Ala Ala
        515                 520                 525

Gly Ala Tyr Thr Ser Gly Pro Gly Thr Ser Gly Pro Tyr Gly Pro Gly
    530                 535                 540

Ala Ser Ala Ala Ala Ala Ala Gly Ala Tyr Gly Ser Gly Pro Gly Thr
545                 550                 555                 560

Ser Gly Pro Tyr Gly Pro Gly Ala Ser Gly Ser Gly Thr Ser Gly Pro
            565                 570                 575

Gly Thr Ser Gly Pro Tyr Ala Ser Ala Ala Ala Ala Gly Pro Gly
            580                 585                 590

Ser Gly Thr Ser Gly Pro Gly Ala Ser
        595                 600


<210>  35
<211>  601
<212>  PRT
<213>  Artificial Sequence
```

<220>
<223>    PRT889

<400>    35

Met His His His His His His Ser Ser Gly Ser Ser Gly Pro Gly Val
1                   5                   10                  15

Leu Gly Pro Tyr Gly Pro Gly Ala Ser Ala Ala Ala Ala Ala Gly Ile
            20              25              30

Asn Gly Pro Gly Ser Gly Val Leu Gly Pro Gly Ile Ser Gly Ile Tyr
            35              40              45

Gly Pro Gly Val Leu Gly Pro Gly Val Leu Gly Pro Gly Ser Ser Ala
    50              55              60

Ala Ala Ala Ala Gly Pro Gly Ile Tyr Gly Pro Gly Val Leu Gly Pro
65              70              75              80

Ser Ala Ser Ala Ala Ala Ala Ala Gly Pro Gly Ser Gly Val Leu Gly
            85              90              95

Pro Gly Ala Ser Gly Ile Tyr Gly Pro Gly Val Leu Gly Pro Gly Val
            100             105             110

Leu Gly Pro Gly Ser Ser Ala Ala Ala Ala Ala Gly Ile Tyr Gly Ser
            115             120             125

Gly Pro Gly Val Leu Gly Pro Tyr Gly Ser Ala Ala Ala Ala Ala Gly
    130             135             140

Pro Gly Ser Gly Ile Tyr Gly Ile Gly Pro Tyr Gly Pro Gly Ala Ser
145             150             155             160

Gly Pro Gly Ile Tyr Gly Pro Gly Val Leu Gly Pro Ser Ala Ser Ala
            165             170             175

Ala Ala Ala Ala Gly Ser Gly Val Leu Gly Pro Gly Ile Tyr Gly Pro
            180             185             190

Tyr Ala Ser Ala Ala Ala Ala Ala Gly Ile Tyr Gly Ser Gly Pro Gly
            195             200             205

Val Leu Gly Pro Tyr Gly Pro Gly Ile Ser Gly Ser Gly Val Leu Gly
            210             215             220

Pro Gly Val Leu Gly Pro Tyr Ala Ser Ala Ala Ala Ala Ala Gly Pro
225             230             235             240

Gly Val Leu Gly Pro Tyr Gly Pro Gly Ser Ser Ala Ala Ala Ala Ala
                245                 250             255

Gly Ile Tyr Gly Tyr Gly Pro Gly Val Leu Gly Pro Tyr Gly Pro Gly
                260                 265             270

Ala Ser Gly Ile Asn Gly Pro Gly Ser Gly Ile Tyr Gly Pro Gly Val
                275             280             285

Leu Gly Pro Gly Ile Ser Ala Ala Ala Ala Gly Pro Gly Val Leu
                290             295             300

Gly Pro Tyr Gly Pro Gly Ala Ser Ala Ala Ala Ala Gly Ile Tyr
305             310             315             320

Gly Pro Gly Val Leu Gly Pro Gly Ile Tyr Gly Pro Gly Ser Ser Gly
                325             330             335

Pro Gly Val Leu Gly Pro Tyr Gly Pro Gly Ser Ser Ala Ala Ala Ala
                340             345             350

Ala Gly Ile Tyr Gly Pro Gly Val Leu Gly Pro Tyr Gly Pro Gly Ile
                355             360             365

Ser Ala Ala Ala Ala Ala Gly Ile Tyr Val Leu Gly Pro Gly Val Leu
370             375             380

Gly Pro Tyr Gly Pro Gly Ala Ser Gly Pro Gly Val Leu Gly Pro Tyr
385             390             395             400

Gly Pro Gly Ala Ser Ala Ala Ala Ala Gly Pro Gly Ile Tyr Gly
                405             410             415

Pro Gly Val Leu Gly Pro Ser Ala Ser Ala Ala Ala Ala Gly Ile
                420             425             430

Tyr Gly Ser Gly Pro Gly Ile Tyr Gly Pro Tyr Gly Pro Gly Ile Ser
                435             440             445

Gly Pro Gly Ser Gly Val Leu Gly Ile Gly Pro Tyr Gly Pro Gly Ala
                450             455             460

Ser Ala Ala Ala Ala Ala Gly Ile Tyr Gly Pro Gly Val Leu Gly Pro
465             470             475             480

Tyr Gly Pro Gly Ile Ser Ala Ala Ala Ala Ala Gly Pro Gly Ser Gly

```
                            485                      490                          495


      Ile Tyr Gly Pro Gly Ala Ser Gly Ile Asn Gly Pro Gly Ser Gly Ile
                  500                 505                 510


      Tyr Gly Pro Gly Val Leu Gly Pro Gly Ile Ser Ala Ala Ala Ala Ala
                  515                 520                 525


      Gly Ile Tyr Val Leu Gly Pro Gly Val Leu Gly Pro Tyr Gly Pro Gly
          530                 535                 540


      Ala Ser Ala Ala Ala Ala Ala Gly Ile Tyr Gly Ser Gly Pro Gly Val
      545                 550                 555                 560


      Leu Gly Pro Tyr Gly Pro Gly Ile Ser Gly Ser Gly Val Leu Gly Pro
                  565                 570                 575


      Gly Val Leu Gly Pro Tyr Ala Ser Ala Ala Ala Ala Gly Pro Gly
                  580                 585                 590


      Ser Gly Val Leu Gly Pro Gly Ala Ser
                  595                 600


      <210>  36
      <211>  601
      <212>  PRT
      <213>  Artificial Sequence

      <220>
      <223>  PRT916

      <400>  36

      Met His His His His His His Ser Ser Gly Ser Ser Gly Pro Gly Val
      1                   5                   10                  15


      Ile Gly Pro Tyr Gly Pro Gly Ala Ser Ala Ala Ala Ala Ala Gly Leu
                  20                  25                  30


      Asn Gly Pro Gly Ser Gly Val Ile Gly Pro Gly Leu Ser Gly Leu Tyr
                  35                  40                  45


      Gly Pro Gly Val Ile Gly Pro Gly Val Ile Gly Pro Gly Ser Ser Ala
          50                  55                  60


      Ala Ala Ala Ala Gly Pro Gly Leu Tyr Gly Pro Gly Val Ile Gly Pro
      65                  70                  75                  80


      Ser Ala Ser Ala Ala Ala Ala Ala Gly Pro Gly Ser Gly Val Ile Gly
                  85                  90                  95
```

```
Pro Gly Ala Ser Gly Leu Tyr Gly Pro Gly Val Ile Gly Pro Gly Val
            100                 105                 110

Ile Gly Pro Gly Ser Ser Ala Ala Ala Ala Ala Gly Leu Tyr Gly Ser
            115                 120                 125

Gly Pro Gly Val Ile Gly Pro Tyr Gly Ser Ala Ala Ala Ala Ala Gly
            130                 135                 140

Pro Gly Ser Gly Leu Tyr Gly Leu Gly Pro Tyr Gly Pro Gly Ala Ser
145                 150                 155                 160

Gly Pro Gly Leu Tyr Gly Pro Gly Val Ile Gly Pro Ser Ala Ser Ala
                165                 170                 175

Ala Ala Ala Ala Gly Ser Gly Val Ile Gly Pro Gly Leu Tyr Gly Pro
            180                 185                 190

Tyr Ala Ser Ala Ala Ala Ala Ala Gly Leu Tyr Gly Ser Gly Pro Gly
            195                 200                 205

Val Ile Gly Pro Tyr Gly Pro Gly Leu Ser Gly Ser Gly Val Ile Gly
            210                 215                 220

Pro Gly Val Ile Gly Pro Tyr Ala Ser Ala Ala Ala Ala Ala Gly Pro
225                 230                 235                 240

Gly Val Ile Gly Pro Tyr Gly Pro Gly Ser Ser Ala Ala Ala Ala Ala
                245                 250                 255

Gly Leu Tyr Gly Tyr Gly Pro Gly Val Ile Gly Pro Tyr Gly Pro Gly
            260                 265                 270

Ala Ser Gly Leu Asn Gly Pro Gly Ser Gly Leu Tyr Gly Pro Gly Val
            275                 280                 285

Ile Gly Pro Gly Leu Ser Ala Ala Ala Ala Gly Pro Gly Val Ile
            290                 295                 300

Gly Pro Tyr Gly Pro Gly Ala Ser Ala Ala Ala Ala Gly Leu Tyr
305                 310                 315                 320

Gly Pro Gly Val Ile Gly Pro Gly Leu Tyr Gly Pro Gly Ser Ser Gly
                325                 330                 335

Pro Gly Val Ile Gly Pro Tyr Gly Pro Gly Ser Ser Ala Ala Ala Ala
```

                340                        345                        350

Ala Gly Leu Tyr Gly Pro Gly Val Ile Gly Pro Tyr Gly Pro Gly Leu
        355                    360                    365

Ser Ala Ala Ala Ala Ala Gly Leu Tyr Val Ile Gly Pro Gly Val Ile
        370                    375                    380

Gly Pro Tyr Gly Pro Gly Ala Ser Gly Pro Gly Val Ile Gly Pro Tyr
385                    390                    395                    400

Gly Pro Gly Ala Ser Ala Ala Ala Ala Ala Gly Pro Gly Leu Tyr Gly
                    405                    410                    415

Pro Gly Val Ile Gly Pro Ser Ala Ser Ala Ala Ala Ala Ala Gly Leu
            420                    425                    430

Tyr Gly Ser Gly Pro Gly Leu Tyr Gly Pro Tyr Gly Pro Gly Leu Ser
        435                    440                    445

Gly Pro Gly Ser Gly Val Ile Gly Leu Gly Pro Tyr Gly Pro Gly Ala
        450                    455                    460

Ser Ala Ala Ala Ala Ala Gly Leu Tyr Gly Pro Gly Val Ile Gly Pro
465                    470                    475                    480

Tyr Gly Pro Gly Leu Ser Ala Ala Ala Ala Gly Pro Gly Ser Gly
                485                    490                    495

Leu Tyr Gly Pro Gly Ala Ser Gly Leu Asn Gly Pro Gly Ser Gly Leu
                500                    505                    510

Tyr Gly Pro Gly Val Ile Gly Pro Gly Leu Ser Ala Ala Ala Ala Ala
        515                    520                    525

Gly Leu Tyr Val Ile Gly Pro Gly Val Ile Gly Pro Tyr Gly Pro Gly
        530                    535                    540

Ala Ser Ala Ala Ala Ala Ala Gly Leu Tyr Gly Ser Gly Pro Gly Val
545                    550                    555                    560

Ile Gly Pro Tyr Gly Pro Gly Leu Ser Gly Ser Gly Val Ile Gly Pro
                565                    570                    575

Gly Val Ile Gly Pro Tyr Ala Ser Ala Ala Ala Ala Gly Pro Gly
        580                    585                    590

Ser Gly Val Ile Gly Pro Gly Ala Ser
        595                 600


<210> 37
<211> 601
<212> PRT
<213> Artificial Sequence

<220>
<223> PRT918

<400> 37

Met His His His His His His Ser Ser Gly Ser Ser Gly Pro Gly Val
1               5                   10                  15


Phe Gly Pro Tyr Gly Pro Gly Ala Ser Ala Ala Ala Ala Ala Gly Ile
            20                  25                  30


Asn Gly Pro Gly Ser Gly Val Phe Gly Pro Gly Ile Ser Gly Ile Tyr
            35                  40                  45


Gly Pro Gly Val Phe Gly Pro Gly Val Phe Gly Pro Gly Ser Ser Ala
        50                  55                  60


Ala Ala Ala Ala Gly Pro Gly Ile Tyr Gly Pro Gly Val Phe Gly Pro
65                  70                  75                  80


Ser Ala Ser Ala Ala Ala Ala Ala Gly Pro Gly Ser Gly Val Phe Gly
            85                  90                  95


Pro Gly Ala Ser Gly Ile Tyr Gly Pro Gly Val Phe Gly Pro Gly Val
            100                 105                 110


Phe Gly Pro Gly Ser Ser Ala Ala Ala Ala Ala Gly Ile Tyr Gly Ser
            115                 120                 125


Gly Pro Gly Val Phe Gly Pro Tyr Gly Ser Ala Ala Ala Ala Ala Gly
        130                 135                 140


Pro Gly Ser Gly Ile Tyr Gly Ile Gly Pro Tyr Gly Pro Gly Ala Ser
145                 150                 155                 160


Gly Pro Gly Ile Tyr Gly Pro Gly Val Phe Gly Pro Ser Ala Ser Ala
            165                 170                 175


Ala Ala Ala Ala Gly Ser Gly Val Phe Gly Pro Gly Ile Tyr Gly Pro
            180                 185                 190


Tyr Ala Ser Ala Ala Ala Ala Ala Gly Ile Tyr Gly Ser Gly Pro Gly

195                    200                    205

Val Phe Gly Pro Tyr Gly Pro Gly Ile Ser Gly Ser Gly Val Phe Gly
    210                    215                    220

Pro Gly Val Phe Gly Pro Tyr Ala Ser Ala Ala Ala Ala Ala Gly Pro
225                    230                    235                    240

Gly Val Phe Gly Pro Tyr Gly Pro Gly Ser Ser Ala Ala Ala Ala Ala
                    245                    250                    255

Gly Ile Tyr Gly Tyr Gly Pro Gly Val Phe Gly Pro Tyr Gly Pro Gly
                    260                    265                    270

Ala Ser Gly Ile Asn Gly Pro Gly Ser Gly Ile Tyr Gly Pro Gly Val
                    275                    280                    285

Phe Gly Pro Gly Ile Ser Ala Ala Ala Ala Gly Pro Gly Val Phe
                    290                    295                    300

Gly Pro Tyr Gly Pro Gly Ala Ser Ala Ala Ala Ala Ala Gly Ile Tyr
305                    310                    315                    320

Gly Pro Gly Val Phe Gly Pro Gly Ile Tyr Gly Pro Gly Ser Ser Gly
                    325                    330                    335

Pro Gly Val Phe Gly Pro Tyr Gly Pro Gly Ser Ser Ala Ala Ala Ala
                    340                    345                    350

Ala Gly Ile Tyr Gly Pro Gly Val Phe Gly Pro Tyr Gly Pro Gly Ile
                    355                    360                    365

Ser Ala Ala Ala Ala Ala Gly Ile Tyr Val Phe Gly Pro Gly Val Phe
                    370                    375                    380

Gly Pro Tyr Gly Pro Gly Ala Ser Gly Pro Gly Val Phe Gly Pro Tyr
385                    390                    395                    400

Gly Pro Gly Ala Ser Ala Ala Ala Ala Ala Gly Pro Gly Ile Tyr Gly
                    405                    410                    415

Pro Gly Val Phe Gly Pro Ser Ala Ser Ala Ala Ala Ala Ala Gly Ile
                    420                    425                    430

Tyr Gly Ser Gly Pro Gly Ile Tyr Gly Pro Tyr Gly Pro Gly Ile Ser
                    435                    440                    445

151

```
Gly Pro Gly Ser Gly Val Phe Gly Ile Gly Pro Tyr Gly Pro Gly Ala
    450                 455                 460

Ser Ala Ala Ala Ala Ala Gly Ile Tyr Gly Pro Gly Val Phe Gly Pro
465                 470                 475                 480

Tyr Gly Pro Gly Ile Ser Ala Ala Ala Ala Gly Pro Gly Ser Gly
                485                 490                 495

Ile Tyr Gly Pro Gly Ala Ser Gly Ile Asn Gly Pro Gly Ser Gly Ile
            500                 505                 510

Tyr Gly Pro Gly Val Phe Gly Pro Gly Ile Ser Ala Ala Ala Ala Ala
            515                 520                 525

Gly Ile Tyr Val Phe Gly Pro Gly Val Phe Gly Pro Tyr Gly Pro Gly
    530                 535                 540

Ala Ser Ala Ala Ala Ala Ala Gly Ile Tyr Gly Ser Gly Pro Gly Val
545                 550                 555                 560

Phe Gly Pro Tyr Gly Pro Gly Ile Ser Gly Ser Gly Val Phe Gly Pro
            565                 570                 575

Gly Val Phe Gly Pro Tyr Ala Ser Ala Ala Ala Ala Ala Gly Pro Gly
            580                 585                 590

Ser Gly Val Phe Gly Pro Gly Ala Ser
        595                 600
```

```
<210>  38
<211>  576
<212>  PRT
<213>  Artificial Sequence

<220>
<223>  PRT699

<400>  38
```

```
Met His His His His His His Ser Ser Gly Ser Ser Gly Pro Gly Val
1               5                   10                  15

Leu Gly Pro Tyr Gly Pro Gly Ala Ser Ala Ala Ala Ala Ala Ala Ala
            20                  25                  30

Gly Ser Asn Gly Pro Gly Ser Gly Val Leu Gly Pro Gly Gln Ser Gly
        35                  40                  45

Gln Tyr Gly Pro Gly Val Leu Gly Pro Gly Val Leu Gly Pro Gly Ser
```

152

50                          55                          60

```
Ser Ala Ala Ala Ala Ala Ala Ala Gly Pro Gly Gln Tyr Gly Pro Gly
65              70              75                      80

Val Leu Gly Pro Ser Ala Ser Ala Ala Ala Ala Ala Ala Ala Gly Pro
                85              90                      95

Gly Ser Gly Val Leu Gly Pro Gly Ala Ser Gly Gln Tyr Gly Pro Gly
                100             105             110

Val Leu Gly Pro Gly Val Leu Gly Pro Gly Ser Ser Ala Ala Ala Ala
            115             120             125

Ala Ala Ala Gly Ser Tyr Gly Ser Gly Pro Gly Val Leu Gly Pro Tyr
    130             135             140

Gly Ser Ala Ala Ala Ala Ala Ala Ala Gly Pro Gly Ser Gly Gln Tyr
145             150             155                     160

Gly Gln Gly Pro Tyr Gly Pro Gly Ala Ser Gly Pro Gly Gln Tyr Gly
                165             170             175

Pro Gly Val Leu Gly Pro Ser Ala Ser Ala Ala Ala Ala Ala Ala Ala
            180             185             190

Gly Ser Gly Val Leu Gly Pro Gly Gln Tyr Gly Pro Tyr Ala Ser Ala
            195             200             205

Ala Ala Ala Ala Ala Gly Ser Tyr Gly Ser Gly Pro Gly Val Leu
    210             215             220

Gly Pro Tyr Gly Pro Gly Gln Ser Gly Ser Gly Val Leu Gly Pro Gly
225             230             235                     240

Val Leu Gly Pro Tyr Ala Ser Ala Ala Ala Ala Ala Ala Gly Pro
            245             250             255

Gly Val Leu Gly Pro Tyr Gly Pro Gly Ser Ser Ala Ala Ala Ala
            260             265             270

Ala Ala Gly Ser Tyr Gly Tyr Gly Pro Gly Val Leu Gly Pro Tyr Gly
    275             280             285

Pro Gly Ala Ser Gly Gln Asn Gly Pro Gly Ser Gly Gln Tyr Gly Pro
    290             295             300
```

```
Gly Val Leu Gly Pro Gly Pro Ser Ala Ala Ala Ala Ala Ala Gly
305             310         315                 320

Pro Gly Val Leu Gly Pro Tyr Gly Pro Gly Ala Ser Ala Ala Ala Ala
            325             330                 335

Ala Ala Ala Gly Ser Tyr Gly Pro Gly Val Leu Gly Pro Gly Gln Tyr
            340             345                 350

Gly Pro Gly Ser Ser Gly Pro Gly Val Leu Gly Pro Tyr Gly Pro Gly
            355             360                 365

Ser Ser Ala Ala Ala Ala Ala Ala Ala Gly Ser Tyr Gly Pro Gly Val
    370             375                 380

Leu Gly Pro Tyr Gly Pro Gly Pro Ser Ala Ala Ala Ala Ala Ala Ala
385             390                 395                 400

Gly Ser Tyr Val Leu Gly Pro Gly Val Leu Gly Pro Tyr Gly Pro Gly
            405             410                 415

Ala Ser Gly Pro Gly Val Leu Gly Pro Tyr Gly Pro Gly Ala Ser Ala
            420             425                 430

Ala Ala Ala Ala Ala Ala Gly Pro Gly Gln Tyr Gly Pro Gly Val Leu
    435             440                 445

Gly Pro Ser Ala Ser Ala Ala Ala Ala Ala Ala Gly Ser Tyr Gly
    450             455                 460

Ser Gly Pro Gly Gln Tyr Gly Pro Tyr Gly Pro Gly Gln Ser Gly Pro
465             470             475                 480

Gly Ser Gly Val Leu Gly Gln Gly Pro Tyr Gly Pro Gly Ala Ser Ala
            485             490                 495

Ala Ala Ala Ala Ala Gly Ser Tyr Gly Pro Gly Val Leu Gly Pro
    500             505                 510

Tyr Gly Pro Gly Pro Ser Ala Ala Ala Ala Ala Ala Gly Pro Gly
    515             520                 525

Ser Gly Gln Tyr Gly Pro Gly Ala Ser Gly Gln Asn Gly Pro Gly Ser
    530             535                 540

Gly Gln Tyr Gly Pro Gly Val Leu Gly Pro Gly Pro Ser Ala Ala Ala
545             550             555                 560
```

154

Ala Ala Ala Ala Gly Pro Gly Ser Gly Val Leu Gly Pro Gly Ala Ser
                565              570              575

<210> 39
<211> 576
<212> PRT
<213> Artificial Sequence

<220>
<223> PRT698

<400> 39

Met His His His His His His Ser Ser Gly Ser Ser Gly Pro Gly Val
1               5               10              15

Leu Gly Pro Tyr Gly Pro Gly Ala Ser Ala Ala Ala Ala Ala Ala Ala
            20              25              30

Gly Ser Asn Gly Pro Gly Ser Gly Val Leu Gly Pro Gly Ile Ser Gly
        35              40              45

Ile Tyr Gly Pro Gly Val Leu Gly Pro Gly Val Leu Gly Pro Gly Ser
    50              55              60

Ser Ala Ala Ala Ala Ala Ala Gly Pro Gly Ile Tyr Gly Pro Gly
65              70              75              80

Val Leu Gly Pro Ser Ala Ser Ala Ala Ala Ala Ala Ala Gly Pro
            85              90              95

Gly Ser Gly Val Leu Gly Pro Gly Ala Ser Gly Ile Tyr Gly Pro Gly
            100             105             110

Val Leu Gly Pro Gly Val Leu Gly Pro Gly Ser Ser Ala Ala Ala Ala
            115             120             125

Ala Ala Ala Gly Ser Tyr Gly Ser Gly Pro Gly Val Leu Gly Pro Tyr
    130             135             140

Gly Ser Ala Ala Ala Ala Ala Ala Gly Pro Gly Ser Gly Ile Tyr
145             150             155             160

Gly Ile Gly Pro Tyr Gly Pro Gly Ala Ser Gly Pro Gly Ile Tyr Gly
            165             170             175

Pro Gly Val Leu Gly Pro Ser Ala Ser Ala Ala Ala Ala Ala Ala
            180             185             190

```
Gly Ser Gly Val Leu Gly Pro Gly Ile Tyr Gly Pro Tyr Ala Ser Ala
    195                 200                 205

Ala Ala Ala Ala Ala Ala Gly Ser Tyr Gly Ser Gly Pro Gly Val Leu
    210                 215                 220

Gly Pro Tyr Gly Pro Gly Ile Ser Gly Ser Gly Val Leu Gly Pro Gly
225                 230                 235                 240

Val Leu Gly Pro Tyr Ala Ser Ala Ala Ala Ala Ala Ala Ala Gly Pro
                245                 250                 255

Gly Val Leu Gly Pro Tyr Gly Pro Gly Ser Ser Ala Ala Ala Ala Ala
                260                 265                 270

Ala Ala Gly Ser Tyr Gly Tyr Gly Pro Gly Val Leu Gly Pro Tyr Gly
            275                 280                 285

Pro Gly Ala Ser Gly Ile Asn Gly Pro Gly Ser Gly Ile Tyr Gly Pro
        290                 295                 300

Gly Val Leu Gly Pro Gly Pro Ser Ala Ala Ala Ala Ala Ala Ala Gly
305                 310                 315                 320

Pro Gly Val Leu Gly Pro Tyr Gly Pro Gly Ala Ser Ala Ala Ala Ala
                325                 330                 335

Ala Ala Ala Gly Ser Tyr Gly Pro Gly Val Leu Gly Pro Gly Ile Tyr
            340                 345                 350

Gly Pro Gly Ser Ser Gly Pro Gly Val Leu Gly Pro Tyr Gly Pro Gly
        355                 360                 365

Ser Ser Ala Ala Ala Ala Ala Ala Gly Ser Tyr Gly Pro Gly Val
    370                 375                 380

Leu Gly Pro Tyr Gly Pro Gly Pro Ser Ala Ala Ala Ala Ala Ala
385                 390                 395                 400

Gly Ser Tyr Val Leu Gly Pro Gly Val Leu Gly Pro Tyr Gly Pro Gly
            405                 410                 415

Ala Ser Gly Pro Gly Val Leu Gly Pro Tyr Gly Pro Gly Ala Ser Ala
        420                 425                 430

Ala Ala Ala Ala Ala Ala Gly Pro Gly Ile Tyr Gly Pro Gly Val Leu
    435                 440                 445
```

```
Gly Pro Ser Ala Ser Ala Ala Ala Ala Ala Ala Gly Ser Tyr Gly
    450             455             460

Ser Gly Pro Gly Ile Tyr Gly Pro Tyr Gly Pro Gly Ile Ser Gly Pro
465             470             475             480

Gly Ser Gly Val Leu Gly Ile Gly Pro Tyr Gly Pro Gly Ala Ser Ala
                485             490             495

Ala Ala Ala Ala Ala Ala Gly Ser Tyr Gly Pro Gly Val Leu Gly Pro
            500             505             510

Tyr Gly Pro Gly Pro Ser Ala Ala Ala Ala Ala Ala Ala Gly Pro Gly
    515             520             525

Ser Gly Ile Tyr Gly Pro Gly Ala Ser Gly Ile Asn Gly Pro Gly Ser
    530             535             540

Gly Ile Tyr Gly Pro Gly Val Leu Gly Pro Gly Pro Ser Ala Ala Ala
545             550             555             560

Ala Ala Ala Ala Gly Pro Gly Ser Gly Val Leu Gly Pro Gly Ala Ser
            565             570             575
```

```
<210>   40
<211>   2372
<212>   PRT
<213>   Artificial Sequence

<220>
<223>   PRT1009

<400>   40
```

```
Met His His His His His His Ser Ser Gly Ser Ser Gly Pro Gly Val
1               5               10              15

Phe Gly Pro Tyr Gly Pro Gly Ala Ser Ala Ala Ala Ala Ala Gly Ile
            20              25              30

Asn Gly Pro Gly Ser Gly Val Phe Gly Pro Gly Ile Ser Gly Ile Tyr
            35              40              45

Gly Pro Gly Val Phe Gly Pro Gly Val Phe Gly Pro Gly Ser Ser Ala
    50              55              60

Ala Ala Ala Gly Pro Gly Ile Tyr Gly Pro Gly Val Phe Gly Pro
65              70              75              80
```

157

Ser Ala Ser Ala Ala Ala Ala Ala Gly Pro Gly Ser Gly Val Phe Gly
                85                      90                      95

Pro Gly Ala Ser Gly Ile Tyr Gly Pro Gly Val Phe Gly Pro Gly Val
            100                     105                     110

Phe Gly Pro Gly Ser Ser Ala Ala Ala Ala Gly Ile Tyr Gly Ser
        115                 120                 125

Gly Pro Gly Val Phe Gly Pro Tyr Gly Ser Ala Ala Ala Ala Gly
    130                 135                 140

Pro Gly Ser Gly Ile Tyr Gly Ile Gly Pro Tyr Gly Pro Gly Ala Ser
145                 150                 155                 160

Gly Pro Gly Ile Tyr Gly Pro Gly Val Phe Gly Pro Ser Ala Ser Ala
                165                 170                 175

Ala Ala Ala Ala Gly Ser Gly Val Phe Gly Pro Gly Ile Tyr Gly Pro
        180                 185                 190

Tyr Ala Ser Ala Ala Ala Ala Ala Gly Ile Tyr Gly Ser Gly Pro Gly
        195                 200                 205

Val Phe Gly Pro Tyr Gly Pro Gly Ile Ser Gly Ser Gly Val Phe Gly
    210                 215                 220

Pro Gly Val Phe Gly Pro Tyr Ala Ser Ala Ala Ala Ala Gly Pro
225                 230                 235                 240

Gly Val Phe Gly Pro Tyr Gly Pro Gly Ser Ser Ala Ala Ala Ala
            245                 250                 255

Gly Ile Tyr Gly Tyr Gly Pro Gly Val Phe Gly Pro Tyr Gly Pro Gly
        260                 265                 270

Ala Ser Gly Ile Asn Gly Pro Gly Ser Gly Ile Tyr Gly Pro Gly Val
        275                 280                 285

Phe Gly Pro Gly Ile Ser Ala Ala Ala Ala Gly Pro Gly Val Phe
    290                 295                 300

Gly Pro Tyr Gly Pro Gly Ala Ser Ala Ala Ala Ala Gly Ile Tyr
305                 310                 315                 320

Gly Pro Gly Val Phe Gly Pro Gly Ile Tyr Gly Pro Gly Ser Ser Gly
            325                 330                 335

Pro Gly Val Phe Gly Pro Tyr Gly Pro Gly Ser Ser Ala Ala Ala Ala
        340             345         350

Ala Gly Ile Tyr Gly Pro Gly Val Phe Gly Pro Tyr Gly Pro Gly Ile
        355             360         365

Ser Ala Ala Ala Ala Ala Gly Ile Tyr Val Phe Gly Pro Gly Val Phe
    370             375         380

Gly Pro Tyr Gly Pro Gly Ala Ser Gly Pro Gly Val Phe Gly Pro Tyr
385             390         395             400

Gly Pro Gly Ala Ser Ala Ala Ala Ala Ala Gly Pro Gly Ile Tyr Gly
                405             410             415

Pro Gly Val Phe Gly Pro Ser Ala Ser Ala Ala Ala Ala Ala Gly Ile
        420             425         430

Tyr Gly Ser Gly Pro Gly Ile Tyr Gly Pro Tyr Gly Pro Gly Ile Ser
        435             440         445

Gly Pro Gly Ser Gly Val Phe Gly Ile Gly Pro Tyr Gly Pro Gly Ala
    450             455         460

Ser Ala Ala Ala Ala Ala Gly Ile Tyr Gly Pro Gly Val Phe Gly Pro
465             470             475             480

Tyr Gly Pro Gly Ile Ser Ala Ala Ala Ala Gly Pro Gly Ser Gly
            485             490             495

Ile Tyr Gly Pro Gly Ala Ser Gly Ile Asn Gly Pro Gly Ser Gly Ile
        500             505         510

Tyr Gly Pro Gly Val Phe Gly Pro Gly Ile Ser Ala Ala Ala Ala Ala
        515             520         525

Gly Ile Tyr Val Phe Gly Pro Gly Val Phe Gly Pro Tyr Gly Pro Gly
    530             535         540

Ala Ser Ala Ala Ala Ala Gly Ile Tyr Gly Ser Gly Pro Gly Val
545             550             555             560

Phe Gly Pro Tyr Gly Pro Gly Ile Ser Gly Ser Gly Val Phe Gly Pro
            565             570             575

Gly Val Phe Gly Pro Tyr Ala Ser Ala Ala Ala Ala Gly Pro Gly
    580             585             590

```
Ser Gly Val Phe Gly Pro Gly Ala Ser Gly Pro Gly Val Phe Gly Pro
        595                 600                 605

Tyr Gly Pro Gly Ala Ser Ala Ala Ala Ala Ala Gly Ile Asn Gly Pro
        610                 615                 620

Gly Ser Gly Val Phe Gly Pro Gly Ile Ser Gly Ile Tyr Gly Pro Gly
625                 630                 635                 640

Val Phe Gly Pro Gly Val Phe Gly Pro Gly Ser Ser Ala Ala Ala Ala
                645                 650                 655

Ala Gly Pro Gly Ile Tyr Gly Pro Gly Val Phe Gly Pro Ser Ala Ser
            660                 665                 670

Ala Ala Ala Ala Ala Gly Pro Gly Ser Gly Val Phe Gly Pro Gly Ala
        675                 680                 685

Ser Gly Ile Tyr Gly Pro Gly Val Phe Gly Pro Gly Val Phe Gly Pro
        690                 695                 700

Gly Ser Ser Ala Ala Ala Ala Ala Gly Ile Tyr Gly Ser Gly Pro Gly
705                 710                 715                 720

Val Phe Gly Pro Tyr Gly Ser Ala Ala Ala Ala Ala Gly Pro Gly Ser
                725                 730                 735

Gly Ile Tyr Gly Ile Gly Pro Tyr Gly Pro Gly Ala Ser Gly Pro Gly
            740                 745                 750

Ile Tyr Gly Pro Gly Val Phe Gly Pro Ser Ala Ser Ala Ala Ala Ala
        755                 760                 765

Ala Gly Ser Gly Val Phe Gly Pro Gly Ile Tyr Gly Pro Tyr Ala Ser
        770                 775                 780

Ala Ala Ala Ala Ala Gly Ile Tyr Gly Ser Gly Pro Gly Val Phe Gly
785                 790                 795                 800

Pro Tyr Gly Pro Gly Ile Ser Gly Ser Gly Val Phe Gly Pro Gly Val
                805                 810                 815

Phe Gly Pro Tyr Ala Ser Ala Ala Ala Ala Ala Gly Pro Gly Val Phe
            820                 825                 830

Gly Pro Tyr Gly Pro Gly Ser Ser Ala Ala Ala Ala Ala Gly Ile Tyr
```

835                    840                    845

Gly Tyr Gly Pro Gly Val Phe Gly Pro Tyr Gly Pro Gly Ala Ser Gly
    850                    855                    860

Ile Asn Gly Pro Gly Ser Gly Ile Tyr Gly Pro Gly Val Phe Gly Pro
865                    870                    875                    880

Gly Ile Ser Ala Ala Ala Ala Ala Gly Pro Gly Val Phe Gly Pro Tyr
                885                    890                    895

Gly Pro Gly Ala Ser Ala Ala Ala Ala Gly Ile Tyr Gly Pro Gly
                900                    905                    910

Val Phe Gly Pro Gly Ile Tyr Gly Pro Gly Ser Ser Gly Pro Gly Val
                915                    920                    925

Phe Gly Pro Tyr Gly Pro Gly Ser Ser Ala Ala Ala Ala Ala Gly Ile
    930                    935                    940

Tyr Gly Pro Gly Val Phe Gly Pro Tyr Gly Pro Gly Ile Ser Ala Ala
945                    950                    955                    960

Ala Ala Ala Gly Ile Tyr Val Phe Gly Pro Gly Val Phe Gly Pro Tyr
                965                    970                    975

Gly Pro Gly Ala Ser Gly Pro Gly Val Phe Gly Pro Tyr Gly Pro Gly
                980                    985                    990

Ala Ser Ala Ala Ala Ala Ala Gly Pro Gly Ile Tyr Gly Pro Gly Val
    995                    1000                   1005

Phe Gly Pro Ser Ala Ser Ala Ala Ala Ala Ala Gly Ile Tyr Gly
    1010                   1015                   1020

Ser Gly Pro Gly Ile Tyr Gly Pro Tyr Gly Pro Gly Ile Ser Gly
    1025                   1030                   1035

Pro Gly Ser Gly Val Phe Gly Ile Gly Pro Tyr Gly Pro Gly Ala
    1040                   1045                   1050

Ser Ala Ala Ala Ala Ala Gly Ile Tyr Gly Pro Gly Val Phe Gly
    1055                   1060                   1065

Pro Tyr Gly Pro Gly Ile Ser Ala Ala Ala Ala Ala Gly Pro Gly
    1070                   1075                   1080

161

```
Ser Gly  Ile Tyr Gly Pro Gly  Ala Ser Gly Ile Asn  Gly Pro Gly
    1085             1090                 1095

Ser Gly  Ile Tyr Gly Pro Gly  Val Phe Gly Pro Gly  Ile Ser Ala
    1100             1105                 1110

Ala Ala  Ala Ala Gly Ile Tyr  Val Phe Gly Pro Gly  Val Phe Gly
    1115             1120                 1125

Pro Tyr  Gly Pro Gly Ala Ser  Ala Ala Ala Ala Ala  Gly Ile Tyr
    1130             1135                 1140

Gly Ser  Gly Pro Gly Val Phe  Gly Pro Tyr Gly Pro  Gly Ile Ser
    1145             1150                 1155

Gly Ser  Gly Val Phe Gly Pro  Gly Val Phe Gly Pro  Tyr Ala Ser
    1160             1165                 1170

Ala Ala  Ala Ala Ala Gly Pro  Gly Ser Gly Val Phe  Gly Pro Gly
    1175             1180                 1185

Ala Ser  Glu Phe Gly Pro Gly  Val Phe Gly Pro Tyr  Gly Pro Gly
    1190             1195                 1200

Ala Ser  Ala Ala Ala Ala Ala  Gly Ile Asn Gly Pro  Gly Ser Gly
    1205             1210                 1215

Val Phe  Gly Pro Gly Ile Ser  Gly Ile Tyr Gly Pro  Gly Val Phe
    1220             1225                 1230

Gly Pro  Gly Val Phe Gly Pro  Gly Ser Ser Ala Ala  Ala Ala Ala
    1235             1240                 1245

Gly Pro  Gly Ile Tyr Gly Pro  Gly Val Phe Gly Pro  Ser Ala Ser
    1250             1255                 1260

Ala Ala  Ala Ala Ala Gly Pro  Gly Ser Gly Val Phe  Gly Pro Gly
    1265             1270                 1275

Ala Ser  Gly Ile Tyr Gly Pro  Gly Val Phe Gly Pro  Gly Val Phe
    1280             1285                 1290

Gly Pro  Gly Ser Ser Ala Ala  Ala Ala Ala Gly Ile  Tyr Gly Ser
    1295             1300                 1305

Gly Pro  Gly Val Phe Gly Pro  Tyr Gly Ser Ala Ala  Ala Ala Ala
    1310             1315                 1320
```

Gly Pro Gly Ser Gly Ile Tyr Gly Ile Gly Pro Tyr Gly Pro Gly
1325 1330 1335

Ala Ser Gly Pro Gly Ile Tyr Gly Pro Gly Val Phe Gly Pro Ser
1340 1345 1350

Ala Ser Ala Ala Ala Ala Ala Gly Ser Gly Val Phe Gly Pro Gly
1355 1360 1365

Ile Tyr Gly Pro Tyr Ala Ser Ala Ala Ala Ala Ala Gly Ile Tyr
1370 1375 1380

Gly Ser Gly Pro Gly Val Phe Gly Pro Tyr Gly Pro Gly Ile Ser
1385 1390 1395

Gly Ser Gly Val Phe Gly Pro Gly Val Phe Gly Pro Tyr Ala Ser
1400 1405 1410

Ala Ala Ala Ala Ala Gly Pro Gly Val Phe Gly Pro Tyr Gly Pro
1415 1420 1425

Gly Ser Ser Ala Ala Ala Ala Ala Gly Ile Tyr Gly Tyr Gly Pro
1430 1435 1440

Gly Val Phe Gly Pro Tyr Gly Pro Gly Ala Ser Gly Ile Asn Gly
1445 1450 1455

Pro Gly Ser Gly Ile Tyr Gly Pro Gly Val Phe Gly Pro Gly Ile
1460 1465 1470

Ser Ala Ala Ala Ala Ala Gly Pro Gly Val Phe Gly Pro Tyr Gly
1475 1480 1485

Pro Gly Ala Ser Ala Ala Ala Ala Ala Gly Ile Tyr Gly Pro Gly
1490 1495 1500

Val Phe Gly Pro Gly Ile Tyr Gly Pro Gly Ser Ser Gly Pro Gly
1505 1510 1515

Val Phe Gly Pro Tyr Gly Pro Gly Ser Ser Ala Ala Ala Ala Ala
1520 1525 1530

Gly Ile Tyr Gly Pro Gly Val Phe Gly Pro Tyr Gly Pro Gly Ile
1535 1540 1545

Ser Ala Ala Ala Ala Ala Gly Ile Tyr Val Phe Gly Pro Gly Val
1550 1555 1560

Phe Gly Pro Tyr Gly Pro Gly   Ala Ser Gly Pro Gly   Val Phe Gly
    1565                1570                1575

Pro Tyr Gly Pro Gly Ala Ser   Ala Ala Ala Ala Ala   Gly Pro Gly
    1580                1585                1590

Ile Tyr Gly Pro Gly Val Phe   Gly Pro Ser Ala Ser   Ala Ala Ala
    1595                1600                1605

Ala Ala Gly Ile Tyr Gly Ser   Gly Pro Gly Ile Tyr   Gly Pro Tyr
    1610                1615                1620

Gly Pro Gly Ile Ser Gly Pro   Gly Ser Gly Val Phe   Gly Ile Gly
    1625                1630                1635

Pro Tyr Gly Pro Gly Ala Ser   Ala Ala Ala Ala Ala   Gly Ile Tyr
    1640                1645                1650

Gly Pro Gly Val Phe Gly Pro   Tyr Gly Pro Gly Ile   Ser Ala Ala
    1655                1660                1665

Ala Ala Ala Gly Pro Gly Ser   Gly Ile Tyr Gly Pro   Gly Ala Ser
    1670                1675                1680

Gly Ile Asn Gly Pro Gly Ser   Gly Ile Tyr Gly Pro   Gly Val Phe
    1685                1690                1695

Gly Pro Gly Ile Ser Ala Ala   Ala Ala Ala Gly Ile   Tyr Val Phe
    1700                1705                1710

Gly Pro Gly Val Phe Gly Pro   Tyr Gly Pro Gly Ala   Ser Ala Ala
    1715                1720                1725

Ala Ala Ala Gly Ile Tyr Gly   Ser Gly Pro Gly Val   Phe Gly Pro
    1730                1735                1740

Tyr Gly Pro Gly Ile Ser Gly   Ser Gly Val Phe Gly   Pro Gly Val
    1745                1750                1755

Phe Gly Pro Tyr Ala Ser Ala   Ala Ala Ala Ala Gly   Pro Gly Ser
    1760                1765                1770

Gly Val Phe Gly Pro Gly Ala   Ser Glu Leu Gly Pro   Gly Val Phe
    1775                1780                1785

Gly Pro Tyr Gly Pro Gly Ala   Ser Ala Ala Ala Ala   Ala Gly Ile

                 1790                          1795                        1800


        Asn Gly Pro Gly Ser Gly Val Phe Gly Pro Gly Ile Ser Gly Ile
            1805            1810            1815


        Tyr Gly Pro Gly Val Phe Gly Pro Gly Val Phe Gly Pro Gly Ser
            1820            1825            1830


        Ser Ala Ala Ala Ala Ala Gly Pro Gly Ile Tyr Gly Pro Gly Val
            1835            1840            1845


        Phe Gly Pro Ser Ala Ser Ala Ala Ala Ala Gly Pro Gly Ser
            1850            1855            1860


        Gly Val Phe Gly Pro Gly Ala Ser Gly Ile Tyr Gly Pro Gly Val
            1865            1870            1875


        Phe Gly Pro Gly Val Phe Gly Pro Gly Ser Ser Ala Ala Ala Ala
            1880            1885            1890


        Ala Gly Ile Tyr Gly Ser Gly Pro Gly Val Phe Gly Pro Tyr Gly
            1895            1900            1905


        Ser Ala Ala Ala Ala Ala Gly Pro Gly Ser Gly Ile Tyr Gly Ile
            1910            1915            1920


        Gly Pro Tyr Gly Pro Gly Ala Ser Gly Pro Gly Ile Tyr Gly Pro
            1925            1930            1935


        Gly Val Phe Gly Pro Ser Ala Ser Ala Ala Ala Ala Gly Ser
            1940            1945            1950


        Gly Val Phe Gly Pro Gly Ile Tyr Gly Pro Tyr Ala Ser Ala Ala
            1955            1960            1965


        Ala Ala Ala Gly Ile Tyr Gly Ser Gly Pro Gly Val Phe Gly Pro
            1970            1975            1980


        Tyr Gly Pro Gly Ile Ser Gly Ser Gly Val Phe Gly Pro Gly Val
            1985            1990            1995


        Phe Gly Pro Tyr Ala Ser Ala Ala Ala Ala Gly Pro Gly Val
            2000            2005            2010


        Phe Gly Pro Tyr Gly Pro Gly Ser Ser Ala Ala Ala Ala Ala Gly
            2015            2020            2025

EP 3 779 000 A1

Ile Tyr Gly Tyr Gly Pro Gly Val Phe Gly Pro Tyr Gly Pro Gly
2030 2035 2040

Ala Ser Gly Ile Asn Gly Pro Gly Ser Gly Ile Tyr Gly Pro Gly
2045 2050 2055

Val Phe Gly Pro Gly Ile Ser Ala Ala Ala Ala Ala Gly Pro Gly
2060 2065 2070

Val Phe Gly Pro Tyr Gly Pro Gly Ala Ser Ala Ala Ala Ala Ala
2075 2080 2085

Gly Ile Tyr Gly Pro Gly Val Phe Gly Pro Gly Ile Tyr Gly Pro
2090 2095 2100

Gly Ser Ser Gly Pro Gly Val Phe Gly Pro Tyr Gly Pro Gly Ser
2105 2110 2115

Ser Ala Ala Ala Ala Ala Gly Ile Tyr Gly Pro Gly Val Phe Gly
2120 2125 2130

Pro Tyr Gly Pro Gly Ile Ser Ala Ala Ala Ala Ala Gly Ile Tyr
2135 2140 2145

Val Phe Gly Pro Gly Val Phe Gly Pro Tyr Gly Pro Gly Ala Ser
2150 2155 2160

Gly Pro Gly Val Phe Gly Pro Tyr Gly Pro Gly Ala Ser Ala Ala
2165 2170 2175

Ala Ala Ala Gly Pro Gly Ile Tyr Gly Pro Gly Val Phe Gly Pro
2180 2185 2190

Ser Ala Ser Ala Ala Ala Ala Ala Gly Ile Tyr Gly Ser Gly Pro
2195 2200 2205

Gly Ile Tyr Gly Pro Tyr Gly Pro Gly Ile Ser Gly Pro Gly Ser
2210 2215 2220

Gly Val Phe Gly Ile Gly Pro Tyr Gly Pro Gly Ala Ser Ala Ala
2225 2230 2235

Ala Ala Ala Gly Ile Tyr Gly Pro Gly Val Phe Gly Pro Tyr Gly
2240 2245 2250

Pro Gly Ile Ser Ala Ala Ala Ala Ala Gly Pro Gly Ser Gly Ile
2255 2260 2265

166

```
Tyr Gly  Pro Gly Ala Ser Gly  Ile Asn Gly Pro Gly  Ser Gly Ile
    2270             2275             2280


Tyr Gly  Pro Gly Val Phe Gly  Pro Gly Ile Ser Ala  Ala Ala Ala
    2285             2290             2295


Ala Gly  Ile Tyr Val Phe Gly  Pro Gly Val Phe Gly  Pro Tyr Gly
    2300             2305             2310


Pro Gly  Ala Ser Ala Ala Ala  Ala Ala Gly Ile Tyr  Gly Ser Gly
    2315             2320             2325


Pro Gly  Val Phe Gly Pro Tyr  Gly Pro Gly Ile Ser  Gly Ser Gly
    2330             2335             2340


Val Phe  Gly Pro Gly Val Phe  Gly Pro Tyr Ala Ser  Ala Ala Ala
    2345             2350             2355


Ala Ala  Gly Pro Gly Ser Gly  Val Phe Gly Pro Gly  Ala Ser
    2360             2365             2370



<210>  41
<211>  559
<212>  PRT
<213>  Artificial Sequence

<220>
<223>  PRT215

<400>  41

Met His His His His His His His His His His Ser Ser Gly Ser Ser
1                 5                 10                15


Leu Glu Val Leu Phe Gln Gly Pro Gly Ala Gly Gly Ser Gly Pro Gly
            20                25                30


Gly Ala Gly Pro Gly Gly Val Gly Pro Gly Gly Ser Gly Pro Gly Gly
        35                40                45


Val Gly Pro Gly Gly Ser Gly Pro Gly Gly Val Gly Pro Gly Gly Ser
    50                55                60


Gly Pro Gly Gly Val Gly Pro Gly Gly Ala Gly Gly Pro Tyr Gly Pro
65                70                75                80


Gly Gly Ser Gly Pro Gly Gly Ala Gly Gly Ala Gly Gly Pro Gly Gly
            85                90                95
```

```
Ala Tyr Gly Pro Gly Gly Ser Tyr Gly Pro Gly Gly Ser Gly Gly Pro
        100                 105             110

Gly Gly Ala Gly Gly Pro Tyr Gly Pro Gly Gly Glu Gly Pro Gly Gly
        115                 120             125

Ala Gly Gly Pro Tyr Gly Pro Gly Gly Ala Gly Gly Pro Tyr Gly Pro
    130             135             140

Gly Gly Ala Gly Gly Pro Tyr Gly Pro Gly Gly Glu Gly Gly Pro Tyr
145             150             155             160

Gly Pro Gly Gly Ser Tyr Gly Pro Gly Gly Ala Gly Gly Pro Tyr Gly
            165             170             175

Pro Gly Gly Pro Tyr Gly Pro Gly Gly Glu Gly Pro Gly Gly Ala Gly
        180             185             190

Gly Pro Tyr Gly Pro Gly Gly Val Gly Pro Gly Gly Gly Gly Pro Gly
    195             200             205

Gly Tyr Gly Pro Gly Gly Ala Gly Pro Gly Gly Tyr Gly Pro Gly Gly
    210             215             220

Ser Gly Pro Gly Gly Tyr Gly Pro Gly Gly Ser Gly Pro Gly Gly Tyr
225             230             235             240

Gly Pro Gly Gly Ser Gly Pro Gly Gly Tyr Gly Pro Gly Gly Ser Gly
            245             250             255

Pro Gly Gly Tyr Gly Pro Gly Gly Ser Gly Pro Gly Gly Ser Gly Pro
        260             265             270

Gly Gly Tyr Gly Pro Gly Gly Ser Gly Pro Gly Gly Ser Gly Pro Gly
    275             280             285

Gly Tyr Gly Pro Gly Gly Ser Gly Pro Gly Gly Tyr Gly Pro Gly Gly
    290             295             300

Ser Gly Pro Gly Gly Ser Gly Pro Gly Gly Tyr Gly Pro Gly Gly Ser
305             310             315             320

Gly Pro Gly Gly Ser Gly Pro Gly Gly Tyr Gly Pro Gly Gly Ser Gly
            325             330             335

Pro Gly Gly Phe Gly Pro Gly Gly Phe Gly Pro Gly Gly Ser Gly Pro
        340             345             350
```

```
Gly Gly Tyr Gly Pro Gly Gly Ser Gly Pro Gly Gly Ala Gly Pro Gly
        355                 360                 365

Gly Val Gly Pro Gly Gly Phe Gly Pro Gly Gly Ala Gly Pro Gly Gly
        370                 375                 380

Ala Gly Pro Gly Gly Ala Gly Pro Gly Gly Ala Gly Pro Gly Gly Ala
385                 390                 395                 400

Gly Pro Gly Gly Ala Gly Pro Gly Gly Ala Gly Pro Gly Gly Ala Gly
                405                 410                 415

Pro Gly Gly Ala Gly Gly Ala Gly Gly Ala Gly Gly Ala Gly Gly Ser
            420                 425                 430

Gly Gly Ala Gly Gly Ser Gly Gly Thr Thr Ile Ile Glu Asp Leu Asp
            435                 440                 445

Ile Thr Ile Asp Gly Ala Asp Gly Pro Ile Thr Ile Ser Glu Glu Leu
    450                 455                 460

Thr Ile Ser Ala Tyr Tyr Pro Ser Ser Arg Val Pro Asp Met Val Asn
465                 470                 475                 480

Gly Ile Met Ser Ala Met Gln Gly Ser Gly Phe Asn Tyr Gln Met Phe
                485                 490                 495

Gly Asn Met Leu Ser Gln Tyr Ser Ser Gly Ser Gly Thr Cys Asn Pro
                500                 505                 510

Asn Asn Val Asn Val Leu Met Asp Ala Leu Leu Ala Ala Leu His Cys
            515                 520                 525

Leu Ser Asn His Gly Ser Ser Ser Phe Ala Pro Ser Pro Thr Pro Ala
    530                 535                 540

Ala Met Ser Ala Tyr Ser Asn Ser Val Gly Arg Met Phe Ala Tyr
545                 550                 555
```

<210> 42
<211> 252
<212> PRT
<213> Artificial Sequence

<220>
<223> Collagen-type4-Kai

<400> 42

```
Met His His His His His His Ser Ser Gly Ser Ser Lys Asp Gly Val
1                   5               10              15

Pro Gly Phe Pro Gly Ser Glu Gly Val Lys Gly Asn Arg Gly Phe Pro
            20              25              30

Gly Leu Met Gly Glu Asp Gly Ile Lys Gly Gln Lys Gly Asp Ile Gly
        35              40              45

Pro Pro Gly Phe Arg Gly Pro Thr Glu Tyr Tyr Asp Thr Tyr Gln Glu
    50              55              60

Lys Gly Asp Glu Gly Thr Pro Gly Pro Pro Gly Pro Arg Gly Ala Arg
65              70              75              80

Gly Pro Gln Gly Pro Ser Gly Pro Pro Gly Val Pro Gly Ser Pro Gly
            85              90              95

Ser Ser Arg Pro Gly Leu Arg Gly Ala Pro Gly Trp Pro Gly Leu Lys
        100             105             110

Gly Ser Lys Gly Glu Arg Gly Arg Pro Gly Lys Asp Ala Met Gly Thr
        115             120             125

Pro Gly Ser Pro Gly Cys Ala Gly Ser Pro Gly Leu Pro Gly Ser Pro
    130             135             140

Gly Pro Pro Gly Pro Pro Gly Asp Ile Val Phe Arg Lys Gly Pro Pro
145             150             155             160

Gly Asp His Gly Leu Pro Gly Tyr Leu Gly Ser Pro Gly Ile Pro Gly
            165             170             175

Val Asp Gly Pro Lys Gly Glu Pro Gly Leu Leu Cys Thr Gln Cys Pro
        180             185             190

Tyr Ile Pro Gly Pro Pro Gly Leu Pro Gly Leu Pro Gly Leu His Gly
        195             200             205

Val Lys Gly Ile Pro Gly Arg Gln Gly Ala Ala Gly Leu Lys Gly Ser
    210             215             220

Pro Gly Ser Pro Gly Asn Thr Gly Leu Pro Gly Phe Pro Gly Phe Pro
225             230             235             240

Gly Ala Gln Gly Asp Pro Gly Leu Lys Gly Glu Lys
            245             250
```

170

<210> 43
<211> 310
<212> PRT
<213> Artificial Sequence

<220>
<223> Resilin-Kai

<400> 43

Met His His His His His His Pro Glu Pro Pro Val Asn Ser Tyr Leu
1               5               10              15


Pro Pro Ser Asp Ser Tyr Gly Ala Pro Gly Gln Ser Gly Pro Gly Gly
            20              25              30


Arg Pro Ser Asp Ser Tyr Gly Ala Pro Gly Gly Gly Asn Gly Gly Arg
            35              40              45


Pro Ser Asp Ser Tyr Gly Ala Pro Gly Gln Gly Gln Gly Gln Gly Gln
        50              55              60


Gly Gln Gly Gly Tyr Ala Gly Lys Pro Ser Asp Ser Tyr Gly Ala Pro
65              70              75              80


Gly Gly Gly Asp Gly Asn Gly Gly Arg Pro Ser Ser Ser Tyr Gly Ala
            85              90              95


Pro Gly Gly Gly Asn Gly Gly Arg Pro Ser Asp Thr Tyr Gly Ala Pro
            100             105             110


Gly Gly Gly Asn Gly Gly Arg Pro Ser Asp Thr Tyr Gly Ala Pro Gly
            115             120             125


Gly Gly Gly Asn Gly Asn Gly Gly Arg Pro Ser Ser Ser Tyr Gly Ala
    130             135             140


Pro Gly Gln Gly Gln Gly Asn Gly Asn Gly Gly Arg Pro Ser Ser Ser
145             150             155             160


Tyr Gly Ala Pro Gly Gly Gly Asn Gly Gly Arg Pro Ser Asp Thr Tyr
            165             170             175


Gly Ala Pro Gly Gly Gly Asn Gly Gly Arg Pro Ser Asp Thr Tyr Gly
            180             185             190


Ala Pro Gly Gly Gly Asn Asn Gly Gly Arg Pro Ser Ser Ser Tyr Gly
            195             200             205

```
Ala Pro Gly Gly Gly Asn Gly Gly Arg Pro Ser Asp Thr Tyr Gly Ala
    210             215             220

Pro Gly Gly Gly Asn Gly Asn Gly Ser Gly Gly Arg Pro Ser Ser Ser
225             230             235                 240

Tyr Gly Ala Pro Gly Gln Gly Gln Gly Gly Phe Gly Gly Arg Pro Ser
            245             250                 255

Asp Ser Tyr Gly Ala Pro Gly Gln Asn Gln Lys Pro Ser Asp Ser Tyr
            260             265             270

Gly Ala Pro Gly Ser Gly Asn Gly Asn Gly Gly Arg Pro Ser Ser Ser
        275             280             285

Tyr Gly Ala Pro Gly Ser Gly Pro Gly Gly Arg Pro Ser Asp Ser Tyr
    290             295             300

Gly Pro Pro Ala Ser Gly
305             310
```

```
<210>   44
<211>   282
<212>   PRT
<213>   Artificial Sequence

<220>
<223>   elastin short

<400>   44
```

```
Met His His His His His His Ser Ser Gly Ser Ser Leu Gly Val Ser
1               5               10              15

Ala Gly Ala Val Val Pro Gln Pro Gly Ala Gly Val Lys Pro Gly Lys
            20              25              30

Val Pro Gly Val Gly Leu Pro Gly Val Tyr Pro Gly Gly Val Leu Pro
        35              40              45

Gly Ala Arg Phe Pro Gly Val Gly Val Leu Pro Gly Val Pro Thr Gly
    50              55              60

Ala Gly Val Lys Pro Lys Ala Pro Gly Val Gly Gly Ala Phe Ala Gly
65              70              75              80

Ile Pro Gly Val Gly Pro Phe Gly Gly Pro Gln Pro Gly Val Pro Leu
            85              90              95
```

```
Gly Tyr Pro Ile Lys Ala Pro Lys Leu Pro Gly Gly Tyr Gly Leu Pro
            100             105             110


Tyr Thr Thr Gly Lys Leu Pro Tyr Gly Tyr Gly Pro Gly Gly Val Ala
            115             120             125


Gly Ala Ala Gly Lys Ala Gly Tyr Pro Thr Gly Thr Gly Val Gly Pro
    130             135             140


Gln Ala Ala Ala Ala Ala Ala Ala Lys Ala Ala Ala Lys Phe Gly Ala
145             150             155             160


Gly Ala Ala Gly Val Leu Pro Gly Val Gly Gly Ala Gly Val Pro Gly
                165             170             175


Val Pro Gly Ala Ile Pro Gly Ile Gly Gly Ile Ala Gly Val Gly Thr
            180             185             190


Pro Ala Ala Ala Ala Ala Ala Ala Ala Ala Lys Ala Ala Lys Tyr
            195             200             205


Gly Ala Ala Ala Gly Leu Val Pro Gly Gly Pro Gly Phe Gly Pro Gly
    210             215             220


Val Val Gly Val Pro Gly Ala Gly Val Pro Gly Val Gly Val Pro Gly
225             230             235             240


Ala Gly Ile Pro Val Val Pro Gly Ala Gly Ile Pro Gly Ala Ala Val
            245             250             255


Pro Gly Val Val Ser Pro Glu Ala Ala Ala Lys Ala Ala Ala Lys Ala
            260             265             270


Ala Lys Tyr Gly Ala Arg Pro Gly Val Gly
    275             280
```

```
<210>   45
<211>   487
<212>   PRT
<213>   Artificial Sequence

<220>
<223>   PRT798

<400>   45

Met His His His His His His Ser Ser Gly Ser Ser Leu Glu Val Leu
1               5               10              15
```

Phe Gln Gly Pro Ser Phe Arg Leu Ser Gly Val Ser Arg Arg Leu Cys
20 25 30

Ser Gln Ala Gly Thr Gly Arg Leu Thr Gly Gly Arg Thr Gly Phe Arg
35 40 45

Ala Gly Asn Val Cys Ser Gly Leu Gly Ala Gly Ser Ser Phe Ser Gly
50 55 60

Pro Leu Gly Ser Val Ser Ser Lys Gly Ser Phe Ser His Gly Gly Gly
65 70 75 80

Gly Leu Gly Ser Gly Val Cys Thr Gly Phe Leu Glu Asn Glu His Gly
85 90 95

Leu Leu Pro Gly Asn Glu Lys Val Thr Leu Gln Asn Leu Asn Asp Arg
100 105 110

Leu Ala Ser Tyr Leu Asp His Val Cys Thr Leu Glu Glu Ala Asn Ala
115 120 125

Asp Leu Glu Gln Lys Ile Lys Gly Trp Tyr Glu Lys Tyr Gly Pro Gly
130 135 140

Ser Gly Arg Gln Leu Ala His Asp Tyr Ser Lys Tyr Phe Ser Val Thr
145 150 155 160

Glu Asp Leu Lys Arg Gln Ile Ile Ser Val Thr Thr Cys Asn Ala Ser
165 170 175

Ile Val Leu Gln Asn Glu Asn Ala Arg Leu Thr Ala Asp Asp Phe Arg
180 185 190

Leu Lys Cys Glu Asn Glu Leu Ala Leu His Gln Ser Val Glu Ala Asp
195 200 205

Ile Asn Gly Leu His Arg Val Met Asp Glu Leu Thr Leu Cys Thr Ser
210 215 220

Asp Leu Glu Met Gln Cys Glu Ala Leu Ser Glu Glu Leu Thr Tyr Leu
225 230 235 240

Lys Lys Asn His Gln Glu Glu Met Lys Val Met Gln Gly Ala Ala Arg
245 250 255

Gly Asn Val Asn Val Glu Ile Asn Ala Ala Pro Gly Val Asp Leu Thr
260 265 270

```
Val Leu Leu Asn Asn Met Arg Ala Glu Tyr Glu Asp Leu Ala Glu Gln
        275                 280             285

Asn His Glu Asp Ala Glu Ala Trp Phe Ser Glu Lys Ser Thr Ser Leu
        290                 295             300

His Gln Gln Ile Ser Asp Asp Ala Gly Ala Ala Met Ala Ala Arg Asn
305                 310                 315                 320

Glu Leu Met Glu Leu Lys Arg Asn Leu Gln Thr Leu Glu Ile Glu Leu
                325                 330                 335

Gln Ser Leu Leu Ala Met Lys His Ser Tyr Glu Cys Ser Leu Ala Glu
                340                 345                 350

Thr Glu Ser Asn Tyr Cys His Gln Leu Gln Gln Ile Gln Glu Gln Ile
            355                 360                 365

Gly Ala Met Glu Asp Gln Leu Gln Gln Ile Arg Met Glu Thr Glu Gly
    370                 375                 380

Gln Lys Leu Glu His Glu Arg Leu Leu Asp Val Lys Ile Phe Leu Glu
385                 390                 395                 400

Lys Glu Ile Glu Met Tyr Cys Lys Leu Ile Asp Gly Glu Gly Arg Lys
                405                 410                 415

Ser Lys Ser Thr Cys Tyr Lys Ser Glu Gly Arg Gly Pro Lys Asn Ser
            420                 425                 430

Glu Asn Gln Val Lys Asp Ser Lys Glu Glu Ala Val Val Lys Thr Val
        435                 440                 445

Val Gly Glu Leu Asp Gln Leu Gly Ser Val Leu Ser Leu Arg Val His
    450                 455                 460

Ser Val Glu Glu Lys Ser Ser Lys Ile Ser Asn Ile Thr Met Glu Gln
465                 470                 475                 480

Arg Leu Pro Ser Lys Val Pro
                485

<210>   46
<211>   1179
<212>   PRT
<213>   Artificial Sequence

<220>
```

<223>  Met-PRT966

<400>  46

Met Gly Pro Gly Val Phe Gly Pro Tyr Gly Pro Gly Ala Ser Ala Ala
1               5                   10                  15

Ala Ala Ala Gly Ile Asn Gly Pro Gly Ser Gly Val Phe Gly Pro Gly
            20                  25                  30

Ile Ser Gly Ile Tyr Gly Pro Gly Val Phe Gly Pro Gly Val Phe Gly
            35                  40                  45

Pro Gly Ser Ser Ala Ala Ala Ala Ala Gly Pro Gly Ile Tyr Gly Pro
        50                  55                  60

Gly Val Phe Gly Pro Ser Ala Ser Ala Ala Ala Ala Gly Pro Gly
65                  70                  75                  80

Ser Gly Val Phe Gly Pro Gly Ala Ser Gly Ile Tyr Gly Pro Gly Val
                85                  90                  95

Phe Gly Pro Gly Val Phe Gly Pro Gly Ser Ser Ala Ala Ala Ala Ala
            100                 105                 110

Gly Ile Tyr Gly Ser Gly Pro Gly Val Phe Gly Pro Tyr Gly Ser Ala
        115                 120                 125

Ala Ala Ala Ala Gly Pro Gly Ser Gly Ile Tyr Gly Ile Gly Pro Tyr
        130                 135                 140

Gly Pro Gly Ala Ser Gly Pro Gly Ile Tyr Gly Pro Gly Val Phe Gly
145                 150                 155                 160

Pro Ser Ala Ser Ala Ala Ala Ala Ala Gly Ser Gly Val Phe Gly Pro
            165                 170                 175

Gly Ile Tyr Gly Pro Tyr Ala Ser Ala Ala Ala Ala Gly Ile Tyr
        180                 185                 190

Gly Ser Gly Pro Gly Val Phe Gly Pro Tyr Gly Pro Gly Ile Ser Gly
        195                 200                 205

Ser Gly Val Phe Gly Pro Gly Val Phe Gly Pro Tyr Ala Ser Ala Ala
        210                 215                 220

Ala Ala Ala Gly Pro Gly Val Phe Gly Pro Tyr Gly Pro Gly Ser Ser
225                 230                 235                 240

Ala Ala Ala Ala Ala Gly Ile Tyr Gly Tyr Gly Pro Gly Val Phe Gly
            245         250                 255

Pro Tyr Gly Pro Gly Ala Ser Gly Ile Asn Gly Pro Gly Ser Gly Ile
            260                 265                 270

Tyr Gly Pro Gly Val Phe Gly Pro Gly Ile Ser Ala Ala Ala Ala Ala
            275                 280                 285

Gly Pro Gly Val Phe Gly Pro Tyr Gly Pro Gly Ala Ser Ala Ala Ala
            290                 295                 300

Ala Ala Gly Ile Tyr Gly Pro Gly Val Phe Gly Pro Gly Ile Tyr Gly
305                 310                 315                 320

Pro Gly Ser Ser Gly Pro Gly Val Phe Gly Pro Tyr Gly Pro Gly Ser
                325                 330                 335

Ser Ala Ala Ala Ala Ala Gly Ile Tyr Gly Pro Gly Val Phe Gly Pro
            340                 345                 350

Tyr Gly Pro Gly Ile Ser Ala Ala Ala Ala Ala Gly Ile Tyr Val Phe
            355                 360                 365

Gly Pro Gly Val Phe Gly Pro Tyr Gly Pro Gly Ala Ser Gly Pro Gly
            370                 375                 380

Val Phe Gly Pro Tyr Gly Pro Gly Ala Ser Ala Ala Ala Ala Ala Gly
385                 390                 395                 400

Pro Gly Ile Tyr Gly Pro Gly Val Phe Gly Pro Ser Ala Ser Ala Ala
            405                 410                 415

Ala Ala Ala Gly Ile Tyr Gly Ser Gly Pro Gly Ile Tyr Gly Pro Tyr
            420                 425                 430

Gly Pro Gly Ile Ser Gly Pro Gly Ser Gly Val Phe Gly Ile Gly Pro
            435                 440                 445

Tyr Gly Pro Gly Ala Ser Ala Ala Ala Ala Gly Ile Tyr Gly Pro
            450                 455                 460

Gly Val Phe Gly Pro Tyr Gly Pro Gly Ile Ser Ala Ala Ala Ala Ala
465                 470                 475                 480

Gly Pro Gly Ser Gly Ile Tyr Gly Pro Gly Ala Ser Gly Ile Asn Gly
            485                 490                 495

Pro Gly Ser Gly Ile Tyr Gly Pro Gly Val Phe Gly Pro Gly Ile Ser
            500                 505                 510

Ala Ala Ala Ala Ala Gly Ile Tyr Val Phe Gly Pro Gly Val Phe Gly
            515                 520                 525

Pro Tyr Gly Pro Gly Ala Ser Ala Ala Ala Ala Gly Ile Tyr Gly
    530                 535                 540

Ser Gly Pro Gly Val Phe Gly Pro Tyr Gly Pro Gly Ile Ser Gly Ser
545                 550                 555                 560

Gly Val Phe Gly Pro Gly Val Phe Gly Pro Tyr Ala Ser Ala Ala Ala
                565                 570                 575

Ala Ala Gly Pro Gly Ser Gly Val Phe Gly Pro Gly Ala Ser Gly Pro
            580                 585                 590

Gly Val Phe Gly Pro Tyr Gly Pro Gly Ala Ser Ala Ala Ala Ala Ala
        595                 600                 605

Gly Ile Asn Gly Pro Gly Ser Gly Val Phe Gly Pro Gly Ile Ser Gly
    610                 615                 620

Ile Tyr Gly Pro Gly Val Phe Gly Pro Gly Val Phe Gly Pro Gly Ser
625                 630                 635                 640

Ser Ala Ala Ala Ala Ala Gly Pro Gly Ile Tyr Gly Pro Gly Val Phe
            645                 650                 655

Gly Pro Ser Ala Ser Ala Ala Ala Ala Ala Gly Pro Gly Ser Gly Val
            660                 665                 670

Phe Gly Pro Gly Ala Ser Gly Ile Tyr Gly Pro Gly Val Phe Gly Pro
        675                 680                 685

Gly Val Phe Gly Pro Gly Ser Ser Ala Ala Ala Ala Gly Ile Tyr
    690                 695                 700

Gly Ser Gly Pro Gly Val Phe Gly Pro Tyr Gly Ser Ala Ala Ala Ala
705                 710                 715                 720

Ala Gly Pro Gly Ser Gly Ile Tyr Gly Ile Gly Pro Tyr Gly Pro Gly
                725                 730                 735

Ala Ser Gly Pro Gly Ile Tyr Gly Pro Gly Val Phe Gly Pro Ser Ala

740 745 750

Ser Ala Ala Ala Ala Ala Gly Ser Gly Val Phe Gly Pro Gly Ile Tyr
    755             760             765

Gly Pro Tyr Ala Ser Ala Ala Ala Ala Ala Gly Ile Tyr Gly Ser Gly
    770             775             780

Pro Gly Val Phe Gly Pro Tyr Gly Pro Gly Ile Ser Gly Ser Gly Val
785             790             795             800

Phe Gly Pro Gly Val Phe Gly Pro Tyr Ala Ser Ala Ala Ala Ala Ala
            805             810             815

Gly Pro Gly Val Phe Gly Pro Tyr Gly Pro Gly Ser Ser Ala Ala Ala
            820             825             830

Ala Ala Gly Ile Tyr Gly Tyr Gly Pro Gly Val Phe Gly Pro Tyr Gly
        835             840             845

Pro Gly Ala Ser Gly Ile Asn Gly Pro Gly Ser Gly Ile Tyr Gly Pro
    850             855             860

Gly Val Phe Gly Pro Gly Ile Ser Ala Ala Ala Ala Ala Gly Pro Gly
865             870             875             880

Val Phe Gly Pro Tyr Gly Pro Gly Ala Ser Ala Ala Ala Ala Ala Gly
            885             890             895

Ile Tyr Gly Pro Gly Val Phe Gly Pro Gly Ile Tyr Gly Pro Gly Ser
        900             905             910

Ser Gly Pro Gly Val Phe Gly Pro Tyr Gly Pro Gly Ser Ser Ala Ala
    915             920             925

Ala Ala Ala Gly Ile Tyr Gly Pro Gly Val Phe Gly Pro Tyr Gly Pro
    930             935             940

Gly Ile Ser Ala Ala Ala Ala Gly Ile Tyr Val Phe Gly Pro Gly
945             950             955             960

Val Phe Gly Pro Tyr Gly Pro Gly Ala Ser Gly Pro Gly Val Phe Gly
            965             970             975

Pro Tyr Gly Pro Gly Ala Ser Ala Ala Ala Ala Ala Gly Pro Gly Ile
    980             985             990

```
Tyr Gly Pro Gly Val Phe Gly Pro  Ser Ala Ser Ala Ala  Ala Ala Ala
        995                 1000              1005

Gly Ile  Tyr Gly Ser Gly Pro  Gly Ile Tyr Gly Pro  Tyr Gly Pro
    1010              1015              1020

Gly Ile  Ser Gly Pro Gly Ser  Gly Val Phe Gly Ile  Gly Pro Tyr
    1025              1030              1035

Gly Pro  Gly Ala Ser Ala Ala  Ala Ala Ala Gly Ile  Tyr Gly Pro
    1040              1045              1050

Gly Val  Phe Gly Pro Tyr Gly  Pro Gly Ile Ser Ala  Ala Ala Ala
    1055              1060              1065

Ala Gly  Pro Gly Ser Gly Ile  Tyr Gly Pro Gly Ala  Ser Gly Ile
    1070              1075              1080

Asn Gly  Pro Gly Ser Gly Ile  Tyr Gly Pro Gly Val  Phe Gly Pro
    1085              1090              1095

Gly Ile  Ser Ala Ala Ala Ala  Ala Gly Ile Tyr Val  Phe Gly Pro
    1100              1105              1110

Gly Val  Phe Gly Pro Tyr Gly  Pro Gly Ala Ser Ala  Ala Ala Ala
    1115              1120              1125

Ala Gly  Ile Tyr Gly Ser Gly  Pro Gly Val Phe Gly  Pro Tyr Gly
    1130              1135              1140

Pro Gly  Ile Ser Gly Ser Gly  Val Phe Gly Pro Gly  Val Phe Gly
    1145              1150              1155

Pro Tyr  Ala Ser Ala Ala Ala  Ala Ala Gly Pro Gly  Ser Gly Val
    1160              1165              1170

Phe Gly  Pro Gly Ala Ser
    1175
```

<210> 47
<211> 1190
<212> PRT
<213> Artificial Sequence

<220>
<223> PRT966

<400> 47

```
Met His His His His His His Ser Ser Gly Ser Ser Gly Pro Gly Val
```

```
       1                 5                      10                         15

       Phe Gly Pro Tyr Gly Pro Gly Ala Ser Ala Ala Ala Ala Ala Gly Ile
                   20                  25                  30

       Asn Gly Pro Gly Ser Gly Val Phe Gly Pro Gly Ile Ser Gly Ile Tyr
                   35                  40                  45

       Gly Pro Gly Val Phe Gly Pro Gly Val Phe Gly Pro Gly Ser Ser Ala
                   50                  55                  60

       Ala Ala Ala Ala Gly Pro Gly Ile Tyr Gly Pro Gly Val Phe Gly Pro
       65                  70                  75                  80

       Ser Ala Ser Ala Ala Ala Ala Ala Gly Pro Gly Ser Gly Val Phe Gly
                       85                  90                  95

       Pro Gly Ala Ser Gly Ile Tyr Gly Pro Gly Val Phe Gly Pro Gly Val
                   100                 105                 110

       Phe Gly Pro Gly Ser Ser Ala Ala Ala Ala Ala Gly Ile Tyr Gly Ser
                   115                 120                 125

       Gly Pro Gly Val Phe Gly Pro Tyr Gly Ser Ala Ala Ala Ala Ala Gly
                   130                 135                 140

       Pro Gly Ser Gly Ile Tyr Gly Ile Gly Pro Tyr Gly Pro Gly Ala Ser
       145                 150                 155                 160

       Gly Pro Gly Ile Tyr Gly Pro Gly Val Phe Gly Pro Ser Ala Ser Ala
                   165                 170                 175

       Ala Ala Ala Ala Gly Ser Gly Val Phe Gly Pro Gly Ile Tyr Gly Pro
                   180                 185                 190

       Tyr Ala Ser Ala Ala Ala Ala Ala Gly Ile Tyr Gly Ser Gly Pro Gly
                   195                 200                 205

       Val Phe Gly Pro Tyr Gly Pro Gly Ile Ser Gly Ser Gly Val Phe Gly
                   210                 215                 220

       Pro Gly Val Phe Gly Pro Tyr Ala Ser Ala Ala Ala Ala Ala Gly Pro
       225                 230                 235                 240

       Gly Val Phe Gly Pro Tyr Gly Pro Gly Ser Ser Ala Ala Ala Ala Ala
                   245                 250                 255
```

Gly Ile Tyr Gly Tyr Gly Pro Gly Val Phe Gly Pro Tyr Gly Pro Gly
                    260                 265                 270

Ala Ser Gly Ile Asn Gly Pro Gly Ser Gly Ile Tyr Gly Pro Gly Val
                275                 280                 285

Phe Gly Pro Gly Ile Ser Ala Ala Ala Ala Gly Pro Gly Val Phe
    290                 295                 300

Gly Pro Tyr Gly Pro Gly Ala Ser Ala Ala Ala Ala Gly Ile Tyr
305                 310                 315                 320

Gly Pro Gly Val Phe Gly Pro Gly Ile Tyr Gly Pro Gly Ser Ser Gly
                325                 330                 335

Pro Gly Val Phe Gly Pro Tyr Gly Pro Gly Ser Ser Ala Ala Ala Ala
                340                 345                 350

Ala Gly Ile Tyr Gly Pro Gly Val Phe Gly Pro Tyr Gly Pro Gly Ile
    355                 360                 365

Ser Ala Ala Ala Ala Ala Gly Ile Tyr Val Phe Gly Pro Gly Val Phe
    370                 375                 380

Gly Pro Tyr Gly Pro Gly Ala Ser Gly Pro Gly Val Phe Gly Pro Tyr
385                 390                 395                 400

Gly Pro Gly Ala Ser Ala Ala Ala Ala Ala Gly Pro Gly Ile Tyr Gly
                405                 410                 415

Pro Gly Val Phe Gly Pro Ser Ala Ser Ala Ala Ala Ala Gly Ile
                420                 425                 430

Tyr Gly Ser Gly Pro Gly Ile Tyr Gly Pro Tyr Gly Pro Gly Ile Ser
    435                 440                 445

Gly Pro Gly Ser Gly Val Phe Gly Ile Gly Pro Tyr Gly Pro Gly Ala
    450                 455                 460

Ser Ala Ala Ala Ala Ala Gly Ile Tyr Gly Pro Gly Val Phe Gly Pro
465                 470                 475                 480

Tyr Gly Pro Gly Ile Ser Ala Ala Ala Ala Gly Pro Gly Ser Gly
                485                 490                 495

Ile Tyr Gly Pro Gly Ala Ser Gly Ile Asn Gly Pro Gly Ser Gly Ile
            500                 505                 510

Tyr Gly Pro Gly Val Phe Gly Pro Gly Ile Ser Ala Ala Ala Ala Ala
        515                 520                 525

Gly Ile Tyr Val Phe Gly Pro Gly Val Phe Gly Pro Tyr Gly Pro Gly
        530                 535                 540

Ala Ser Ala Ala Ala Ala Ala Gly Ile Tyr Gly Ser Gly Pro Gly Val
545                 550                 555                 560

Phe Gly Pro Tyr Gly Pro Gly Ile Ser Gly Ser Gly Val Phe Gly Pro
                565                 570                 575

Gly Val Phe Gly Pro Tyr Ala Ser Ala Ala Ala Ala Ala Gly Pro Gly
                580                 585                 590

Ser Gly Val Phe Gly Pro Gly Ala Ser Gly Pro Gly Val Phe Gly Pro
                595                 600                 605

Tyr Gly Pro Gly Ala Ser Ala Ala Ala Ala Gly Ile Asn Gly Pro
        610                 615                 620

Gly Ser Gly Val Phe Gly Pro Gly Ile Ser Gly Ile Tyr Gly Pro Gly
625                 630                 635                 640

Val Phe Gly Pro Gly Val Phe Gly Pro Gly Ser Ser Ala Ala Ala Ala
                645                 650                 655

Ala Gly Pro Gly Ile Tyr Gly Pro Gly Val Phe Gly Pro Ser Ala Ser
                660                 665                 670

Ala Ala Ala Ala Ala Gly Pro Gly Ser Gly Val Phe Gly Pro Gly Ala
        675                 680                 685

Ser Gly Ile Tyr Gly Pro Gly Val Phe Gly Pro Gly Val Phe Gly Pro
        690                 695                 700

Gly Ser Ser Ala Ala Ala Ala Gly Ile Tyr Gly Ser Gly Pro Gly
705                 710                 715                 720

Val Phe Gly Pro Tyr Gly Ser Ala Ala Ala Ala Gly Pro Gly Ser
                725                 730                 735

Gly Ile Tyr Gly Ile Gly Pro Tyr Gly Pro Gly Ala Ser Gly Pro Gly
        740                 745                 750

Ile Tyr Gly Pro Gly Val Phe Gly Pro Ser Ala Ser Ala Ala Ala Ala
        755                 760                 765

```
Ala Gly Ser Gly Val Phe Gly Pro Gly Ile Tyr Gly Pro Tyr Ala Ser
    770                 775                 780

Ala Ala Ala Ala Ala Gly Ile Tyr Gly Ser Gly Pro Gly Val Phe Gly
785                 790                 795                 800

Pro Tyr Gly Pro Gly Ile Ser Gly Ser Gly Val Phe Gly Pro Gly Val
            805                 810                 815

Phe Gly Pro Tyr Ala Ser Ala Ala Ala Ala Gly Pro Gly Val Phe
        820                 825                 830

Gly Pro Tyr Gly Pro Gly Ser Ser Ala Ala Ala Ala Gly Ile Tyr
        835                 840                 845

Gly Tyr Gly Pro Gly Val Phe Gly Pro Tyr Gly Pro Gly Ala Ser Gly
    850                 855                 860

Ile Asn Gly Pro Gly Ser Gly Ile Tyr Gly Pro Gly Val Phe Gly Pro
865                 870                 875                 880

Gly Ile Ser Ala Ala Ala Ala Gly Pro Gly Val Phe Gly Pro Tyr
            885                 890                 895

Gly Pro Gly Ala Ser Ala Ala Ala Ala Gly Ile Tyr Gly Pro Gly
            900                 905                 910

Val Phe Gly Pro Gly Ile Tyr Gly Pro Gly Ser Ser Gly Pro Gly Val
        915                 920                 925

Phe Gly Pro Tyr Gly Pro Gly Ser Ser Ala Ala Ala Ala Gly Ile
    930                 935                 940

Tyr Gly Pro Gly Val Phe Gly Pro Tyr Gly Pro Gly Ile Ser Ala Ala
945                 950                 955                 960

Ala Ala Ala Gly Ile Tyr Val Phe Gly Pro Gly Val Phe Gly Pro Tyr
            965                 970                 975

Gly Pro Gly Ala Ser Gly Pro Gly Val Phe Gly Pro Tyr Gly Pro Gly
            980                 985                 990

Ala Ser Ala Ala Ala Ala Ala Gly  Pro Gly Ile Tyr Gly  Pro Gly Val
        995                 1000                1005

Phe Gly  Pro Ser Ala Ser Ala  Ala Ala Ala Ala Gly  Ile Tyr Gly
```

```
           1010                    1015                    1020

           Ser Gly  Pro Gly Ile Tyr Gly  Pro Tyr Gly Pro Gly  Ile Ser Gly
               1025                    1030                    1035

           Pro Gly  Ser Gly Val Phe Gly  Ile Gly Pro Tyr Gly  Pro Gly Ala
               1040                    1045                    1050

           Ser Ala  Ala Ala Ala Ala Gly  Ile Tyr Gly Pro Gly  Val Phe Gly
               1055                    1060                    1065

           Pro Tyr  Gly Pro Gly Ile Ser  Ala Ala Ala Ala Ala  Gly Pro Gly
               1070                    1075                    1080

           Ser Gly  Ile Tyr Gly Pro Gly  Ala Ser Gly Ile Asn  Gly Pro Gly
               1085                    1090                    1095

           Ser Gly  Ile Tyr Gly Pro Gly  Val Phe Gly Pro Gly  Ile Ser Ala
               1100                    1105                    1110

           Ala Ala  Ala Ala Gly Ile Tyr  Val Phe Gly Pro Gly  Val Phe Gly
               1115                    1120                    1125

           Pro Tyr  Gly Pro Gly Ala Ser  Ala Ala Ala Ala Ala  Gly Ile Tyr
               1130                    1135                    1140

           Gly Ser  Gly Pro Gly Val Phe  Gly Pro Tyr Gly Pro  Gly Ile Ser
               1145                    1150                    1155

           Gly Ser  Gly Val Phe Gly Pro  Gly Val Phe Gly Pro  Tyr Ala Ser
               1160                    1165                    1170

           Ala Ala  Ala Ala Ala Gly Pro  Gly Ser Gly Val Phe  Gly Pro Gly
               1175                    1180                    1185

           Ala Ser
               1190
```

## Claims

1. A side-by-side type composite fiber having a latent crimping ability, comprising:

   a first component containing a modified fibroin; and
   a second component containing a structural protein,
   wherein the first component and the second component are joined to each other.

2. The composite fiber according to claim 1, wherein a composition ratio of the first component to the second component is 9:1 to 1:9 based on a mass of the composite fiber.

3. The composite fiber according to claim 1 or 2, wherein the structural protein is at least one selected from the group consisting of a silk fibroin, a spider silk fibroin, collagen, resilin, elastin, and keratin.

4. The composite fiber according to any one of claims 1 to 3, wherein the structural protein is at least one selected from the group consisting of a silk fibroin, a spider silk fibroin, and keratin.

5. The composite fiber according to any one of claims 1 to 4, wherein the modified fibroin is a modified fibroin including a domain sequence represented by Formula 1: $[(A)_n \text{ motif - REP}]_m$, and
the domain sequence has an amino acid sequence in which a content of the $(A)_n$ motif is reduced, the amino acid sequence being equivalent to an amino acid sequence in which at least one or a plurality of the $(A)_n$ motifs are deleted, as compared with a naturally occurring fibroin,
in Formula 1, the $(A)_n$ motif represents an amino acid sequence composed of 2 to 27 amino acid residues and the number of alanine residues with respect to a total number of amino acid residues in the $(A)_n$ motif is 83% or more, the REP represents an amino acid sequence composed of 10 to 200 amino acid residues, m represents an integer of 2 to 300, a plurality of the $(A)_n$ motifs may have the same amino acid sequence or amino acid sequences different from each other, and a plurality of the REPs may have the same amino acid sequence or amino acid sequences different from each other.

6. The composite fiber according to any one of claims 1 to 4, wherein the modified fibroin includes a domain sequence represented by Formula 1: $[(A)_n \text{ motif - REP}]_m$, and
the domain sequence has an amino acid sequence in which a content of a glycine residue is reduced, the amino acid sequence being equivalent to an amino acid sequence in which at least one or a plurality of the glycine residues in the REP are substituted with other amino acid residues, as compared with a naturally occurring fibroin,
in Formula 1, the $(A)_n$ motif represents an amino acid sequence composed of 2 to 27 amino acid residues and the number of alanine residues with respect to a total number of amino acid residues in the $(A)_n$ motif is 83% or more, the REP represents an amino acid sequence composed of 10 to 200 amino acid residues, m represents an integer of 2 to 300, a plurality of the $(A)_n$ motifs may have the same amino acid sequence or amino acid sequences different from each other, and a plurality of the REPs may have the same amino acid sequence or amino acid sequences different from each other.

7. The composite fiber according to any one of claims 1 to 4, wherein the modified fibroin includes a domain sequence represented by Formula 1: $[(A)_n \text{ motif - REP}]_m$, and
the domain sequence has an amino acid sequence locally including a region having a high hydropathy index, the amino acid sequence being equivalent to an amino acid sequence in which one or a plurality of amino acid residues in the REP are substituted with amino acid residues having a high hydropathy index and/or one or a plurality of amino acid residues having a high hydropathy index are inserted into the REP, as compared with a naturally occurring fibroin,
[in Formula 1, the $(A)_n$ motif represents an amino acid sequence composed of 2 to 27 amino acid residues and the number of alanine residues with respect to a total number of amino acid residues in the $(A)_n$ motif is 83% or more, the REP represents an amino acid sequence composed of 10 to 200 amino acid residues, m represents an integer of 2 to 300, a plurality of the $(A)_n$ motifs may have the same amino acid sequence or amino acid sequences different from each other, and a plurality of the REPs may have the same amino acid sequence or amino acid sequences different from each other].

8. The composite fiber according to any one of claims 1 to 4, wherein the modified fibroin includes a domain sequence represented by Formula 1: $[(A)_n \text{ motif - REP}]_m$ or Formula 2: $[(A)_n \text{ motif - REP}]_m - (A)_n$ motif, and
the domain sequence has an amino acid sequence in which a content of a glutamine residue is reduced, the amino acid sequence being equivalent to an amino acid sequence in which one or a plurality of the glutamine residues in the REP are deleted or substituted with other amino acid residues, as compared with a naturally occurring fibroin,
in Formula 1 and Formula 2, the $(A)_n$ motif represents an amino acid sequence composed of 2 to 27 amino acid residues and the number of alanine residues with respect to a total number of amino acid residues in the $(A)_n$ motif is 80% or more, the REP represents an amino acid sequence composed of 10 to 200 amino acid residues, m represents an integer of 2 to 300, a plurality of the $(A)_n$ motifs may have the same amino acid sequence or amino acid sequences different from each other, and a plurality of the REPs may have the same amino acid sequence or amino acid sequences different from each other.

9. The composite fiber according to any one of claims 1 to 4, wherein the modified fibroin has a limiting oxygen index (LOI) value of 26.0 or more.

**10.** The composite fiber according to any one of claims 1 to 4, wherein the modified fibroin has a highest hygroscopic heat generation degree of more than 0.025 °C/g, the highest hygroscopic heat generation degree being determined according to Expression A,

$$\text{Expression A: highest hygroscopic heat generation degree} = $$
$$\{(\text{highest temperature of a sample when the sample has been transferred}$$
$$\text{to a high humidity environment after being placed in a low humidity}$$
$$\text{environment until a temperature of the sample reaches equilibrium}) - $$
$$(\text{temperature of the sample when the sample is being transferred to the}$$
$$\text{high humidity environment after being placed in the low humidity}$$
$$\text{environment until the temperature of the sample reaches equilibrium})\}$$
$$(^{\circ}\text{C})/\text{sample weight (g)},$$

in Expression A, the low humidity environment means an environment of a temperature of 20°C and a relative humidity of 40%, and the high humidity environment means an environment of a temperature of 20°C and a relative humidity of 90%.

**11.** A method for manufacturing a composite fiber, comprising:

preparing a first doping liquid containing a modified fibroin and a solvent;
preparing a second doping liquid containing a structural protein and a solvent;
discharging the first doping liquid and the second doping liquid from a spinneret and joining the first doping liquid and the second doping liquid to form an undrawn composite fiber in a coagulation liquid; and
bringing the undrawn composite fiber into contact with an aqueous medium.

**12.** The method according to claim 11, further comprising:
drawing the undrawn composite fiber.

**13.** The method according to claim 11 or 12, wherein a concentration of the modified fibroin in the first doping liquid is 5% to 40% by mass based on a total mass of the first doping liquid, and
a concentration of the structural protein in the second doping liquid is 5% to 40% by mass based on a total mass of the second doping liquid.

**14.** The method according to any one of claims 11 to 13, wherein the solvent contains at least one selected from the group consisting of hexafluoroisopropanol, hexafluoroacetone, dimethyl sulfoxide, N,N-dimethylformamide, N,N-dimethylacetamide, 1,3-dimethyl-2-imidazolidone, N-methyl-2-pyrrolidone, acetonitrile, N-methylmorpholine-N-oxide, formic acid, and an aqueous solution containing at least one selected from the group consisting of urea, guanidine, sodium dodecyl sulfate, lithium bromide, calcium chloride, and lithium thiocyanate.

**15.** The method according to any one of claims 11 to 14, wherein the coagulation liquid is at least one selected from the group consisting of a lower alcohol having 1 to 5 carbon atoms and acetone.

**16.** The method according to any one of claims 11 to 15, wherein the structural protein is at least one selected from the group consisting of a silk fibroin, a spider silk fibroin, collagen, resilin, elastin, and keratin.

**17.** The method according to any one of claims 11 to 16, wherein the structural protein is at least one selected from the group consisting of a silk fibroin, a spider silk fibroin, and keratin.

**18.** The method according to any one of claims 11 to 17, wherein the modified fibroin is a modified fibroin including a domain sequence represented by Formula 1: $[(A)_n$ motif - $REP]_m$, and

the domain sequence has an amino acid sequence in which a content of the $(A)_n$ motif is reduced, the amino acid sequence being equivalent to an amino acid sequence in which at least one or a plurality of the $(A)_n$ motifs are deleted, as compared with a naturally occurring fibroin,

in Formula 1, the $(A)_n$ motif represents an amino acid sequence composed of 2 to 27 amino acid residues and the number of alanine residues with respect to a total number of amino acid residues in the $(A)_n$ motif is 83% or more, the REP represents an amino acid sequence composed of 10 to 200 amino acid residues, m represents an integer of 2 to 300, a plurality of the $(A)_n$ motifs may have the same amino acid sequence or amino acid sequences different from each other, and a plurality of the REPs may have the same amino acid sequence or amino acid sequences different from each other.

19. The method according to any one of claims 11 to 17, wherein the modified fibroin includes a domain sequence represented by Formula 1: $[(A)_n$ motif - $REP]_m$, and

the domain sequence has an amino acid sequence in which a content of a glycine residue is reduced, the amino acid sequence being equivalent to an amino acid sequence in which at least one or a plurality of the glycine residues in the REP are substituted with other amino acid residues, as compared with a naturally occurring fibroin,

in Formula 1, the $(A)_n$ motif represents an amino acid sequence composed of 2 to 27 amino acid residues and the number of alanine residues with respect to a total number of amino acid residues in the $(A)_n$ motif is 83% or more, the REP represents an amino acid sequence composed of 10 to 200 amino acid residues, m represents an integer of 2 to 300, a plurality of the $(A)_n$ motifs may have the same amino acid sequence or amino acid sequences different from each other, and a plurality of the REPs may have the same amino acid sequence or amino acid sequences different from each other.

20. The method according to any one of claims 11 to 17, wherein the modified fibroin includes a domain sequence represented by Formula 1: $[(A)_n$ motif - $REP]_m$, and

the domain sequence has an amino acid sequence locally including a region having a high hydropathy index, the amino acid sequence being equivalent to an amino acid sequence in which one or a plurality of amino acid residues in the REP are substituted with amino acid residues having a high hydropathy index and/or one or a plurality of amino acid residues having a high hydropathy index are inserted into the REP, as compared with a naturally occurring fibroin.

in Formula 1, the $(A)_n$ motif represents an amino acid sequence composed of 2 to 27 amino acid residues and the number of alanine residues with respect to a total number of amino acid residues in the $(A)_n$ motif is 83% or more, the REP represents an amino acid sequence composed of 10 to 200 amino acid residues, m represents an integer of 2 to 300, a plurality of the $(A)_n$ motifs may have the same amino acid sequence or amino acid sequences different from each other, and a plurality of the REPs may have the same amino acid sequence or amino acid sequences different from each other.

21. The method according to any one of claims 11 to 17, wherein the modified fibroin includes a domain sequence represented by Formula 1: $[(A)_n$ motif - $REP]_m$ or Formula 2: $[(A)_n$ motif - $REP]_m$ - $(A)_n$ motif, and

the domain sequence has an amino acid sequence in which a content of a glutamine residue is reduced, the amino acid sequence being equivalent to an amino acid sequence in which one or a plurality of the glutamine residues in the REP are deleted or substituted with other amino acid residues, as compared with a naturally occurring fibroin, in Formula 1 and Formula 2, the $(A)_n$ motif represents an amino acid sequence composed of 2 to 27 amino acid residues and the number of alanine residues with respect to a total number of amino acid residues in the $(A)_n$ motif is 80% or more, the REP represents an amino acid sequence composed of 10 to 200 amino acid residues, m represents an integer of 2 to 300, a plurality of the $(A)_n$ motifs may have the same amino acid sequence or amino acid sequences different from each other, and a plurality of the REPs may have the same amino acid sequence or amino acid sequences different from each other.

22. The method according to any one of claims 11 to 17, wherein the modified fibroin has a limiting oxygen index (LOI) value of 26.0 or more.

23. The method according to any one of claims 11 to 17, wherein the modified fibroin has a highest hygroscopic heat generation degree of more than 0.025 °C/g, the highest hygroscopic heat generation degree being determined according to Expression A,

Expression A: highest hygroscopic heat generation degree = {(highest temperature of a sample when the sample has been transferred to a high humidity environment after being placed in a low humidity environment until a temperature of the sample reaches equilibrium) - (temperature of the sample when the sample is being transferred to the high humidity environment after being placed in the low humidity environment until the temperature of the sample reaches equilibrium)} (°C)/sample weight (g),

in Expression A, the low humidity environment means an environment of a temperature of 20°C and a relative humidity of 40%, and the high humidity environment means an environment of a temperature of 20°C and a relative humidity of 90%.

24. A product comprising:

the composite fiber according to any one of claims 1 to 10,
wherein the product is one selected from the group consisting of fiber, yarn, fabric, knit, braid, non-woven fabric, paper, and cotton.

25. A side-by-side type composite fiber comprising:

a first component; and
a second component,
wherein the first component and the second component are joined to each other, and one of the first component and the second component contains a modified fibroin.

26. A side-by-side type composite fiber comprising:

a first component; and
a second component,
wherein the first component and the second component are joined to each other, and one of the first component and the second component contains a modified fibroin having a latent crimping ability.

Fig.1

EP 3 779 000 A1

## *Fig.2*

EP 3 779 000 A1

Fig.3

EP 3 779 000 A1

# Fig.4

# Fig.5

EP 3 779 000 A1

# Fig.6

*Fig.7*

(a)

50

51            52

(b)

50

51            52

EP 3 779 000 A1

Fig.8

## INTERNATIONAL SEARCH REPORT

| International application No. |
|---|
| PCT/JP2019/014867 |

**A. CLASSIFICATION OF SUBJECT MATTER**
Int.Cl. D01F8/02(2006.01)i, C07K14/435(2006.01)i, D01F4/02(2006.01)i

According to International Patent Classification (IPC) or to both national classification and IPC

**B. FIELDS SEARCHED**

Minimum documentation searched (classification system followed by classification symbols)
Int.Cl. D01F8/02, C07K14/435, D01F4/02

| Documentation searched other than minimum documentation to the extent that such documents are included in the fields searched | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)
CAplus/ BIOSIS/WPIDS (STN)

**C. DOCUMENTS CONSIDERED TO BE RELEVANT**

| Category* | Citation of document, with indication, where appropriate, of the relevant passages | Relevant to claim No. |
|---|---|---|
| X<br>Y<br>A | PENG, Ling. et al., "Two-in-One Composite Fibers With Side-by-Side Arrangement of Silk Fibroin and Poly(L-lactide) by Electrospinning", Macromol.Mater.Eng., 2016, 301, 48-55, abstract | 1, 2, 24-26<br>5, 6<br>3, 4, 7-23 |
| Y<br>A | WO 2017/188434 A1 (SPIBER INC.) 02 November 2017, claims 1, 11 & EP 3450452 A1 (claims 1, 11) & CA 3012821 A & AU 2017257942 A & CN 109071619 A & KR 10-2019-0003583 A | 5, 6<br>1-4, 7-26 |
| P, A | WO 2019/022163 A1 (SPIBER INC.) 31 January 2019, entire text (Family: none) | 1-26 |

☐ Further documents are listed in the continuation of Box C.   ☐ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 10 June 2019 (10.06.2019) | 18 June 2019 (18.06.2019) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**EP 3 779 000 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H593317 B
- JP 2016023389 A

- JP 2002238569 A

**Non-patent literature cited in the description**

- **LING PENG et al.** *Macromol. Mater. Eng.,* 2016, vol. 301, 48-55
- *Nucleic Acid Res.,* 1982, vol. 10, 6487
- *Methods in Enzymology,* 1983, vol. 100, 448
- **KYTE J ; DOOLITTLE R.** A simple method for displaying the hydropathic character of a protein. *J. Mol. Biol.,* 1982, vol. 157, 105-132

- *Fire and Disaster Management Agency, Dangerous Materials Regulation Section Manager, Fire and Disaster No. 50,* 31 May 1995
- *Proc. Natl. Acad. Sci. USA,* 1972, vol. 69, 2110
- Molecular Cloning
- *Fire and Disaster No. 50,* 31 May 1995